# EUROPEAN PATENT APPLICATION

(11) **EP 4 342 896 A1**
(43) Date of publication of application: **27.03.2024**
(21) Application number: 22804069.7
(22) Date of filing: 20.05.2022
(51) Int. Cl.: C07D 471/04, A61K 31/4985, A61P 35/00

(54) **PIPERAZINE DERIVATIVE AND USE THEREOF IN MEDICINE**

(30) Priority: 21.05.2021 CN 202110550437; 24.05.2021 CN 202110562428; 03.08.2021 CN 202110885041; 12.11.2021 CN 202111331120; 25.01.2022 CN 202210084201
(71) Applicant: Chengdu Baiyu Pharmaceutical Co., Ltd., Chengdu, Sichuan 611130 (CN)
(72) Inventor: WEI, Yonggang, Chengdu, Sichuan 610000 (CN); ZHU, Yuqin, Chengdu, Sichuan 610000 (CN); CHU, Hongzhu, Chengdu, Sichuan 610000 (CN); YE, Fei, Chengdu, Sichuan 610000 (CN); DENG, Wutong, Chengdu, Sichuan 610000 (CN); LIU, Wei, Chengdu, Sichuan 610000 (CN); SUN, Yi, Chengdu, Sichuan 610000 (CN)
(74) Representative: Patentanwälte Olbricht Buchhold Keulertz
(86) International application number: PCT/CN2022/094124
(87) International publication number: WO 2022/242750

(57) **Abstract**

The present application relates to a piperazine-based compound and a composition thereof, and the use thereof in the manufacture of an anti-tumor medicament.

## Description

### Technical Field

The present invention generally relates to the field of pharmaceutical chemistry, and particularly to a piperazine derivative or a pharmaceutically acceptable salt, a stereoisomer, or a deuteriated agent thereof, and the use thereof in medicine.

### Background

Adenosine diphosphate-ribosylation (ADP-ribosylation) is a protein post-transcriptional modification process, in which one or more adenosine diphosphate-ribose (ADP-ribose) groups are inserted in amino acid residues of proteins. ADP-ribosylation is a reversible process involving physiological regulation such as cell signal transduction, DNA damage repair, transcription, regulation of gene expression and apoptosis. ADP-ribose is derived from a redox cofactor: nicotinamide adenine dinucleotide (NAD+), and the enzyme that mediates the modification of ADP-ribose insertion is ADP-ribosylase. In the regulation of this physiological response, the N-glycosidic linkage of NAD+ connecting an ADP-ribose molecule and a nicotinamide group is cleaved, which is then captured to form a linkage with the corresponding amino acid residue of the target protein. ADP-ribosylases can perform two types of modifications: mono-ADP ribosylation and poly-ADP ribosylation. When DNA is damaged or cells are stressed, PARP is activated, resulting in an increase in the amount of poly ADP-ribose and a decrease in the amount of NAD+. For more than a decade, PARP1 was considered the only poly-ADP-ribose polymerase in mammalian cells and thus studied most. To date, scientists have identified 17 different PARPs. MonoPARP accounts for a majority of the PARP family and mediates important biological functions and various stress responses, for example, unfolded protein response, NF-κB signaling, antiviral response and cytokine signaling. 2,3,7,8-tetrachlorodibenzo-p-dioxin (TCDD)-inducible poly(ADP-ribose) polymerase (PARP-7) is a member of the MonoPARP family, and its expression is regulated by TCDD-activated aromatic hydrocarbon receptor (AHR), which is a ligand-activated transcription factor that mediates the toxic activity of many environmental xenobiotics. AHR up-regulates the expression of PARP-7, and PARP-7 enables the ADP-ribosylation of the kinase TBK1 by their interaction, resulting in the inhibition of TBK1 activity and the down-regulation of IFN-I (type I interferon) response, which in turn leads to the suppression of antiviral and tumor immune responses of the body.

### Summary of the Invention

One or more embodiments of the present application provide a selective PARP7 inhibitor or a pharmaceutically acceptable salt, a stereoisomer or a deuterated compound thereof, and the use thereof in medicine, e.g., in anticancer application.

One or more embodiments of the present application provide a compound of formula (I) or a pharmaceutically acceptable salt, a stereoisomer or a deuterated compound thereof: 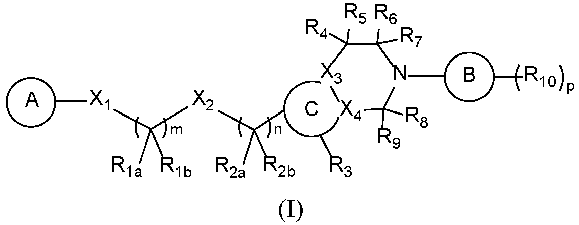 wherein:
X₁ is NH, O, or a 4 to 6-membered heterocycle containing 1 to 3 heteroatoms selected from N and O;
X₂ is O or a bond;
X₃ and X₄ are each independently C or N;
R₁ₐ and R_{1b} are each independently H, D or a C₁₋₆ alkyl; or R₁ₐ and R_{1b} form a 3 to 5-membered cycloalkyl together with the carbon atom connected thereto;
R₂ₐ and R_{2b} are each independently H, D or a C₁₋₆ alkyl; or R₂ₐ and R_{2b} form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H, D or a C₁₋₆ alkyl; or R₄ and R₅ form a 3 to 5-membered cycloalkyl together with the carbon atom connected thereto;
R₆ and R₇ are each independently H, D or a C₁₋₆ alkyl; or R₆ and R₇ form =O on the carbon atom connected thereto;
R₈ and R₉ are each independently H, D or a C₁₋₆ alkyl; or R₈ and R₉ form =O on the carbon atom connected thereto; or R₈ and R₉ form a 3 to 5-membered cycloalkyl together with the carbon atom connected thereto;
each R₁₀ is independently a C₁₋₆ alkyl, a C₁₋₆ alkoxy, CONR₁₀ₐR_{10b}, halogen, cyano, S(O)₂R_{10c}, SR_{10d} or a 3 to 5-membered cycloalkyl, where the C₁₋₆ alkyl and C₁₋₆ alkoxy are optionally substituted with 1 to 3 halogens;
R₁₀ₐ, R_{10b}, R_{10c} and R_{10d} are each independently H, D or a C₁₋₆ alkyl;
A is 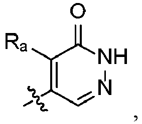 Rₐ is a C₁₋₆ alkyl, a C₃₋₅ cycloalkyl, halogen or cyano, where the C₁₋₆ alkyl is optionally substituted with 1 to 3 halogens;
B is a 5 to 10-membered carbocycle or heterocycle containing 1 to 3 heteroatoms selected from N, O and S;
C is a 5 to 6-membered heterocycle containing 1 to 3 heteroatoms of N;
m is 1, 2, or 3;
n is 0, 1, 2, or 3; and
p is 0, 1, 2, or 3.

In one or more embodiments:
X₁ is NH, O, or a 4 to 6-membered heterocycle containing 1 to 3 heteroatoms selected from N and O;
X₂ is O or a bond;
X₃ and X₄ are each independently C or N;
R₁ₐ and R_{1b} are each independently H, D or a C₁₋₆ alkyl;
R₂ₐ and R_{2b} are each independently H, D or a C₁₋₆ alkyl; or R₂ₐ and R_{2b} form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H, D or a C₁₋₆ alkyl; or R₄ and R₅ form a 3 to 5-membered cycloalkyl together with the carbon atom connected thereto;
R₆ and R₇ are each independently H, D or a C₁₋₆ alkyl;
R₈ and R₉ are each independently H, D or a C₁₋₆ alkyl; or R₈ and R₉ form =O on the carbon atom connected thereto;
each R₁₀ is independently a C₁₋₆ alkyl, a C₁₋₆ alkoxy, CONR₁₀ₐR_{10b}, halogen, cyano, S(O)₂R_{10c}, SR_{10d} or a 3 to 5-membered cycloalkyl, where the C₁₋₆ alkyl and C₁₋₆ alkoxy are optionally substituted with 1 to 3 halogens;
R₁₀ₐ, R_{10b}, R_{10c} and R_{10d} are each independently H, D or a C₁₋₆ alkyl;
A is 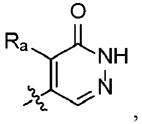 Rₐ is a C₁₋₆ alkyl, a C₃₋₅ cycloalkyl, halogen, or cyano, where the C₁₋₆ alkyl is optionally substituted with 1 to 3 halogens;
B is a 5 to 6-membered carbocycle or heterocycle containing 1 to 3 heteroatoms selected from N, O and S;
C is a 5 to 6-membered heterocycle containing 1 to 3 heteroatoms of N;
m is 1, 2, or 3;
n is 0, 1, 2, or 3; and
p is 0, 1, 2, or 3.

In one or more embodiments, the compound of the present application has the structure according to formula (I-1): 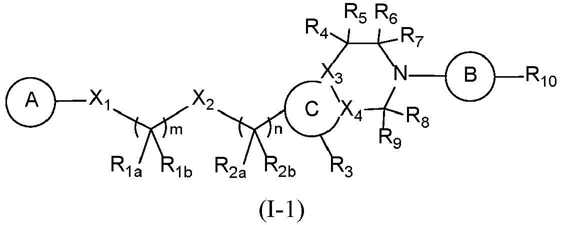 wherein:
X₁ is NH or a 4 to 6-membered heterocycle containing 1 to 3 heteroatoms selected from N and O;
X₂ is O;
X₃ and X₄ are each independently C or N;
R₁ₐ and R_{1b} are each independently H, D or a C₁₋₆ alkyl;
R₂ₐ and R_{2b} are each independently H, D or a C₁₋₆ alkyl;
R₃ is H, D, a C₁₋₆ alkyl, or halogen, where the C₁₋₆ alkyl is optionally substituted with 1 to 3 halogens;
R₄ and R₅ are each independently H, D or a C₁₋₆ alkyl;
R₆ and R₇ are each independently H, D or a C₁₋₆ alkyl;
R₈ and R₉ are each independently H, D or a C₁₋₆ alkyl; or R₈ and R₉ form =O on the carbon atom connected thereto;
R₁₀ is a C₁₋₆ alkyl, a C₁₋₆ alkoxy, cyano, or SR_{10d}, where the C₁₋₆ alkyl and C₁₋₆ alkoxy are optionally substituted with 1 to 3 halogens;
R_{10d} is H, D or a C₁₋₆ alkyl;
A is 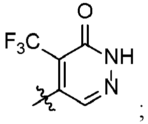
B is a 5 to 6-membered carbocycle or heterocycle containing 1 to 3 heteroatoms selected from N, O and S;
C is a 5 to 6-membered heterocycle containing 1 to 3 heteroatoms of N;
m is 1, 2, or 3; and
n is 0, 1, 2, or 3.

In one or more embodiments, the compound of the present application has the structure according to formula (I-2): 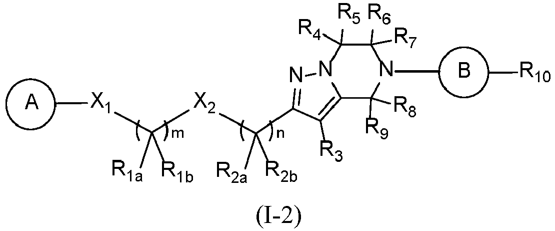 wherein:
X₁ is NH;
X₂ is O;
R₁ₐ and R_{1b} are each independently H, D or a C₁₋₆ alkyl;
R₂ₐ and R_{2b} are each independently H, D or a C₁₋₆ alkyl;
R₃ is H, D, a C₁₋₆ alkyl, or halogen, where the C₁₋₆ alkyl is optionally substituted with 1 to 3 halogens;
R₄ and R₅ are each independently H, D or a C₁₋₆ alkyl;
R₆ and R₇ are each independently H, D or a C₁₋₆ alkyl;
R₈ and R₉ are each independently H, D or a C₁₋₆ alkyl; or R₈ and R₉ form =O on the carbon atom connected thereto;
R₁₀ is a C₁₋₆ alkyl, a C₁₋₆ alkoxy, cyano, or SR_{10d}, where the C₁₋₆ alkyl and C₁₋₆ alkoxy are optionally substituted with 1 to 3 halogens;
R_{10d} is H, D or a C₁₋₆ alkyl;
A is 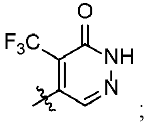
B is a 5 to 6-membered carbocycle or heterocycle containing 1 to 3 heteroatoms selected from N, O and S;
m is 1, 2, or 3; and
n is 0, 1, 2, or 3.

In one or more embodiments:
X₁ is NH;
X₂ is O;
R₁ₐ and R_{1b} are each independently H, D or a C₁₋₆ alkyl;
R₂ₐ and R_{2b} are each independently H, D or a C₁₋₆ alkyl;
R₃ is H, D, C₁₋₆ alkyl, or halogen, where the C₁₋₆ alkyl is optionally substituted with 1 to 3 halogens;
R₄ and R₅ are each independently H, D or a C₁₋₆ alkyl;
R₆ and R₇ are each independently H, D or a C₁₋₆ alkyl;
R₈ and R₉ are each independently H, D or a C₁₋₆ alkyl, or R₈ and R₉ form =O on the carbon atom connected thereto;
R₁₀ is a C₁₋₆ alkyl, cyano, or SR_{10d}, where the C₁₋₆ alkyl is optionally substituted with 1 to 3 halogens;
R_{10d} is H, D or a C₁₋₆ alkyl;
A is 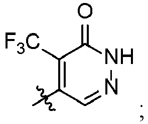
B is a 5 to 6-membered carbocycle or heterocycle containing 1 to 3 heteroatoms selected from N, O and S;
m is 1, 2, or 3;
n is 0, 1, 2, or 3.

In one or more embodiments:
X₁ is selected from NH;
X₂ is selected from O;
R₁ₐ and R_{1b} are each independently selected from H, D or a C₁₋₆ alkyl;
R₂ₐ and R_{2b} are each independently selected from H, D or a C₁₋₆ alkyl;
R₃ is H, D, a C₁₋₆ alkyl, or halogen, where the C₁₋₆ alkyl is optionally substituted with 1 to 3 halogens;
R₄ and R₅ are each independently H or D;
R₆ and R₇ are each independently H or D;
R₈ and R₉ are each independently H or D;
R₁₀ is CF₃ or SR_{10d};
R_{10d} is H, D or a C₁₋₆ alkyl;
A is 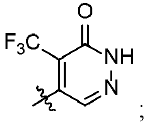
B is 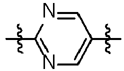 or 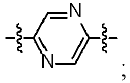
m is 1, 2, or 3; and
n is 0, 1, 2, or 3.

In one or more embodiments:
X₁ is selected from NH;
X₂ is selected from O;
R₁ₐ and R_{1b} are each independently selected from H, D, or a C₁₋₃ alkyl;
R₂ₐ and R_{2b} are each independently selected from H, D, or a C₁₋₃ alkyl;
R₃ is selected from H, D, or CF₃;
R₄ and R₅ are each independently selected from H or D;
R₆ and R₇ are each independently selected from H or D;
R₈ and R₉ are each independently selected from H or D;
R₁₀ is selected from CF₃;
A is 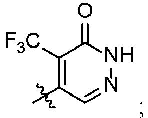
B is 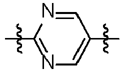 or 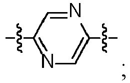
m is 1, 2, or 3; and
n is 0, 1, or 2.

In one or more embodiments, the compound of the present application has the structure according to formula (I-3): 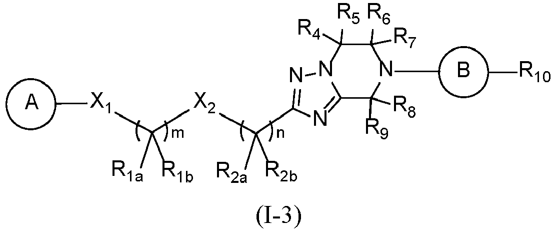 wherein:
X₁ is NH or a 4 to 6-membered heterocycle containing 1 to 3 heteroatoms selected from N and O;
X₂ is O;
R₁ₐ and R_{1b} are each independently H, D or a C₁₋₆ alkyl;
R₂ₐ and R_{2b} are each independently H, D or a C₁₋₆ alkyl;
R₄ and R₅ are each independently H, D or a C₁₋₆ alkyl;
R₆ and R₇ are each independently H, D or a C₁₋₆ alkyl;
R₈ and R₉ are each independently H, D or a C₁₋₆ alkyl; or R₈ and R₉ form =O on the carbon atom connected thereto;
R₁₀ is a C₁₋₆ alkyl, a C₁₋₆ alkoxy, cyano, or SR_{10d}, where the C₁₋₆ alkyl and C₁₋₆ alkoxy are optionally substituted with 1 to 3 halogens;
R_{10d} is selected from H, D or a C₁₋₆ alkyl;
A is 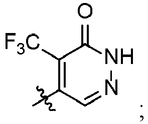
B is a 5 to 6-membered carbocycle or heterocycle containing 1 to 3 heteroatoms selected from N, O and S;
m is 1, 2, or 3; and
n is 0, 1, 2, or 3.

In one or more embodiments:
X₁ is NH or a 4 to 6-membered heterocycle containing 1 to 3 heteroatoms selected from N and O;
X₂ is O;
R₁ₐ and R_{1b} are each independently H, D, or a C₁₋₃ alkyl;
R₂ₐ and R_{2b} are each independently H, D, or a C₁₋₃ alkyl;
R₄ and R₅ are each independently H, D, or a C₁₋₃ alkyl;
R₆ and R₇ are each independently H, D, or a C₁₋₃ alkyl;
R₈ and R₉ are each independently H, D, or a C₁₋₃ alkyl; or R₈ and R₉ form =O on the carbon atom connected thereto;
R₁₀ is a C₁₋₆ alkyl, cyano, or SR_{10d}, where the C₁₋₆ alkyl is optionally substituted with 1 to 3 halogens;
R_{10d} is selected from H, D or a C₁₋₆ alkyl;
A is 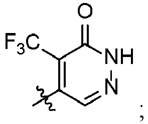
B is 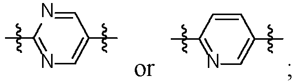 m is 1, 2, or 3; and
n is 0, 1, or 2.

In one or more embodiments:
X₁ is NH;
X₂ is O;
R₁ₐ and R_{1b} are each independently H, D, or a C₁₋₃ alkyl;
R₂ₐ and R_{2b} are each independently H or D;
R₄ and R₅ are each independently H or D;
R₆ and R₇ are each independently H or D;
R₈ and R₉ are each independently H or D;
R₁₀ is CF₃;
A is 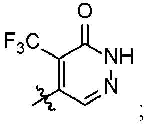
B is 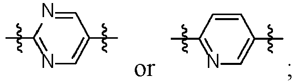
m is 1, 2, or 3; and
n is 0, 1, or 2.

One or more embodiments of the present application provide a compound of formula (I') or a stereoisomer thereof: 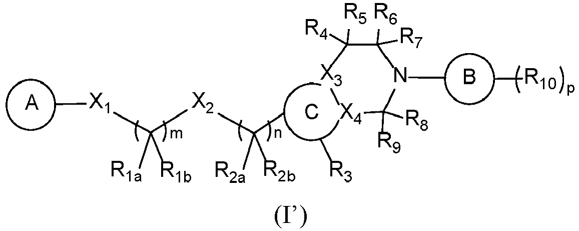 wherein:
X₁ is NH or a 4 to 6-membered heterocycle containing 1 to 3 heteroatoms selected from N and O;
X₂ is O or a bond;
X₃ and X₄ are C or N;
R₁ₐ and R_{1b} are each independently H, D or a C₁₋₆ alkyl; or R₁ₐ and R_{1b} optionally form a 3 to 5-membered cycloalkyl together with the carbon atom connected thereto;
R₂ₐ and R_{2b} are each independently H, D or a C₁₋₆ alkyl; or R₂ₐ and R_{2b} optionally form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H or a C₁₋₆ alkyl; or R₄ and R₅ form a 3 to 5-membered cycloalkyl together with the carbon atom connected thereto;
R₆ and R₇ are each independently H or a C₁₋₆ alkyl; or R₆ and R₇ form =O on the carbon atom connected thereto;
R₈ and R₉ are each independently H, D or a C₁₋₆ alkyl; or R₈ and R₉ form =O on the carbon atom connected thereto; or R₈ and R₉ form a 3 to 5-membered cycloalkyl together with the carbon atom connected thereto;
each R₁₀ is independently a halogen-substituted C₁₋₆ alkyl, a halogen-substituted C₁₋₆ alkoxy, CONR₁₀ₐR_{10b}, cyano, S(O)₂R_{10c}, SR_{10d} or a 3 to 5-membered cycloalkyl;
R₁₀ₐ, R_{10b}, R_{10c} and R_{10d} are each independently H, D or a C₁₋₆ alkyl; A is 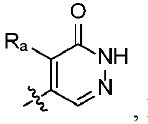 Rₐ is a C₁₋₆ alkyl, a C₃₋₅ cycloalkyl, halogen or cyano, where the C₁₋₆ alkyl is optionally substituted with 1 to 3 halogens;
B is a 5 to 10-membered carbocycle or heterocycle containing 1 to 3 heteroatoms selected from N, O and S;
C is a 5 to 6-membered heterocycle containing 1 to 3 heteroatoms of N;
m is 1, 2, or 3;
n is 0, 1, 2, or 3; and
p is 0, 1, 2, or 3.

One or more embodiments of the present application provide a compound of formula (I-1') or a stereoisomer thereof: 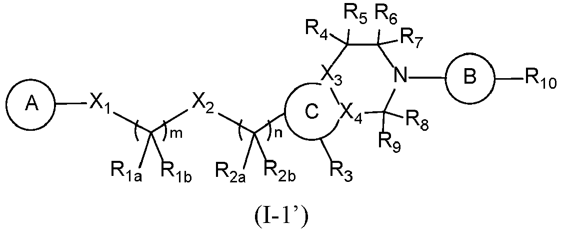 wherein:
X₁ is NH or a 4 to 6-membered heterocycle containing 1 to 3 heteroatoms selected from N and O;
X₂ is O or a bond;
X₃ and X₄ are each independently C or N;
R₁ₐ and R_{1b} are each independently H, D or a C₁₋₆ alkyl; or R₁ₐ and R_{1b} optionally form a 3 to 5-membered cycloalkyl together with the carbon atom connected thereto;
R₂ₐ and R_{2b} are each independently H, D or a C₁₋₆ alkyl; or R₂ₐ and R_{2b} optionally form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H or a C₁₋₆ alkyl; or R₄ and R₅ form a 3 to 5-membered cycloalkyl together with the carbon atom connected thereto;
R₆ and R₇ are each independently H or a C₁₋₆ alkyl; or R₆ and R₇ form =O on the carbon atom connected thereto;
R₈ and R₉ are each independently H, D or a C₁₋₆ alkyl; or R₈ and R₉ form =O on the carbon atom connected thereto; or R₈ and R₉ form a 3 to 5-membered cycloalkyl together with the carbon atom connected thereto;
R₁₀ is a halogen-substituted C₁₋₆ alkyl, CONR₁₀ₐR_{10b}, cyano, or a 3 to 5-membered cycloalkyl;
R₁₀ₐ and R_{10b} are each independently H, D or a C₁₋₆ alkyl;
A is 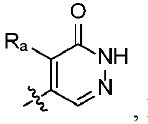 Rₐ is a C₁₋₆ alkyl, a C₃₋₅ cycloalkyl, halogen or cyano, where the C₁₋₆ alkyl is optionally substituted with 1 to 3 halogens;
B is a 5 to 6-membered carbocycle or heterocycle containing 1 to 3 heteroatoms selected from N, O and S;
C is a 5 to 6-membered heterocycle containing 1 to 3 heteroatoms of N;
m is 1, 2, or 3; and
n is 0, 1, 2, or 3.

One or more embodiments of the present application provide a compound of formula (I-2') or a stereoisomer thereof: 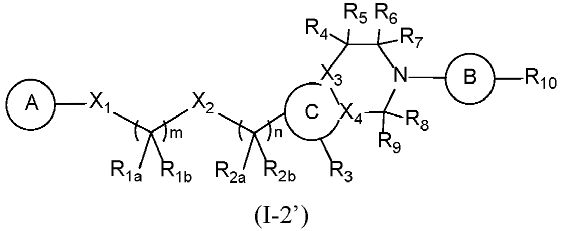 wherein:
X₁ is NH;
X₂ is O or a bond;
X₃ and X₄ are C or N;
R₁ₐ and R_{1b} are each independently H, D or a C₁₋₆ alkyl; or R₁ₐ and R_{1b} optionally form a 3 to 5-membered cycloalkyl together with the carbon atom connected thereto;
R₂ₐ and R_{2b} are each independently H, D or a C₁₋₆ alkyl; or R₂ₐ and R_{2b} optionally form a 3 to 5-membered cycloalkyl together with the carbon atom connected thereto;
R₃ is H, a C₁₋₆ alkyl, halogen or cyano, where the C₁₋₆ alkyl is optionally substituted with 1 to 3 halogens;
R₄ and R₅ are each independently H or a C₁₋₆ alkyl;
R₆ and R₇ are each independently H or a C₁₋₆ alkyl; or R₆ and R₇ form =O on the carbon atom connected thereto;
R₈ and R₉ are each independently H, D or a C₁₋₆ alkyl; or R₈ and R₉ form =O on the carbon atom connected thereto;
R₁₀ is CF₃, cyano, or a 3 to 5-membered cycloalkyl;
A is 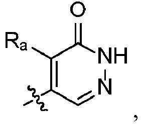 Rₐ is a C₁₋₆ alkyl, a C₃₋₅ cycloalkyl, halogen or cyano, where the C₁₋₆ alkyl is optionally substituted with 1 to 3 halogens;
B is a 5 to 6-membered carbocycle or heterocycle containing 1 to 3 heteroatoms selected from N, O and S;
C is a 5 to 6-membered heterocycle containing 1 to 3 heteroatoms of N;
m is 1, 2, or 3; and
n is 0, 1, 2, or 3.

One or more embodiments of the present application provide a compound of formula (I-3') or a stereoisomer thereof: 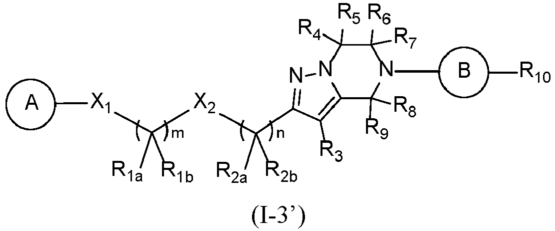 wherein
X₁ is NH;
X₂ is O or a bond;
R₁ₐ and R_{1b} are each independently H, D or a C₁₋₆ alkyl; or R₁ₐ and R_{1b} optionally form a 3 to 5-membered cycloalkyl together with the carbon atom connected thereto;
R₂ₐ and R_{2b} are each independently H, D or a C₁₋₆ alkyl; or R₂ₐ and R_{2b} optionally form a 3 to 5-membered cycloalkyl together with the carbon atom connected thereto;
R₃ is H or a C₁₋₆ alkyl;
R₄ and R₅ are each independently H or a C₁₋₆ alkyl;
R₆ and R₇ are each independently H or a C₁₋₆ alkyl;
R₈ and R₉ are each independently H or a C₁₋₆ alkyl;
R₁₀ is CF₃ or a 3 to 5-membered cycloalkyl;
A is 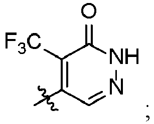
B is 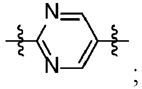
m is 1, 2, or 3; and
n is 0, 1, 2, or 3.

In one or more embodiments:
X₁ is NH;
X₂ is O;
R₁ₐ and R_{1b} are each independently H or a C₁₋₆ alkyl;
R₂ₐ and R_{2b} are each independently H or a C₁₋₆ alkyl;
R₃ is H or a C₁₋₆ alkyl;
R₄ and R₅ are each independently H or a C₁₋₆ alkyl;
R₆ and R₇ are each independently H or a C₁₋₆ alkyl;
R₈ and R₉ are each independently H or a C₁₋₆ alkyl;
R₁₀ is CF₃ or a 3 to 5-membered cycloalkyl;
A is 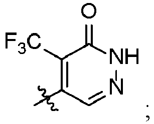
B is 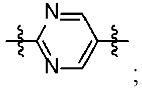
m is 1 or 2; and
n is 0 or 1.

In one or more embodiments of the present application, the compound of the present application has the following structure: 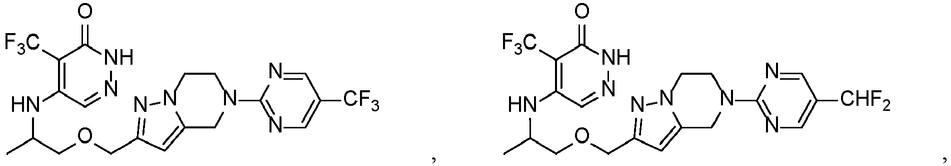 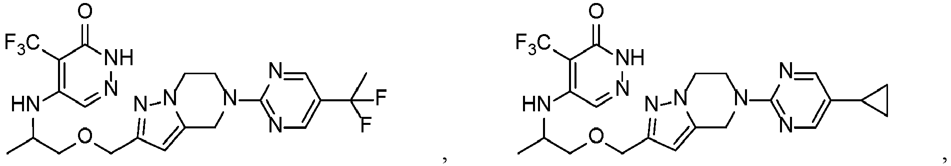 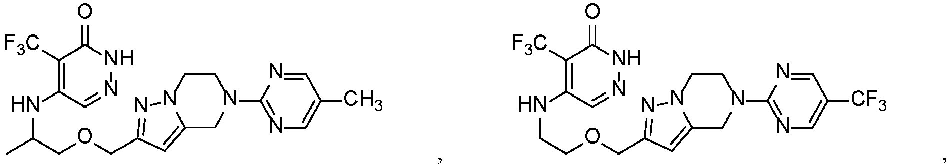 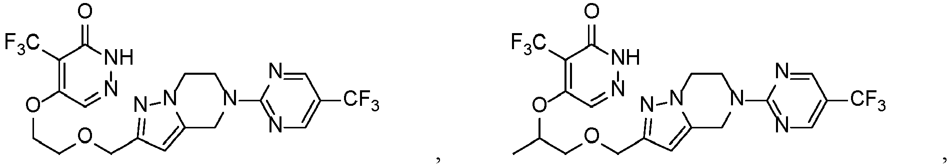 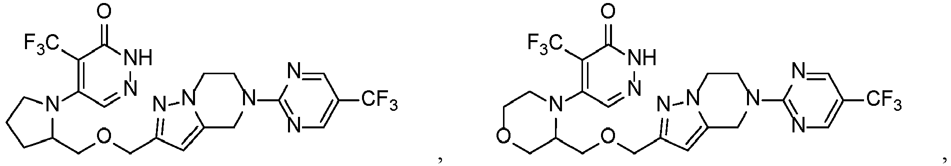 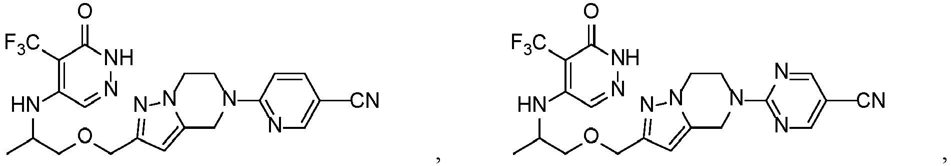 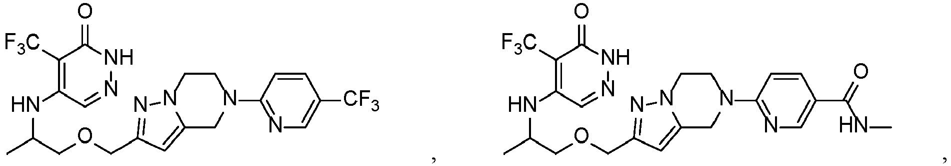 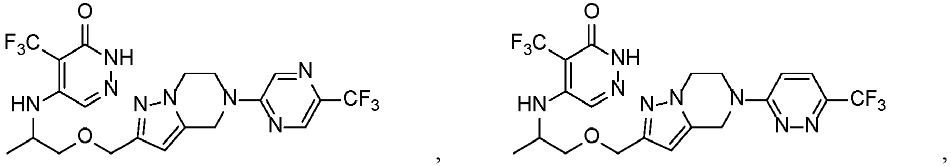 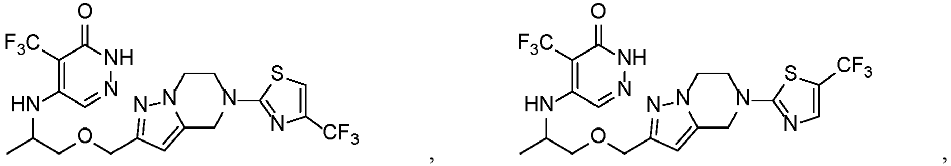 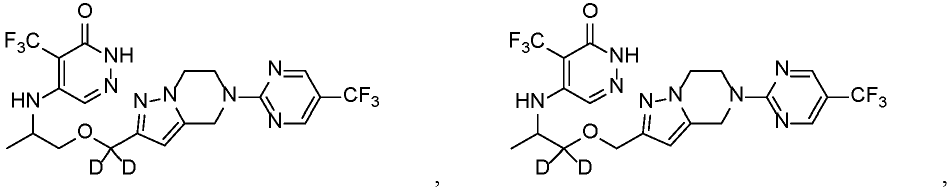 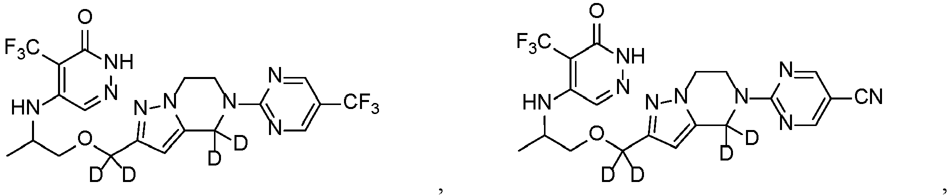 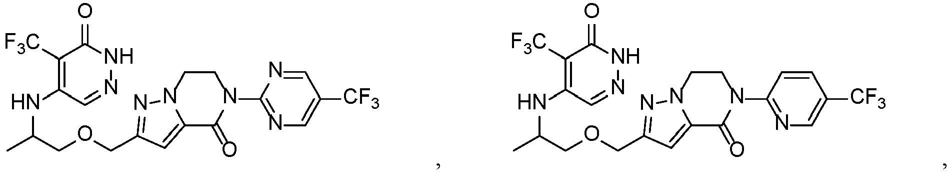 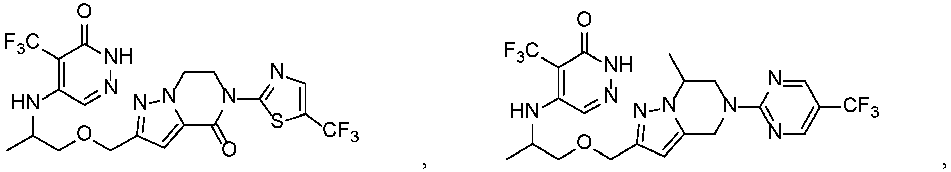 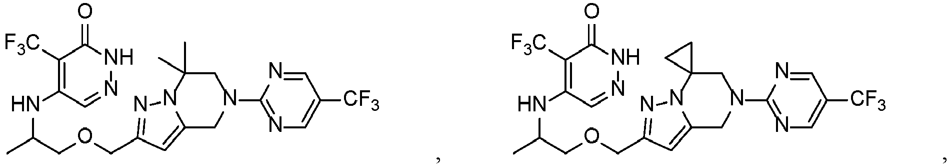 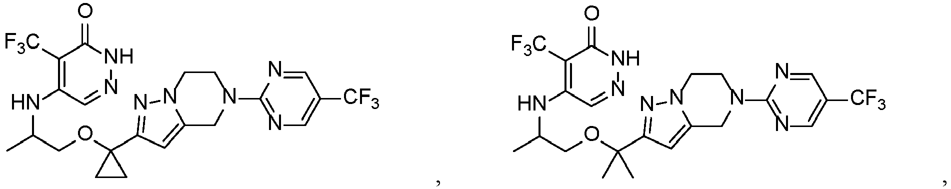 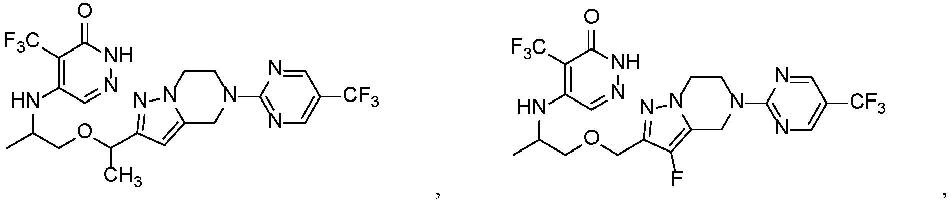 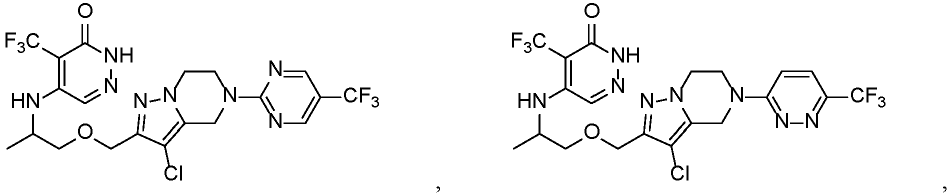 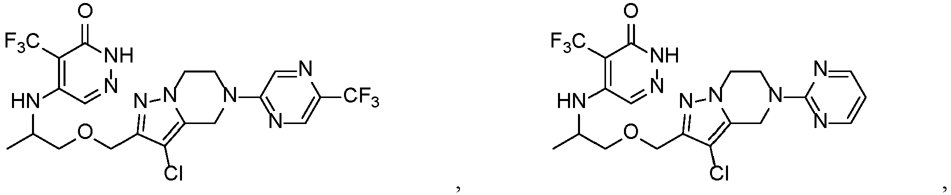 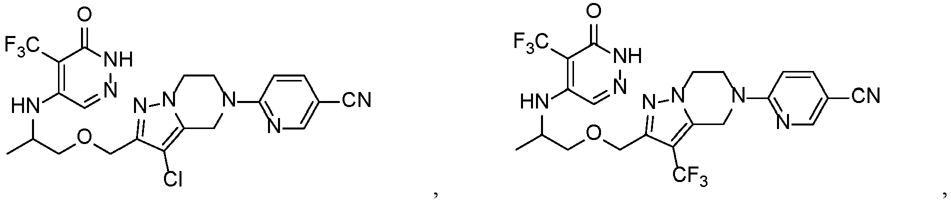 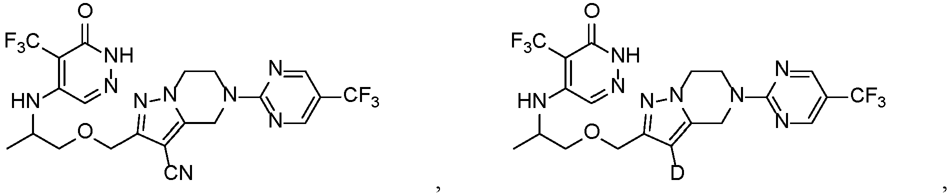 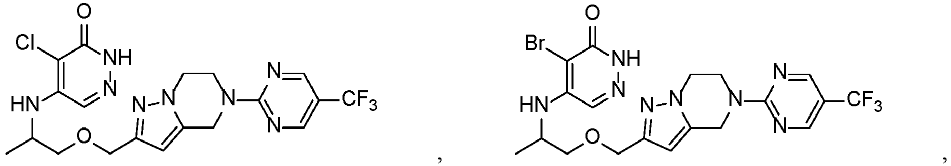 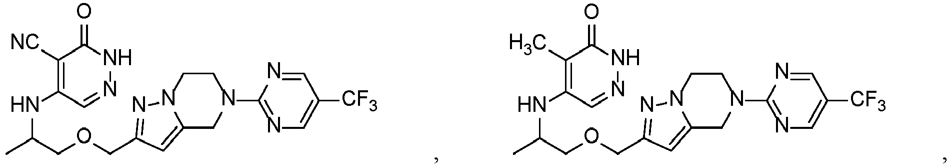 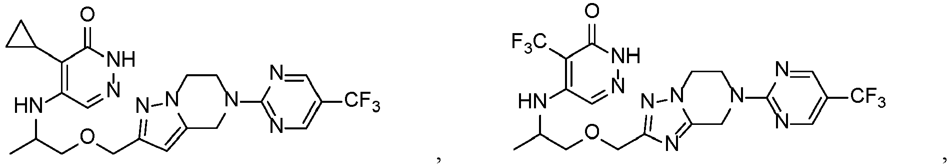 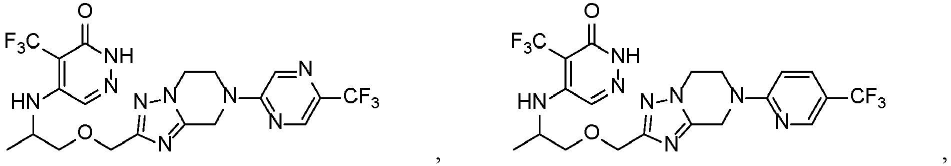 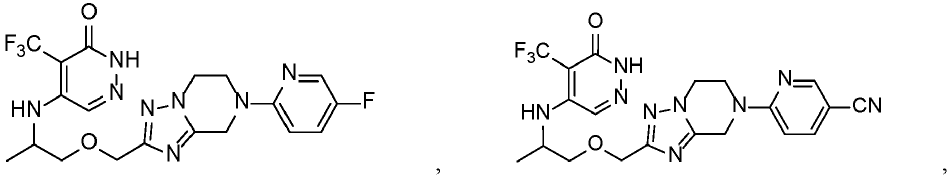 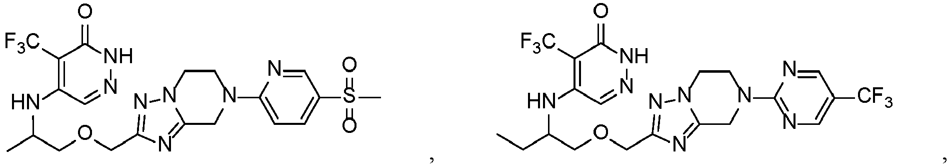 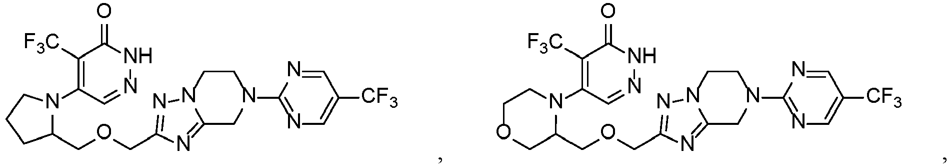 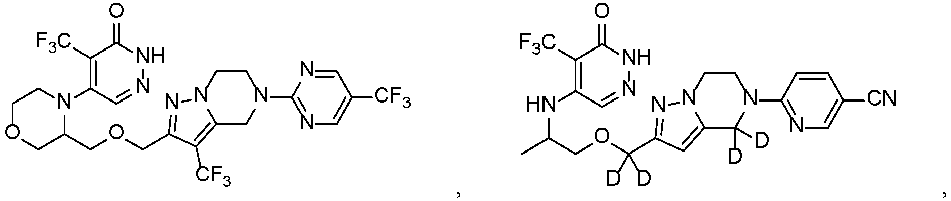 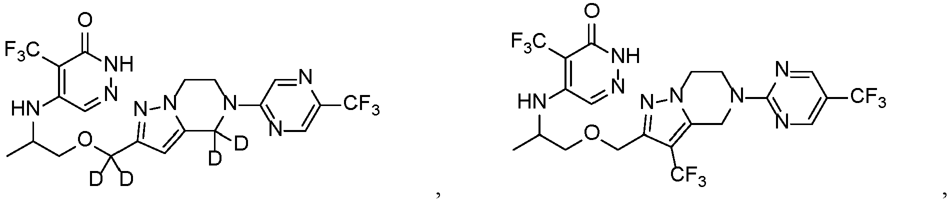 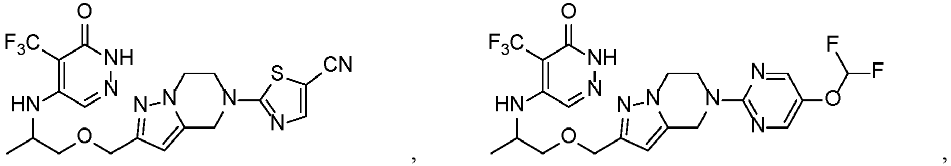 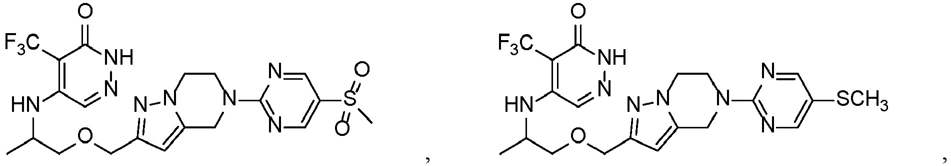 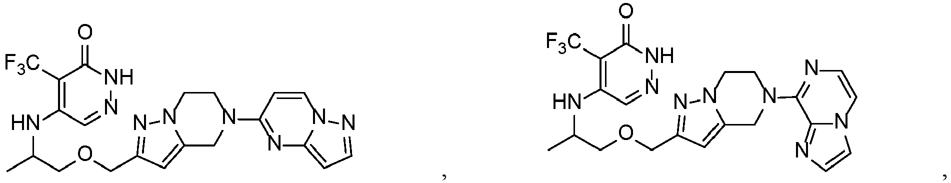 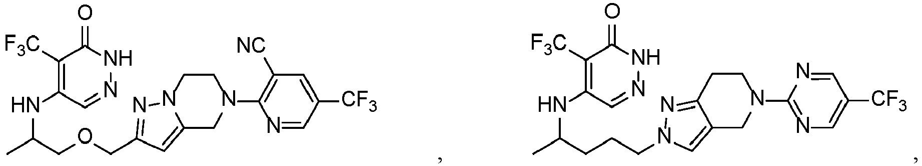 or

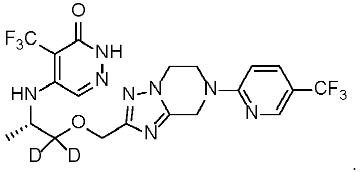

One or more embodiments of the present application provide an intermediate for preparing the compound according to formula (I), (I-1), (I-2), (I-3), (I'), (I-1'), (I-2') or (I-3') or a pharmaceutically acceptable salt, a stereoisomer or a deuterated compound thereof, wherein the intermediate or a pharmaceutically acceptable salt, a stereoisomer or a deuterated compound thereof has the structure according to formula (I-4): 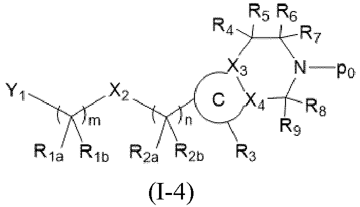 wherein:
Y₁ is -NH₂, -OH, -NHPi or a 4 to 6-membered heterocycle containing 1 to 3 heteroatoms selected from N and O;
P₁ is an amino-protecting group, preferably -Boc;
P₀ is H or an amino-protecting group, where the amino-protecting group is preferably - PMB;
X₂ is O or a bond;
X₃ and X₄ are each independently C or N;
R₁ₐ and R_{1b} are each independently H, D or a C₁₋₆ alkyl;
R₂ₐ and R_{2b} are each independently H, D or a C₁₋₆ alkyl; or R₂ₐ and R_{2b} form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H, D or a C₁₋₆ alkyl; or R₄ and R₅ form a 3 to 5-membered cycloalkyl together with the carbon atom connected thereto;
R₆ and R₇ are each independently H, D or a C₁₋₆ alkyl;
R₈ and R₉ are each independently H, D or a C₁₋₆ alkyl; or R₈ and R₉ form =O on the carbon atom connected thereto;
C is a 5 to 6-membered heterocycle containing 1 to 3 heteroatoms of N;
m is 1, 2, or 3; and
n is 0, 1, 2, or 3.

In one or more embodiments of the present application, the compound of the present application has the following structure: 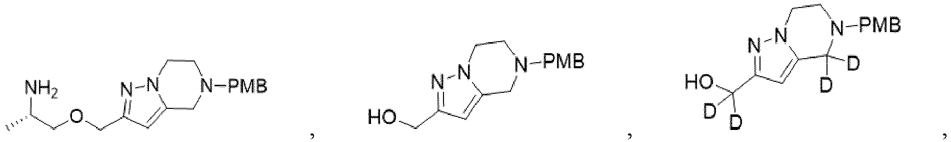 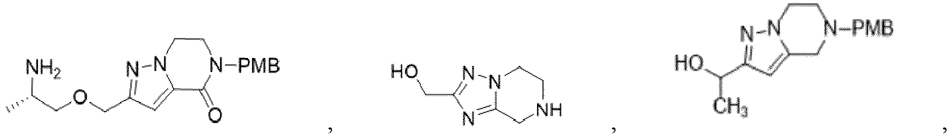 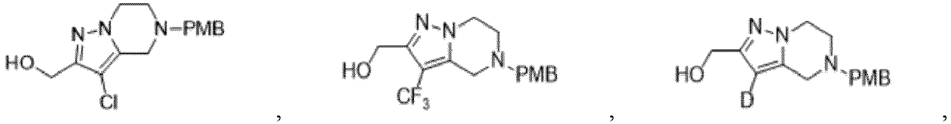 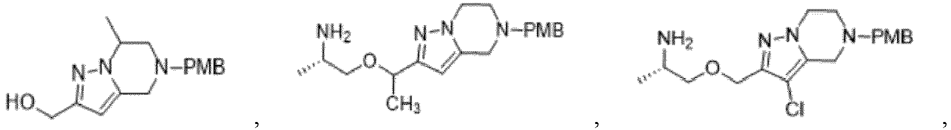 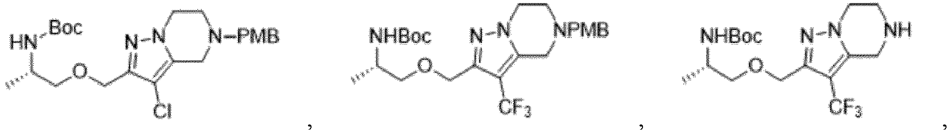 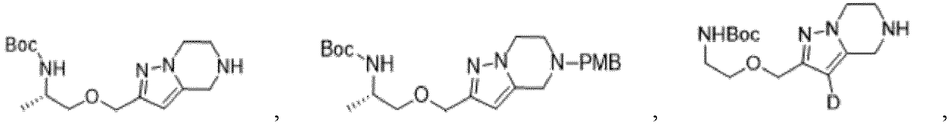 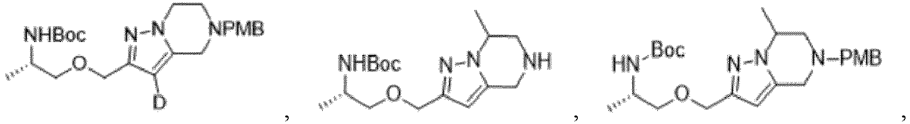 or

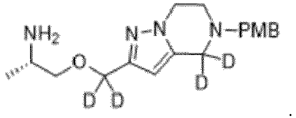

One or more embodiments of the present application provide an intermediate for preparing the compound according to formula (I), (I-1), (I-2), (I-3), (I'), (I-1'), (I-2') or (I-3') or a pharmaceutically acceptable salt, a stereoisomer or a deuterated compound thereof, wherein the intermediate or a pharmaceutically acceptable salt, a stereoisomer or a deuterated compound thereof has the structure according to formula (I-5): 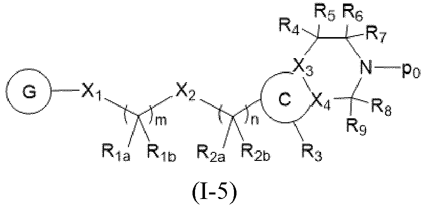 wherein:
X₁ is NH, O or a 4 to 6-membered heterocycle containing 1 to 3 heteroatoms selected from N and O;
P₀ is H or an amino-protecting group, where the amino-protecting group is preferably - PMB;
X₂ is O or a bond;
X₃ and X₄ are each independently C or N;
R₁ₐ and R_{1b} are each independently H, D or a C₁₋₆ alkyl;
R₂ₐ and R_{2b} are each independently H, D or a C₁₋₆ alkyl; or R₂ₐ and R_{2b} form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H, D or a C₁₋₆ alkyl; or R₄ and R₅ form a 3 to 5-membered cycloalkyl together with the carbon atom connected thereto;
R₆ and R₇ are each independently H, D or a C₁₋₆ alkyl;
R₈ and R₉ are each independently H, D or a C₁₋₆ alkyl; or R₈ and R₉ form =O on the carbon atom connected thereto;
Gis 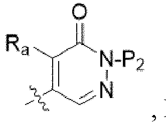 Rₐ is a C₁₋₆ alkyl, a C₃₋₅ cycloalkyl, halogen or cyano, where the C₁₋₆ alkyl is optionally substituted with 1 to 3 halogens;
P₂ is H or an amino-protecting group, where the amino-protecting group is preferably - SEM or -PMB;
C is a 5 to 6-membered heterocycle containing 1 to 3 heteroatoms of N;
m is 1, 2, or 3; and
n is 0, 1, 2, or 3.

In one or more embodiments of the present application, the compound of the present application has the following structure:

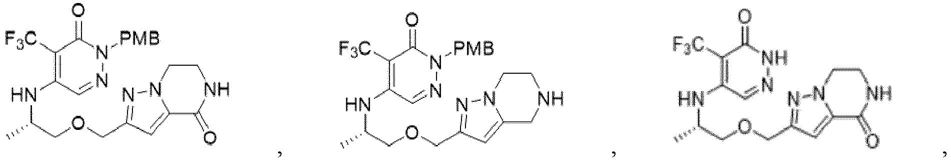

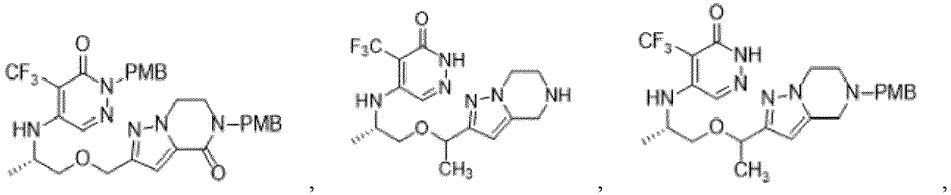

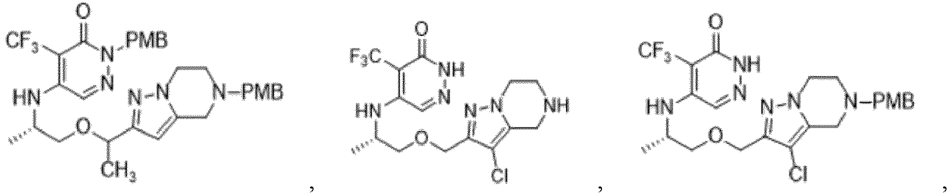

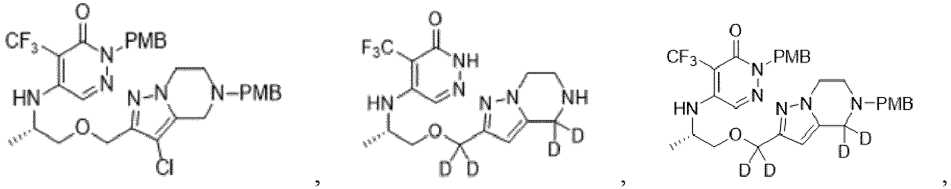

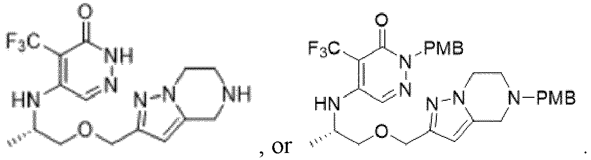

One or more embodiments of the present application provide an intermediate for preparing the compound according to formula (I), (I-1), (I-2), (I-3), (I'), (I-1'), (I-2') or (I-3') or a pharmaceutically acceptable salt, a stereoisomer or a deuterated compound thereof, wherein the intermediate or a pharmaceutically acceptable salt, a stereoisomer or a deuterated compound thereof has the structure according to formula (I-6): 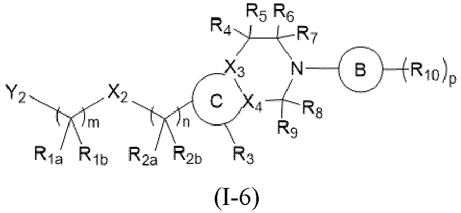 wherein:
Y₂ is NHR_{Y}, OH or a 4 to 6-membered heterocycle containing 1 to 3 heteroatoms selected from N and O;
R_{Y} is H or an amino-protecting group, where the amino-protecting group is preferably - Boc;
X₂ is O or a bond;
X₃ and X₄ are each independently C or N;
R₁ₐ and R_{1b} are each independently H, D or a C₁₋₆ alkyl;
R₂ₐ and R_{2b} are each independently H, D or a C₁₋₆ alkyl; or R₂ₐ and R_{2b} form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H, D or a C₁₋₆ alkyl; or R₄ and R₅ form a 3 to 5-membered cycloalkyl together with the carbon atom connected thereto;
R₆ and R₇ are each independently H, D or a C₁₋₆ alkyl;
R₈ and R₉ are each independently H, D or a C₁₋₆ alkyl; or R₈ and R₉ form =O on the carbon atom connected thereto;
each R₁₀ is independently a C₁₋₆ alkyl, a C₁₋₆ alkoxy, CONR₁₀ₐR_{10b}, halogen, cyano, S(O)₂R_{10c}, SR_{10d} or a 3 to 5-membered cycloalkyl, where the C₁₋₆ alkyl and C₁₋₆ alkoxy are optionally substituted with 1 to 3 halogens;
R₁₀ₐ, R_{10b}, R_{10c} and R_{10d} are each independently H, D or a C₁₋₆ alkyl;
B is a 5 to 10-membered carbocycle or heterocycle containing 1 to 3 heteroatoms selected from N, O and S;
C is a 5 to 6-membered heterocycle containing 1 to 3 heteroatoms of N;
m is 1, 2, or 3;
n is 0, 1, 2, or 3; and
p is 0, 1, 2, or 3.

In one or more embodiments of the present application, the compound of the present application has the following structure:

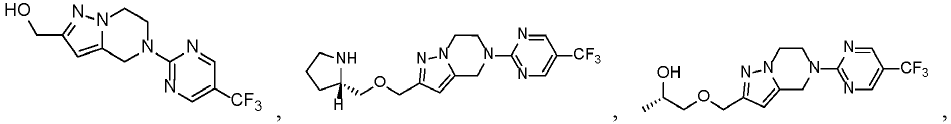

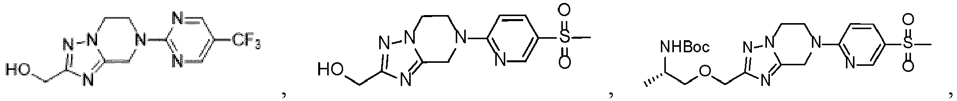

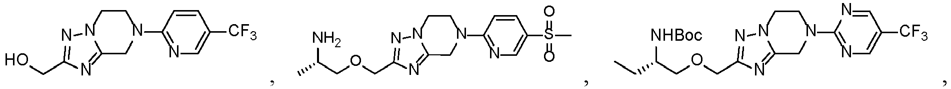

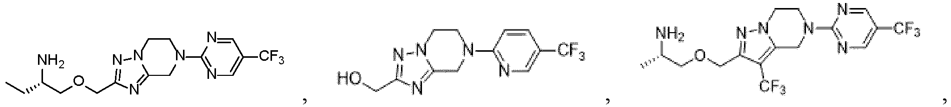

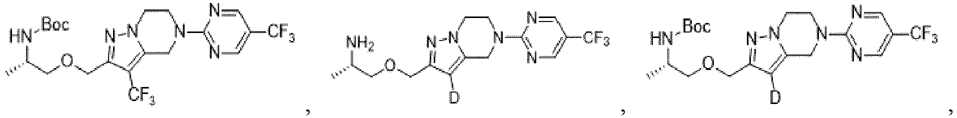

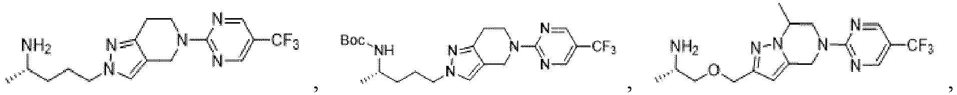

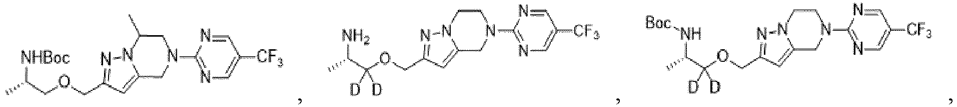

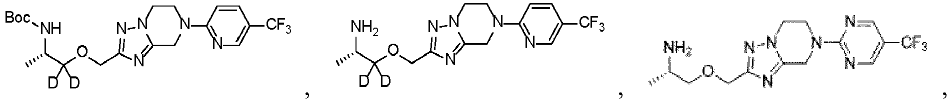

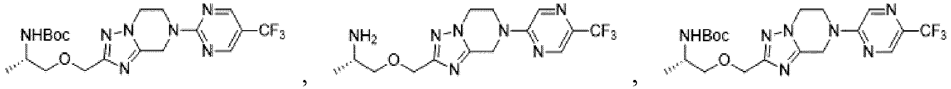

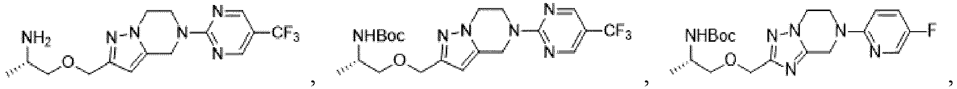

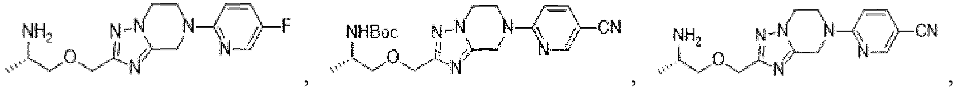

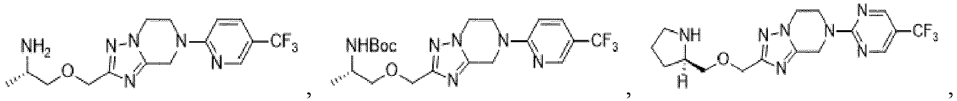

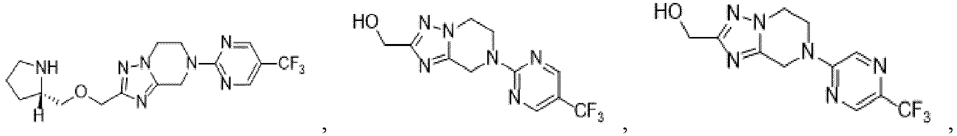

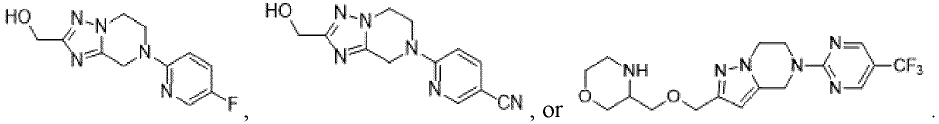

One or more embodiments of the application provide an intermediate for preparing the compound according to formula (I), (I-1), (I-2), (I-3), (I'), (I-1'), (I-2') or (I-3') or a pharmaceutically acceptable salt, a stereoisomer or a deuterated compound thereof, wherein the intermediate or a pharmaceutically acceptable salt, a stereoisomer or a deuterated compound thereof has the structure according to formula (I-7): 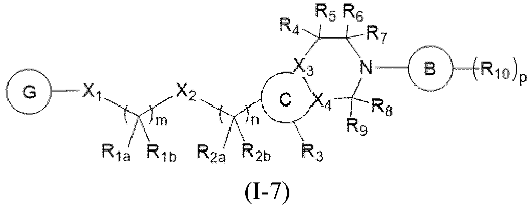 wherein:
X₁ is NH, O or a 4 to 6-membered heterocycle containing 1 to 3 heteroatoms selected from N and O;
X₂ is O or a bond;
X₃ and X₄ are each independently C or N;
R₁ₐ and R_{1b} are each independently H, D or a C₁₋₆ alkyl;
R₂ₐ and R_{2b} are each independently H, D or a C₁₋₆ alkyl; or R₂ₐ and R_{2b} form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H, D or a C₁₋₆ alkyl; or R₄ and R₅ form a 3 to 5-membered cycloalkyl together with the carbon atom connected thereto;
R₆ and R₇ are each independently H, D or a C₁₋₆ alkyl;
R₈ and R₉ are each independently H, D or a C₁₋₆ alkyl; or R₈ and R₉ form =O on the carbon atom connected thereto;
each R₁₀ is independently a C₁₋₆ alkyl, a C₁₋₆ alkoxy, CONR₁₀ₐR_{10b}, halogen, cyano, S(O)₂R_{10c}, SR_{10d} or a 3 to 5-membered cycloalkyl, where the C₁₋₆ alkyl and C₁₋₆ alkoxy are optionally substituted with 1 to 3 halogens;
R₁₀ₐ, R_{10b}, R_{10c} and R_{10d} are each independently H, D or a C₁₋₆ alkyl;
Gis 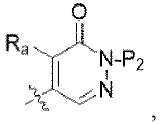 Rₐ is a C₁₋₆ alkyl, a C₃₋₅ cycloalkyl, halogen or cyano, where the C₁₋₆ alkyl is optionally substituted with 1 to 3 halogens;
P₂ is an amino-protecting group, preferably -SEM or -PMB;
B is a 5 to 10-membered carbocycle or heterocycle containing 1 to 3 heteroatoms selected from N, O and S;
C is a 5 to 6-membered heterocycle containing 1 to 3 heteroatoms of N;
m is 1, 2, or 3;
n is 0, 1, 2, or 3; and
p is 0, 1, 2, or 3.

In one or more embodiments of the present application, the compound of the present application has the following structure: 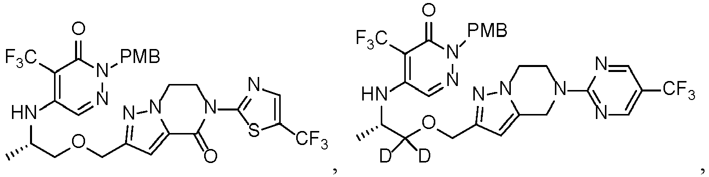 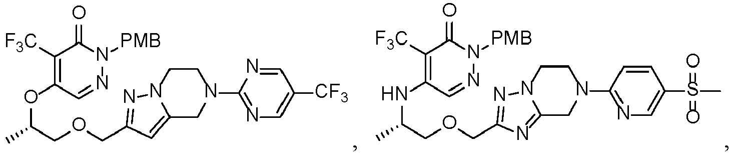 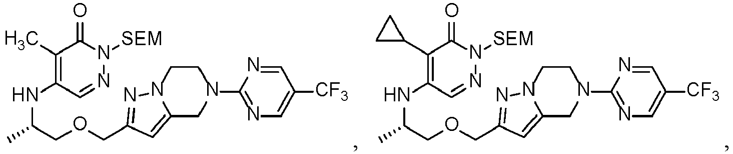 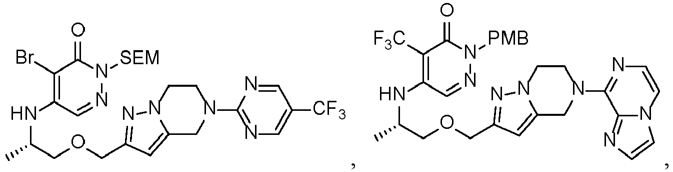 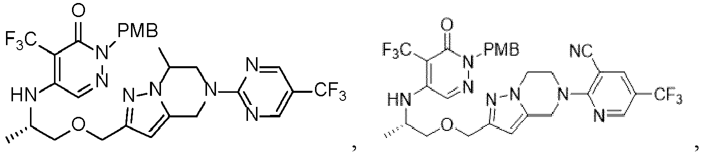 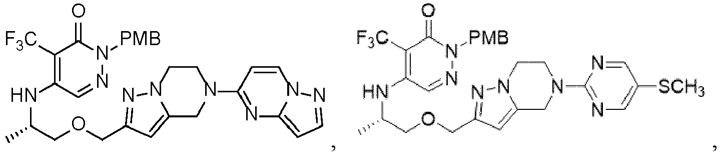 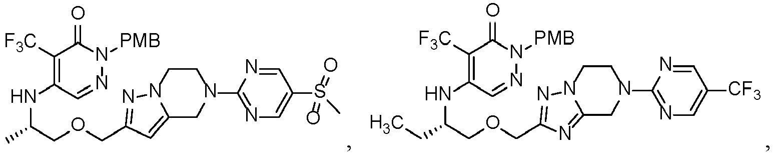 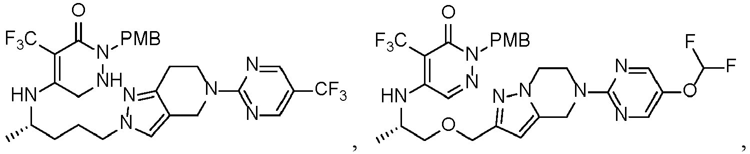 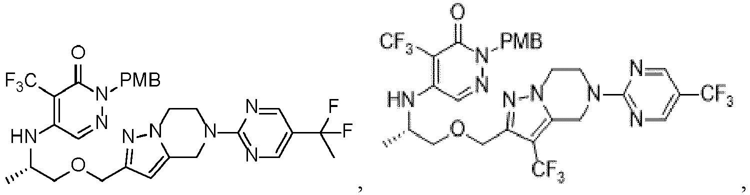 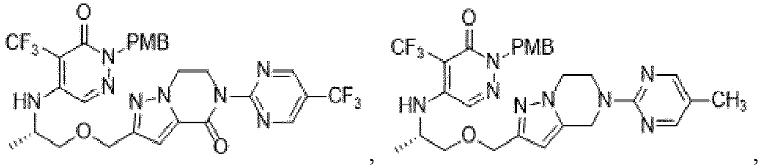 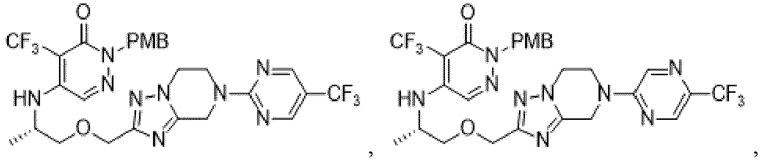 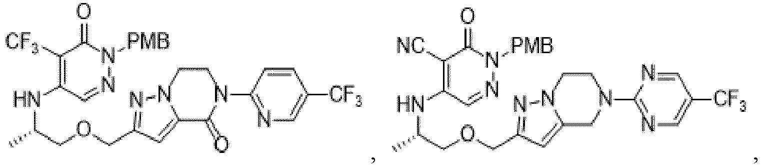 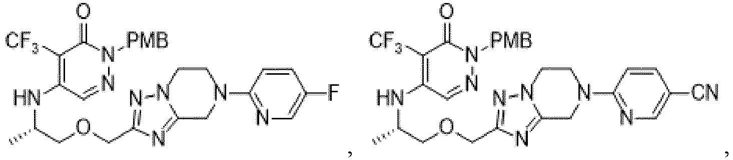 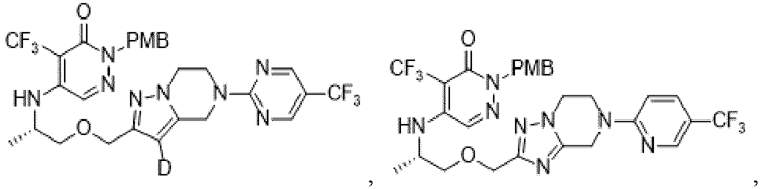 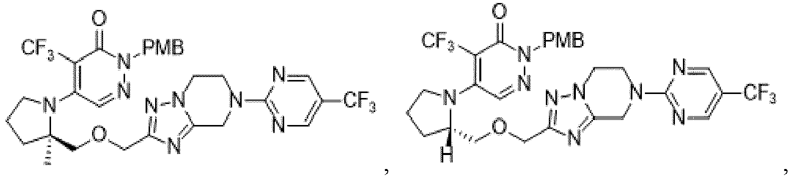 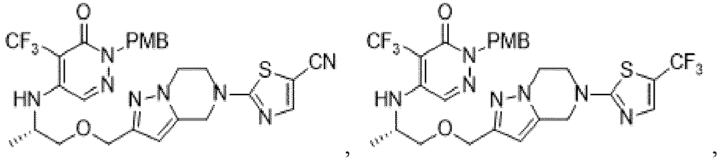 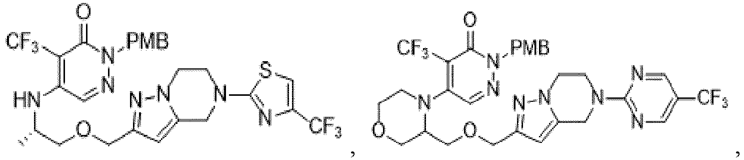 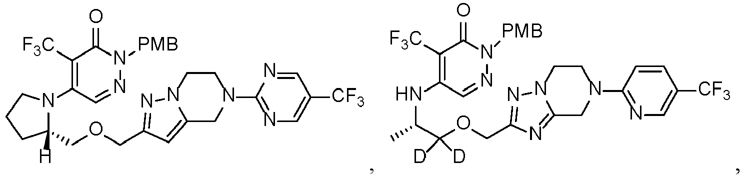 or

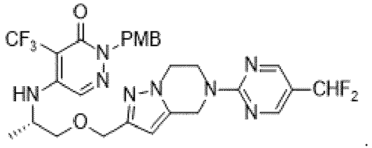

One or more embodiments of the present application provide a pharmaceutical composition comprising:
(1) the compound of the present application or a pharmaceutically acceptable salt, a stereoisomer or a deuterated compound thereof;
(2) optionally one or more additional active ingredients; and
(3) a pharmaceutically acceptable carrier and/or excipient.

One or more embodiments of the present application provide use of the compound of the present application or a pharmaceutically acceptable salt, a stereoisomer or a deuterated compound thereof, or the pharmaceutical composition of the present application in the manufacture of an antitumor medicament.

One or more embodiments of the present application provide the compound of the present application or a pharmaceutically acceptable salt, a stereoisomer or a deuterated compound thereof, or the composition of the present application for use as a medicament.

One or more embodiments of the present application provide the compound of the present application or a pharmaceutically acceptable salt, a stereoisomer or a deuterated compound thereof, or the composition of the present application for use in a method for treating/preventing cancer.

One or more embodiments of the present application provide a method for treating/preventing cancer including administering the compound of the present application or a pharmaceutically acceptable salt, a stereoisomer or a deuterated compound thereof, or the composition of the present application to a subject in need thereof.

Unless stated to the contrary, the terms used in the specification and claims have the following meanings.

Carbon, hydrogen, oxygen, sulfur, nitrogen, F, Cl, Br and I involved in the groups and compounds described herein are each inclusive of isotopes thereof, and carbon, hydrogen, oxygen, sulfur or nitrogen involved in the groups and compounds described herein is optionally further replaced by one or more isotopes thereof corresponding thereto, wherein isotopes of carbon include ¹²C, ¹³C and ¹⁴C, isotopes of hydrogen include protium (H), deuterium (D, also called heavy hydrogen) and tritium (T, also called superheavy hydrogen), isotopes of oxygen include ¹⁶O, ¹⁷O and ¹⁸O, isotopes of sulfur include ³²S, ³³S, ³⁴S and ³⁶S, isotopes of nitrogen include ¹⁴N and ¹⁵N, isotopes of fluorine include ¹⁷F and ¹⁹F, isotopes of chlorine include ³⁵C1 and ³⁷Cl, and isotopes of bromine include ⁷⁹Br and ⁸¹Br.

"Alkyl" refers to a linear or branched saturated aliphatic hydrocarbon group consisting of 1 to 20 carbon atoms, preferably an alkyl group consisting of 1 to 8 (e.g., 1, 2, 3, 4, 5, 6, 7 or 8) carbon atoms, more preferably an alkyl group consisting of 1 to 6 carbon atoms, and further preferably an alkyl group consisting of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched chain isomers thereof; when the alkyl is substituted, it may be optionally further substituted with 1 or more substituents.

"Alkoxy" refers to a group formed by substitution of at least 1 carbon atom of an alkyl group with an oxygen atom. Non-limiting examples include methoxy, ethoxy, n-propoxy, isopropoxy, n-butoxy, sec-butoxy, tert-butoxy, n-pentoxy, n-hexoxy, cyclopropoxy and cyclobutoxy. The alkyl is defined in the same way as for the "alkyl" described above.

"Alkenyl" refers to a linear or branched unsaturated aliphatic hydrocarbon group containing 1 to 10 (e.g., 1, 2, 3, 4, 5, 6, 7, 8, 9 or 10) carbon-carbon double bonds and consisting of 2 to 20 carbon atoms, preferably an alkenyl group consisting of 2 to 12 (e.g., 2, 3, 4, 5, 6, 7, 8, 9, 10, 11 or 12) carbon atoms, more preferably an alkenyl group consisting of 2 to 8 carbon atoms, and further preferably an alkenyl group consisting of 2 to 6 carbon atoms. Non-limiting examples include vinyl, propen-2-yl, buten-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl, and undecen-3-yl. The alkenyl may be optionally further substituted with 1 or more substituents.

"Alkynyl" refers to a linear or branched unsaturated aliphatic hydrocarbon group containing 1 to 10 (e.g., 1, 2, 3, 4, 5, 6, 7, 8, 9 or 10) carbon-carbon triple bonds and consisting of 2 to 20 carbon atoms, preferably an alkynyl group consisting of 2 to 12 (e.g., 2, 3, 4, 5, 6, 7, 8, 9, 10, 11 or 12) carbon atoms, more preferably an alkynyl group consisting of 2 to 8 carbon atoms, and further preferably an alkynyl group consisting of 2 to 6 carbon atoms. Non-limiting examples include ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyn-3-yl, nonyn-3-yl, decyn-4-yl, undec-3-yl, and dodecyn-4-yl. The alkynyl may be optionally further substituted with one or more substituents.

"Aryl" refers to a substituted or unsubstituted aromatic ring. It may be a 5-8 membered (e.g., 5, 6, 7 or 8 membered) monocyclic ring system, a 5-12 membered (e.g., 5, 6, 7, 8, 9, 10, 11 or 12 membered) bicyclic ring system or a 10-15 membered (e.g., 10, 11, 12, 13, 14 or 15 membered) tricyclic ring system, and may be a bridged ring or a spiro ring. Non-limiting examples include phenyl and naphthyl. The aryl may be optionally further substituted with 1 or more substituents.

"Heteroaryl" refers to a substituted or unsubstituted aromatic ring. It may be a 3-8 membered (e.g., 3, 4, 5, 6, 7 or 8 membered) monocyclic ring system, a 5-12 membered (e.g., 5, 6, 7, 8, 9, 10, 11 or 12 membered) bicyclic ring system or a 10-15 membered (e.g., 10, 11, 12, 13, 14 or 15 membered) tricyclic ring system, and it contains 1 to 6 (e.g., 1, 2, 3, 4, 5 or 6) heteroatoms selected from N, O and S, and is preferably 5-8 membered heteroaryl. 1 to 4 (e.g., 1, 2, 3 or 4) N and S optionally substituted in the ring of the heteroaryl can be oxidized to various oxidation states. Heteroaryl may be attached to a heteroatom or carbon atom and it may be a bridged ring or a spiro ring. Non-limiting examples include cyclic pyridinyl, furanyl, thienyl, pyranyl, pyrrolyl, pyrimidinyl, pyrazinyl, pyridazinyl, imidazolyl, piperidinyl, benzimidazolyl, benzopyridinyl and pyrrolopyridinyl. Heteroaryl is optionally further substituted with 1 or more substituents.

"Carbocyclyl" or "carbocycle" refers to a saturated or unsaturated, aromatic or non-aromatic ring. When being an aromatic ring, it is defined in the same way as for the "aryl" described above; when being an non-aromatic ring, it may be a 3-10 membered (e.g., 3, 4, 5, 6, 7, 8, 9 or 10 membered) monocyclic ring system, a 4-12 membered (e.g., 4, 5, 6, 7, 8, 9, 10, 11 or 12-membered) bicyclic ring system or a 10-15 membered (e.g., 10, 11, 12, 13, 14 or 15 membered) tricyclic ring system, and it may be a bridged ring or a spiro ring. Non-limiting examples include cyclopropyl, cyclobutyl, cyclopentyl, 1-cyclopentyl-1-alkenyl, 1-cyclopentyl-2-alkenyl, 1-cyclopentyl-3-alkenyl, cyclohexyl, 1-cyclohexyl-2-alkenyl, 1-cyclohexyl-3-alkenyl, cyclohexenyl, cyclohexadienyl, cycloheptyl, cyclooctyl, cyclononyl, cyclodecyl, cycloundecyl, cyclododecyl, 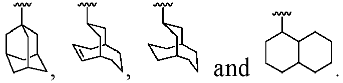 The "carbocyclyl" or "carbocycle" is optionally further substituted with 1 or more substituents.

"Heterocyclyl" or "heterocycle" refers to a saturated or unsaturated, aromatic or non-aromatic heterocycle. When being an aromatic heterocycle, it is defined in the same way as for the "heteroaryl" described above; when being a non-aromatic heterocycle, it may be a 3-10 membered (e.g., 3, 4, 5, 6, 7, 8, 9 or 10 membered) monocyclic ring system, a 4-12 membered (e.g., 4, 5, 6, 7, 8, 9, 10, 11 or 12 membered) bicyclic ring system or a 10-15 membered (e.g., 10, 11, 12, 13, 14 or 15 membered) tricyclic ring system, and it contains 1 to 4 (e.g., 1, 2, 3 or 4) heteroatoms selected from N, O and S, and is preferably 3-8 membered heterocyclyl. 1 to 4 (e.g., 1, 2, 3 or 4) N and S optionally substituted in the ring of the "heterocyclyl" or "heterocycle" can be oxidized to various oxidation states; "heterocyclyl" or "heterocycle" may be attached to a heteroatom or a carbon atom, and may be a bridged ring or a spiro ring. Non-limiting examples of "heterocyclyl" or "heterocycle" include epoxyethyl, epoxypropyl, aziridinyl, oxetanyl, azetidinyl, thietanyl, 1,3-dioxolanyl, 1,4-dioxolanyl, 1,3-dioxanyl, azepanyl, oxepanyl, thiepanyl, oxoazepinyl, diazepinyl, thiazepinyl, pyridinyl, homopiperidinyl, furanyl, thienyl, pyranyl, N-alkylpyrrolyl, pyrimidinyl, pyrazinyl, pyridazinyl, piperazinyl, homopiperazinyl, imidazolyl, piperidinyl, morpholinyl, thiomorpholinyl, oxathianyl, 1,3-dithianyl, dihydrofuranyl, dithiacyclopentyl, tetrahydrofuranyl, tetrahydrothienyl, tetrahydropyridyl, tetrahydrothiopyranyl, tetrahydropyrrolyl, tetrahydroimidazolyl, tetrahydrothiazolyl, tetrahydropyranyl, benzimidazolyl, benzopyridinyl, pyrrolopyridinyl, pyrazolopyrimidinyl, imidazopyrazinyl, benzodihydrofuranyl, 2-pyrrolinyl, 3-pyrrolinyl, indolinyl, 2H-pyranyl, 4H-pyranyl, dioxacyclohexyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3H-indolylquinolizinyl, N-pyridylurea, 1,1-dioxothiomorpholinyl, azabicyclo[3.2.1]octyl, azabicyclo[5.2.0]nonyl, oxatricyclo[5.3.1.1]dodecyl, aza-adamantyl and oxaspiro[3.3]heptyl. The "heterocyclyl" or "heterocycle" may be optionally further substituted with 1 or more substituents.

"Cycloalkyl" refers to a saturated cyclic hydrocarbon group, the ring of which may be a 3-10 membered (e.g., 3, 4, 5, 6, 7, 8, 9 or 10 membered) monocyclic ring system, a 4-12 membered (e.g., 4, 5, 6, 7, 8, 9, 10, 11 or 12 membered) bicyclic ring system or a 10-20 membered (e.g., 10, 11, 12, 13, 14, 15, 16, 17, 18, 19 or 20 membered) polycyclic ring system. The ring carbon atoms are preferably 3 to 10 carbon atoms, further preferably 3 to 8 carbon atoms. Non-limiting examples of "cycloalkyl" include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl, cycloheptatrienyl, and the like. When the cycloalkyl is substituted, it may be optionally further substituted with 1 or more substituents.

"Heterocycloalkyl" refers to a substituted or unsubstituted saturated non-aromatic cyclic group. It may be a 3-8 membered (e.g., 3, 4, 5, 6, 7 or 8 membered) monocyclic ring system, a 4-12 membered (e.g., 4, 5, 6, 7, 8, 9, 10, 11 or 12 membered) bicyclic ring system or a 10-15 membered (e.g., 10, 11, 12, 13, 14 or 15 membered) tricyclic ring system, and it contains 1, 2 or 3 heteroatoms selected from N, O and S, and is preferably 3-8 membered heterocyclyl. 1, 2 or 3 N and S optionally substituted in the ring of "heterocycloalkyl" can be oxidized to various oxidation states; "heterocycloalkyl" may be attached to a heteroatom or a carbon atom and may be a bridged ring or a spiro ring. Non-limiting examples of "heterocycloalkyl" include epoxyethyl, aziridinyl, oxetanyl, azetidinyl, 1,3-dioxolanyl, 1,4-dioxolanyl, 1,3-dioxanyl, azepanyl, piperidinyl, morpholinyl, thiomorpholinyl, 1,3-dithianyl, tetrahydrofuranyl, tetrahydropyrrolyl, tetrahydroimidazolyl, tetrahydrothiazolyl, tetrahydropyranyl, azabicyclo[3.2.1]octyl, azabicyclo[5.2.0]nonyl, oxatricyclo[5.3.1.1]dodecyl, aza-adamantyl and oxaspiro[3.3]heptyl.

When the "alkyl", "alkoxy", "alkenyl", "alkynyl", "aryl", "heteroaryl", "carbocyclyl", "carbocycle", "heterocyclyl", "heterocycle", "cycloalkyl", "heterocycloalkyl" or "heterocyclyl" described above is substituted, it may be optionally further substituted with 0, 1, 2, 3, 4, 5, 6, 7, 8, 9 or 10 substituents selected from F, Cl, Br, I, hydroxy, mercapto, nitro, cyano, amino, C₁₋₆ alkylamino, =O, C₁₋₆ alkyl, C₁₋₆ alkoxy, C₂₋₆ alkenyl, C₂₋₆ alkynyl, -NR_{q4}R_{q5}, =NR_{q6}, - C(=O)OC₁₋₆ alkyl, -OC(=O)C₁₋₆ alkyl, -C(=O)NR_{q4}R_{q5}, C₃₋₈ cycloalkyl, C₃₋₈ heterocycloalkyl, C₆₋₁₀ aryl, C₅₋₁₀ heteroaryl, -C(=O)OC₆₋₁₀ aryl, -OC(=O)C₆₋₁₀ aryl, -OC(=O)C₅₋₁₀ heteroaryl, - C(=O)OC₅₋₁₀ heteroaryl, -OC(=O)C₃₋₈ heterocycloalkyl, -C(=O)OC₃₋₈ heterocycloalkyl, - OC(=O)C₃₋₈ cycloalkyl, -C(=O)OC₃₋₈ cycloalkyl, -NHC(=O)C₃₋₈ heterocycloalkyl, - NHC(=O)C₆₋₁₀ aryl, -NHC(=O)C₅₋₁₀ heteroaryl, -NHC(=O)C₃₋₈ cycloalkyl, -NHC(=O)C₃₋₈ heterocycloalkyl, -NHC(=O)C₂₋₆ alkenyl and -NHC(=O)C₂₋₆ alkynyl, wherein the substituent C₁₋₆ alkyl, C₁₋₆ alkoxy, C₂₋₆ alkenyl, C₂₋₆ alkynyl, C₃₋₈ cycloalkyl, C₃₋₈ heterocycloalkyl, C₆₋₁₀ aryl, C₅₋₁₀ heteroaryl, -NHC(=O)C₆₋₁₀ aryl, -NHC(=O)C₅₋₁₀ heteroaryl, -NHC(=O)C₃₋₈ heterocycloalkyl or -NHC(=O)C₃₋₈ cycloalkyl is optionally further substituted with 1 to 3 substituents selected from OH, F, Cl, Br, I, C₁₋₆ alkyl, C₁₋₆ alkoxy, -NR_{q4}R_{q5} and =O, R_{q1} is selected from C₁₋₆ alkyl, C₁₋₆ alkoxy and C₆₋₁₀ aryl, and R_{q2} and R_{q3} are selected from H and C₁-₆ alkyl, wherein R_{q4} and R_{q5} are selected from H, C₁₋₆ alkyl, -NH(C=NR_{q1})NR_{q2}R_{q3}, - S(=O)₂NR_{q2}R_{q3}, -C(=O)R_{q1} and -C(=O)NR_{q2}R_{q3}, wherein the C₁₋₆ alkyl is optionally further substituted with 1 or more substituents selected from OH, F, Cl, Br, I, C₁₋₆ alkyl, C₁₋₆ alkoxy, C₆₋₁₀ aryl, C₅₋₁₀ heteroaryl, C₃₋₈ cycloalkyl and C₃₋₈ heterocycloalkyl; or R_{q4} and R_{q5}, together with an N atom, form a 3-8 membered heterocycle, which may contain 1 or more heteroatoms selected from N, O and S.

Halogen includes F, Cl, Br and I.

"Pharmaceutically acceptable salt" or "pharmaceutically acceptable salt thereof' refers to a salt obtained by reaction of a compound disclosed herein in a free acid form with a nontoxic inorganic or organic base or by reaction of a compound disclosed herein in a free base form with a nontoxic inorganic or organic acid, and in this salt, the bioavailability and characteristics of the compound disclosed herein in the free acid or free base form is retained.

"Pharmaceutical composition" refers to a mixture of one or more compounds described herein or pharmaceutically acceptable salts or prodrugs thereof and other chemical components, wherein the "other chemical components" refer to pharmaceutically acceptable carriers, excipients and/or one or more other therapeutic agents.

"Carrier" refers to a material that does not cause significant irritation to an organism and does not eliminate the biological activity and characteristics of the administered compound.

"Excipient" refers to an inert substance added to a pharmaceutical composition to facilitate administration of a compound. Non-limiting examples include calcium carbonate, calcium phosphate, sugars, starches, cellulose derivatives (including microcrystalline cellulose), gelatin, vegetable oils, polyethylene glycols, diluents, granulating agents, lubricants, binders and disintegrants.

"Stereoisomer" refers to isomers resulting from different spatial arrangements of atoms in a molecule, including cis-trans isomers, enantiomers and conformers.

"Optional", "optionally", "selective" or "selectively" means that the subsequently described event or circumstance may, but does not necessarily, occur, and the description includes cases where the event or circumstance occurs and cases where it does not. For example, "heterocyclyl optionally substituted with alkyl" means that the alkyl may, but does not necessarily, be present, and the description includes the case where the heterocyclyl is substituted with alkyl and the case where the heterocyclyl is not substituted with alkyl.

### DETAILED DESCRIPTION

The following examples illustrate the technical schemes of the present invention in detail, but the protection scope of the present invention includes but is not limited thereto.

The reaction temperature is room temperature and the optimum reaction temperature of room temperature is 20-30 °C if not otherwise specified in examples.

### Intermediate 1

### 5-Chloro-2-(4-methoxybenzyl)-4-(trifluoromethyl)pyridazin-3(2H)-one (Intermediate 1)

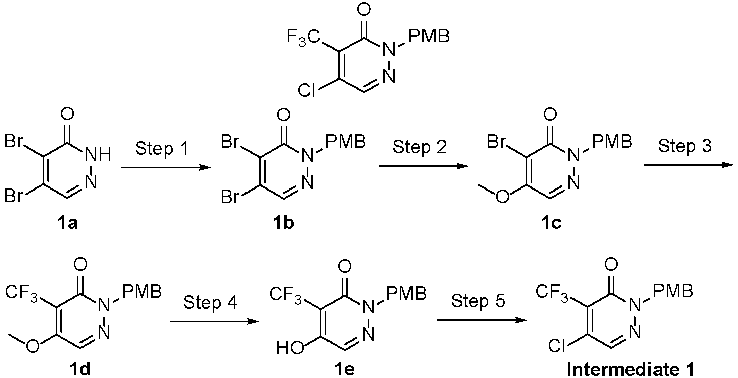

### Step 1:

### 4,5-Dibromo-2-(4-methoxybenzyl)pyridazin-3(2H)-one (1b)

To a solution of 4,5-dibromo-2,3-dihydropyridazin-3-one (**1a,** 50 g, 196.94 mmol, 1.0 equiv) in *N*,*N-*dimethylformamide (500 mL) was added sodium hydride (11.82 g, 295.41 mmol, 1.5 equiv, 60%) in portions at 0-10 °C, followed by the addition of 1-(chloromethyl)-4-methoxybenzene (46.06 g, 294.11 mmol, 1.49 equiv) at 0 °C. After the addition was completed, the reaction mixture was stirred at room temperature for 3 h. After the reaction was completed, the reaction mixture was slowly poured into 1.0 L of an ice-water mixture to quench the reaction and extracted with dichloromethane (2 × 500 mL). The organic layers were combined and concentrated. The solid was washed with methanol (500 mL × 2) to obtain **1b** in the form of a yellow solid (48.4 g, 66% yield).

LC-MS m/z (ESI) = 375.00 [M+1].

### Step 2:

### 4-Bromo-5-methoxy-2-(4-methoxybenzyl)pyridazin-3(2H)-one (1c)

**1b** (48.4 g, 129.40 mmol, 1.0 equiv) and potassium hydroxide (21.78 g, 388.30 mmol, 3.00 equiv) were dissolved in methanol (417 mL), and the reaction mixture was stirred at room temperature for 2 h. The resulting reaction mixture was concentrated to 80 mL and filtered to obtain a crude product. The resulting filter cake was slurried in water (160 mL) for 1 h and filtered to obtain **1c** in the form of a white solid (38.72 g, 92% yield).

LC-MS m/z (ESI) = 326.30 [M+1].

### Step 3:

### 5-Methoxy-2-(4-methoxybenzyl)-4-(trifluoromethyl)pyridazin-3(2H)-one (1d)

**1c** (14 g, 43.04 mmol, 1.0 equiv) and CuI (4.10 g, 21.52 mmol, 0.50 equiv) were weighed into a 250 mL reaction flask, dissolved with *N*-methylpyrrolidinone (72 mL), followed by the slow addition of methyl 2,2-difluoro-2-(fluorosulfonyl)acetate (16.4 mL, 129.11 mmol, 3.0 equiv). After the addition was completed, the reaction mixture was placed in a 100 °C oil bath and stirred for 3 h. After the reaction was completed, 90 mL of water was added to the reaction mixture to quench the reaction. The resulting solution was extracted with dichloromethane (3 × 60 mL). The organic layers were combined, dried over anhydrous sodium sulfate and concentrated *in vacuo,* and the residue was purified by column chromatography (petroleum ether:ethyl acetate = 1:1) to obtain **1d** in the form of a white solid (12.1 g, 89% yield).

LC-MS m/z (ESI) = 315.10 [M+1].

### Step 4:

### 5-Hydroxy-2-(4-methoxybenzyl)-4-(trifluoromethyl)pyridazin-3(2H)-one (1e)

To a solution of **1d** (12.1 g, 38.52 mmol, 1.0 equiv) in *N*,*N-*dimethyl-formamide (60 mL) was added dropwise iodotrimethylsilane (9.97 g, 50.07 mmol, 1.3 equiv) at room temperature. The resulting reaction mixture was stirred at 85 °C for 20 h. After the reaction was completed, 60 mL of water was added to the reaction mixture to quench the reaction, and then the resulting solution was extracted with dichloromethane (3 × 60 mL). The organic phases were combined, dried over anhydrous sodium sulfate and concentrated *in vacuo,* and the residue was purified by column chromatography (petroleum ether:ethyl acetate = 1:1) to obtain **1e** in the form of a white solid (10.4 g, 90% yield).

LC-MS m/z (ESI) = 301.07 [M+1].

### Step 5:

### 5-Chloro-2-(4-methoxybenzyl)-4-(trifluoromethyl)pyridazin-3(2H)-one (Intermediate 1)

To a solution of compound **1e** (10.4 g, 34.66 mmol, 1.0 equiv) in *N*,*N*-dimethylformamide (52 mL) at 0 °C was slowly added dropwise oxalyl dichloride (8.79 g, 69.32 mmol, 2.0 equiv). After the addition was completed, the reaction mixture was stirred at room temperature for 8 h. After the reaction was completed, 550 mL of water was added to the reaction mixture to quench the reaction. The reaction mixture was filtered to obtain **intermediate** 1 in the form of a white solid (11.04 g, 99% yield).

LC-MS m/z (ESI) = 319.68 [M+1].

### Intermediate 2

### (S)-1-((5-(4-methoxybenzyl)-4,5,6,7-tetrahydropyrazolo[1,5-a]pyrazin-2-yl)methoxy)propan-2-amine (Intermediate 2)

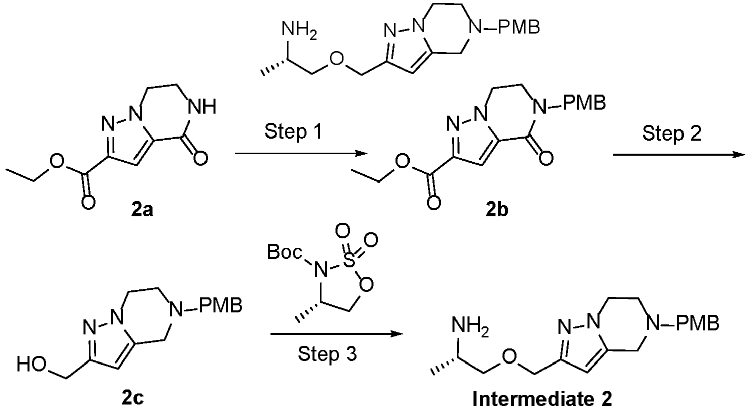

### Step 1:

### Ethyl 5-(4-methoxybenzyl)-4-oxo-4, 5,6,7-tetrahydropyrazolo[1,5-a]pyrazine-2-carboxylate (2b)

Ethyl 4-oxo-4,5,6,7-tetrahydropyrazolo[1,5-*a*]pyrazine-2-carboxylate (**2a,** 5.0 g, 24 mmol, 1.0 equiv) and *p*-methoxybenzyl bromide (4.2 mL, 28.8 mmol, 1.2 equiv) were weighed out and dissolved in *N*,*N-*dimethylformamide (50 mL), followed by the slow addition of sodium hydride (1.15 g, 28.8 mmol, 1.2 equiv) under an ice bath; after the addition was completed, the reaction mixture was reacted at room temperature for 3 h. After the reaction was completed, water was added to quench the reaction. The reaction mixture was extracted with ethyl acetate and dried over anhydrous sodium sulfate, and an organic phase was concentrated to dryness by rotary evaporation. The crude product was purified by flash column chromatography (dichloromethane:methanol = 20:1) to obtain **2b** in the form of a white solid (7.5 g, 92% yield).

¹H NMR (400 MHz, DMSO-*d*₆): δ 7.33 - 7.23 (m, 2H), 7.14 (s, 1H), 6.94 - 6.87 (m, 2H), 4.62 (s, 2H), 4.50 - 4.38 (m, 2H), 4.28 (q, 2H), 3.76 - 3.69 (m, 5H), 1.29 (t, 3H).

LC-MS m/z (ESI) = 330.10 [M+1].

### Step 2:

### (5-(4-Methoxybenzyl)-4,5,6,7-tetrahydropyrazolo[1,5-a]pyrazin-2-yl)methanol (2c)

**2b** (4.1 g, 12.5 mmol, 1.0 equiv) was weighed out and dissolved in tetrahydrofuran (100 mL). Under nitrogen atmosphere, a solution of lithium aluminum hydride in tetrahydrofuran (50 mL, 50 mmol, 4.0 equiv) was slowly added dropwise under an ice bath. After the dropwise addition was completed, the reaction mixture was heated to 70 °C and reacted for 10 min. After the reaction was completed, the reaction mixture was cooled to room temperature, placed in an ice-water bath to quench the reaction, and filtered under vacuum. The filtrate was concentrated to dryness by rotary evaporation, and the crude product was purified by flash column chromatography (dichloromethane:methanol = 10:1) to obtain **2c** in the form of a yellow solid (2.45 g, 71% yield).

LC-MS m/z (ESI) = 274.10 [M+1].

### Step 3:

### (S)-1-((5-(4-methoxybenzyl)-4,5,6,7-tetrahydropyrazolo[1,5-a]pyrazin-2-yl)methoxy)propan-2-amine (Intermediate 2)

**2c** (864 mg, 3.16 mmol, 1.0 equiv) was added to a 25 mL reaction flask and dissolved in anhydrous *N*,*N*-dimethylformamide (18 mL). Under N₂ atmosphere, sodium hydride (300 mg, 7.51 mmol, 2.5 equiv) was added in portions at 0 °C, and after the addition was completed, the reaction mixture was stirred for another 30 min at this temperature. Subsequently, a solution of *tert*-butyl (*S*)-4-methyl-1,2,3-oxathiazolidine-3-carboxylate 2,2-dioxide in *N,N-*dimethylformamide (18 mL) was slowly added dropwise to the reaction system with the temperature maintained at 0 °C, and the reaction mixture was stirred for another 2 h. After the reaction was completed, the pH of the reaction system was adjusted to 3, and the reaction mixture was stirred at room temperature for 0.5 h. The reaction mixture was extracted with EA (3 × 120 mL). The organic layers were combined, dried over anhydrous sodium sulfate and concentrated in *vacuo* to obtain a crude product. The crude product was purified by column chromatography (dichloromethane:methanol = 40:1) to obtain **intermediate 2** in the form of a white solid (252 mg, 24% yield).

LC-MS m/z (ESI) = 331.50 [M+1].

### Intermediate 3

### (S)-5-((1-((4,5,6,7-tetrahydropyrazolo[1,5-a]pyrazin-2-yl)methoxy)propan-2-yl)amino)-4-(trifluoromethyl)pyridazin-3(2H)-one (Intermediate 3)

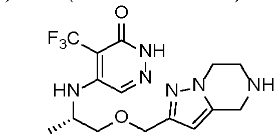

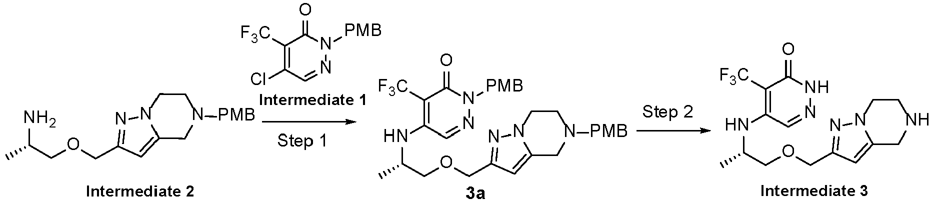

Step 1:

### (S)-2-(4-methoxybenzyl)-5-((1-((5-(4-methoxybenzyl)-4,5,6,7-tetrahydropyrazolo[1,5-a]pyrazin-2-yl)methoxy)propan-2-yl)amino)-4-(trifluoromethyl)pyridazin-3(2H)-one (3a)

**Intermediate 2** (252 mg, 0.764 mmol, 1.0 equiv) and **intermediate 1** (291.4 mg, 0.916 mmol, 1.1 equiv) were weighed into a 10 mL reaction flask and dissolved by the addition of *N*,*N*-dimethylformamide (3.0 mL). Subsequently, *N,N*-diisopropylethylamine (0.5 mL, 3.06 mmol, 4.0 equiv) was added successively. The reaction mixture was stirred at 100 °C for 4 h. After the reaction was completed, the reaction mixture was concentrated *in vacuo,* and the residue was purified by column chromatography (petroleum ether:ethyl acetate = 1:1.5) to obtain **3a** in the form of a white solid (359.8 mg, 77% yield).

LC-MS m/z (ESI) = 613.62 [M+1].

Step 2:

### (S)-5-((1-(((4,5,6,7-tetrahydropyrazolo[1,5-a]pyrazin-2-yl)methoxy)propan-2-yl)amino)-4-(trifluoromethyl)pyridazin-3(2H)-one (Intermediate 3)

To a 10 mL reaction flask with **3a** weighed out (359.8 mg, 0.588 mmol, 1.0 equiv) was added trifluoroacetic acid (3.4 mL) and trifluoromethanesulfonic acid (0.42 mL, 4.7 mmol, 8.0 equiv) successively. After the addition was completed, the reaction mixture was stirred at 25 °C for 1 h. Subsequently, the reaction mixture was stirred in a 70 °C oil bath. After the reaction was completed, 15 mL of water was added to the reaction mixture to quench the reaction. The resulting solution was extracted with ethyl acetate (3 × 15 mL). The pH of the organic layer was adjusted to 8 to 9 by an aqueous potassium carbonate solution. The organic layers were combined and concentrated *in vacuo,* and the residue was purified by MPLC (water/acetonitrile = 1:1) to obtain **intermediate 3** in the form of a white solid (48 mg, 22% yield).

¹H NMR (400 MHz, DMSO-*d*₆) δ 12.46 (s, 1H), 8.81 (s, 2H), 7.90 (s, 1H), 6.28 (dd, 1H), 6.11 (s, 1H), 5.01 (s, 2H), 4.41 (d, 2H), 4.32 (t, 2H), 4.16 (t, 3H), 3.55 - 3.45 (m, 2H), 1.15 (d, 3H).

LC-MS m/z (ESI) = 373.1 [M+1].

### Intermediate 4

### (S)-1-((5-(4-methoxybenzyl)-4,5,6,7-tetrahydropyrazolo[1,5-a]pyrazin-2-yl-4,4-d₂)methoxy-d₂)propan-2-amine (Intermediate 4)

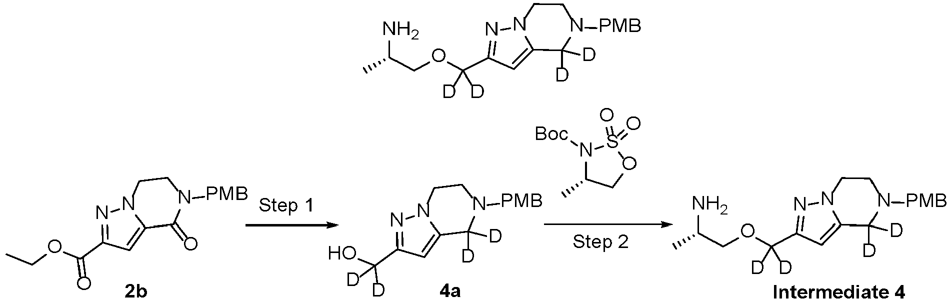

### Step 1:

### (5-(4-Methoxybenzyl)-4,5,6,7-tetrahydropyrazolo[1,5-a]pyrazin-2-yl-4,4-d₂)methan-d₂-ol (4a)

**2b** (3.3 g, 10.0 mmol, 1.0 equiv) was weighed out and dissolved in tetrahydrofuran (60 mL). Under nitrogen atmosphere, lithium aluminum deuteride (1.68 g, 40.0 mmol, 4.0 equiv) was added slowly under an ice bath. After the dropwise addition was completed, the reaction mixture was heated to 70 °C and reacted for 10 min. After the reaction was completed, the reaction mixture was cooled to room temperature, placed in an ice-water bath to quench the reaction, and filtered under vacuum. The filtrate was concentrated to dryness by rotary evaporation, and the crude product was purified by flash column chromatography (dichloromethane:methanol = 10:1) to obtain **4a** in the form of a white solid (2.60 g, 93.8% yield).

¹H NMR (400 MHz, Chloroform-*d*): δ 7.28 (d, 1H), 6.89 (d, 1H), 5.95 (s, 1H), 4.16 (t, 1H), 3.81 (s, 2H), 3.68 (s, 1H).

LC-MS m/z (ESI) = 278.10 [M+1].

### Step 2:

### (S)-1-((5-(4-methoxybenzyl)-4,5,6,7-tetrahydropyrazolo[1,5-a]pyrazin-2-yl-4,4-d₂)methoxy-d₂)propan-2-amine (Intermediate 4)

**4a** (2.6 g, 9.3 mmol, 1.0 equiv) was added to a 25 mL reaction flask and dissolved in anhydrous *N*,*N*-dimethylformamide (26 mL). Under N₂ atmosphere, sodium hydride (720 mg, 18.6 mmol, 2.0 equiv) was added in portions at 0 °C, and after the addition was completed, the reaction mixture was stirred for another 10 min at this temperature. Subsequently, a solution of *tert*-butyl (*S*)-4-methyl-1,2,3-oxathiazolidine-3-carboxylate 2,2-dioxide in *N,N-*dimethylformamide (3.0 g in 18 mL) was slowly added dropwise to the reaction system with the temperature maintained at 0 °C, and the reaction mixture was stirred for another 2 h. After the reaction was completed, the pH of the reaction system was adjusted to 3 with hydrochloric acid solution (2 M) and stirred at room temperature for 0.5 h. The reaction mixture was extracted with EA (3 × 120 mL). The organic layers were combined, dried over anhydrous sodium sulfate and concentrated in *vacuo* to obtain a crude product. The crude product was purified by column chromatography (dichloromethane:methanol = 40:1) to obtain **intermediate 4** in the form of a white solid (2.8 g, 94% yield).

LC-MS m/z (ESI) = 335.10 [M+1].

### Intermediate 5

### (S)-5-((1-((4,5,6,7-tetrahydropyrazolo[1,5-a]pyrazin-2-yl-4,4-d₂)methoxy-d₂)propan-2-yl)amino)-4-(trifluoromethyl)pyridazin-3(2H)-one (Intermediate 5)

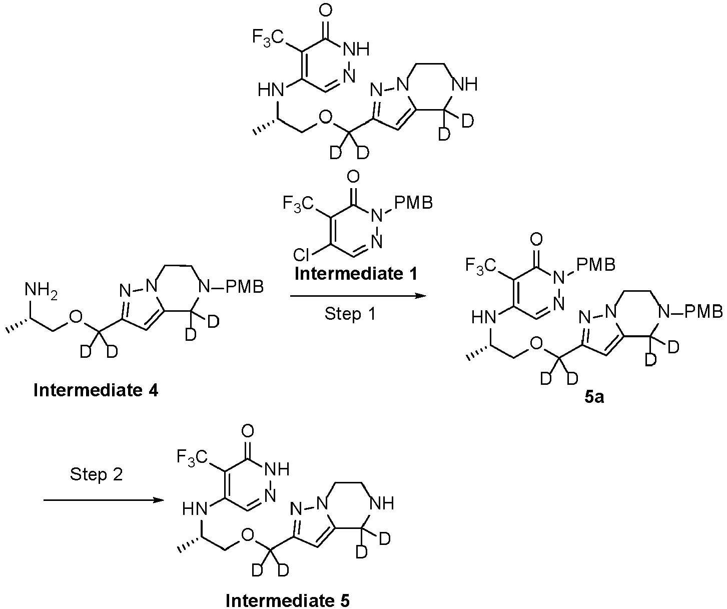

### Step 1:

### (S)-2-(4-methoxybenzyl)-5-((1-((5-(4-methoxybenzyl)-4,5,6,7-tetrahydropyrazolo[1,5-a]pyrazin-2-yl-4,4-d₂)methoxy-d₂)propan-2-yl)amino)-4-(trifluoromethyl)pyridazin-3(2H)-one (5a)

**Intermediate 4** (6 g, 16.2 mmol, 1.5 equiv) and **intermediate 1** (3.4 g, 10.7 mmol, 1.0 equiv) were weighed into a 10 mL reaction flask and dissolved by the addition of acetonitrile (30 mL). Subsequently, triethylamine (6.2 mL, 45 mmol, 4.5 equiv) was added. The reaction mixture was stirred at 80 °C for 4 h. After the reaction was completed, the reaction mixture was concentrated *in vacuo,* and the residue was purified by column chromatography (petroleum ether:ethyl acetate = 1:1.5) to obtain **5a** in the form of a white solid (2.0 g, 30% yield).

LC-MS m/z (ESI) = 617.10 [M+1].

### Step 2:

### (S)-5-((1-((4,5,6,7-tetrahydropyrazolo[1,5-a]pyrazin-2-yl-4,4-d₂)methoxy-d₂)propan-2-yl)amino)-4-(trifluoromethyl)pyridazin-3(2H)-one (Intermediate 5)

To a 10 mL reaction flask with **5a** weighed out (2.0 g, 3.2 mmol, 1.0 equiv) was added trifluoroacetic acid (8 mL) and trifluoromethanesulfonic acid (1.4 mL, 16 mmol, 5.0 equiv) successively. After the addition was completed, the reaction mixture was stirred at 25 °C for 1 h. Subsequently, the reaction mixture was stirred in a 70 °C oil bath. After the reaction was completed, 15 mL of water was added to the reaction mixture to quench the reaction. The resulting solution was extracted with ethyl acetate (3 × 15 mL). The pH of the organic layer was adjusted to 8 to 9 by an aqueous potassium carbonate solution. The organic layers were combined and concentrated *in vacuo,* and the residue was purified by MPLC (water/acetonitrile = 1: 1) to obtain **intermediate** 5 in the form of a white solid (500 mg, 42% yield).

### Intermediate 6

### (S)-2-((2-aminopropoxy)methyl)-5-(4-methoxybenzyl)-6,7-dihydropyrazolo[1,5-a]pyrazin-4(5H)-one (Intermediate 6)

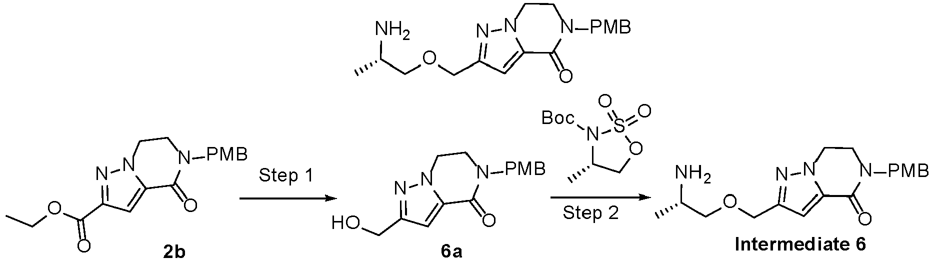

### Step 1:

### 2-(Hydroxymethyl)-5-(4-methoxybenzyl)-6,7-dihydropyrazolo[1,5-a]pyrazin-4(5H)-one (6a)

**2b** (4.0 g, 12.0 mmol, 1.0 equiv) was weighed out and dissolved in absolute methanol (40 mL). Under nitrogen atmosphere, sodium borohydride (9.8 g, 259.0 mmol, 21.5 equiv) was added slowly under an ice bath. The reaction mixture was reacted at room temperature for 48 h, placed in an ice-water bath to quench the reaction, and extracted with dichloromethane. The organic phase was concentrated. The crude product was purified by flash column chromatography (dichloromethane:methanol = 10:1) to obtain **6a** in the form of a white solid (2.80 g, 81% yield).

¹H NMR (400 MHz, Chloroform-*d*): δ 7.26 - 7.21 (m, 2H), 6.91 - 6.84 (m, 3H), 4.70 (s, 2H), 4.68 (s, 2H), 4.31 - 4.20 (m, 2H), 3.80 (s, 3H), 3.66 - 3.58 (m, 2H).

LC-MS m/z (ESI) = 288.10 [M+1].

### Step 2:

### (S)-2-((2-aminopropoxy)methyl)-5-(4-methoxybenzyl)-6,7-dihydropyrazolo[1,5-a]pyrazin-4(5H)-one (Intermediate 6)

**6a** (2.6 g, 9.0 mmol, 1.0 equiv) was added to a 25 mL reaction flask and dissolved in anhydrous *N*,*N*-dimethylformamide (20 mL). Under N₂ atmosphere, sodium hydride (540 mg, 13.5 mmol, 1.5 equiv) was added in portions at 0 °C, and after the addition was completed, the reaction mixture was stirred for another 10 min at this temperature. Subsequently, a solution of *tert*-butyl (*S*)-4-methyl-1,2,3-oxathiazolidine-3-carboxylate 2,2-dioxide in *N,N-*dimethylformamide (4.2 g in 20 mL) was slowly added dropwise to the reaction system with the temperature maintained at 0 °C, and the reaction mixture was stirred for another 2 h. After the reaction was completed, the pH of the reaction system was adjusted to 3 with hydrochloric acid solution (2 M) and stirred at room temperature for 0.5 h. The reaction mixture was extracted with EA (3 × 120 mL). The organic layers were combined, dried over anhydrous sodium sulfate and concentrated in *vacuo* to obtain a crude product. The crude product was purified by column chromatography (dichloromethane:methanol = 40:1) to obtain **intermediate 6** in the form of a white solid (3.0 g, 97% yield).

LC-MS m/z (ESI) = 345.10 [M+1].

### Intermediate 7

### (S)-2-((2-((6-oxo-5-(trifluoromethyl)-1,6-dihydropyridazin-4-yl)amino)propoxy)methyl)-6,7-dihydropyrazolo[1,5-a]pyrazin-4(5H)-one (Intermediate 7)

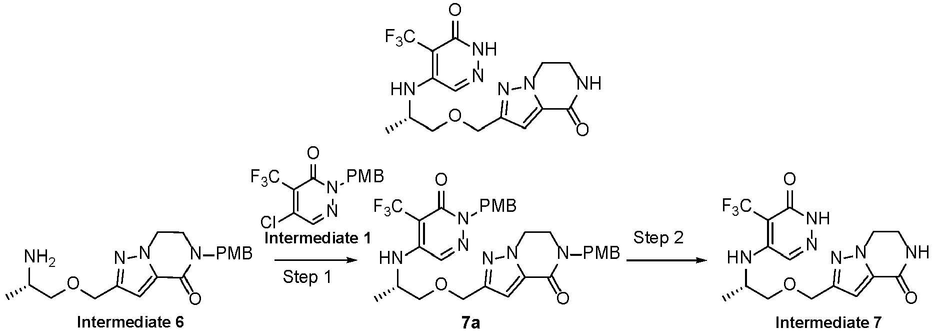

### Step 1:

### (S)-5-(4-methoxybenzyl)-2-((2-((1-(4-methoxybenzyl)-6-oxo-5-(trifluoromethyl)-1,6-dihydropyridazin-4-yl)amino)propoxy)methyl)-6,7-dihydropyrazolo[1,5-a]pyrazin-4(5H)-one (7a)

**Intermediate 6** (3.7 g, 10.7 mmol, 1.5 equiv) and **intermediate 1** (2.3 g, 7.2 mmol, 1.0 equiv) were weighed into a 100 mL reaction flask and dissolved by the addition of acetonitrile (20 mL). Subsequently, triethylamine (4.5 mL, 32.4 mmol, 4.5 equiv) was added. The reaction mixture was stirred at 80 °C for 4 h. After the reaction was completed, the reaction mixture was concentrated *in vacuo,* and the residue was purified by column chromatography (petroleum ether:ethyl acetate = 1:1.5) to obtain **7a** in the form of a white solid (5.0 g, 74% yield).

LC-MS m/z (ESI) = 627.10 [M+1].

### Step 2:

### (S)-2-((2-((6-oxo-5-(trifluoromethyl)-1,6-dihydropyridazin-4-yl)amino)propoxy)methyl)-6,7-dihydropyrazolo[1,5-a]pyrazin-4(5H)-one (Intermediate 7)

To a 10 mL reaction flask with **7a** weighed out (4.7 g, 7.5 mmol, 1.0 equiv) was added trifluoroacetic acid (18 mL) and trifluoromethanesulfonic acid (4.0 mL, 45 mmol, 6 equiv) successively. After the addition was completed, the reaction mixture was stirred at 25 °C for 2 h. After the reaction was completed, 15 mL of water was added to the reaction mixture to quench the reaction. The resulting solution was extracted with ethyl acetate (3 × 15 mL). The pH of the organic layer was adjusted to 8 to 9 by an aqueous potassium carbonate solution. The organic layers were combined and concentrated *in vacuo,* and the residue was purified by MPLC (water/acetonitrile = 1:1) to obtain **intermediate** 7 in the form of a white solid (2.6 g, 89% yield).

LC-MS m/z (ESI) = 387.10 [M+1].

### Intermediate 8

### (2S)-1-(1-(5-(4-methoxybenzyl)-4,5,6,7-tetrahydropyrazolo[1,5-a]pyrazin-2-yl)ethoxy)propan-2-amine (Intermediate 8)

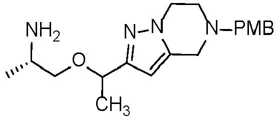

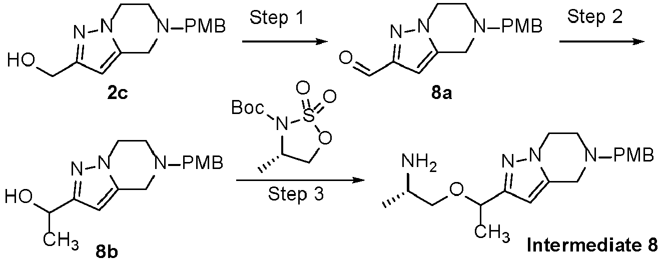

### Step 1:

### 5-(4-Methoxybenzyl)-4,5,6,7-tetrahydropyrazolo[1,5-a]pyrazine-2-carbaldehyde (8a)

(5-(4-Methoxybenzyl)-4,5,6,7-tetrahydropyrazolo[1,5-*a*]pyrazin-2-yl)methanol (**2c,** 1.4 g, 5.1 mmol, 1.0 equiv) and Dess-Martin periodinane (2.6 g, 6.2 mmol, 1.2 equiv) were weighed out and dissolved in dichloromethane (20 mL), and after the addition was completed, the reaction mixture was reacted at room temperature for 3 h. After the reaction was completed, the reaction mixture was filtered, and the organic phase was concentrated to dryness by rotary evaporation to obtain crude **8a** in the form of a pale red oil, which was directly used in the next step (1.0 g, 71.4% yield).

LC-MS m/z (ESI) = 272.4 [M+1].

### Step 2:

### 1-(5-(4-Methoxybenzyl)-4,5,6,7-tetrahydropyrazolo[1,5-a]pyrazin-2-yl)ethan-1-ol (8b)

**8a** (1.0 g, 3.7 mmol, 1.0 equiv) was weighed out and dissolved in tetrahydrofuran (20 mL). Under nitrogen atmosphere, a solution of methylmagnesium bromide in tetrahydrofuran (3.7 mL, 11 mmol, 3.0 equiv) was added slowly dropwise under a dry ice/ethanol bath. The reaction mixture was reacted for 2 h, and the reaction was completed. The reaction was quenched with saturated ammonium chloride, and the reaction mixture was extracted with ethyl acetate (3 × 60 mL). The organic layers were combined, dried over anhydrous sodium sulfate and concentrated *in vacuo,* and the residue was purified by column chromatography (petroleum ether:ethyl acetate = 1:2) to obtain **8b** in the form of a pale yellow oil (600 mg, 57% yield).

LC-MS m/z (ESI) = 288.10 [M+1].

### Step 3:

### (2S)-1-(1-(5-(4-methoxybenzyl)-4,5,6,7-tetrahydropyrazolo[1,5-a]pyrazin-2-yl)ethoxy)propan-2-amine (Intermediate 8)

**8b** (600 mg, 2 mmol, 1.0 equiv) was added to a 25 mL reaction flask and dissolved in anhydrous *N,N*-dimethylformamide (10 mL). Under N₂ atmosphere, sodium hydride (120 mg, 5 mmol, 2.5 equiv) was added in portions at 0 °C, and after the addition was completed, the reaction mixture was stirred for another 30 min at this temperature. Subsequently, a solution of *tert*-butyl (*S*)-4-methyl-1,2,3-oxathiazolidine-3-carboxylate 2,2-dioxide in *N,N-*dimethylformamide (10 mL) was slowly added dropwise to the reaction system with the temperature maintained at 0 °C, and the reaction mixture was stirred for another 2 h. After the reaction was completed, the pH of the reaction system was adjusted to 3, and the reaction mixture was stirred at room temperature for 0.5 h. The reaction mixture was extracted with EA (3 × 120 mL). The organic layers were combined, dried over anhydrous sodium sulfate and concentrated *in vacuo* to obtain a crude product. The crude product was purified by column chromatography (ethyl acetate:petroleum ether = 1:0) to obtain **intermediate 8** in the form of a pale yellow solid (600 mg, 90% yield).

LC-MS m/z (ESI) = 334.50 [M+1].

### Intermediate 9

### 5-(((2S)-1-(1-(4,5,6,7-tetrahydropyrazolo[1,5-a]pyrazin-2-yl)ethoxy)propan-2-yl)amino)-4-(trifluoromethyl)pyridazin-3(2H)-one (Intermediate 9)

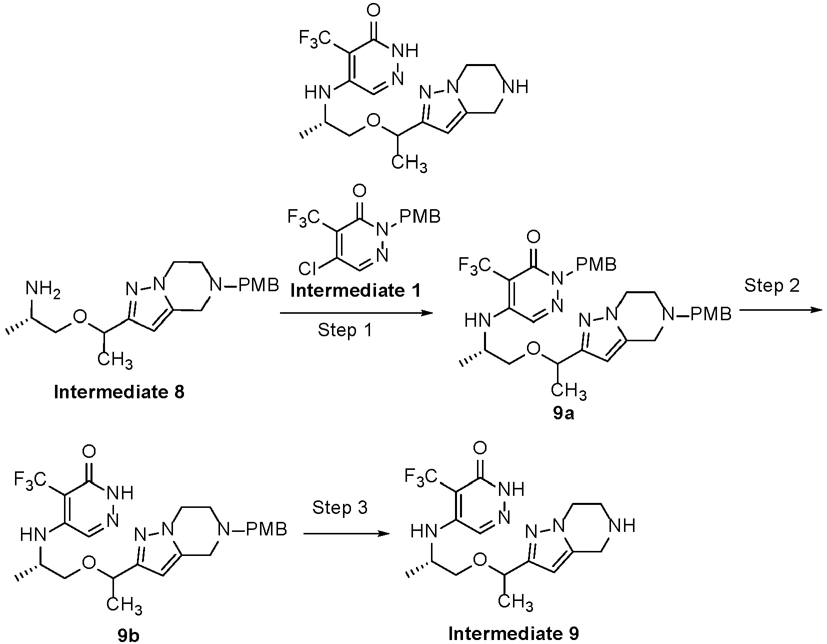

### Step 1:

### 2-(4-Methoxybenzyl)-5-(((2S)-1-(1-(5-(4-methoxybenzyl)-4,5,6,7-tetrahydropyrazolo [1,5-a]pyrazin-2-yl)ethoxy)propan-2-yl)amino)-4-(trifluoromethyl)pyridazin-3(2H)-one (9a)

**Intermediate 8** (600 mg, 1.75 mmol, 1.0 equiv) and **intermediate 1** (660 mg, 1.92 mmol, 1.1 equiv) were weighed into a 10 mL reaction flask and dissolved by the addition of *N,N-*dimethylformamide (5.0 mL). Subsequently, triethylamine (1.21 mL, 8.75 mmol, 5.0 equiv) was added successively. The reaction mixture was stirred at 100 °C for 2 h. After the reaction was completed, the reaction mixture was concentrated *in vacuo,* and the residue was purified by column chromatography (petroleum ether:ethyl acetate = 0:1) to obtain **9a** in the form of a white solid (630 mg, 52% yield).

LC-MS m/z (ESI) = 627.28 [M+1].

### Step 2:

### (S)-5-((1-((4,5,6,7-tetrahydropyrazolo[1,5-a]pyrazin-2-yl)methoxy)propan-2-yl)amino)-4-(trifluoromethyl)pyridazin-3(2H)-one (9b)

To a 25 mL reaction flask with **9a** weighed out (630 mg, 1 mmol, 1.0 equiv) was added trifluoroacetic acid (5 mL) and trifluoromethanesulfonic acid (1 mL, 8 mmol, 8.0 equiv) successively. After the addition was completed, the reaction mixture was stirred at room temperature for 1 h. After the reaction was completed, 15 mL of water was added to the reaction mixture to quench the reaction. The resulting solution was extracted with ethyl acetate (3 × 15 mL). The pH of the organic layer was adjusted to 8 to 9 by an aqueous potassium carbonate solution. The organic layers were combined and concentrated *in vacuo,* and the residue was purified by MPLC (water/acetonitrile = 4:6) to obtain **9b** in the form of a white solid (370 mg, 73.21% yield).

LC-MS m/z (ESI) = 507.23 [M+1].

### Step 3:

### 5-(((2S)-1-(1-(4,5,6,7-tetrahydropyrazolo[1,5-a]pyrazin-2-yl)ethoxy)propan-2-yl)amino)-4-(trifluoromethyl)pyridazin-3(2H)-one (Intermediate 9)

To a 25 mL reaction flask with **9b** weighed out (370 mg, 0.73 mmol, 1.0 equiv) were added 1,2 dichloroethane (5 mL), 1-chloroethyl chloroformate (0.78 mL, 7.3 mmol, 10.0 equiv) and potassium carbonate (203 mg, 1.46 mmol, 2.0 equiv) successively. After the addition was completed, the reaction mixture was stirred at room temperature for 8 h. After the reaction was completed, 1,2-dichloroethane was concentrated to dryness by rotary evaporation, and the reaction mixture was added with methanol (10 mL) and refluxed for 2 h. The reaction mixture was concentrated *in vacuo,* and the residue was purified by MPLC (water/acetonitrile = 4:6) to obtain **intermediate 9** in the form of a white solid (100 mg, 35.40% yield).

LC-MS m/z (ESI) = 387.17 [M+1].

### Intermediate 10

### (S)-2-((2-((1-(4-methoxybenzyl)-6-oxo-5-(trifluoromethyl)-1,6-dihydropyridazin-4-yl)amino)propoxy)methyl)-6,7-dihydropyrazolo[1,5-a]pyrazin-4(5H)-one (Intermediate 10)

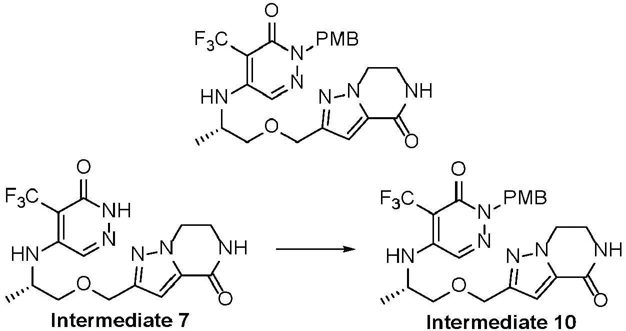

To a solution of **intermediate 7** (386 mg, 1.0 mmol, 1.0 equiv) in *N*,*N*-dimethylformamide (5 mL) was added sodium hydride (60 mg, 1.5 mmol, 1.5 equiv, 60%) at 10 °C, followed by the addition of 1-(chloromethyl)-4-methoxybenzene (187 mg, 1.2 mmol, 1.2 equiv) at 0 °C. After the addition was completed, the reaction mixture was stirred at room temperature for 3 h. After the reaction was completed, the reaction mixture was slowly poured into an ice-water mixture to quench the reaction and extracted with dichloromethane (2 × 5 mL), and the organic phase was concentrated. The crude product was subjected to column chromatography (dichloromethane:methanol = 20: 1) to obtain **intermediate 10** in the form of a white oil (300 mg, 59% yield).

LC-MS m/z (ESI) = 507.1 [M+1].

### Intermediate 11

### (S)-5-((1-((3-chloro-4,5,6,7-tetrahydropyrazolo[1,5-a]pyrazin-2-yl)methoxy)propan-2-yl)amino)-4-(trifluoromethyl)pyridazin-3(2H)-one (Intermediate 11)

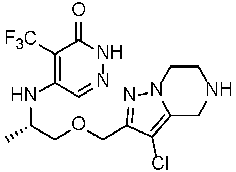

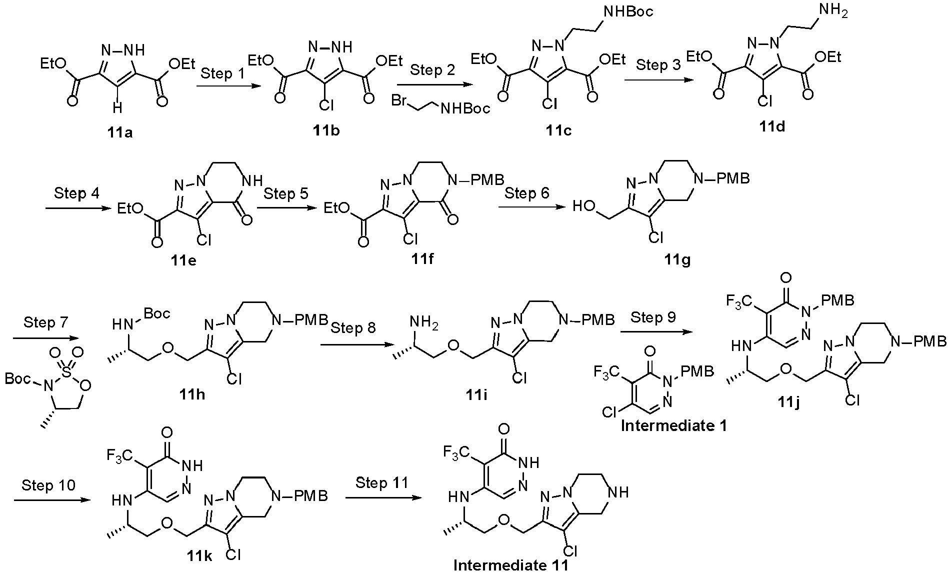

### Step 1:

### Diethyl 4-chloro-1H-pyrazole-3,5-dicarboxylate (11b)

**11a** (16.0 g, 75.47 mmol, 1.0 equiv) was added in a 500 mL single-necked flask at room temperature and dissolved in 200 mL of acetic acid. The reaction mixture was added with 10% sodium hypochlorite (100 mL) and reacted at room temperature for 3 h. After the reaction was completed, the reaction mixture was poured into 600 mL of water and filtered, and the solid was washed and dried to obtain **11b** in the form of a white solid (13 g, 70% yield).

LC-MS m/z (ESI) = 246.04 [M+1].

### Step 2:

### Diethyl 1-(2-((tert-butoxycarbonyl)amino)ethyl)-4-chloro-1H-pyrazole-3,5-dicarboxylate (11c)

**11b** (13 g, 52.8 mmol, 1.0 equiv) was weighed into a 500 mL three-necked flask, and dissolved by the addition of anhydrous *N*,*N*-dimethylformamide (150 mL). The reaction mixture was slowly added with *tert*-butyl (2-bromoethyl)carbamate (13 g, 58.1 mmol, 1.1 equiv), and after the addition was completed, the reaction mixture was added with cesium carbonate (25.8 g, 79.2 mmol, 1.5 equiv), heated to 50 °C and reacted for 3 h. After the reaction was completed, the reaction mixture was poured into 500 mL of water and filtered, and the solid was washed and dried to obtain **11c** in the form of a white solid (12.2 g, 59% yield).

LC-MS m/z (ESI) = 389.13 [M+1].

### Step 3:

### Diethyl-1-(2-aminoethyl)-4-chloro-1H-pyrazole-3,5-dicarboxylate (11d)

**11c** (12.2 g, 30.77 mmol, 1.0 equiv) was added to a 500 mL reaction flask and dissolved by the addition of dioxane hydrochloride solution (4N, 100 mL) under an ice bath. The reaction mixture was stirred for 1 h. After the reaction was completed, the reaction mixture was concentrated *in vacuo* to obtain **11d** in the form of a white solid (11 g, 99% yield).

LC-MS m/z (ESI) = 289.08 [M+1].

### Step 4:

### Ethyl-3-chloro-4-oxo-4,5,6,7-tetrahydropyrazolo[1,5-a]pyrazine-2-carboxylate (11e)

**11d** (12.2 g, 41.4 mmol, 1.0 equiv) was added to a 500 mL reaction flask, and dissolved by the addition of a solution of potassium carbonate (17.25 g, 124 mmol, 3.0 equiv) in water (250 mL). The reaction mixture was stirred for 1 h. The reaction mixture was filtered, and the solid was washed and dried to obtain **11e** in the form of a white solid (8.0 g, 79% yield).

LC-MS m/z (ESI) = 243.04 [M+1].

### Step 5:

### Ethyl-3-chloro-5-(4-methoxybenzyl)-4-oxo-4,5,6,7-tetrahydropyrazolo[1,5-a]pyrazine-2-carboxylate (11f)

The synthesis method of **2b** described above was referred to obtain **11f** in the form of a white solid (7 g, 54% yield).

LC-MS m/z (ESI) = 363.10 [M+1].

### Step 6:

### (3-Chloro-5-(4-methoxybenzyl)-4,5,6,7-tetrahydropyrazolo[1,5-a]pyrazin-2-yl)methanol (11g)

The synthesis method of **2c** described above was referred to obtain **11g** in the form of a white solid (4.2 g, 65% yield).

LC-MS m/z (ESI) = 307.11 [M+1].

### Step 7:

### Tert-butyl-(S)-(1-((3-chloro-5-(4-methoxybenzyl)-4,5,6,7-tetrahydropyrazolo[1,5-a]pyrazin-2-yl)methoxy)propan-2-yl)carbamate (11h)

**11g** (4.2 g, 11.5 mmol, 1.0 equiv) was added to a 250 mL reaction flask and dissolved in anhydrous *N*,*N*-dimethylformamide (100 mL). Under nitrogen atmosphere, sodium hydride (552 mg, 23 mmol, 2.0 equiv) was added in portions at 0 °C, and after the addition was completed, the reaction mixture was stirred for another 30 min at this temperature. Subsequently, a solution of *tert*-butyl (*S*)-4-methyl-1,2,3-oxathiazolidine-3-carboxylate 2,2-dioxide (4.1 g, 17.3 mmol, 1.5 equiv) in *N*,*N*-dimethylformamide (50 mL) was slowly added dropwise to the reaction system with the temperature maintained at 0 °C, and the reaction mixture was stirred for another 2 h. After the reaction was completed, the pH of the reaction system was adjusted to 3, and the reaction mixture was stirred at room temperature for 0.5 h. The reaction mixture was extracted with ethyl acetate (3 × 120 mL). The organic layers were combined, dried over anhydrous sodium sulfate and concentrated in *vacuo* to obtain a crude product. The crude product was purified by column chromatography (dichloromethane:methanol = 40:1) to obtain **11h** in the form of a yellow oil (3.6 g, 67% yield).

LC-MS m/z (ESI) = 464.22 [M+1].

### Step 8:

### (S)-1-((3-chloro-5-(4-methoxybenzyl)-4,5,6,7-tetrahydropyrazolo[1,5-a]pyrazin-2-yl)methoxy)propan-2-amine (11i)

**11h** (3.6 g, 9.13 mmol, 1.0 equiv) was added to a 100 mL reaction flask and dissolved by the addition of hydrochloric acid (4 N, 30 mL) under an ice bath. The reaction mixture was stirred for 1 h. After the reaction was completed, the reaction mixture was concentrated *in vacuo* to obtain **11i** in the form of a yellow oil (3.2 g, 92% yield).

LC-MS m/z (ESI) = 364.17 [M+1].

### Step 9:

### (S)-5-((1-((3-chloro-5-(4-methoxybenzyl)-4,5,6,7-tetrahydropyrazolo[1,5-a]pyrazin-2-yl)methoxy)propan-2-yl)amino)-2-(4-methoxybenzyl)-4-(trifluoromethyl)pyridazin-3(2H)-one (11j)

The synthesis method of **3a** described above was referred to obtain **11j** in the form of a white solid (2.2 g, 76% yield).

LC-MS m/z (ESI) = 646.29 [M+1].

### Step 10:

### (S)-5-((1-((3-chloro-5-(4-methoxybenzyl)-4,5,6,7-tetrahydropyrazolo[1,5-a]pyrazin-2-yl)methoxy)propan-2-yl)amino)-4-(trifluoromethyl)pyridazin-3(2H)-one (11k)

To a 100 mL reaction flask with **11j** weighed out (2.6 g, 4.0 mmol, 1.0 equiv) was added trifluoroacetic acid (20 mL) and trifluoromethanesulfonic acid (1.37 mL, 16 mmol, 4.0 equiv) successively. After the addition was completed, the reaction mixture was stirred at 25 °C for 1 h. After the reaction was completed, the resulting solution was quenched with saturated potassium carbonate, adjusted to pH 8 to 9, and extracted with ethyl acetate (3 × 50 mL). The organic layers were combined and concentrated *in vacuo* to obtain **11k** in the form of a white solid (2.1 g, 99% yield).

LC-MS m/z (ESI) = 526.17 [M+1].

Step 11:

### (S)-5-((1-((3-chloro-4,5,6,7-tetrahydropyrazolo[1,5-a]pyrazin-2-yl)methoxy)propan-2-yl)amino)-4-(trifluoromethyl)pyridazin-3(2H)-one (Intermediate 11)

To a 25 mL reaction flask with **11k** weighed out (1.8 g, 3.4 mmol, 1.0 equiv) was added 1,2-dichloroethane (20 mL), 1-chloroethyl chloroformate (2.45 mL, 17.1 mmol, 5.0 equiv) and *N*,*N*-diisopropylethylamine (2.24 mL, 13.6 mmol, 4 equiv) successively. After the addition was completed, the reaction mixture was stirred at room temperature for 3 h. After the reaction was completed, 1,2-dichloroethane was concentrated to dryness by rotary evaporation, and the reaction mixture was added with methanol (20 mL) and refluxed for 2 h. The reaction mixture was concentrated *in vacuo,* and the residue was purified by MPLC (water/acetonitrile = 4:6) to obtain **intermediate 11** in the form of a white solid (400 mg, 29% yield).

LC-MS m/z (ESI) = 406.11 [M+1].

### Intermediate 12

### (5,6,7,8-Tetrahydro-[1,2,4]triazolo[1,5-a]pyrazin-2-yl)methanol (Intermediate 12)

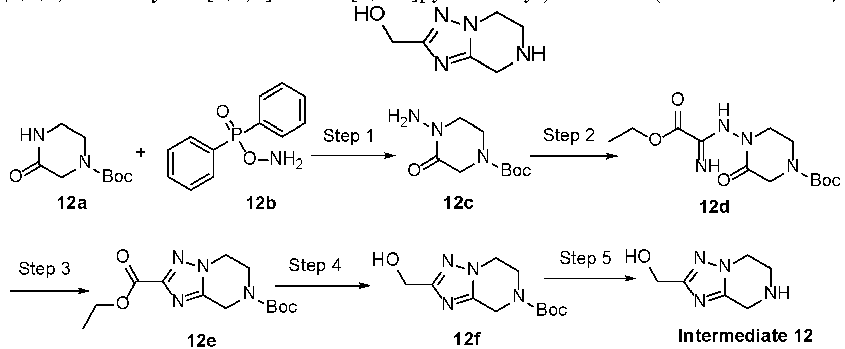

### Step 1:

### Tert-butyl 4-amino-3-oxopiperazine-1-carboxylate (12c)

**12a** (2.0 g, 10 mmol, 1.0 equiv) was weighed out and dissolved in *N*,*N*-dimethylformamide (50 mL). The reaction mixture was added with sodium hydride (600 mg, 15 mmol, 1.5 equiv) under an ice bath, reacted for 20 min, added with **12b** (3.0 g, 13 mmol, 1.3 equiv) in portions, stirred at room temperature for 24 h and filtered under vacuum. The filtrate was concentrated to dryness by rotary evaporation to obtain crude **12c** (2.0 g, 93% yield), which was directly used in the next step.

LC-MS m/z (ESI) = 216.10 [M+1].

### Step 2:

### Tert-butyl 4-(2-ethoxy-2-oxoacetimidamido)-3-oxopiperazine-1-carboxylate (12d)

**12c** (1.3 g, 6.0 mmol, 1.0 equiv) was dissolved in absolute ethanol, and the reaction mixture was added with ethyl 2-ethoxy-2-iminoacetate (2.0 mL, 15 mmol, 2.5 equiv) and reacted at 90 °C for 3 h. The reaction mixture was cooled to room temperature and concentrated to dryness by rotary evaporation to obtain crude **12d** (1.8 g, 95% yield), which was used directly in the next step.

LC-MS m/z (ESI) = 315.10 [M+1].

### Step 3:

### 7-(Tert-butyl) 2-ethyl 5,6-dihydro-[1,2,4]triazolo[1,5-a]pyrazine-2,7(8H)-dicarboxylate (12e)

**12d** (4.4 g, 14 mmol, 1.0 equiv) was weighed out and dissolved in toluene (60 mL), and the reaction mixture was added with *p*-toluenesulfonic acid (3.1 g, 16.7 mmol, 1.2 equiv), and condensed under reflux (a water separator to separate water) at 130 °C for 2 h. After the reaction was completed, the reaction mixture was cooled to room temperature and concentrated, and the crude product was subj ected to column chromatography (*n*-hexane: ethyl acetate = 2:1) to obtain **12e** in the form of a brown oil (1.5 g, 36% yield).

LC-MS m/z (ESI) = 297.10 [M+1].

### Step 4:

### Tert-butyl 2-(hydroxymethyl)-5,6-dihydro-[1,2,4]triazolo[1,5-a]pyrazine-7(8H)-carboxylate (12f)

**12e** (0.9 g, 3 mmol, 1.0 equiv) was dissolved in absolute methanol (10 mL), and the reaction mixture was added with sodium borohydride (1.1 g, 30 mmol, 10 equiv) and reacted at room temperature for 24 h. The reaction was quenched with saturated ammonium chloride. The reaction mixture was extracted with dichloromethane (10 mL). The organic phase was concentrated and the crude product was subjected to column chromatography (*n*-hexane:ethyl acetate = 1:1) to obtain **12f** in the form of a brown oil (0.7 g, 92% yield).

LC-MS m/z (ESI) = 255.10 [M+1].

### Step 5:

### (5,6,7,8-Tetrahydro-[1,2,4]triazolo[1,5-a]pyrazin-2-yl)methanol (Intermediate 12)

**12f** (300 mg, 1.2 mmol, 1.0 equiv) was weighed out and dissolved in dioxane hydrochloride (3 mL, 4 M), and the reaction mixture was reacted at room temperature for 2 h and filtered under vacuum. The filter cake was dried to obtain **intermediate 12** in the form of a colorless liquid (120 mg, 65% yield).

LC-MS m/z (ESI) = 155.10 [M+1].

### Intermediate 13

### (S)-1-((3-(trifluoromethyl)-5-(5-(trifluoromethyl)pyrimidin-2-yl)-4,5,6,7-tetrahydropyrazolo[1,5-a]pyrazin-2-yl)methoxy)propan-2-amine (Intermediate 13)

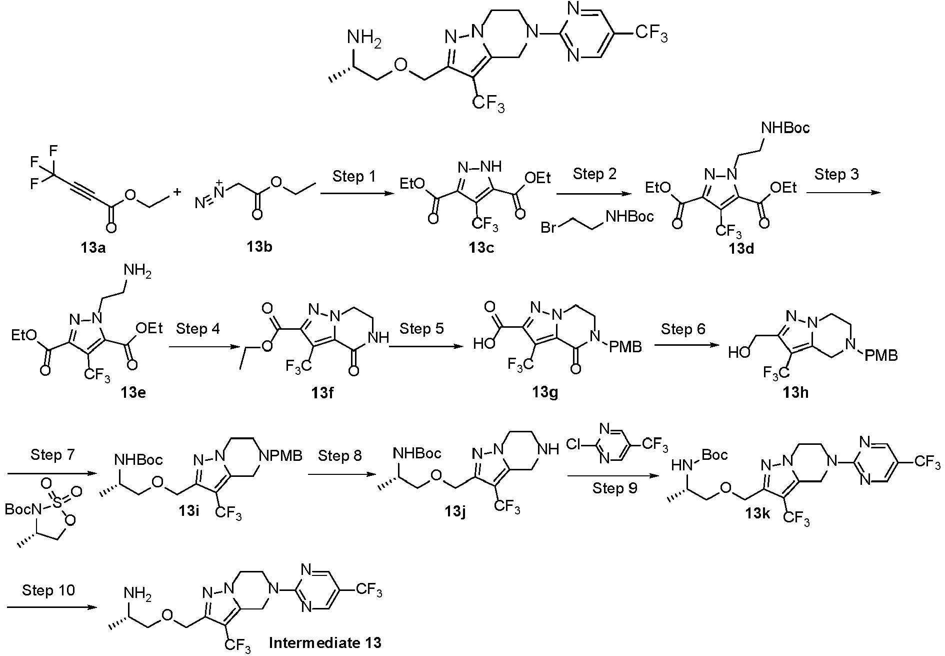

### Step 1:

### Diethyl 4-(trifluoromethyl)-1H-pyrazole-3,5-dicarboxylate (13c)

**13a** (12.0 g, 75 mmol, 2.0 equiv) was added to a 500 mL single-necked flask at room temperature and dissolved in 200 mL tetrahydrofuran, and the reaction mixture was added with 13b (4.275 g, 37.5 mmol, 1.0 equiv) and reacted at room temperature for 2 h. After the reaction was completed, the organic layers were combined, and the reaction mixture was concentrated *in vacuo* and subjected to column chromatography (petroleum ether:ethyl acetate = 4:1) to obtain **13c** in the form of a yellow oil (12.1 g, 58% yield).

LC-MS m/z (ESI) = 280.07 [M+1].

### Step 2:

### Diethyl-1-(2-((tert-butoxycarbonyl)amino)ethyl)-4-(trifluoromethyl)-1H-pyrazole-3,5-dicarboxylate (13d)

The synthesis method of **11c** described above was referred to obtain **13d** in the form of a pale yellow solid (15 g, 63% yield).

LC-MS m/z (ESI) = 423.16 [M+1].

### Step 3:

### Diethyl-1-(2-aminoethyl)-4-(trifluoromethyl)-1H-pyrazole-3,5-dicarboxylate (13e)

The synthesis method of **11d** described above was referred to obtain crude **13e** in the form of a white solid (15 g, 62% yield).

LC-MS m/z (ESI) = 323.11 [M+1].

### Step 4:

### Ethyl-4-oxo-3-(trifluoromethyl)-4,5,6,7-tetrahydropyrazolo[1,5-a]pyrazine-2-carboxylate (13f)

The synthesis method of **11e** described above was referred to obtain crude **13f** in the form of a white solid (12 g, 75% yield).

LC-MS m/z (ESI) = 277.07 [M+1].

### Step 5:

### 5-(4-Methoxybenzyl)-4-oxo-3-(trifluoromethyl)-4,5,6,7-tetrahydropyrazolo[1,5-a]pyrazine-2-carboxylic acid (13g)

The synthesis method of **2b** described above was referred to obtain **13g** in the form of a yellow oil (12 g, 68% yield).

LC-MS m/z (ESI) = 369.09 [M+1].

### Step 6:

### (5-(4-Methoxybenzyl)-3-(trifluoromethyl)-4,5,6,7-tetrahydropyrazolo[1,5-a]pyrazin-2-yl)methanol (13h)

The synthesis method of **2c** described above was referred to obtain **13h** in the form of a white solid (500 mg, 35% yield).

LC-MS m/z (ESI) = 341.14 [M+1].

### Step 7:

### Tert-butyl-(S)-(1-((5-(4-methoxybenzyl)-3-(trifluoromethyl)-4,5,6,7-tetrahydropyrazolo[1,5-a]pyrazin-2-yl)methoxy)propan-2-yl)carbamate (13i)

**13h** (500 mg, 1.47 mmol, 1.0 equiv) was added to a 100 mL reaction flask and dissolved in anhydrous *N*,*N*-dimethylformamide (10 mL). Under nitrogen atmosphere, sodium hydride (70 mg, 2.93 mmol, 2.0 equiv) was added in portions at 0 °C, and after the addition was completed, the reaction mixture was stirred for another 30 min at this temperature. Subsequently, a solution of *tert*-butyl (*S*)-4-methyl-1,2,3-oxathiazolidine-3-carboxylate 2,2-dioxide (696.78 mg, 2.93 mmol, 2.0 equiv) in *N*,*N*-dimethylformamide (10 mL) was slowly added dropwise to the reaction system with the temperature maintained at 0 °C, and the reaction mixture was stirred for another 2 h. After the reaction was completed, the pH of the reaction system was adjusted to 3, and the reaction mixture was stirred at room temperature for 0.5 h. The reaction mixture was extracted with EA (3 × 120 mL). The organic layers were combined, dried over anhydrous sodium sulfate and concentrated in *vacuo* to obtain a crude product. The crude product was purified by column chromatography (dichloromethane:methanol = 40:1) to obtain **13i** in the form of a white solid (300 mg, 41% yield).

LC-MS m/z (ESI) = 498.24 [M+1].

### Step 8:

### Tert-butyl-(S)-(1-((3-(trifluoromethyl)-4,5,6,7-tetrahydropyrazolo[1,5-a]pyrazin-2-yl)methoxy)propan-2-yl)carbamate (13j)

The similar synthesis method of **intermediate 11** described above was referred to obtain crude **13j** in the form of a yellow oil (300 mg, 70% yield).

LCMS m/z = 378.19 [M+1].

### Step 9:

### Tert-butyl-(S)-(1-((3-(trifluoromethyl)-5-(5-(trifluoromethyl)pyrimidin-2-yl)-4,5,6,7-tetrahydropyrazolo[1,5-a]pyrazin-2-yl)methoxy)propan-2-yl)carbamate (13k)

**13j** (300 mg, 0.6 mmol, 1.0 equiv) and 2-chloro-5-trifluoromethylpyrimidine (163 mg, 0.9 mmol, 1.5 equiv) were weighed into a 50 mL reaction flask and dissolved in *N,N-*dimethylformamide (10.0 mL). The reaction mixture was added with *N*,*N*-diisopropylethylamine (0.495 mL, 3 mmol, 5.0 equiv) and stirred at 90 °C for 1 h. After the reaction was completed, the reaction mixture was concentrated *in vacuo.* The residue was purified by MPLC (water/acetonitrile = 1:1) to obtain **13k** in the form of a yellow oil (200 mg, 64% yield).

LCMS m/z = 524.20 [M+1].

### Step 10:

### (S)-1-((3-(trifluoromethyl)-5-(5-(trifluoromethyl)pyrimidin-2-yl)-4,5,6,7-tetrahydropyrazolo[1,5-a]pyrazin-2-yl)methoxy)propan-2-amine (Intermediate 13)

**13k** (200 g, 0.38 mmol, 1.0 equiv) was added to a 100 mL reaction flask and dissolved by the addition of hydrochloric acid (4 N, 5 mL) under an ice bath. The reaction mixture was stirred for 1 h. After the reaction was completed, the reaction mixture was concentrated *in vacuo* to obtain **intermediate 13** in the form of a white solid (161.8 mg, 98.3% yield).

LCMS m/z = 424.20 [M+1].

### Intermediate 14

### 2-Chloro-5-cyclopropylpyrimidine (Intermediate 14)

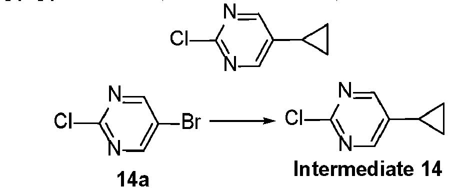

**14a** (2.0 g, 10.34 mmol, 1.0 equiv) was added to the mixed solution of toluene/water (50 mL/2.5 mL), and the reaction mixture was added with cyclopropylboronic acid (1.15 g, 13.44 mmol, 1.3 equiv), Pd(dppf)Cl₂ (0.422 g, 0.517 mmol, 0.05 equiv) and cesium carbonate (10.11 g, 31.019 mmol, 3.0 equiv) successively. After the mixture was completed, the reaction mixture was reacted at 80 °C for 5.0 h. After the reaction was completed, 30 mL of water was added to the reaction mixture, and the reaction mixture was extracted with 3 × 40 mL ethyl acetate (3 × 40 mL). The organic layers were combined, dried over anhydrous sodium sulfate and concentrated *in vacuo,* and the residue was purified by column chromatography (petroleum ether:ethyl acetate = 10:1) to obtain **intermediate 14** in the form of a white solid (1.32 g, 83% yield).

¹H NMR (400 MHz, DMSO-*d*₆): δ 8.55 (s, 2H), 2.00 - 1.93 (m, 1H), 1.09 - 1.02 (m, 2H), 0.90 - 0.84 (m, 2H).

LC-MS m/z (ESI) = 155.03 [M+1].

### Intermediate 15

### Tert-butyl-(S)-(1-((4,5,6,7-tetrahydropyrazolo[1,5-a]pyrazin-2-yl)methoxy)propan-2-yl)carbamate (Intermediate 15)

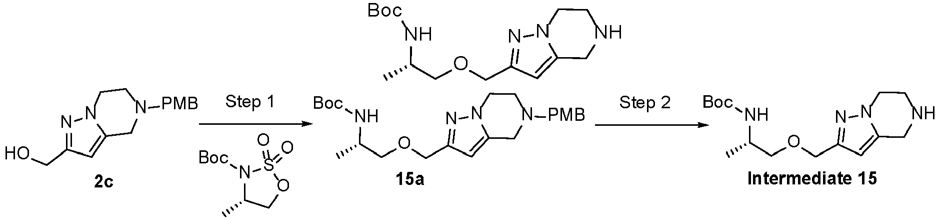

### Step 1:

### Tert-butyl (S)-(1-((5-(4-methoxybenzyl)-4,5,6,7-tetrahydropyrazolo[1,5-a]pyrazin-2-yl)methoxy)propan-2-yl)carbamate (15a)

**2c** (864 mg, 3.16 mmol, 1.0 equiv) was added to a 50 mL reaction flask and dissolved in anhydrous *N*,*N-*dimethylformamide (18 mL). Under nitrogen atmosphere, sodium hydride (300 mg, 7.51 mmol, 2.5 equiv) was added in portions at 0 °C, and after the addition was completed, the reaction mixture was stirred for another 30 min at this temperature. A solution of *tert*-butyl (*S*)-4-methyl-1,2,3-oxathiazolidine-3-carboxylate 2,2-dioxide in *N*,*N-*dimethylformamide (18 mL) was slowly added dropwise to the reaction system with the temperature maintained at 0 °C, and the reaction mixture was stirred for another 2 h. After the reaction was completed, the pH of the reaction system was adjusted to 6, a portion of *N*,*N-*dimethylformamide was removed by concentration *in vacuo,* and the reaction mixture was added with 40 mL of water and extracted with EA (3 × 120 mL). The organic layers were combined, dried over anhydrous sodium sulfate and concentrated *in vacuo,* and the reaction mixture was subjected to column chromatography (ethyl acetate:petroleum ether = 1:1) to obtain **15a** in the form of a yellow oil (544 mg, 40% yield).

LC-MS m/z (ESI) = 431.55 [M+1].

### Step 2:

### Tert-butyl-(S)-(1-((4,5,6,7-tetrahydropyrazolo[1,5-a]pyrazin-2-yl)methoxy)propan-2-yl)carbamate (Intermediate 15)

The synthesis method of **intermediate 11** was referred to obtain crude **intermediate 15** in the form of a brown-black oil (280 mg, 72% yield).

### Intermediate 16

### 5-Chloro-2-(4-methoxybenzyl)-3-oxo-2,3-dihydropyridazine-4-carbonitrile (Intermediate 16)

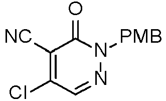

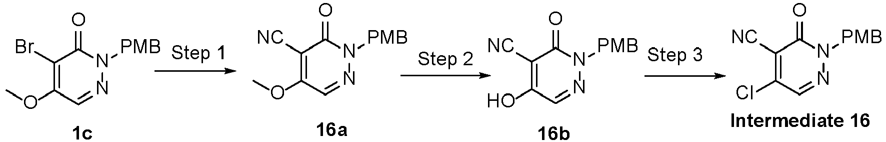

### Step 1:

### 5-Methoxy-2-(4-methoxybenzyl)-3-oxo-2,3-dihydropyridazine-4-carbonitrile (16a)

**1c** (650 mg, 2.0 mmol, 1.0 equiv) was weighed out and dissolved in anhydrous tetrahydrofuran (8 mL). The reaction mixture was added dropwise with isopropyl magnesium chloride (1 mL, 2.0 mmol, 1.0 equiv) in an ice water bath under nitrogen protection, reacted for 10 min, added with *p*-toluenesulfonyl cyanide (360 mg, 2.0 mmol, 1.0 equiv), and reacted for 2 h. The reaction was quenched with saturated aqueous ammonium chloride solution, and the reaction mixture was extracted with ethyl acetate. The organic phase was concentrated, and the crude product was subj ected to column chromatography (*n*-hexane: ethyl acetate = 1:1) to obtain **16a** in the form of a yellow oil (160 mg, 30% yield).

¹H NMR (400 MHz, DMSO-*d*₆): δ 8.35 (s, 1H), 7.25 (d, 2H), 6.89 (d, 2H), 5.18 (s, 2H), 4.15 (s, 3H), 3.72 (s, 3H).

LC-MS m/z (ESI) = 272.10 [M+1].

### Step 2:

### 5-Hydroxy-2-(4-methoxybenzyl)-3-oxo-2,3-dihydropyridazine-4-carbonitrile (16b)

The similar synthesis method of **1e** described above was referred to obtain **16b** in the form of a white solid (90 mg, 81% yield).

LC-MS m/z (ESI) = 258.10 [M+1].

### Step 3:

### 5-Chloro-2-(4-methoxybenzyl)-3-oxo-2,3-dihydropyridazine-4-carbonitrile (Intermediate 16)

The similar synthesis method of **intermediate 1** described above was referred to obtain **intermediate 16** in the form of a white solid (30 mg, 50% yield).

LC-MS m/z (ESI) = 276.10 [M+1].

### Intermediate 17

### (S)-1-((5-(5-(trifluoromethyl)pyrimidin-2-yl)-4,5,6,7-tetrahydropyrazolo[1,5-a]pyrazin-2-yl-3-d)methoxy)propan-2-amine (Intermediate 17)

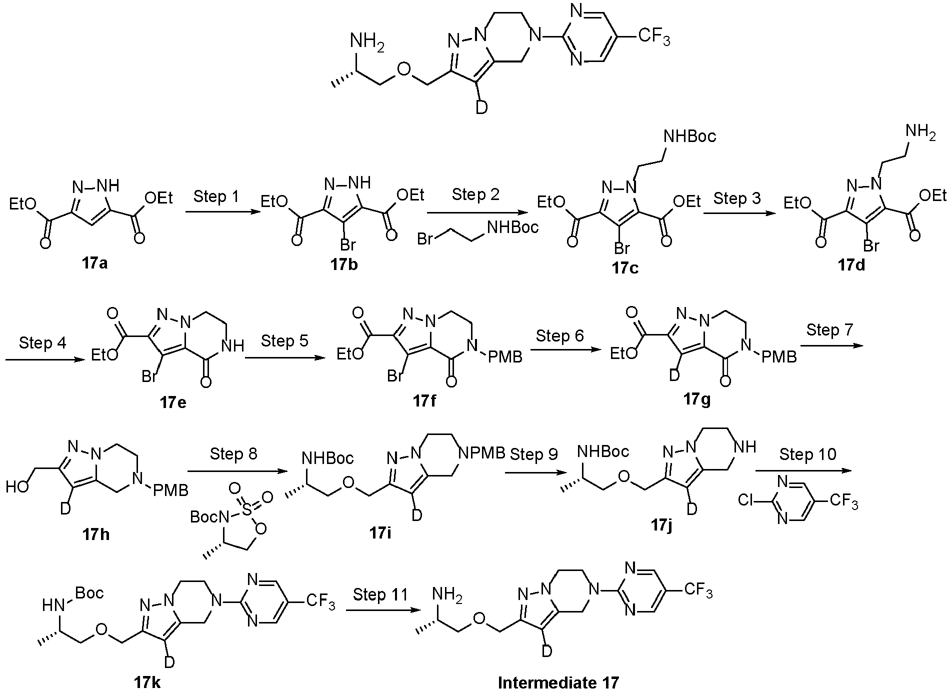

### Step 1:

### Diethyl-4-bromo-1H-pyrazole-3,5-dicarboxylate (17b)

**17a** (15.0 g, 70 mmol, 1.0 equiv) was added to a 500 mL single-necked flask at room temperature and dissolved in 150 mL of acetic acid, and the reaction mixture was added with N-bromosuccinimide (15.1 g, 84 mmol, 1.2 equiv), added with 15 mL of diluted nitric acid under an ice bath, and reacted at 120 °C for 2 h. After the reaction was completed, the organic layers were combined, and the reaction mixture was concentrated *in vacuo* and subjected to column chromatography (petroleum ether:ethyl acetate = 4:1) to obtain **17b** in the form of a white solid (17.6 g, 87% yield).

LC-MS m/z (ESI) = 289.99 [M+1].

### Step 2:

### 4-Bromo-1-(2-((tert-butoxycarbonyl)amino)ethyl)-1H-pyrazole-3,5-dicarboxylate (17c)

The similar synthesis method of **11c** described above was referred to obtain **17c** in the form of a white solid (7.2 g, 81% yield).

LC-MS m/z (ESI) = 433.08 [M+1].

### Step 3:

### 4-Bromo-1-(2-aminoethyl)-4-(trifluoromethyl)-1H-pyrazole-3,5-dicarboxylate (17d)

The similar synthesis method of **11d** described above was referred to obtain crude **17d** in the form of a white solid (6.4g, 97% yield).

LC-MS m/z (ESI) = 333.08 [M+1].

### Step 4:

### Ethyl-3-bromo-4-oxo-4,5,6,7-tetrahydropyrazolo[1,5-a]pyrazine-2-carboxylate (17e)

The similar synthesis method of **11e** described above was referred to obtain **17e** in the form of a white solid (2.6 g, 64% yield).

LC-MS m/z (ESI) = 286.99 [M+1].

### Step 5:

### Ethyl-3-bromo-5-(4-methoxybenzyl)-4-oxo-4,5,6,7-tetrahydropyrazolo[1,5-a]pyrazine-2-carboxylate (17f)

The similar synthesis method **of 2b** described above was referred to obtain **17f** in the form of a white solid (3.2 g, 78% yield).

LC-MS m/z (ESI) = 407.04 [M+1].

### Step 6:

### Ethyl-5-(4-methoxybenzyl)-4-oxo-4,5,6,7-tetrahydropyrazolo[1,5-a]pyrazine-2-carboxylate-3-d (17g)

**17f** (3 g, 7.4 mmol, 1.0equiv), deuterated sodium formate (2.4 g, 37 mmol, 5.0 equiv), tris(dibenzylideneacetone)dipalladium (703 mg, 0.74 mmol, 0.1 equiv) and *tri-tert-*butylphosphine (594.96 mg, 1.48 mmol, 0.2 equiv) were sequentially added into a 250 mL three-necked flask under nitrogen protection and dissolved in dimethyl sulfoxide (30 mL), and the reaction mixture was reacted at 85 °C and stirred for 5 h. The reaction mixture was cooled to room temperature, poured into water and extracted with ethyl acetate (50 mL × 3). The organic layers were combined, concentrated *in vacuo* and purified by column chromatography (petroleum ether:ethyl acetate = 2:1) to obtain **17g** in the form of a white solid (1.3 g, 53% yield).

LC-MS m/z (ESI) = 330.14 [M+1].

### Step 7:

### (5-(4-Methoxybenzyl)-4,5,6,7-tetrahydropyrazolo[1,5-a]pyrazin-2-yl-3-d)methanol (17h)

The similar synthesis method of **2c** described above was referred to obtain crude **17h** in the form of a pale yellow oil (1.02 g, 74% yield).

LC-MS m/z (ESI) = 274.15 [M+1].

### Step 8:

### Tert-butyl-(S)-(1-((5-(4-methoxybenzyl)-4,5,6,7-tetrahydropyrazolo[1,5-a]pyrazin-2-yl-3-d)methoxy)propan-2-yl)carbamate (17i)

The similar synthesis method of **13i** described above was referred to obtain **17i** in the form of a pale yellow oil (1.07 g, 56% yield).

LC-MS m/z (ESI) = 431.26 [M+1].

### Step 9:

### Tert-butyl-(S)-(1-((4,5,6,7-tetrahydropyrazolo[1,5-a]pyrazin-2-yl-3-d)methoxy)propan-2-yl)carbamate (17j)

The similar synthesis method of **intermediate 11** described above was referred to obtain crude **17j** in the form of a pale yellow oil (300 mg, 65% yield).

LCMS m/z = 311.14 [M+l].

### Step 10:

### Tert-butyl-(S)-(1-((5-(trifluoromethyl)pyrimidin-2-yl)-4,5,6,7-tetrahydropyrazolo[1,5-a]pyrazin-2-yl-3-d)methoxy)propan-2-yl)carbamate (17k)

The similar synthesis method of **13k** described above was referred to obtain crude **17k** in the form of a pale yellow oil (100 mg, 24% yield).

LCMS m/z = 457.22 [M+l].

### Step 11:

### (S)-1-((5-(5-(trifluoromethyl)pyrimidin-2-yl)-4,5,6,7-tetrahydropyrazolo[1,5-a]pyrazin-2-yl-3-d)methoxy)propan-2-amine (Intermediate 17)

The synthesis method of **intermediate 13** described above was referred to obtain **intermediate 17** in the form of a pale yellow oil (80 mg, 90% yield).

### Intermediate 18

### (S)-2-(4-methoxybenzyl)-5-((1-((4,5,6,7-tetrahydropyrazolo[1,5-a]pyrazin-2-yl)methoxy)propan-2-yl)amino)-4-(trifluoromethyl)pyridazin-3(2H)-one (Intermediate 18)

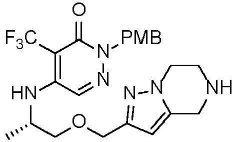

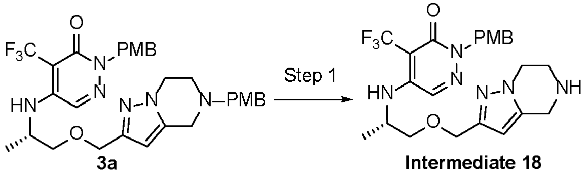

**3a** (8.0 g, 13 mmol, 1.0 equiv) was added to a 250 mL single-necked flask and dissolved with 60 mL of dichloroethane, and then the reaction system was added successively with 1-chloroethyl chloroformate (8 mL, 65 mmol, 5.0 equiv) and *N*,*N-*diisopropylethylamine (6 mL, 39 mmol, 3.0 equiv). After the addition was completed, the reaction mixture was reacted at room temperature for 3 h. The reaction mixture was concentrated *in vacuo* to remove the solvent, 60 mL of methanol was added to the residue, and the reaction mixture was refluxed for 2 h. The reaction mixture was concentrated *in vacuo* and subjected to column chromatography (petroleum ether:ethyl acetate = 1:1) to obtain **intermediate 18** in the form of a yellow solid (5.0 g, 78% yield).

¹H NMR (400 MHz, DMSO-*d*₆) δ 7.98 (s, 1H), 7.22 (d, 2H), 6.89 (d, 2H), 6.38 - 6.35 (m, 1H), 6.05 (s, 1H), 5.12 - 4.95 (m, 2H), 4.40 (d, 2H), 4.20 (s, 2H), 4.17 - 4.14 (m, 3H), 3.72 (d, 4H), 3.54 - 3.41 (m, 4H), 1.14 (d, 3H).

LCMS m/z = 493.10 [M+l].

### Intermediate 19

### (S)-4-((tert-butoxycarbonyl)amino)pentyl 4-methylbenzenesulfonate (Intermediate 19)

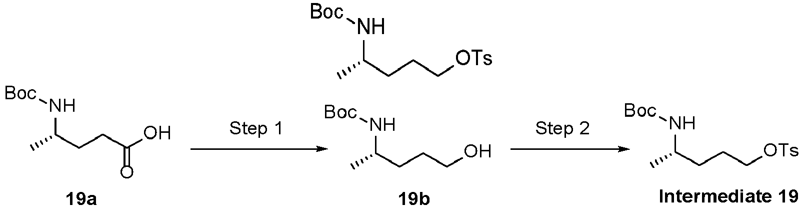

### Step 1

### Tert-butyl (S)-(5-hydroxypentan-2-yl)carbamate (19b)

**19a** (1.05 g, 5 mmol, 1.0 equiv) was added to a 50 mL three-necked reaction flask and dissolved in anhydrous tetrahydrofuran (20 mL), and lithium aluminum hydride (1 N, 10 mL) was added in portions to the reaction system under nitrogen protection in an ice bath. After the addition was completed, the reaction mixture was stirred at room temperature for 1 h. After the reaction was completed, the reaction mixture was concentrated *in vacuo* to obtain 19b in the form of a pale yellow oil (800 mg, 90% yield).

LC-MS m/z (ESI) = 204.15 [M+1].

### Step 2

### (S)-4-((tert-butoxycarbonyl)amino)pentyl 4-methylbenzenesulfonate (Intermediate 19)

**19b** (609 mg, 3 mmol, 1.0 equiv) was weighed into a 50 mL three-necked reaction flask and dissolved with anhydrous tetrahydrofuran (20 mL); sodium hydride (216 mg, 9 mmol, 3.0 equiv) was added in portions under nitrogen protection in an ice bath, and after the addition was completed, the reaction mixture was stirred at room temperature for 20 min. Subsequently, *p-*toluenesulfonyl chloride (801 mg, 4.5 mmol, 1.5 equiv) was added to the reaction system. The reaction mixture was stirred at room temperature for 3 h. After the reaction was completed, the reaction mixture was concentrated *in vacuo* and subjected to column chromatography (petroleum ether:ethyl acetate = 10: 1) to obtain **intermediate 19** in the form of a white oil (600 mg, 56% yield).

LC-MS m/z (ESI) = 358.16 [M+1].

### Intermediate 20

### (S)-5-(5-(5-(trifluoromethyl)pyrimidin-2-yl)-4,5,6,7-tetrahydro-2H-pyrazolo[4,3-c]pyridine-2-yl)pentan-2-amine (Intermediate 20)

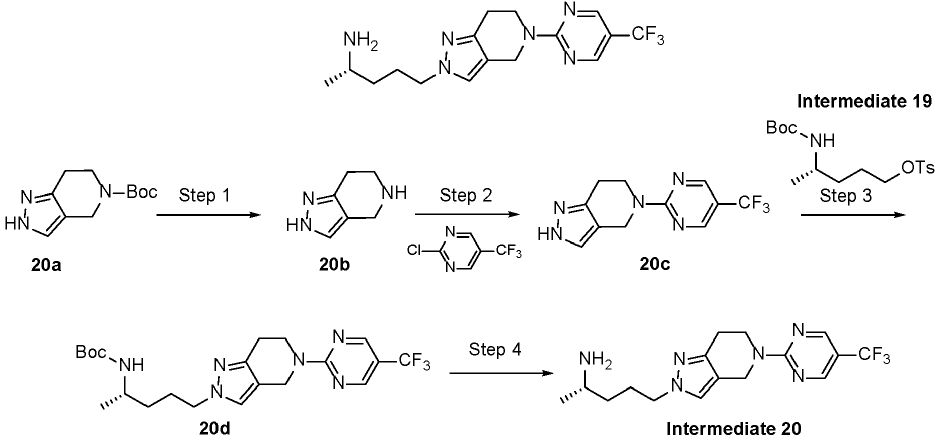

### Step 1:

### 4,5,6,7-Tetrahydro-2H-pyrazolo[4,3-c]pyridine (20b)

**20a** (1.1 g, 5 mmol, 1.0 equiv) was weighed into a 50 mL reaction flask; dioxane hydrochloride (4 N, 10 mL) was added to the reaction system under an ice bath, and after the addition was completed, the reaction mixture was stirred for 1 h. After the reaction was completed, the reaction mixture was concentrated *in vacuo* to obtain crude **20b** in the form of a white solid (1 g, 99% yield).

LC-MS m/z (ESI) = 124.08 [M+1].

### Step 2:

### 5-(5-(Trifluoromethyl)pyrimidin-2-yl)-4,5,6,7-tetrahydro-27/-pyrazolo[4,3-c]pyridine (20c)

The similar synthesis method of **13k** described above was referred to perform purification by preparative medium pressure liquid chromatography to obtain **20c** in the form of a white solid (800 mg, 75% yield).

¹H NMR (400 MHz, DMSO-*d*₆) δ 12.55 (s, 1H), 8.73 (s, 2H), 7.58 (s, 1H), 4.87 (s, 2H), 4.15 (t, 2H), 2.74 (s, 2H).

LC-MS m/z (ESI) = 269.09 [M+1].

### Step 3:

### Tert-butyl-(S)-(5-(5-(5-(trifluoromethyl)pyrimidin-2-yl)-4,5,6,7-tetrahydro-2H-pyrazolo [4,3-c]pyridin-2-yl)pentan-2-yl)carbamate (20d)

**20c** (124 mg, 0.46 mmol, 1.5 equiv) was weighed into a 50 mL reaction flask and dissolved with anhydrous *N*,*N-*dimethylacetamide (3 mL), followed by the addition of **intermediate 19** (110 mg, 0.31 mmol, 1.0 equiv) and cesium carbonate (293 mg, 0.93 mmol, 3.0 equiv) to the system successively. After the addition was completed, the reaction mixture was stirred at 80 °C for 1 h. When the reaction was completed, the reaction mixture was purified by preparative medium pressure liquid chromatography (water:acetonitrile = 75:25) to obtain **20d** in the form of a white solid (58 mg, 75% yield).

LC-MS m/z (ESI) = 455.23 [M+1].

### Step 4

### (S)-5-(5-(5-(trifluoromethyl)pyrimidin-2-yl)-4,5,6,7-tetrahydro-2H-pyrazolo[4,3-c]pyridin-2-yl)pentan-2-amine (Intermediate 20)

The similar synthesis method of **intermediate 13** was referred to obtain **intermediate 20** in the form of a white solid (48 mg, 99% yield).

LC-MS m/z (ESI) = 355.23 [M+1].

### Intermediate 21

### (2S)-1-((7-methyl-5-(5-(trifluoromethyl)pyrimidin-2-yl)-4,5,6,7-tetrahydropyrazolo[1,5-a]pyrazin-2-yl)methoxy)propan-2-amine (Intermediate 21)

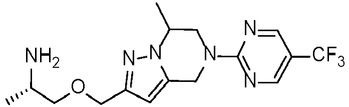

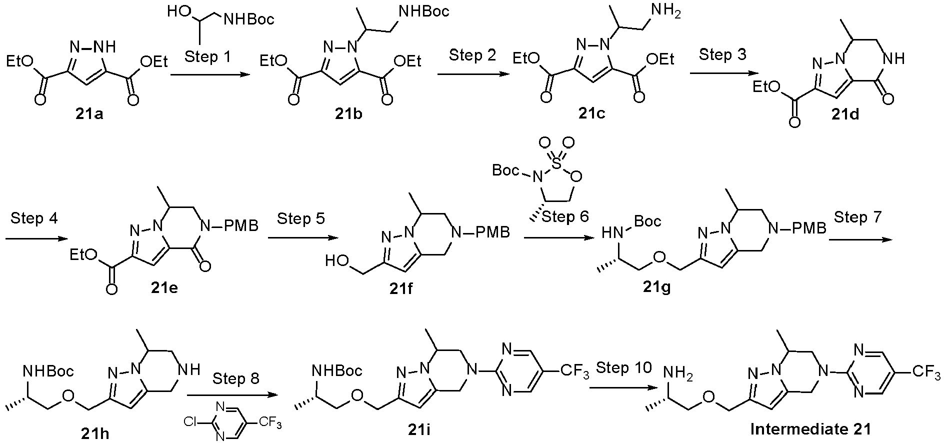

### Step 1:

### Diethyl 1-(1-((tert-butoxycarbonyl)amino)propan-2-yl)-1H-pyrazole-3,5-dicarboxylate (21b)

**21a** (1.06 g, 5 mmol, 1.0 equiv) was weighed into a 100 mL three-necked flask and dissolved by the addition of anhydrous tetrahydrofuran (20 mL); the reaction mixture was added with te*r*t-butyl(2-hydroxypropyl)carbamate (1.75 g, 10 mmol, 2.0 equiv), and after the addition was completed, the reaction mixture was added with di-*tert*-butyl azodicarboxylate (2.07 g, 9 mmol, 1.8 equiv), slowly added with triphenylphosphine (2.36 g, 9 mmol, 1.8 equiv), heated to 70 °C and reacted for 3 h. After the reaction was completed, the reaction mixture was extracted with ethyl acetate (50 mL), concentrated and subjected to column chromatography (PE:EA = 5:1) to obtain **21b** in the form of a white solid (2.2 g, 99% yield).

LC-MS m/z (ESI) = 369.19 [M+1].

### Step 2:

### Diethyl 1-(1-aminopropan-2-yl)-1H-pyrazole-3,5-dicarboxylate (21c)

The similar synthesis method of intermediate **11d** was referred to obtain **21c** in the form of a white solid (1.8 g, 99% yield).

LC-MS m/z (ESI) = 269.19 [M+1].

### Step 3:

### Ethyl 7-methyl-4-oxo-4,5,6,7-tetrahydropyrazolo[1,5-a]pyrazine-2-carboxylate (21d)

The similar synthesis method of intermediate **11e** was referred to obtain **21d** in the form of a white solid (820 mg, 79% yield).

LC-MS m/z (ESI) = 224.10 [M+1].

### Step 4:

### Ethyl 5-(4-methoxybenzyl)-7-methyl-4-oxo-4,5,6,7-tetrahydropyrazolo[1,5-a]pyrazine-2-carboxylate (21e)

The similar synthesis method of **2b** described above was referred to obtain **21e** in the form of a white solid (780 mg, 54% yield).

¹H NMR (400 MHz, DMSO-*d*₆) δ 7.52 (d, 2H), 6.89 (d, 2H), 6.15 (s, 1H), 4.82 (d, 2H), 4.50 - 4.37 (m, 4H), 3.66 - 3.53 (m, 1H), 3.57 (d, 3H), 1.45 (d, 3H), 1.32 (d, 3H).

LC-MS m/z (ESI) = 344.15 [M+1].

### Step 5:

### (5-(4-Methoxybenzyl)-7-methyl-4,5,6,7-tetrahydropyrazolo[1,5-a]pyrazin-2-yl)methanol (21f)

The synthesis method of **2c** described above was referred to obtain **21f** in the form of a white solid (510 mg, 65% yield).

¹H NMR (400 MHz, DMSO- *d₆*) δ 7.52 (d, 2H), 6.89 (d, 2H), 6.15 (s, 1H), 4.82 (d, 2H), 4.50 - 4.37 (m, 4H), 3.66 - 3.53 (m, 1H), 3.57 (d, 3H) 1.45 (d, 3H).

LC-MS m/z (ESI) = 307.11 [M+1].

### Step 6:

### Tert-butyl((2S)-1-((5-(4-methoxybenzyl)-7-methyl-4,5,6,7-tetrahydropyrazolo[1,5-a]pyrazin-2-yl)methoxy)propan-2-yl)carbamate (21g)

The similar synthesis method of intermediate **11h** was referred to obtain **21g** in the form of a white solid (380 mg, 67% yield).

¹H NMR (400 MHz, DMSO- *d₆*) δ 8.78 (s, 2H), 7.52 (d, 2H), 6.89 (d, 2H), 6.15 (s, 1H), 5.163- 4.99 (m, 2H), 4.50 - 4.37 (m, 4H), 4.10-4.01 (m, 2H), 3.66 - 3.53 (m, 1H),3.57 (d, 3H) 3.23 (q, 1H), 1.45 (d, 3H), 1.36 (s, 9H), 1.01 (d, 3H).

LC-MS m/z (ESI) = 444.27 [M+1].

### Step 7:

### Tert-butyl((2S)-1-((7-methyl-4,5,6,7-tetrahydropyrazolo[1,5-a]pyrazin-2-yl)methoxy)propan-2-yl)carbamate (21h)

The similar synthesis method of **13j** described above was referred to obtain crude **21h** in the form of a pale yellow oil (400 mg, 90% yield).

¹H NMR (400 MHz, DMSO-d6) δ.74 (s, 2H), 6.15 (s, 1H), 5.16 - 4.90 (m, 2H), 4.50 - 4.30 (m, 4H), 4.05-4.01 (m, 2H), 3.66 - 3.53 (m, 1H), 3.20 (q, 1H), 1.41 (d, 3H), 1.36 (s, 9H), 0.99 (d, 3H).

LCMS m/z = 457.16 [M+l].

### Step 8:

### Tert-butyl((2S)-1-((7-methyl-5-(5-(trifluoromethyl)pyrimidin-2-yl)-4,5,6,7-tetrahydro pyrazolo[1,5-a]pyrazin-2-yl)methoxy)propan-2-yl)carbamate (21i)

The synthesis method of **13k** described above was referred to obtain **21i** in the form of a pale yellow solid (250 mg, 63% yield).

LCMS m/z = 471.50 [M+l].

### Step 9:

### (2S)-1-((7-methyl-5-(5-(trifluoromethyl)pyrimidin-2-yl)-4,5,6,7-tetrahydropyrazolo[1,5-a]pyrazin-2-yl)methoxy)propan-2-amine (Intermediate 21)

The similar synthesis method of **11d** was referred to obtain **intermediate 21** in the form of a white solid (190 mg, 99% yield).

LC-MS m/z (ESI) = 357.16 [M+1].

### Intermediate 22

### (5-(5-(Trifluoromethyl)pyrimidin-2-yl)-4,5,6,7-tetrahydropyrazolo[1,5-a]pyrazin-2-yl)methanol (Intermediate 22)

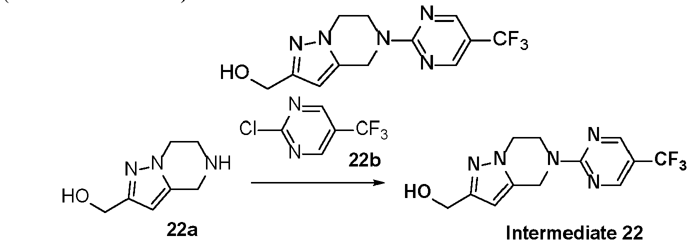

**22a** (1.5 g, 10 mmol, 1 equiv) was weighed out and dissolved in *N,N-*dimethylformamide (10 mL), and the reaction mixture was added with **22b** (1.8 g, 10 mmol, 1 equiv) and *N*,*N-*diisopropylethylamine (2.4 g, 20 mmol, 2 equiv) and reacted at 100 °C for 4 h; the reaction mixture was cooled and poured into ice water, a solid precipitated and filtered under vacuum, and the filter cake was dried to obtain **intermediate 22** in the form of a white solid (2.9 g, 99% yield).

LC-MS m/z (ESI) = 300.10[M+1].

### Intermediate 23

### (S)-1-((5-(5-(trifluoromethyl)pyrimidin-2-yl)-4,5,6,7-tetrahydropyrazolo[1,5-a]pyrazin-2-yl)methoxy)propan-1,1-d₂-2-amine (Intermediate 23)

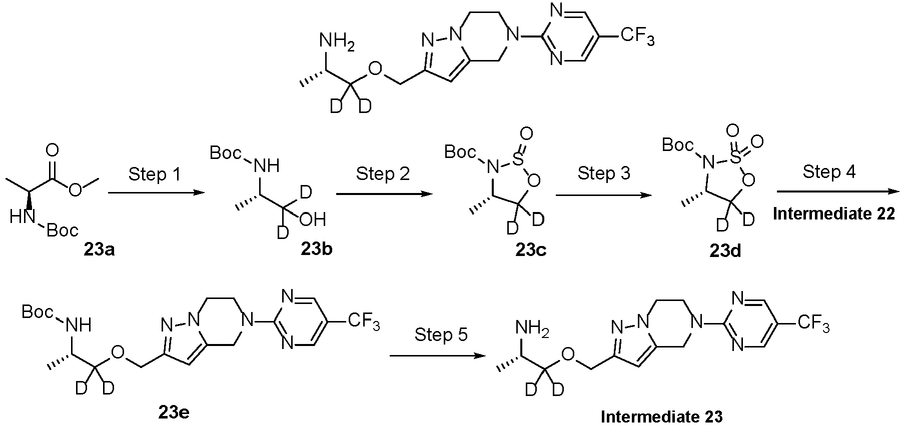

### Step 1:

### Tert-butyl (S)-(1-hydroxypropan-2-yl-1,1-d₂)carbamate (23b)

**23a** (2.5 g, 17 mmol, 1.0 equiv) was dissolved in anhydrous tetrahydrofuran (30 mL), and the reaction mixture was slowly added with deuterated lithium aluminum (880 mg, 21 mmol, 1.2 equiv) in portions under an ice bath under nitrogen protection; after the addition was completed, the reaction mixture was reacted for 1 h. After the reaction was completed, sodium sulfate decahydrate was added to quench the reaction, the reaction mixture was filtered under vacuum, the filter cake was washed with ethyl acetate and the filtrate was concentrated to obtain crude **23b** in the form of a yellow oil (2.0 g, 66% yield).

### Step 2:

### Tert-butyl (4S)-4-methyl-1,2,3-oxathiazolidine-3-carboxylate-5,5-d₂-oxide (23c)

Imidazole (2.0 g, 30 mmol, 6 equiv) was weighed out and dissolved in dichloromethane (15 mL), and reaction mixture was added dropwise with thionyl chloride (650 µL, 9 mmol, 1.8 equiv) in ice bath and reacted at room temperature for 2 h. The reaction mixture was placed in the ice bath, added dropwise with a solution of compound **23b** (890 mg, 5 mmol, 1.0 equiv) in dichloromethane (10 mL), and reacted at room temperature overnight. The reaction mixture was added with 10% citric acid to quench the reaction and extracted with dichloromethane; the organic phase was washed with saturated brine, dried over anhydrous sodium sulfate and concentrated to obtain crude **23c** in the form of a yellow oil (900 mg, 80% yield).

### Step 3:

### Tert-butyl (S)-4-methyl-1,2,3-oxathiazolidine-3-carboxylate-5,5-d₂ 2,2-dioxide (23d)

Compound **22c** (1.1 g, 5 mmol, 1 equiv) was weighed out and dissolved in acetonitrile (10 mL) and water (5 mL), and then the reaction mixture was added with sodium periodate (1.0 g, 5 mmol, 1 equiv) and ruthenium trichloride (55 mg, 0.25 mmol, 0.05equiv) successively and reacted at room temperature for 1 h. The reaction mixture was added with water (10 mL) and ethyl acetate (10 mL) and extracted, and the organic phase was concentrated to obtain the crude **23d** in the form of a brown oil (900 mg, 75% yield).

### Step 4:

### Tert-butyl (S)-(1-((5-(5-(trifluoromethyl)pyrimidin-2-yl)-4,5,6,7-tetrahydropyrazolo[1,5-a]pyrazin-2-yl)methoxy)propan-2-yl-1,1-d₂)carbamate (23e)

The similar synthesis method of intermediate **11h** was referred to obtain **23e** in the form of a white solid (480 mg, 47% yield).

LC-MS m/z (ESI) = 459.10 [M+1].

### Step 5:

### (S)-1-((5-(5-(trifluoromethyl)pyrimidin-2-yl)-4,5,6,7-tetrahydropyrazolo[1,5-a]pyrazin-2-yl)methoxy)propan-1,1-d₂-2-amine (Intermediate 23)

The similar synthesis method of intermediate **11d** was referred to obtain **intermediate 23** in the form of a white solid (190 mg, 99% yield).

LC-MS m/z (ESI) = 359.10 [M+1].

### Intermediate 24

### (S)-1-((7-(5-(trifluoromethyl)pyridin-2-yl)-5,6,7,8-tetrahydro-[1,2,4]triazolo[1,5-a]pyrazin-2-yl)methoxy)propan-1,1-d₂-2-amine (Intermediate 24)

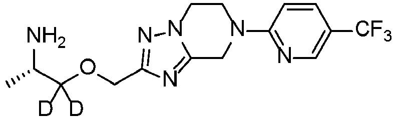

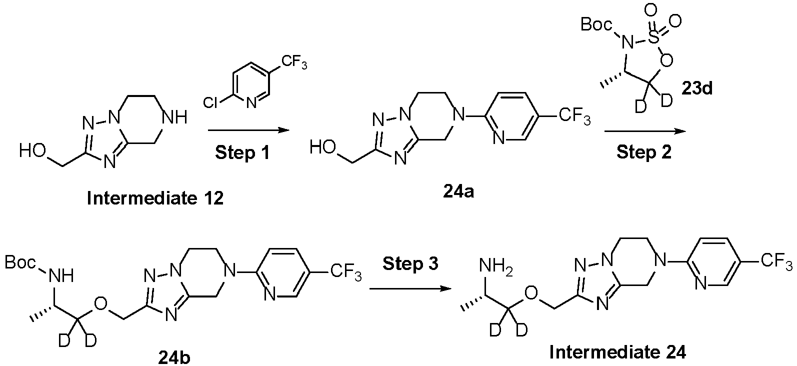

### Step 1:

### (7-(5-(Trifluoromethyl)pyridin-2-yl)-5,6,7,8-tetrahydro-[1,2,4]triazolo[1,5-a]pyrazin-2-yl)methanol (24a)

The similar synthesis method of **intermediate 22** was referred to obtain **24a** in the form of a white solid (443 mg, 86% yield).

LC-MS m/z (ESI) = 301.22 [M+1].

### Step 2:

### Tert-butyl (S)-(1-((7-(5-(trifluoromethyl)pyridin-2-yl)-5,6,7,8-tetrahydro-[1,2,4]triazolo[1,5-a]pyrazin-2-yl)methoxy)propan-2-yl-1,1-d₂)carbamate (24b)

The similar synthesis method of **intermediate 11h** was referred to obtain **24b** in the form of a white solid (360 mg, 52% yield).

LC-MS m/z (ESI) = 460.11 [M+1].

### Step 3:

### (S)-1-((7-(5-(trifluoromethyl)pyridin-2-yl)-5,6,7,8-tetrahydro-[1,2,4]triazolo[1,5-a]pyrazin-2-yl)methoxy)propan-1,1-d₂-2-amine (Intermediate 24)

The similar synthesis method of **intermediate 11d** was referred to obtain **intermediate 24** in the form of a white solid (260 mg, 78% yield).

LC-MS m/z (ESI) = 360.12 [M+1].

### Example 1

### (S)-4-(trifluoromethyl)-5-((1-((5-(5-(5-(trifluoromethyl)pyrimidin-2-yl)-4,5,6,7-tetrahydro pyrazolo[1,5-a]pyrazin-2-yl)methoxy)propan-2-yl)amino)pyridazin-3(2H)-one (Compound 1)

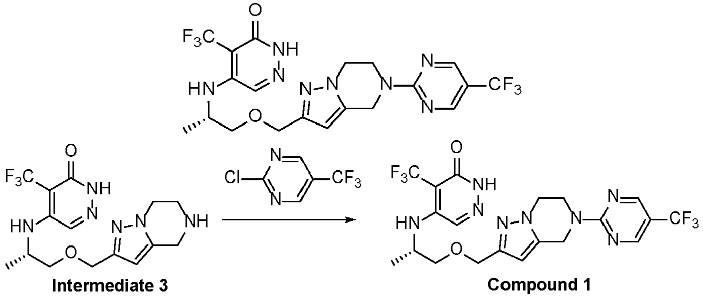

**Intermediate 3** (48 mg, 0.13 mmol, 1.0 equiv) and 2-chloro-5-trifluoromethylpyrimidine (24 mg, 0.13 mmol, 1.0 equiv) were weighed into a 10 mL reaction flask and dissolved in *N,N-*dimethylformamide (4.0 mL). The reaction mixture was added with *N*,*N*-diisopropylethylamine (0.086 mL, 0.52 mmol, 4 equiv), reacted at 90 °C and stirred for 1 h. After the reaction was completed, the reaction mixture was concentrated *in vacuo.* The residue was purified by MPLC (water/acetonitrile = 1:1) to obtain **compound 1** in the form of a white solid (40 mg, 59% yield).

¹H NMR (400 MHz, DMSO-*d*₆): δ 12.46 (s, 1H), 8.81 (s, 2H), 7.90 (s, 1H), 6.28 (dd, 1H), 6.11 (s, 1H), 5.01 (s, 2H), 4.41 (d, 2H), 4.32 (t, 2H), 4.16 (t, 3H), 3.55 - 3.45 (m, 2H), 1.15 (d, 3H).

LCMS m/z = 519.40 [M+l].

### Example 2

### (S)-4-(trifluoromethyl)-5-((1-((5-(5-(trifluoromethyl)pyrimidin-2-yl)-4,5,6,7-tetrahydropyrazolo[1,5-a]pyrazin-2-yl-4,4-d₂)methoxy-d₂)propan-2-yl)amino)pyridazin-3(2H)-one (Compound 2)

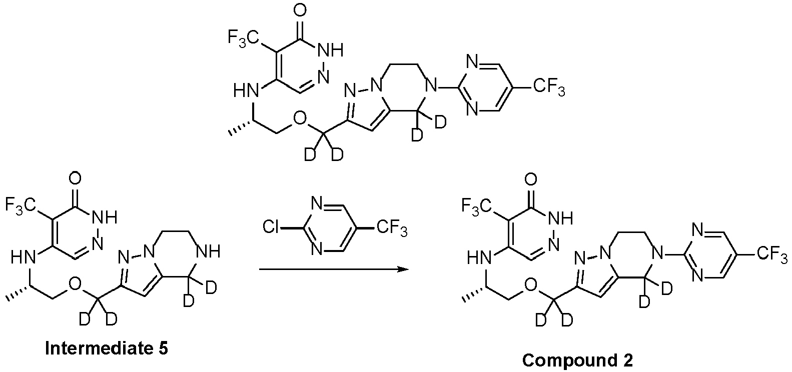

The similar synthesis method of **compound 1** was referred to obtain **compound 2** in the form of a white solid (500 mg, 72% yield).

¹H NMR (400 MHz, DMSO-*d*₆): δ 12.45 (s, 1H), 8.81 (s, 2H), 7.90 (s, 1H), 6.27 (dd, 1H), 6.11 (s, 1H), 4.32 (t, 2H), 4.17 - 4.14 (m, 3H), 3.56 - 3.41 (m, 2H), 1.15 (d, 3H).

LCMS m/z = 523.10 [M+l].

### Example 3

### 4-(Trifluoromethyl)-5-(((S)-1-((S)-1-(5-(5-(trifluoromethyl)pyrimidin-2-yl)-4,5,6,7-tetrahydropyrazolo[1,5-a]pyrazin-2-yl)ethoxy)propan-2-yl)amino)pyridazin-3(2H)-one (compound 3-1)

### 4-(Trifluoromethyl)-5-(((S)-1-((R)-1-(5-(5-(trifluoromethyl)pyrimidin-2-yl)-4,5,6,7-tetrahydropyrazolo[1,5-a]pyrazin-2-yl)ethoxy)propan-2-yl)amino)pyridazin-3(2H)-one (compound 3-II)

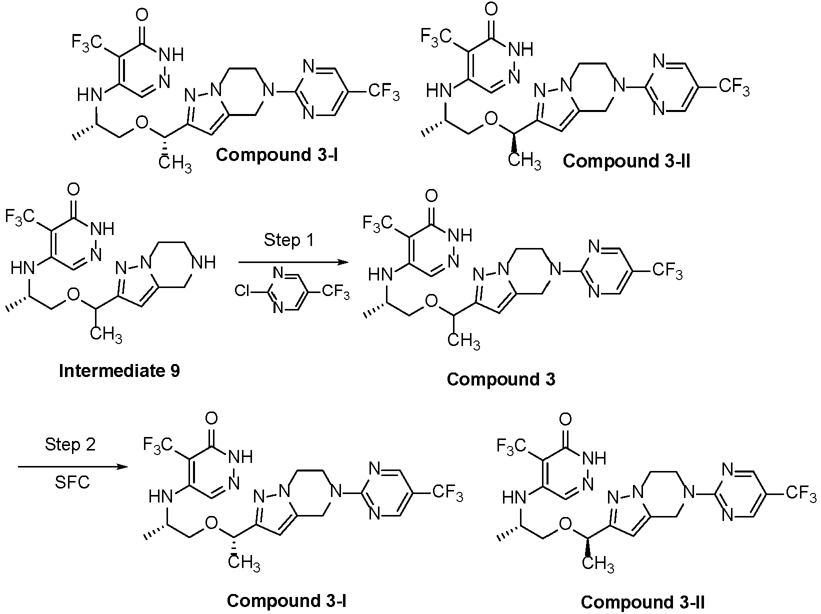

**Intermediate 9** (100 mg, 0.26 mmol, 1.0 equiv) and 2-chloro-5-trifluoromethylpyrimidine (24 mg, 0.26 mmol, 1.0 equiv) were weighed into a 10 mL reaction flask and dissolved with *N,N-*dimethylformamide (5.0 mL). The reaction mixture was added with *N,N-*diisopropylethylamine (0.18 mL, 1.04 mmol, 4 equiv), reacted at 90 °C and stirred for 1 h. After the reaction was completed, the reaction mixture was concentrated *in vacuo.* The residue was purified by C18 reverse phase chromatography (water/acetonitrile = 1: 1) to obtain **compound 3** in the form of a white solid (80 mg, 59% yield).

LCMS m/z = 532.45 [M+l].

**Compound 3** (80 mg) was resolved by SFC to obtain **compound 3-1** (38 mg, 47.2% yield, RT = 7.684 min, 100% ee) and **compound 3-II** (35 mg, 46.5% yield, RT = 12.365 min, 100% ee). Chiral HPLC (AS) mobile phase: n-hexane/ethanol = 90/10; column temperature: 35 °C; column pressure: 80 bar; flow rate: 1 mL/min; detector signal channel: 215 nm@4.8 nm; start/stop wavelength of diode array detector: 200-400 nm.

**Compound 3-1:** ¹H NMR (400 MHz, DMSO-*d*₆) δ 8.80 (s, 2H), 7.87 (s, 1H), 6.23 (dd, 1H), 6.05 (s, 1H), 5.00 (s, 2H), 4.45 (dd, 1H), 4.36-4.26 (m, 2H), 4.15-4.09 (m, 2H), 3.33-3.18 (m, 2H), 1.33 (d, 3H), 1.12 (d, 3H).

**Compounds 3-II:** ¹H NMR (400 MHz, DMSO-*d*₆) δ 8.79 (s, 2H), 7.86 (s, 1H), 6.25 (dd, 1H), 6.10 (s, 1H), 5.08 (s, 2H), 4.43 (dd, 1H), 4.31-4.24 (m, 2H), 4.14-4.06 (m, 2H), 3.39 - 3.24(m, 2H), 1.31 (d, 3H), 1.13 (d, 3H).

### Example 4

### (S)-6-(2-((2-((6-oxo-5-(trifluoromethyl)-1,6-dihydropyridazin-4-yl)amino)propoxy) methyl)-6,7-dihydropyrazolo[1,5-a]pyrazin-5(4H-yl)nicotinonitrile (Compound 4)

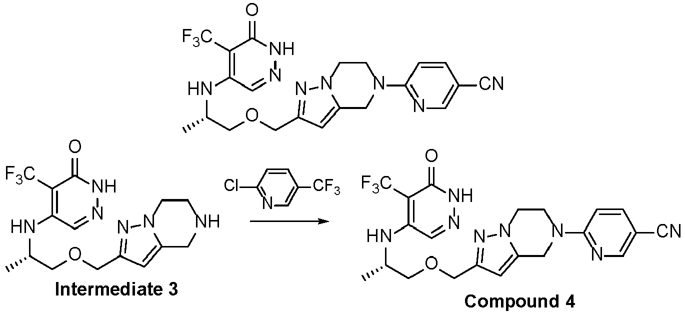

The similar synthesis method of **compound 1** was referred to obtain **compound 4** in the form of a white solid (20 mg, 33% yield).

¹H NMR (400 MHz, DMSO- *d₆*): δ 12.46 (s, 1H), 8.57 (d, 1H), 7.97 (m, 1H), 7.90 (s, 1H), 7.10 (d, 1H), 6.28 (dd, 1H), 6.08 (s, 1H), 4.87 (s, 2H), 4.41 (d, 2H), 4.15 (s, 4H), 3.49 (d, 2H), 1.23 (s, 1H), 1.15 (d, 3H).

LCMS m/z = 475.45 [M+l].

### Example 5

### (S)-4-(trifluoromethyl)-5-((1-((5-(5-(trifluoromethyl)pyrazin-2-yl)-4,5,6,7-tetrahydropyrazolo[1,5-a]pyrazin-2-yl)methoxy)propan-2-yl)amino)pyridazin-3(2H)-one (Compound 5)

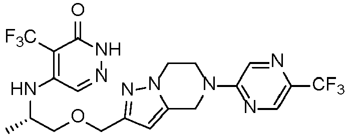

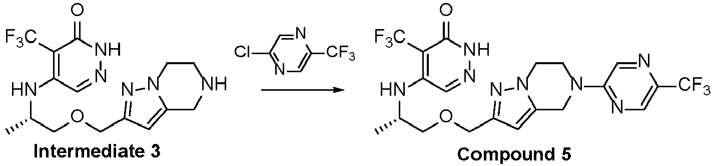

The similar synthesis method of **compound 1** was referred to obtain **compound 5** in the form of a white solid (25 mg, 36% yield).

¹H NMR (400 MHz, DMSO-*d*₆) δ 12.46 (s, 1H), 8.57 (s, 2H), 7.90 (s, 1H), 6.28 (s, 1H), 6.10 (s, 1H), 4.94 (s, 2H), 4.48 - 4.32 (d, 2H), 4.27 - 4.18 (m, 4H), 4.16 (m, 1H), 3.53 - 3.46 (m, 2H), 1.15 (d, 3H).

LCMS m/z = 519.42 [M+l].

### Example 6

### (S)-2-((2-((6-oxo-5-(trifluoromethyl)-1,6-dihydropyridazin-4-yl)amino)propoxy)methyl)-5-(5-(trifluoromethyl)pyrimidin-2-yl)-6,7-dihydropyrazolo[1,5-a]pyrazin-4(5H)-one (Compound 6)

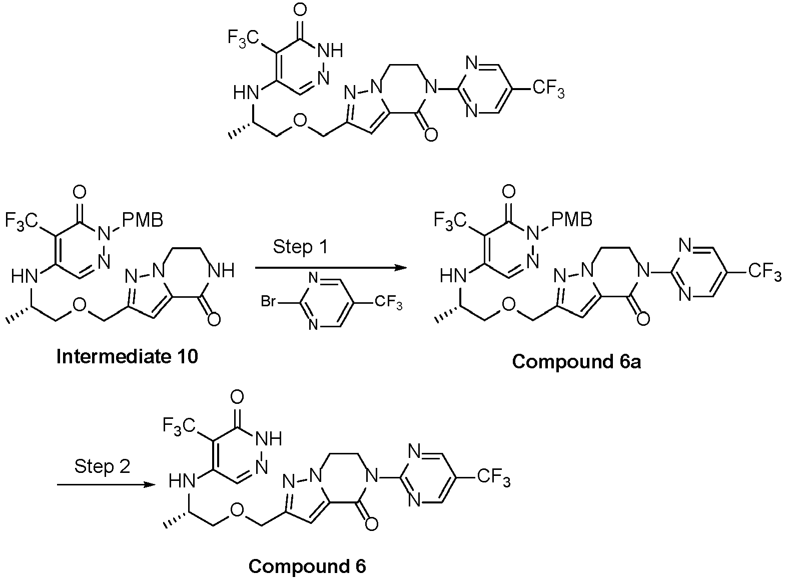

### Step 1:

### (S)-2-((2-((1-(4-methoxybenzyl)-6-oxo-5-(trifluoromethyl)-1,6-dihydropyridazin-4-yl)amino)propoxy)methyl)-5-(5-(trifluoromethyl)pyrimidin-2-yl)-6,7-dihydropyrazolo[1,5-a]pyrazin-4(5H)-one (Compound 6a)

**Intermediate 10** (200 mg, 0.4 mmol, 1.0 equiv) was weighed out and dissolved in dioxane (5.0 mL); the reaction mixture was added with potassium phosphate (255 mg, 1.2 mmol, 3.0 equiv), 2-bromo-5-trifluoromethylpyrimidine (136 mg, 0.6 mmol, 1.5 equiv), cuprous iodide (12 mg, 0.06 mmol, 0.15 equiv), trans-(1*R*,2*R*)-*N*,*N*-dimethyl-1,2-cyclohexanediamine (20 µL, 0.12 mmol, 0.3 equiv) successively under nitrogen, and reacted at 110 °C for 24 h. The reaction mixture was concentrated *in vacuo* and the residue was purified by MPLC (water/acetonitrile = 1: 1) to obtain **compound 6a** in the form of a white solid (20 mg, 7% yield).

LCMS m/z = 653.10 [M+l].

### Step 2:

### (S)-2-((2-((6-oxo-5-(trifluoromethyl)-1,6-dihydropyridazin-4-yl)amino)propoxy)methyl)-5-(5-(trifluoromethyl)pyrimidin-2-yl)-6,7-dihydropyrazolo[1,5-a]pyrazin-4(5H)-one (Compound 6)

To a 25 mL reaction flask with **compound 6a** weighed out (20 mg, 0.03 mmol, 1.0 equiv) was added trifluoroacetic acid (2 mL) and trifluoromethanesulfonic acid (0.2 mL, 0.24 mmol, 8.0 equiv) successively. After the addition was completed, the reaction mixture was stirred at room temperature for 1 h. After the reaction was completed, 5 mL of water was added to the reaction mixture to quench the reaction. The resulting solution was extracted with ethyl acetate (3 × 15 mL). The pH of the organic layer was adjusted to 8 to 9 by an aqueous potassium carbonate solution. The organic layers were combined and concentrated *in vacuo,* and the residue was purified by MPLC (water/acetonitrile = 4:6) to obtain **compound 6** in the form of a white solid (8 mg, 50% yield).

¹H NMR (400 MHz, DMSO-*d*₆): δ 12.46 (s, 1H), 9.28 (d, 2H), 7.93 (s, 1H), 6.91 (s, 1H), 6.33-6.31 (m, 1H), 4.52-4.50 (m, 6H), 4.25-4.15 (m, 1H), 3.55 (d, 2H), 1.17 (d, 3H).

LC-MS m/z (ESI) = 533.10 [M+1].

### Example 7

### (S)-2-(2-((2-((6-oxo-5-(trifluoromethyl)-1,6-dihydropyridazin-4-yl)amino)propoxy)methyl)-6,7-dihydropyrazolo[1,5-a]pyrazin-5(4H)-yl)pyrimidine-5-carbonitrile (Compound 7)

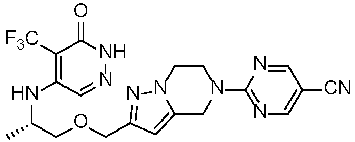

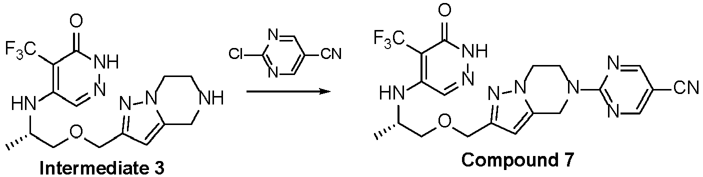

The similar synthesis method of **compound 1** was referred to obtain **compound 7** in the form of a white solid (30 mg, 48% yield).

¹H NMR (400 MHz, DMSO-*d*₆): δ 12.45 (s, 1H), 8.87 (s, 2H), 7.90 (s, 1H), 6.27 (d, 1H), 6.11 (s, 1H), 5.01 (s, 2H), 4.41 (d, 1H), 4.32 (d, 2H), 4.16 (t, 2H), 4.03 (q, 2H), 3.49 (d, 2H), 1.16 (d, 3H).

LCMS m/z = 476.44 [M+l].

### Example 8

### (S)-5-((1-((5-(5-methylpyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 8)

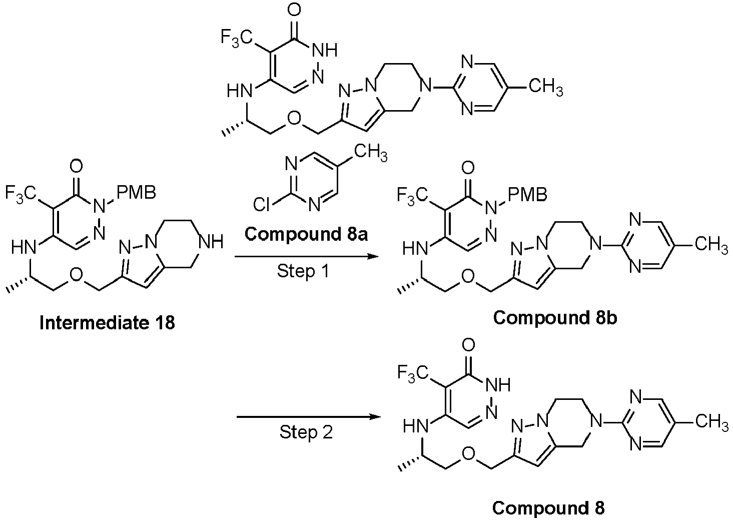

### Step 1:

### (S)-2-(4-methoxybenzyl)-4-(trifluoromethyl)-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 8b)

The similar synthesis method of **compound 1** was referred to obtain **compound 8b** in the form of a yellow solid (68 mg, 29% yield).

LCMS m/z (ESI) = 585.6 [M+l].

### Step 2:

### (S)-5-((1-((5-(5-methylpyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 8)

The similar synthesis method in the second step of the preparative route of **compound 6** was referred to obtain **compound 8 in** the form of a white solid (21 mg, 39% yield).

¹H NMR (400 MHz, DMSO-*d*₆) δ 12.46 (s, 1H), 8.31 (s, 2H), 7.90 (s, 1H), 6.28 (dd, 1H), 6.06 (s, 1H), 4.87 (s, 2H), 4.55 - 4.32 (m, 2H), 4.20 (dd, 3H), 4.09 (t, 2H), 3.48 (d, 2H), 2.11 (s, 3H), 1.15 (d, 4H).

¹⁹F NMR (377 MHz, DMSO-*d*₆) δ -55.43 (s, 3H).

LCMS m/z (ESI) = 465.4 [M+l].

### Example 9

### (S)-4-(trifluoromethyl)-5-((1-((7-(5-(trifluoromethyl)pyrimidin-2-yl)-5,6,7,8-tetrahydro-[1,2,4]triazolo[1,5-a]pyrazin-2-yl)methoxy)propan-2-yl)amino)pyridazin-3(2H)-one (Compound 9)

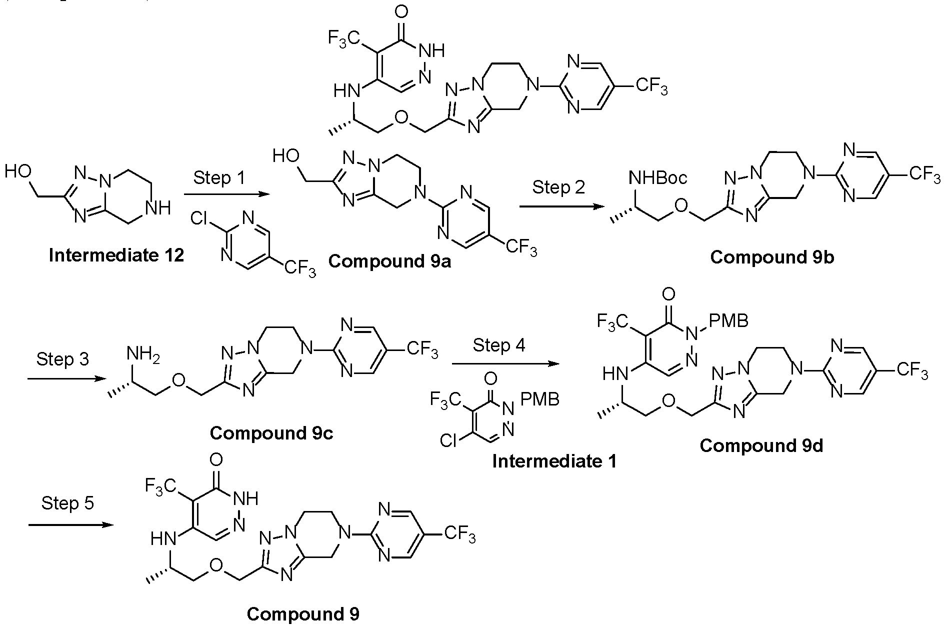

### Step 1:

### (7-(5-(Trifluoromethyl)pyrimidin-2-yl)-5,6,7,8-tetrahydro-[1,2,4]triazolo[1,5-a]pyrazin-2-yl)methanol (Compound 9a)

**Intermediate 12** (186 mg, 1.2 mmol, 1.0 equiv) was weighed out and dissolved in *N,N-*dimethylformamide (4 mL), and the reaction mixture was added with 2-chloro-5-(trifluoromethyl) pyrimidine (214 mg, 1.2 mmol, 1.0 equiv) and *N*,*N-*diisopropylethylamine (1.0 mL, 6.0 mmol, 5.0 equiv) and reacted at 100 °C for 2 h. After the reaction was completed, the reaction mixture was concentrated *in vacuo.* The residue was subjected to C18 reverse phase chromatography (water/acetonitrile = 1: 1) to obtain **compound 9a** in the form of a white solid (340 mg, 94% yield).

¹H NMR (400 MHz, DMSO-*d*₆): δ 8.92 - 8.70 (m, 2H), 5.07 (s, 2H), 4.45 - 4.32 (m, 4H), 4.23 (t, 2H).

LC-MS m/z (ESI) = 301.10 [M+1].

### Step 2:

### Tert-butyl-(S)-(1-((7-(5-(trifluoromethyl)pyrimidin-2-yl)-5,6,7,8-tetrahydro-[1,2,4]triazolo[1,5-a]pyrazin-2-yl)methoxy)propan-2-yl)carbamate (Compound 9b)

The similar synthesis method of **11h** was referred to obtain **compound 9b** in the form of a yellow oil (200 mg, 60% yield).

LC-MS m/z (ESI) = 458.10 [M+1].

### Step 3:

### (S)-1-((7-(5-(trifluoromethyl)pyrimidin-2-yl)-5,6,7,8-tetrahydro-[1,2,4]triazolo[1,5-a]pyrazin-2-yl)methoxy)propan-2-amine (Compound 9c)

The similar synthesis method of **11i** was referred to obtain **compound 9c** in the form of a white solid (50 mg, 90% yield), and the crude product was used directly in the next step.

LC-MS m/z (ESI) = 358.10 [M+1].

### Step 4:

### (S)-2-(4-methoxybenzyl)-4-(trifluoromethyl)-5-((1-((7-(5-(trifluoromethyl)pyrimidin-2-yl)-5,6,7,8-tetrahydro-[1,2,4]triazolo[1,5-a]pyrazin-2-yl)methoxy)propan-2-yl)amino)pyridazin-3(2H)-one (Compound 9d)

The similar synthesis **of 3a** was referred to obtain **compound 9d** in the form of a yellow oil (250 mg, 78% yield).

LC-MS m/z (ESI) = 640.10 [M+1].

### Step 5:

### (S)-4-(trifluoromethyl)-5-((1-((7-(5-(trifluoromethyl)pyrimidin-2-yl)-5,6,7,8-tetrahydro-[1,2,4]triazolo[1,5-a]pyrazin-2-yl)methoxy)propan-2-yl)amino)pyridazin-3(2H)-one (Compound 9)

The similar synthesis method of **compound 6** was referred to obtain **compound 9** in the form of a white solid (50 mg, 60% yield).

¹H NMR (400 MHz, Chloroform-*d*): δ 11.45 (s, 1H), 8.76 - 8.38 (m, 2H), 7.69 (s, 1H), 5.87 (d, 1H), 5.19 (s, 2H), 4.64 (d, 2H), 4.47 (t, 2H), 4.30 (t, 2H), 4.04 - 3.97 (m, 1H), 3.75 - 3.61 (m, 2H), 1.33 (d, 3H).

LC-MS m/z (ESI) = 520.10 [M+1].

### Example 10

### (S)-4-(trifluoromethyl)-5-((1-((7-(5-(trifluoromethyl)pyrazin-2-yl)-5,6,7,8-tetrahydro-[1,2,4]triazolo[1,5-a]pyrazin-2-yl)methoxy)propan-2-yl)amino)pyridazin-3(2H)-one (Compound 10)

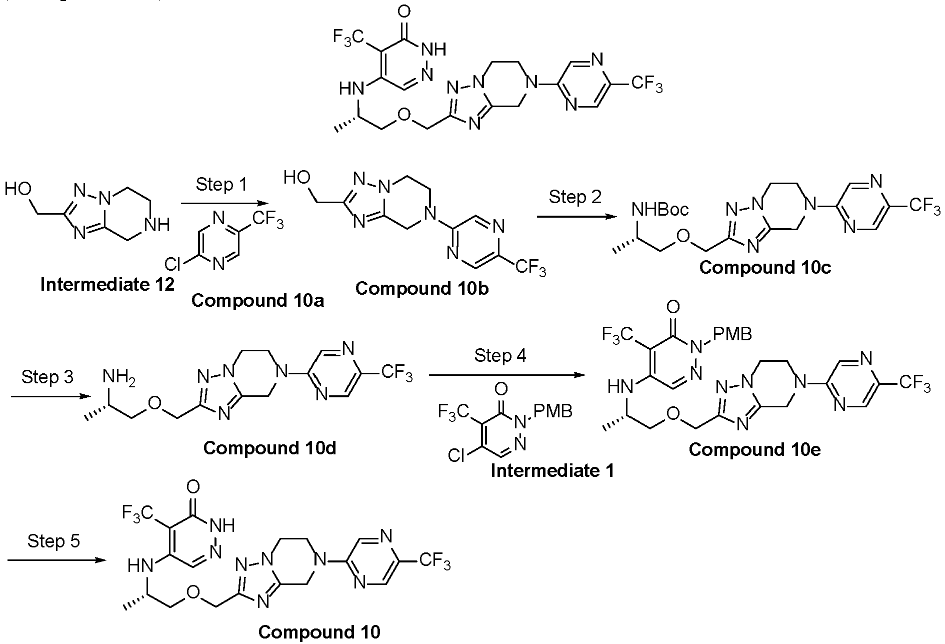

### Step 1:

### (7-(5-(Trifluoromethyl)pyrazin-2-yl)-5,6,7,8-tetrahydro-[1,2,4]triazolo[1,5-a]pyrazin-2-yl)methanol (Compound 10b)

The similar synthesis method of **compound 9a** was referred to obtain **compound 10b** in the form of a white solid (200 mg, 90% yield).

LC-MS m/z (ESI) = 301.10 [M+1].

### Step 2:

### Tert-butyl (S)-(1-((7-(5-(trifluoromethyl)pyrazin-2-yl)-5,6,7,8-tetrahydro-[1,2,4]triazolo [1,5-a]pyrazin-2-yl)methoxy)propan-2-yl)carbamate (Compound 10c)

The similar synthesis method of **intermediate 11h** was referred to obtain **compound 10c** in the form of a yellow oil (170 mg, 80% yield).

LC-MS m/z (ESI) = 458.10 [M+1].

### Step 3:

### (S)-1-((7-(5-(trifluoromethyl)pyrazin-2-yl)-5,6,7,8-tetrahydro-[1,2,4]triazolo[1,5-a]pyrazin-2-yl)methoxy)propan-2-amine (Compound 10d)

The similar synthesis method of **intermediate 11i** was referred to obtain **compound 10d** in the form of a white solid (120 mg, 97% yield), and the crude product was used directly in the next step.

LC-MS m/z (ESI) = 358.10 [M+1].

### Step 4:

### (S)-2-(4-methoxybenzyl)-4-(trifluoromethyl)-5-((1-((7-(5-(trifluoromethyl)pyrimidin-2-yl)-5,6,7,8-tetrahydro-[1,2,4]triazolo[1,5-a]pyrazin-2-yl)methoxy)propan-2-yl)amino) pyridazin-3(2H)-one (Compound 10e)

The method of **3a** was referred to synthesize **compound 10e** in the form of a yellow oil (180 mg, 76% yield).

¹H NMR (400 MHz, Chloroform-*d*): δ 8.48 (d, 1H), 8.25 (d, 1H), 7.62 (s, 1H), 7.38 (d, 2H), 6.84 (d, 2H), δ 5.77-5.70 (m, 1H), 5.13 (d, 2H), 4.93 (s, 2H), 4.62 (d, 2H), 4.27 (s, 3H), 3.92 - 3.87 (m, 1H), 3.80-3.77 (m, 4H), 3.73 - 3.70 (m, 1H), 3.64 - 3.60 (m, 1H), 1.30 (d, 3H).

LC-MS m/z (ESI) = 640.10 [M+1].

### Step 5:

### (S)-4-(trifluoromethyl)-5-((1-((7-(5-(trifluoromethyl)pyrazin-2-yl)-5,6,7,8-tetrahydro-[1,2,4]triazolo[1,5-a]pyrazin-2-yl)methoxy)propan-2-yl)amino)pyridazin-3(2H)-one (Compound 10)

The similar synthesis method of **compound 6** was referred to obtain **compound 10** in the form of a white solid (38 mg, 62% yield).

¹H NMR (400 MHz, Chloroform-*d*): δ 11.07 (s, 1H), 8.48 (s, 1H), 8.32 (s, 1H), 7.69 (s, 1H), 5.82 (s, 1H), 4.99 (d, 2H), 4.76 - 4.56 (m, 2H), 4.52 - 4.25 (m, 4H), 3.98 (s, 1H), 3.78-3.62 (m, 2H), 1.33 (d, 3H).

LC-MS m/z (ESI) = 520.10 [M+1].

### Example 11

### (S)-2-((2-((6-oxo-5-(trifluoromethyl)-1,6-dihydropyridazin-4-yl)amino)propoxy) methyl)-5-(5-(trifluoromethyl)pyridin-2-yl)-6,7-dihydropyrazolo[1,5-a]pyrazin-4(5H)-one (Compound 11)

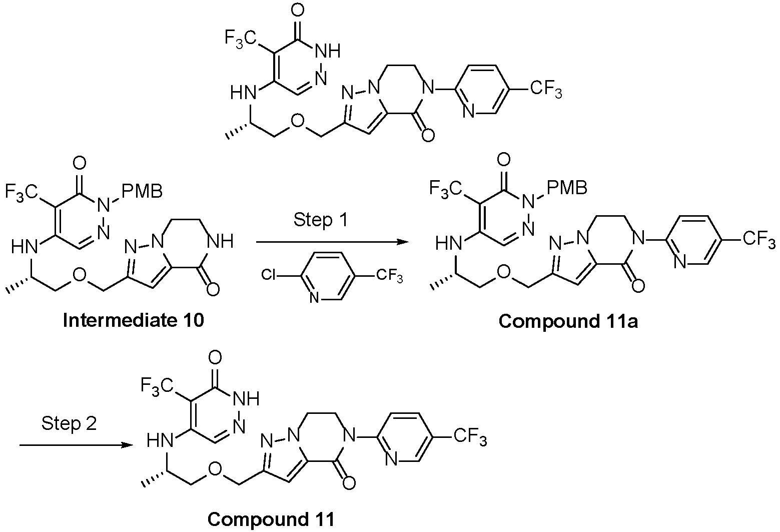

### Step 1:

### (S)-2-((2-((1-(4-methoxybenzyl)-6-oxo-5-(trifluoromethyl)-1,6-dihydropyridazin-4-yl)amino)propoxy)methyl)-5-(5-(trifluoromethyl)pyridin-2-yl)-6,7-dihydropyrazolo[1,5-a]pyrazin-4(5H)-one (Compound 11a)

The similar synthesis method of **compound 6a** was referred to obtain **compound 11a** in the form of a white solid (100 mg, 50% yield).

LC-MS m/z (ESI) = 652.10 [M+1].

### Step 2:

### (S)-2-((2-((6-oxo-5-(trifluoromethyl)-1,6-dihydropyridazin-4-yl)amino)propoxy)methyl)-5-(5-(trifluoromethyl)pyridin-2-yl)-6,7-dihydropyrazolo[1,5-a]pyrazin-4(5H)-one (Compound 11)

The similar synthesis method of **compound 6** was referred to obtain **compound 11** in the form of a white solid (40 mg, 50% yield).

¹H NMR (400 MHz, DMSO-*d*₆): δ 12.46 (s, 1H), 8.89 - 8.87 (m, 1H), 8.32 - 8.23 (m, 1H), 8.18 - 8.16 (m, 1H), 7.93 (s, 1H), 6.89 (s, 1H), 6.31 (dd, 1H), 4.65 - 4.43 (m, 6H), 4.30 - 4.09 (m, 1H), 3.55 (d 2H), 1.17 (d, 3H).

LC-MS m/z (ESI) = 532.10 [M+1].

### Example 12

### (S)-5-((1-((5-(5-cyc1opropylpyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 12)

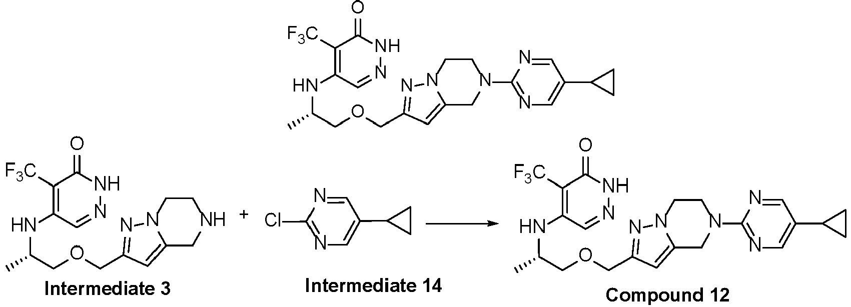

**Intermediate 3** (50 mg, 0.13 mmol, 1.0 equiv) and **intermediate 14** (23 mg, 0.15 mmol, 1.1 equiv) were weighed into a 10 mL reaction flask and dissolved in *N*,*N-*dimethylformamide (2.0 mL). The reaction mixture was added with *N*,*N*-diisopropyl-ethylamine (0.111 mL, 0.67 mmol, 5.0 equiv) and stirred at 120 °C for 5 h. After the reaction was completed, the reaction mixture was concentrated *in vacuo.* The residue was purified by MPLC (water/acetonitrile = 2:3) to obtain **compound 12** in the form of a white solid (29 mg, 44% yield).

¹H NMR (400 MHz, DMSO-*d*₆): δ 12.43 (s, 1H), 8.80 (s, 2H), 7.91 (s, 1H), 6.25 (dd, 1H), 6.10 (s, 1H), 4.99 (s, 2H), 4.41 (d, 2H), 4.32 (t, 2H), 4.16 (t, 3H), 3.55 - 3.45 (m, 2H), 2.88 - 2.73(m, 1H), 1.15 (d, 3H), 0.91 - 0.80 (m, 2H), 0.62 - 0.55 (m, 2H).

LCMS m/z = 491.49 [M+l].

### Example 13

### (S)-5-((1-((3-chloro-5-(5-(trifluoromethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 13)

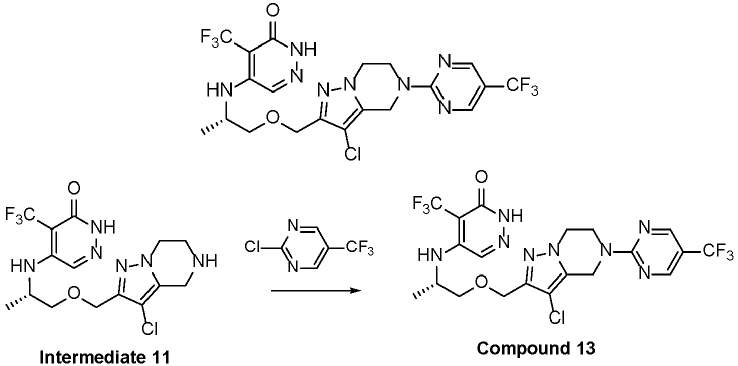

The similar synthesis method of **compound 1** was referred to obtain **compound 13** in the form of a white solid (40 mg, 29% yield).

¹H NMR (400 MHz, DMSO-*d*₆): δ 12.44 (s, 1H), 8.93 (s, 2H), 7.88 (s, 1H), 6.24 (dd, 1H), 4.94 (s, 2H), 4.47 - 4.39 (m, 2H), 4.34 (t, 2H), 4.17 (t, 2H), 3.52 (d, 2H), 1.15 (d, 3H).

LCMS m/z = 552.12 [M+l].

### Example 14

### (S)-5-((1-((3-chloro-5-(5-(trifluoromethyl)pyrazin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 14)

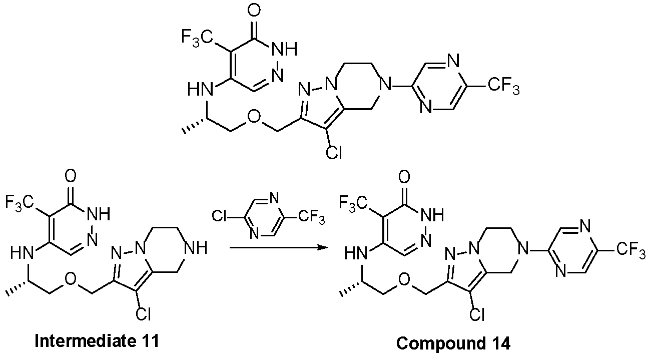

The similar synthesis method of **compound 1** was referred to obtain **compound 14** in the form of a white solid (30 mg, 21.73% yield).

¹H NMR (400 MHz, DMSO- *d*₆): δ 12.45 (s, 1H), 8.74 (s, 2H), 7.88 (s, 1H), 6.25 (dd, 1H), 4.87 (s, 2H), 4.43 - 4.39 (m, 2H), 4.23 (t, 2H), 4.15 (t, 2H), 3.55 (d, 2H), 1.15 (d, 3H).

LCMS m/z = 552.12 [M+l].

### Example 15

### (S)-6-(3-chloro-2-((2-((6-oxo-5-(trifluoromethyl)-1,6-dihydropyridazin-4-yl)amino)propoxy)methyl)-6,7-dihydropyrazolo[1,5-a]pyrazin-5(4H)-yl)nicotinonitrile (Compound 15)

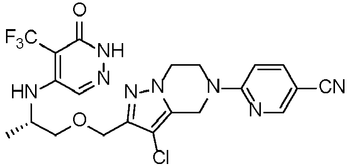

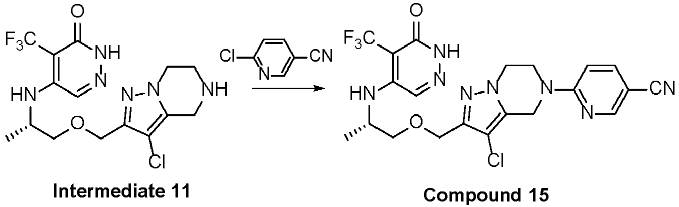

The similar synthesis method of **compound 1** was referred to obtain **compound 15** in the form of a white solid (18 mg, 35% yield).

¹H NMR (400 MHz, DMSO-*d*₆): δ 12.45 (s, 1H), 8.72 (s, 1H), 8.22 (d, 1H), 7.88 (s, 1H), 6.28 (dd, 1H), 4.86 (s, 2H), 4.44 - 4.39 (m, 2H), 4.23 (t, 2H), 4.17 (t, 2H), 3.60 (d, 2H), 1.15 (d, 3H).

LCMS m/z = 508.13 [M+l].

### Example 16

### (S)-5-((1-((3-chloro-5-(pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 16)

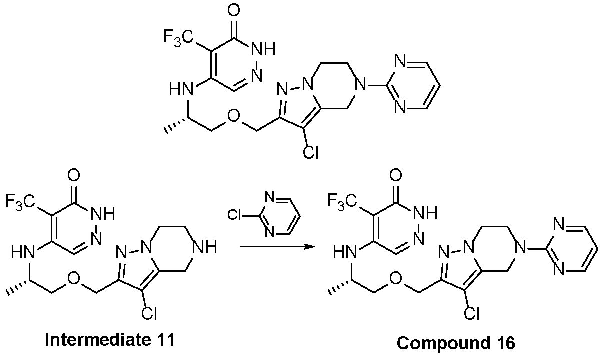

The similar synthesis method of **compound 1** was referred to obtain **compound 16** in the form of a white solid (12 mg, 25% yield).

¹H NMR (400 MHz, DMSO-*d*₆): δ 12.45 (s, 1H), 8.76 (d, 2H), 7.87 (s, 1H), 6.24 (dd, 1H), 4.82 (s, 2H), 4.44 - 4.40 (m, 2H), 4.25 (d, 2H), 4.17 (d, 2H), 3.62 (d, 2H), 1.15 (d, 3H).

LCMS m/z = 484.13 [M+l].

### Example 17

### (S)-5-((1-((3-chloro-5-(6-(trifluoromethyl)pyridazin-3-yl)-4,5,6,7-tetrahydropyrazolo[1,5-a]pyrazin-2-yl)methoxy)propan-2-yl)amino)-4-(trifluoromethyl)pyridazin-3(2H)-one (Compound 17)

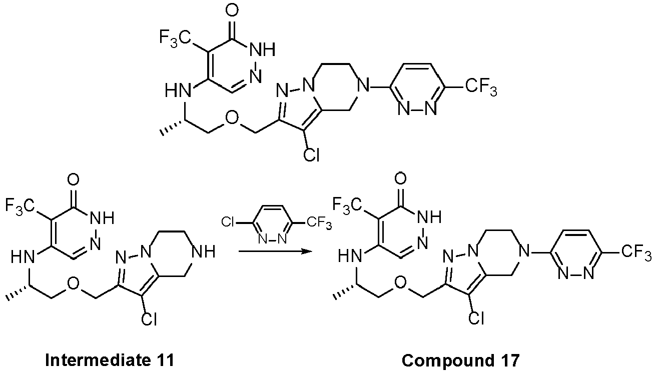

The similar synthesis method of **compound 1** was referred to obtain **compound 17** in the form of a white solid (16 mg, 29% yield).

¹H NMR (400 MHz, DMSO-*d*₆): δ 12.45 (s, 1H), 8.72 (s, 1H), 7.88 (s, 1H), 7.18 (d, 1H), 6.86 (d, 1H), 4.86 (s, 2H), 4.46 - 4.39 (m, 2H), 4.23 (t, 2H), 4.17 (t, 2H), 3.60 (d, 2H), 1.15 (d, 3H).

LCMS m/z = 552.12 [M+l].

### Example 18

### (S)-3-oxo-5-((1-((5-(5-(trifluoromethyl)pyrimidin-2-yl)-4,5,6,7-tetrahydropyrazolo[1,5-a]pyrazin-2-yl)methoxy)propan-2-yl)amino)-2,3-dihydropyridazine-4-carbonitrile (Compound 18)

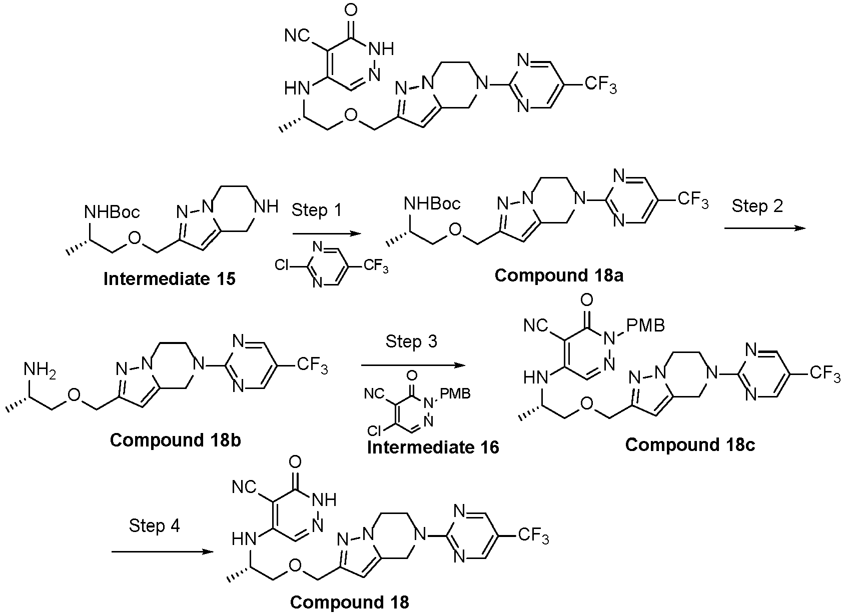

### Step 1:

### Tert-butyl-(S)-(1-((5-(5-(trifluoromethyl)pyrimidin-2-yl)-4,5,6,7-tetrahydropyrazolo[1,5-a]pyrazin-2-yl)methoxy)propan-2-yl)carbamate (Compound 18a)

The similar synthesis method of **compound 9a** was referred to obtain **compound 18a** in the form of a white solid (200 mg, 90% yield).

LC-MS m/z (ESI) = 458.10 [M+1].

### Step 2:

### (S)-1-((5-(5-(trifluoromethyl)pyrimidin-2-yl)-4,5,6,7-tetrahydropyrazolo[1,5-a]pyrazin-2-yl)methoxy)propan-2-amine (Compound 18b)

The similar synthesis method of **11i** was referred to obtain **compound 18b** in the form of a white solid (100 mg, 90% yield), and the crude product was used directly in the next step.

LC-MS m/z (ESI) = 358.10 [M+1].

### Step 3:

### (S)-2-(4-methoxybenzyl)-3-oxo-5-((1-((5-(5-(trifluoromethyl)pyrimidin-2-yl)-4,5,6,7-tetrahydropyrazolo[1,5-a]pyrazin-2-yl)methoxy)propan-2-yl)amino)-2,3-dihydropyridazine-4-carbonitrile (Compound 18c)

The similar synthesis method of **3a** was referred to obtain **compound 18c** in the form of a yellow oil (20 mg, 55% yield).

LC-MS m/z (ESI) = 596.10 [M+1].

### Step 4:

### (S)-3-oxo-5-((1-((5-(5-(trifluoromethyl)pyrimidin-2-yl)-4,5,6,7-tetrahydropyrazolo[1,5-a]pyrazin-2-yl)methoxy)propan-2-yl)amino)-2,3-dihydropyridazine-4-carbonitrile (Compound 18)

The similar synthesis method of **compound 6** was referred to obtain **compound 18** in the form of a white solid (8.0 mg, 50% yield).

¹H NMR (400 MHz, DMSO-*d*₆) δ 12.47 (s, 1H), 8.91 - 8.74 (m, 2H), 7.53 (s, 1H), 6.14 (s, 1H), 5.01 (d, 2H), 4.41 (s, 2H), 4.33 (t, 2H), 4.16 (t, 2H), 4.03 (q, 2H), 3.49 (d, 2H), 1.17 (d, 3H).

¹⁹F NMR (376 MHz, DMSO-*d*₆) δ -57.26 (3F), -59.38 (3F).

LC-MS m/z (ESI) = 476.10 [M+1].

### Example 19

### (S)-4-(trifluoromethyl)-5-((1-((3-(trifluoromethyl)-5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 19)

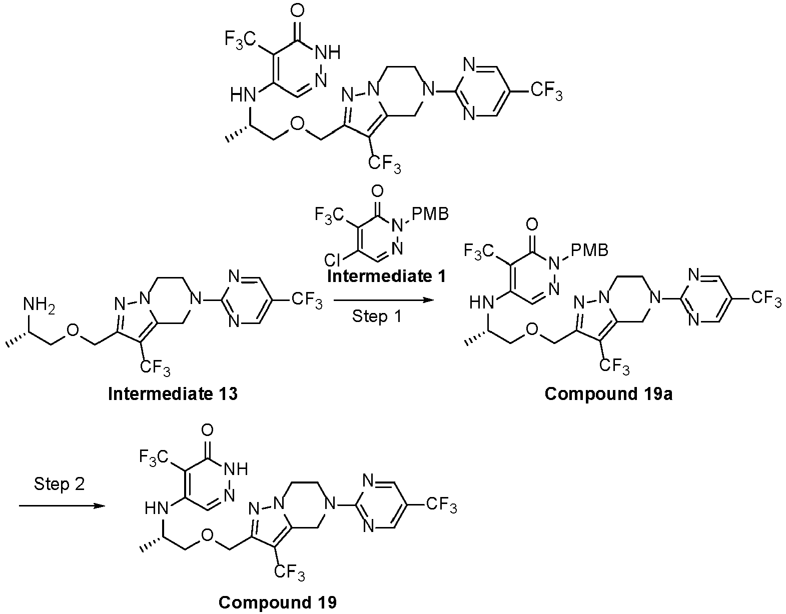

### Step 1:

### (S)-2-(4-methoxybenzyl)-4-(trifluoromethyl)-5-((1-((3-(trifluoromethyl)-5-(5-(trifluoromethyl)pyrimidin-2-yl)-4,5,6,7-tetrahydropyrazolo[1,5-a]pyrazin-2-yl)methoxy) propan-2-yl)amino)pyridazin-3(2H)-one (19a)

**Intermediate 13** (161.8 mg, 0.38 mmol, 1.0 equiv) and **intermediate 1** (145 mg, 0.46 mmol, 1.2 equiv) were weighed into a 25 mL reaction flask and dissolved in acetonitrile (10 mL). The reaction mixture was added with triethylamine (0.26 mL, 1.9 mmol, 5 equiv), reacted at 90 °C and stirred for 1 h. After the reaction was completed, the reaction mixture was concentrated *in vacuo.* The residue was purified by column chromatography (petroleum ether/ethyl acetate = 1:1) to obtain **compound 19a** in the form of a pale yellow solid (130 mg, 48% yield).

LCMS m/z = 706.21 [M+l].

### Step 2:

### (S)-4-(trifluoromethyl)-5-((1-((3-(trifluoromethyl)-5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 19)

To a 10 mL reaction flask with compound **19a** weighed out (130 mg, 0.18 mmol, 1.0 equiv) was added trifluoroacetic acid (3 mL) and trifluoromethanesulfonic acid (0.1 mL, 0.72 mmol, 4.0 equiv) successively. After the addition was completed, the reaction mixture was stirred at 25 °C for 1 h. After the reaction was completed, the resulting solution was quenched with saturated potassium carbonate, adjusted to pH 8 to 9, and extracted with ethyl acetate (3 × 50 mL). The organic layers were combined and concentrated *in vacuo,* and the residue was purified by MPLC (water/acetonitrile = 1: 1) to obtain **compound 19** in the form of a white solid (70 mg, 56% yield).

¹H NMR (400 MHz, DMSO-*d*₆): δ 8.85 (s, 2H), 7.88 (s, 1H), 6.22 (dd, 1H), 5.14 (s, 2H), 4.49 (s, 2H), 4.37 (t, 2H), 4.24 (t, 2H), 4.16 (d, 1H), 3.52 (d, 2H), 1.14 (d, 3H).

LC-MS m/z (ESI) = 586.15 [M+1].

### Example 20

### (S)-5-((1-((7-(5-fluoropyridin-2-yl)-5,6,7,8-tetrahydro-[1,2,4]triazolo[1,5-a]pyrazin-2-yl)methoxy)propan-2-yl)amino)-4-(trifluoromethyl)pyridazin-3(2H)-one (Compound 20)

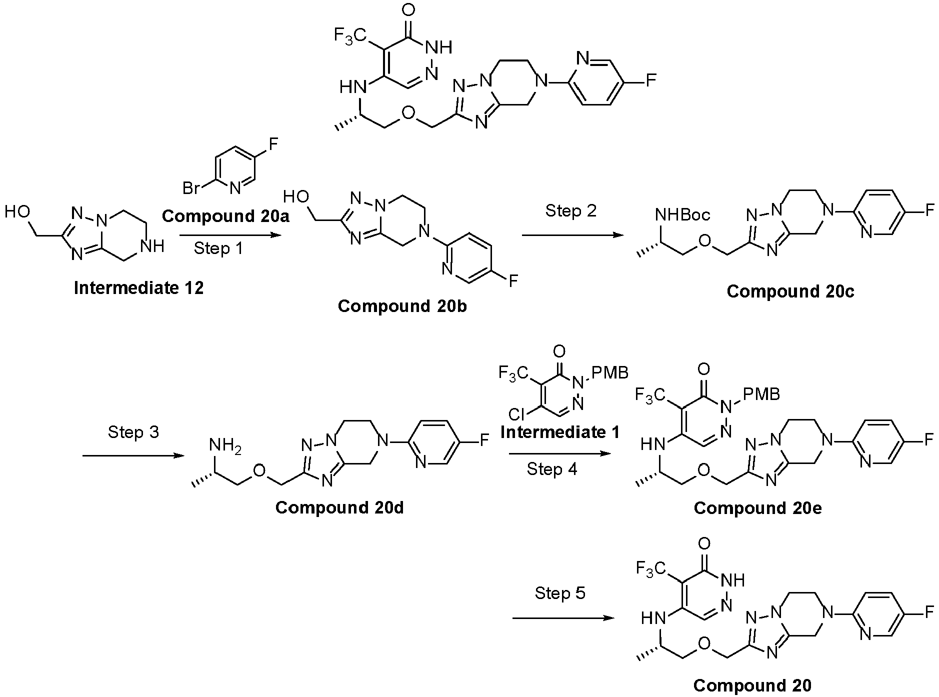

### Step 1:

### (7-(5-Fluoropyridin-2-yl)-5,6,7,8-tetrahydro-[1,2,4]triazolo[1,5-a]pyrazin-2-yl)methanol (Compound 20b)

The similar synthesis method of **compound 9a** was referred to obtain **compound 20b** in the form of a white solid (125 mg, 62% yield).

LC-MS m/z (ESI) = 250.10 [M+1].

### Step 2:

### Tert-butyl (S)-(1-((7-(5-fluoropyridin-2-yl)-5,6,7,8-tetrahydro-[1,2,4]triazolo[1,5-a]pyrazin-2-yl)methoxy)propan-2-yl)carbamate (Compound 20c)

The similar synthesis method of **intermediate 11h** was referred to obtain **compound 20c** in the form of a yellow oil (100 mg, 52% yield).

LC-MS m/z (ESI) = 407.21 [M+1].

### Step 3:

### (S)-1-((7-(5-fluoropyridin-2-yl)-5,6,7,8-tetrahydro-[1,2,4]triazolo[1,5-a]pyrazin-2-yl)methoxy)propan-2-amine (Compound 20d)

The similar synthesis method of **intermediate 11i** was referred to obtain crude **compound 20d** in the form of a white solid (87 mg), and the crude product was used directly in the next step without further purification.

LC-MS m/z (ESI) = 307.21 [M+1].

### Step 4:

### (S)-5-((1-((7-(5-fluoropyridin-2-yl)-5,6,7,8-tetrahydro-[1,2,4]triazolo[1,5-a]pyrazin-2-yl)methoxy)propan-2-yl)amino)-2-(4-methoxybenzyl)-4-(trifluoromethyl)pyridazin-3(2H)-one (Compound 20e)

The similar method of **3a** was referred to obtain **compound 20e** in the form of a yellow oil (75 mg, 48% yield).

LC-MS m/z (ESI) = 589.22 [M+1].

### Step 5:

### (S)-5-((1-((7-(5-fluoropyridin-2-yl)-5,6,7,8-tetrahydro-[1,2,4]triazolo[1,5-a]pyrazin-2-yl)methoxy)propan-2-yl)amino)-4-(trifluoromethyl)pyridazin-3(2H)-one (Compound 20)

The similar synthesis method of **compound 6** was referred to obtain **compound 20** in the form of a white solid (42 mg, 62% yield).

¹H NMR (400 MHz, DMSO-*d*₆) δ 12.47 (s, 1H), 8.17 (d, 1H), 7.91 (s, 1H), 7.64 - 7.59 (m, 1H), 7.12 (dd, 1H), 6.32 (s, 1H), 4.75 (s, 2H), 4.54 - 4.42 (m, 2H), 4.20 - 4.15(m, 3H), 4.08 (t, 2H), 3.57 (d, 2H), 1.15 (d, 3H).

¹⁹F NMR (376 MHz, DMSO-*d*₆) δ -55.45 (3F), -141.97 (F).

LC-MS m/z (ESI) = 469.22 [M+1].

### Example 21

### (S)-4-(trifluoromethyl)-5-((1-((5-(5-(trifluoromethyl)pyrazin-2-yl)-4,5,6,7-tetrahydropyrazolo[1,5-a]pyrazin-2-yl-4,4-d₂)methoxy-d₂)propan-2-yl)amino)pyridazin-3(2H)-one (Compound 21)

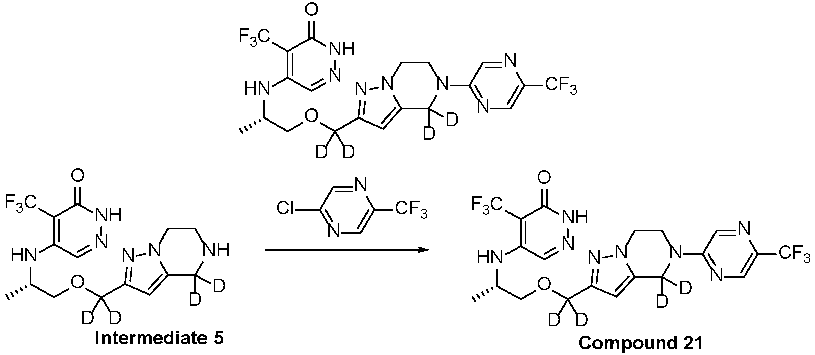

The similar synthesis method of **compound 1** was referred to obtain **compound 21** in the form of a white solid (37 mg, 65% yield).

¹H NMR (400 MHz, DMSO-*d*₆) δ 12.44 (s, 1H), 8.57 (s, 2H), 7.90 (s, 1H), 6.42 - 6.21 (m, 1H), 6.10 (s, 1H), 4.38 - 3.94 (m, 5H), 3.56 - 3.44 (m, 2H), 1.15 (d, 3H).

LC-MS m/z (ESI) = 523.10 [M+1].

### Example 22

### (S)-2-(2-((2-((6-oxo-5-(trifluoromethyl)-1,6-dihydropyridazin-4-yl)amino)propoxy)methyl- d₂)-6,7-dihydropyrazolo[1,5-a]pyrazin-5(4H)-yl-4,4-d₂)pyrimidine-5-carbonitrile (Compound 22)

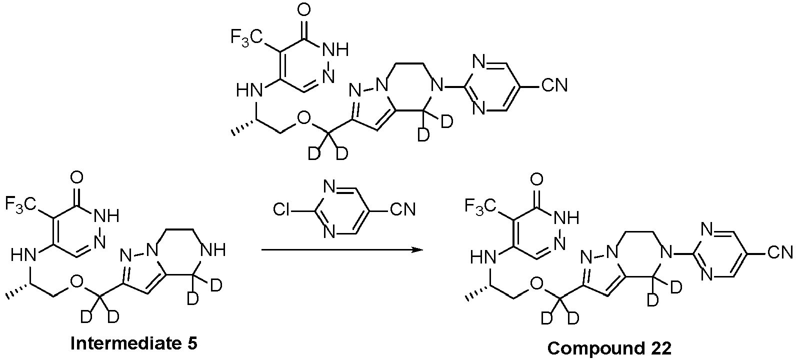

The similar synthesis method of **compound 1** was referred to obtain **compound 22** in the form of a white solid (50 mg, 65% yield).

¹H NMR (400 MHz, DMSO-*d*₆) δ 12.44 (s, 1H), 8.86 (s, 2H), 7.89 (s, 1H), 6.28 - 6.25 (m, 1H), 6.11 (s, 1H), 4.34 - 4.31 (m, 2H), 4.17 - 4.14 (m, 3H), 3.63 - 3.41 (m, 2H), 1.15 (d, 3H).

LC-MS m/z (ESI) = 480.10 [M+1].

### Example 23

### (S)-6-(2-((2-((6-oxo-5-(trifluoromethyl)-1,6-dihydropyridazin-4-yl)amino)propoxy)methyl-d₂)-6,7-dihydropyrazolo[1,5-a]pyrazin-5(4H)-yl-4,4-d₂)nicotinonitrile (Compound 23)

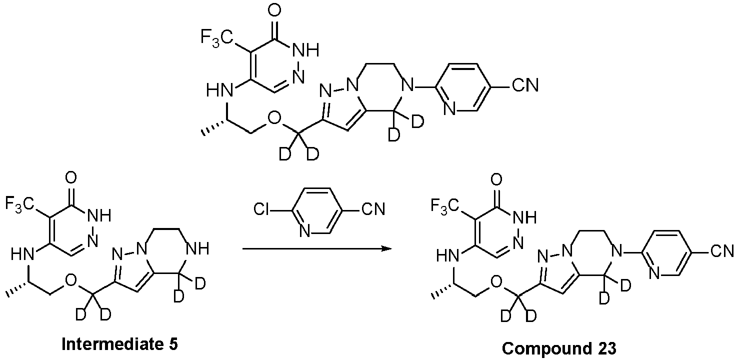

The similar synthesis method of **compound** 1 was referred to obtain **compound 23** in the form of a white solid (24 mg, 48% yield).

¹H NMR (400 MHz, DMSO-*d*₆): δ 12.41 (s, 1H), 8.56 (d, 1H), 7.97 - 7.94 (m, 1H), 7.90 (s, 1H), 7.09 (d, 1H), 6.25 - 6.29 (m, 1H), 6.08 (s, 1H), 4.20 - 4.13 (m, 5H), 3.49 (d, 2H), 1.15 (d, 3H).

LC-MS m/z (ESI) = 478.10 [M+1].

### Example 24

### (S)-6-(2-((2-((6-oxo-5-(trifluoromethyl)-1,6-dihydropyridazin-4-yl)amino)propoxy)methyl)-5,6-dihydro-[1,2,4]triazolo[1,5-a]pyrazin-7(8H)-yl)nicotinonitrile (Compound 24)

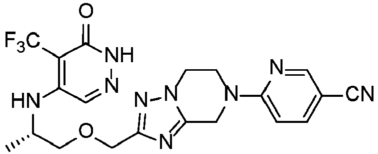

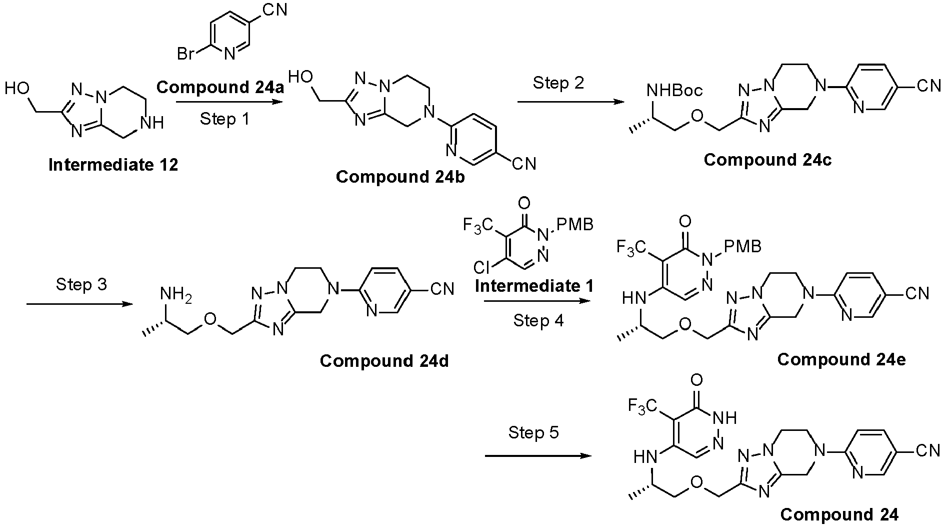

### Step 1:

### 6-(2-(Hydroxymethyl)-5,6-dihydro-[1,2,4]triazolo[1,5-a]pyrazin-7(8H)-yl)nicotinonitrile (Compound 24b)

The similar synthesis method of **compound 9a** was referred to obtain **compound 24b** in the form of a white solid (130 mg, 42% yield).

¹H NMR (400 MHz, DMSO-*d*₆) δ 8.59 (d, 1H), 7.99 (dd, 1H), 7.18 (d, 1H), 5.17 (s, 1H), 4.94 (s, 2H), 4.40 (s, 2H), 4.26 (dd, 2H), 4.21 (dd, 2H).

LC-MS m/z (ESI) = 257.11 [M+1].

### Step 2:

### Tert-butyl-(S)-(1-((7-(5-cyanopyridin-2-yl)-5,6,7,8-tetrahydro-[1,2,4]triazolo[1,5-a]pyrazin-2-yl)methoxy)propan-2-yl)carbamate (Compound 24c)

The similar synthesis method of **intermediate 11h** was referred to obtain **compound 24c** in the form of a yellow oil (210 mg, 75% yield).

LC-MS m/z (ESI) = 414.21 [M+1].

### Step 3:

### (S)-1-((7-(5-fluoropyridin-2-yl)-5,6,7,8-tetrahydro-[1,2,4]triazolo[1,5-a]pyrazin-2-yl)methoxy)propan-2-amine (Compound 24d)

The similar synthesis method of **intermediate 11i** was referred to obtain **compound 24d** in the form of a white solid (150 mg, 90% yield), and the crude product was used directly in the next step.

LC-MS m/z (ESI) = 314.21 [M+1].

### Step 4:

### (S)-5-((1-((7-(5-fluoropyridin-2-yl)-5,6,7,8-tetrahydro-[1,2,4]triazolo[1,5-a]pyrazin-2-yl)methoxy)propan-2-yl)amino)-2-(4-methoxybenzyl)-4-(trifluoromethyl)pyridazin-3(2H)-one (Compound 24e)

The method of **3a** was referred to synthesize **compound 24e** in the form of a yellow oil (130 mg, 42% yield).

LC-MS m/z (ESI) = 596.23 [M+1].

### Step 5:

### (S)-6-(2-((2-((6-oxo-5-(trifluoromethyl)-1,6-dihydropyridazin-4-yl)amino)propoxy)methyl)-5,6-dihydro-[1,2,4]triazolo[1,5-a]pyrazin-7(8H)-yl)nicotinonitrile (Compound 24)

The similar synthesis method of **compound 6** was referred to obtain **compound 24** in the form of a white solid (42 mg, 62% yield).

¹H NMR (400 MHz, DMSO-*d*₆) δ 12.47 (s, 1H), 8.59 (d, 1H), 8.00 (dd, 1H), 7.91 (s, 1H), 7.17 (d, 1H), 6.39 - 6.27 (m, 1H), 4.94 (s, 2H), 4.54 - 4.42 (m, 2H), 4.28 - 4.10 (m, 5H), 3.57 (d, 2H), 1.15 (d, 3H).

¹⁹F NMR (376 MHz, DMSO-*d*₆) δ -55.48 (3F).

LC-MS m/z (ESI) = 476.17 [M+1].

### Example 25

### (S)-4-(trifluoromethyl)-5-((1-((5-(5-(trifluoromethyl)pyrimidin-2-yl)-4,5,6,7-tetrahydropyrazolo[1,5-a]pyrazin-2-yl-d)methoxy)propan-2-yl)amino)pyridazin-3(2H)-one (Compound 25)

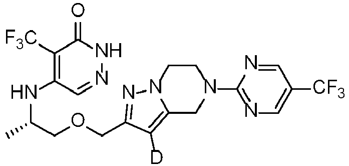

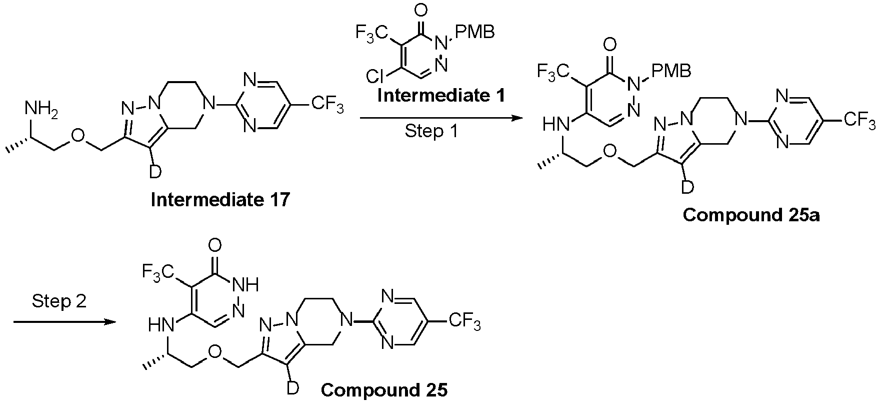

### Step 1:

### (S)-2-(4-methoxybenzyl)-4-(trifluoromethyl)-5-((1-((5-(5-(trifluoromethyl)pyrimidin-2-yl)-4,5,6,7-tetrahydropyrazolo[1,5-a]pyrazin-2-yl-3-d)methoxy)propan-2-yl)amino)pyridazin-3(2H)-one (Compound 25a)

The synthesis method of **19a** was referred to obtain **compound 25a** in the form of a pale yellow solid (32 mg, 24% yield).

LCMS m/z = 639.22 [M+l].

### Step 2:

### (S)-4-(trifluoromethyl)-5-((1-((5-(5-(trifluoromethyl)pyrimidin-2-yl)-4,5,6,7-tetrahydropyrazolo[1,5-a]pyrazin-2-yl-d)methoxy)propan-2-yl)amino)pyridazin-3(2H)-one (Compound 25)

The similar synthesis method of **compound 19** was referred to obtain **compound 25** in the form of a white solid (15 mg, 72% yield).

¹H NMR (400 MHz, DMSO-*d*₆): δ 12.47 (s, 1H), 8.81 (s, 2H), 7.93 (s, 1H), 6.26 (dd, 1H), 5.06 (s, 2H), 4.41 (d, 2H), 4.32 (t, 2H), 4.16 (t, 3H), 3.53 - 3.47 (m, 2H), 1.15 (d, 3H).

LCMS m/z = 519.42 [M+l].

### Example 26

### (S)-4-(trifluoromethyl)-5-((1-((7-(5-(trifluoromethyl)pyridin-2-yl)-5,6,7,8-tetrahydro[1,2,4]triazolo[1,5-a]pyrazin-2-yl)methoxy)propan-2-yl)amino)pyridazin-3(2H)one (Compound 26)

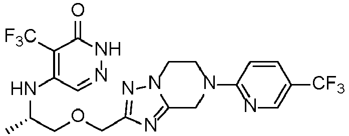

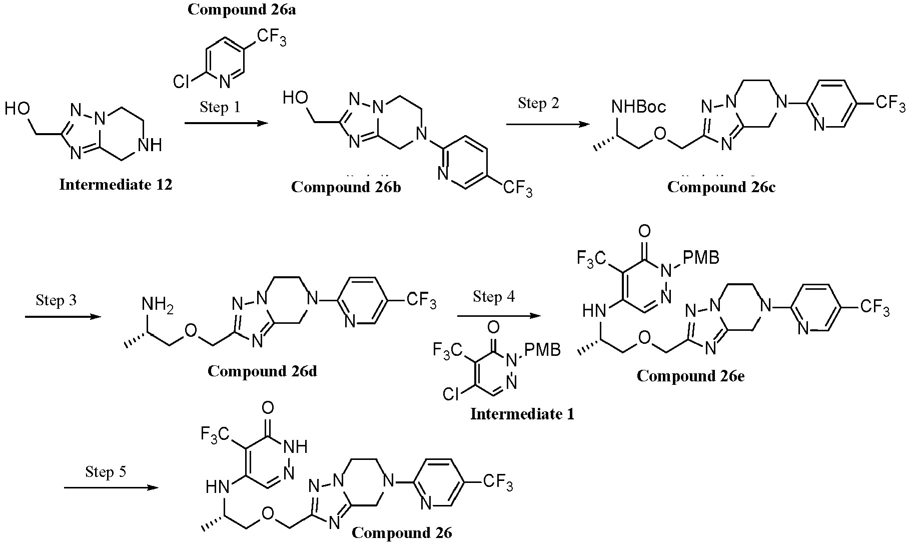

### Step 1:

### (7-(5-(Trifluoromethyl)pyridin-2-yl)-5,6,7,8-tetrahydro-[1,2,4]triazolo[1,5-a]pyrazin-2-yl)methanol (Compound 26b)

The similar synthesis method of **compound 9a** was referred to obtain **compound 26b** in the form of a white solid (80 mg, 75% yield).

LC-MS m/z (ESI) = 300.10 [M+1].

### Step 2:

### Tert-butyl-(1-((7-(5-(trifluoromethyl)pyridin-2-yl)-5,6,7,8-tetrahydro-[1,2,4]triazolo[1,5-a]pyrazin-2-yl)methoxy)propan-2-yl)carbamate (Compound 26c)

The similar synthesis method of **intermediate 11h** was referred to obtain **compound 26c** in the form of a yellow oil (40 mg, 32% yield).

LC-MS m/z (ESI) = 457.10 [M+1].

### Step 3:

### (S)-1-((7-(5-(trifluoromethyl)pyridin-2-yl)-5,6,7,8-tetrahydro-[1,2,4]triazolo[1,5-a]pyrazin-2-yl)methoxy)propan-2-amine (Compound 26d)

The similar synthesis method of intermediate **11i** was referred to obtain **compound 26d** in the form of a white solid (30 mg, 96% yield), and the crude product was used directly in the next step.

LC-MS m/z (ESI) = 357.10 [M+1].

### Step 4:

### (S)-2-(4-methoxybenzyl)-4-(trifluoromethyl)-5-((1-((7-(5-(trifluoromethyl)pyridin-2-yl)-5,6,7,8-tetrahydro-[1,2,4]triazolo[1,5-a]pyrazin-2-yl)methoxy)propan-2-yl)amino)pyridazin-3(2H)-one (Compound 26e)

The method of **3a** was referred to synthesize **compound 26e** in the form of a yellow oil (40 mg, 76% yield).

LC-MS m/z (ESI) = 639.10 [M+1].

### Step 5:

### (S)-4-(trifluoromethyl)-5-((1-((7-(5-(trifluoromethyl)pyridin-2-yl)-5,6,7,8-tetrahydro[1,2,4]triazolo[1,5-a]pyrazin-2-yl)methoxy)propan-2-yl)amino)pyridazin-3(2H)one (Compound 26)

The similar synthesis method of **compound 6** was referred to obtain **compound 26** in the form of a white solid (10 mg, 62% yield).

¹H NMR (400 MHz, DMSO-*d*₆) δ 12.47 (s, 1H), 8.51 (d, 1H), 7.95 - 7.90 (m, 2H), 7.20 (d, 1H), 6.38 - 6.23 (m, 1H), 4.92 (s, 2H), 4.57 - 4.34 (m, 2H), 4.25 - 4.20 (m, 4H), 4.17 - 4.13 (m, 1H), 3.57 (d, 2H), 1.15 (d, 3H).

¹⁹F NMR (376 MHz, DMSO-*d*₆) δ -55.46 (3F),-59.48 (3F).

LC-MS m/z (ESI) = 519.10 [M+1].

### Example 27

### (R)-4-(trifluoromethyl)-5-(2-(((7-(5-(trifluoromethyl)pyrimidin-2-yl)-5,6,7,8-tetrahydro-[1,2,4]triazolo[1,5-a]pyrazin-2-yl)methoxy)methyl)pyrrolidin-1-yl)pyridazin-3(2H)-one (Compound 27)

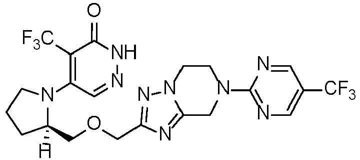

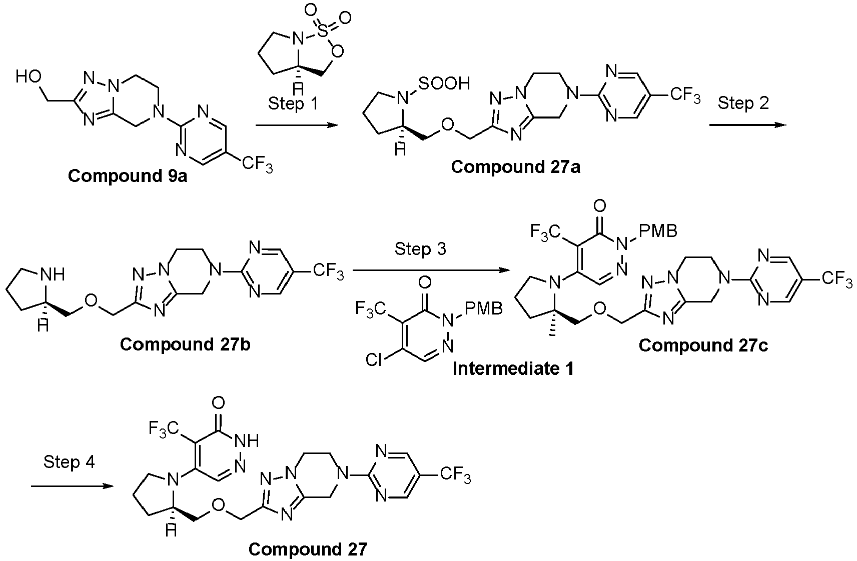

### Step 1:

### (2R)-2-(((7-(5-(trifluoromethyl)pyrimidin-2-yl)-5,6,7,8-tetrahydro-[1,2,4]triazolo[1,5-a]pyrazin-2-yl)methoxy)methyl)pyrrolidine-1-sulfinic acid (Compound 27a)

**Compound 9a** (300 mg, 1.0 mmol, 1.0 equiv) was added to a 50 mL reaction flask and dissolved in anhydrous *N,N-*dimethylformamide (3 mL). Under nitrogen atmosphere, sodium hydride (60 mg, 1.5 mmol, 1.5 equiv) was added in portions at 0 °C. After the addition was completed, the reaction mixture was stirred for another 20 min at this temperature. A solution of (*R*)-1,1-dioxide-tetrahydro-3*H*-pyrrolo [1,2-*c*][1,2,3]oxathiazole (326 mg, 2 mmol, 2.0 equiv) in *N,N-*dimethylformamide (3 mL) was slowly added dropwise to the reaction system with the temperature maintained at 0 °C, and the reaction mixture was stirred for another 2 h. After the reaction was completed, the pH of the reaction system was adjusted to 3, and the reaction mixture was stirred at room temperature for 0.5 h. The reaction mixture was extracted with ethyl acetate (3 × 120 mL). The organic layers were combined, dried over anhydrous sodium sulfate and concentrated in *vacuo* to obtain a crude product. The crude product was purified by column chromatography (dichloromethane:methanol = 40:1) to obtain **compound 27a** in the form of a yellow oil (200 mg, 52% yield).

¹H NMR (400 MHz, DMSO-*d*₆) δ 8.84 (s, 2H), 5.09 (s, 2H), 4.46 - 4.36 (m, 4H), 4.25 (t, 2H), 3.64 - 3.61 (m, 1H), 3.48 - 3.39 (m, 1H), 3.13 (t, 1H), 3.03 - 2.85 (m, 2H), 1.77 - 1.51 (m, 4H).

LC-MS m/z (ESI) = 464.22 [M+1].

### Step 2:

### (R)-2-((pyrrolidin-2-ylmethoxy)methyl)-7-(5-(trifluoromethyl)pyrimidin-2-yl)-5,6,7,8-tetrahydro-[1,2,4]triazolo[1,5-a]pyrazine (Compound 27b)

**Compound 27a** (200 mg, 0.4 mmol, 1.0 equiv) was dissolved in anhydrous *N,N-*dimethylformamide (3 mL) in a 50 mL reaction flask and iodotrimethylsilane (140 µL, 1.0 mmol, 2.5 equiv) was added to the reaction system. After the addition was completed, the reaction mixture was stirred at 110 °C for another 40 min until the reaction was completed, cooled to room temperature, washed with anhydrous sodium sulfite solution and extracted with ethyl acetate (3 × 60 mL). The organic phase was concentrated to obtain the crude **compound 27b** in the form of a yellow oil (150 mg, 99% yield).

LC-MS m/z (ESI) = 384.10[M+1].

### Step 3:

### (R)-2-(4-methoxybenzyl)-5-(2-methyl-2-(((7-(5-(trifluoromethyl)pyrimidin-2-yl)-5,6,7,8-tetrahydro-[1,2,4]triazolo[1,5-a]pyrazin-2-yl)methoxy)methyl)pyrrolidin-1-yl)-4-(trifluoromethyl)pyridazin-3(2H)-one (Compound 27c)

The method of **intermediate 3a** was referred to synthesize **compound 27c** in the form of a yellow oil (40 mg, 76% yield).

LC-MS m/z (ESI) = 666.20 [M+1].

### Step 4

### (R)-4-(trifluoromethyl)-5-(2-(((7-(5-(trifluoromethyl)pyrimidin-2-yl)-5,6,7,8-tetrahydro-[1,2,4]triazolo[1,5-a]pyrazin-2-yl)methoxy)methyl)pyrrolidin-1-yl)pyridazin-3(2H)-one (Compound 27)

The similar synthesis method of **compound** 6 was referred to obtain **compound** 27 in the form of a white solid (68 mg, 52% yield).

¹H NMR (400 MHz, DMSO-*d*₆) δ 12.36 (s, 1H), 8.85 (s, 2H), 8.02 (s, 1H), 5.05 (d, 2H), 4.51 (s, 1H), 4.44 (s, 2H), 4.38 (t, 2H), 4.21 (t, 2H), 3.63 - 3.59 (m, 1H), 3.52 - 3.48 (m, 2H), 3.18 (d, 1H), 2.08 (s, 1H), 1.86 (d, 1H), 1.66 (d, 2H).

¹⁹F NMR (376 MHz, DMSO-*d*₆) δ -53.83 (3F), -59.42 (3F).

LC-MS m/z (ESI) = 546.20 [M+1].

### Example 28

### (S)-4-(trifluoromethyl)-5-(2-(((7-(5-(trifluoromethyl)pyrimidin-2-yl)-5,6,7,8-tetrahydro-[1,2,4]triazolo[1,5-a]pyrazin-2-yl)methoxy)methyl)pyrrolidin-1-yl)pyridazin-3(2H)-one (Compound 28)

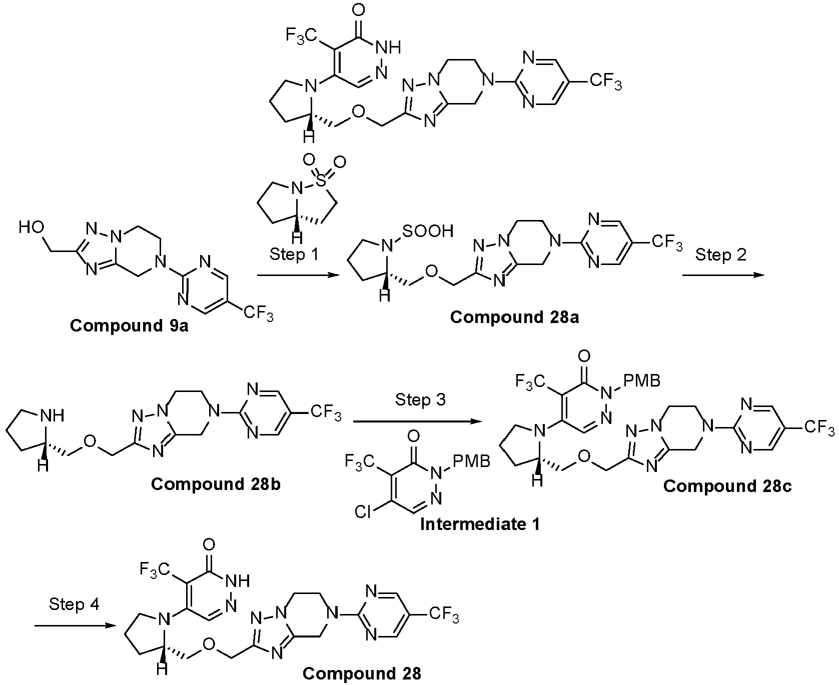

### Step 1:

### (2S)-2-(((7-(5-(trifluoromethyl)pyrimidin-2-yl)-5,6,7,8-tetrahydro-[1,2,4]triazolo[1,5-a]pyrazin-2-yl)methoxy)methyl)pyrrolidine-1-sulfinic acid (Compound 28a)

The similar synthesis method of **compound 27a** was referred to obtain **compound 28a** in the form of a yellow oil (400 mg, 88% yield).

¹H NMR (400 MHz, DMSO-*d*₆) δ 8.84 (s, 2H), 5.08 (s, 2H), 4.41- 4.38 (m, 4H), 4.25 (t, 2H), 3.64 - 3.61(m, 1H), 3.46 - 3.42(m, 1H), 3.12 (t, 1H), 3.02 - 2.81 (m, 2H), 1.78 - 1.51 (m, 4H).

LC-MS m/z (ESI) = 464.22 [M+1].

### Step 2:

### (S)-2-((pyrrolidin-2-ylmethoxy)methyl)-7-(5-(trifluoromethyl)pyrimidin-2-yl)-5,6,7,8-tetrahydro-[1,2,4]triazolo[1,5-a]pyrazine (Compound 28b)

The similar synthesis method of **compound 27b** was referred to obtain **compound 28b** in the form of a yellow oil (300 mg, 98% yield), and the crude product was used directly in the next step.

LC-MS m/z (ESI) = 384.10 [M+1].

### Step 3:

### (S)-2-(4-methoxybenzyl)-5-(2-methyl-2-(((7-(5-(trifluoromethyl)pyrimidin-2-yl)-5,6,7,8-tetrahydro-[1,2,4]triazolo[1,5-a]pyrazin-2-yl)methoxy)methyl)pyrrolidin-1-yl)-4-(trifluoromethyl)pyridazin-3(2H)-one (Compound 28c)

The similar synthesis method of **3a** was referred to obtain **compound 28c** in the form of a yellow oil (500 mg, 76% yield).

LC-MS m/z (ESI) = 666.20 [M+1].

### Step 4:

### (S)-4-(trifluoromethyl)-5-(2-(((7-(5-(trifluoromethyl)pyrimidin-2-yl)-5,6,7,8-tetrahydro-[1,2,4]triazolo[1,5-a]pyrazin-2-yl)methoxy)methyl)pyrrolidin-1-yl)pyridazin-3(2H)-one (Compound 28)

The similar synthesis method of **compound 6** was referred to obtain **compound 28** in the form of a white solid (80 mg, 56% yield).

¹H NMR (400 MHz, DMSO-*d*₆) δ 12.36 (s, 1H), 8.85 (s, 2H), 8.02 (s, 1H), 5.05 (d, 2H), 4.51 (s, 1H), 4.44 (s, 2H), 4.38 (t, 2H), 4.21 (t, 2H), 3.63 - 3.59 (m, 1H), 3.52 - 3.48 (m, 2H), 3.19 (s, 1H), 2.08 (s, 1H), 1.88 (s, 1H), 1.66 (d, 2H).

¹⁹F NMR (376 MHz, DMSO-*d*₆) δ -53.83 (3F), -59.42 (3F).

LC-MS m/z (ESI) = 546.20 [M+1].

### Example 29

### (S)-2-(2-((2-((6-oxo-5-(trifluoromethyl)-1,6-dihydropyridazin-4-yl)amino)propoxy)methyl)-6,7-dihydropyrazolo[1,5-a]pyrazin-5(4H)-yl)thiazole-5-carbonitrile (Compound 29)

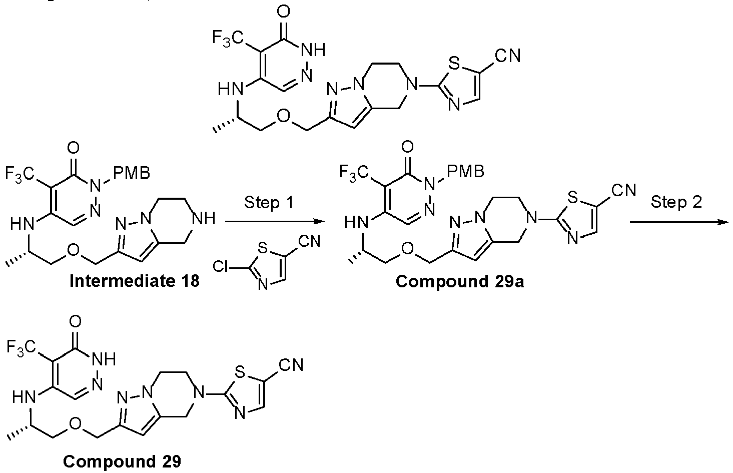

### Step 1:

### (S)-2-(2-((2-((1-(4-methoxybenzyl)-6-oxo-5-(trifluoromethyl)-1,6-dihydropyridazin-4-yl)amino)propoxy)methyl)-6,7-dihydropyrazolo[1,5-a]pyrazin-5(4H)-yl)thiazole-5-carbonitrile (Compound 29a)

The similar synthesis method of **compound 1** was referred to obtain **compound 29a** in the form of a yellow solid (480 mg, 88% yield).

¹H NMR (400 MHz, DMSO-*d*₆) δ 8.12 (s, 1H), 7.96 (s, 1H), 7.22 (d, 2H), 6.92 - 6.84 (m, 2H), 6.42 - 6.30 (m, 1H), 6.11 (s, 1H), 5.06 - 4.99 (m, 2H), 4.76 (s, 2H), 4.41 (d, 2H), 4.27 - 4.01 (m, 5H), 3.72 (s, 3H), 3.48 (d, 2H), 1.14 (d, 3H).

LC-MS m/z (ESI) = 601.20 [M+1].

### Step 2:

### (S)-2-(2-((2-((6-oxo-5-(trifluoromethyl)-1,6-dihydropyridazin-4-yl)amino)propoxy)-methyl)-6,7-dihydropyrazolo[1,5-a]pyrazin-5(4H)-yl)thiazole-5-carbonitrile (Compound29)

The similar synthesis method of **compound 6** was referred to obtain **compound 29** in the form of a white solid (98 mg, 56% yield).

¹H NMR (400 MHz, DMSO-*d*₆) δ 12.46 (s, 1H), 8.12 (s, 1H), 7.90 (s, 1H), 6.30 - 6.27 (m, 1H), 6.13 (s, 1H), 4.79 (s, 2H), 4.42 (d, 2H), 4.30 - 4.01 (m, 5H), 3.50 (d, 2H), 1.15 (d, 3H).

¹⁹F NMR (376 MHz, DMSO-*d*₆) δ -55.43 (3F).

LC-MS m/z (ESI) = 481.20 [M+1].

### Example 30

### (S)-4-(trifluoromethyl)-5-((1-((5-(trifluoromethyl)thiazol-2-yl)-4,5,6,7-tetrahydropyrazolo[1,5-a]pyrazin-2-yl)methoxy)propan-2-yl)amino)pyridazin-3(2H)-one (Compound 30)

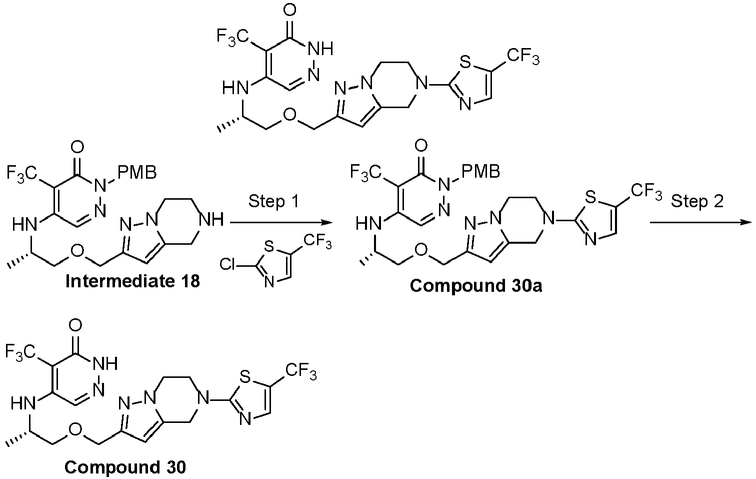

### Step 1:

### (S)-2-(4-methoxybenzyl)-4-(trifluoromethyl)-5-((1-((5-(5-(trifluoromethyl)thiazol-2-yl)-4,5,6,7-tetrahydropyrazolo[1,5-a]pyrazin-2-yl)methoxy)propan-2-yl)amino)pyridazin-3(2H)-one (Compound 30a)

The similar synthesis method of **compound 1** was referred to obtain **compound 30a** in the form of a yellow solid (90 mg, 78% yield).

¹H NMR (400 MHz, DMSO-*d*₆) δ 7.96 (s, 1H), 7.83 (d, 1H), 7.21 (d, 2H), 6.88 (d, 2H), 6.38 - 6.34 (m, 1H), 6.11 (s, 1H), 5.02 (d, 2H), 4.73 (s, 2H), 4.41 (d, 2H), 4.22 - 4.15 (m, 3H), 4.03 (t, 2H), 3.71 (s, 3H), 3.48 (d, 2H), 1.14 (d, 3H).

LC-MS m/z (ESI) = 644.20 [M+1].

### Step 2:

### (S)-4-(trifluoromethyl)-5-((1-((5-(trifluoromethyl)thiazol-2-yl)-4,5,6,7-tetrahydropyrazolo-[1,5-a]pyrazin-2-yl)methoxy)propan-2-yl)amino)pyridazin-3(2H)-one (Compound 30)

The similar synthesis method of **compound 6** was referred to obtain **compound 30** in the form of a white solid (38 mg, 52% yield).

¹H NMR (400 MHz, DMSO-*d*₆) δ 12.47 (s, 1H), 7.91 (s, 1H), 7.84 (d, 1H), 6.31 - 6.28 (m, 1H), 6.12 (s, 1H), 4.76 (s, 2H), 4.42 (d, 2H), 4.23 - 4.15 (m, 3H), 4.06 - 4.04 (m, 2H), 3.50 (d, 2H), 1.15 (d, 3H).

¹⁹F NMR (376 MHz, DMSO-*d*₆) δ -52.92 (3F), -55.43 (3F).

LC-MS m/z (ESI) = 524.20 [M+1].

### Example 31

### (S)-4-(trifluoromethyl)-5-((1-((5-(4-(trifluoromethyl)thiazol-2-yl)-4,5,6,7-tetrahydropyrazolo[1,5-a]pyrazin-2-yl)methoxy)propan-2-yl)amino)pyridazin-3(2H)-one (Compound 31)

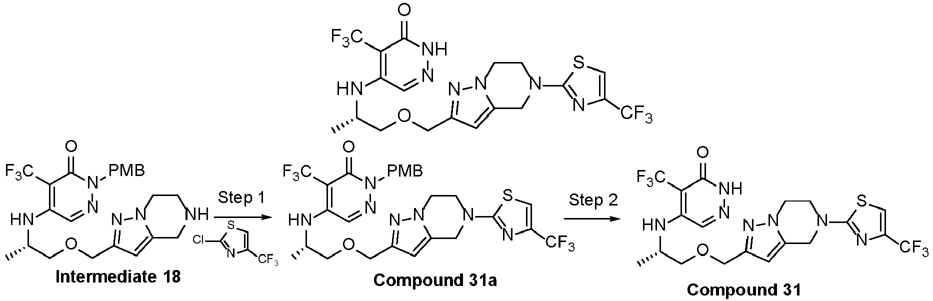

### Step 1:

### (S)-2-(4-methoxybenzyl)-4-(trifluoromethyl)-5-((1-((5-(4-(trifluoromethyl)thiazol-2-yl)-4,5,6,7-tetrahydropyrazolo[1,5-a]pyrazin-2-yl)methoxy)propan-2-yl)amino)pyridazin-3(2H)-one (Compound 31a)

The similar synthesis method of **compound 1** was referred to obtain **compound 31a** in the form of a yellow solid (120 mg, 88% yield).

¹H NMR (400 MHz, DMSO-*d*₆) δ 7.96 (s, 1H), 7.68 (d, 1H), 7.21 (d, 2H), 6.88 (d, 2H), 6.47 - 6.27 (m, 1H), 6.11 (s, 1H), 5.02 (d, 2H), 4.69 (s, 2H), 4.41 (d, 2H), 4.20 - 4.15 (m, 3H), 3.98 (t, 2H), 3.71 (s, 3H), 3.48 (d, 2H), 1.14 (d, 3H).

LC-MS m/z (ESI) = 644.20 [M+1].

### Step 2:

### (S)-4-(trifluoromethyl)-5-((1-((5-(4-(trifluoromethyl)thiazol-2-yl)-4,5,6,7-tetrahydropyrazolo[1,5-a]pyrazin-2-yl)methoxy)propan-2-yl)amino)pyridazin-3(2H)-one (Compound 31)

The similar synthesis method of **compound 6** was referred to obtain **compound 31** in the form of a white solid (58 mg, 48% yield).

¹H NMR (400 MHz, DMSO-*d*₆) δ 12.46 (s, 1H), 7.91 (s, 1H), 7.68 (d, 1H), 6.42 - 6.18 (m, 1H), 6.12 (s, 1H), 4.71 (s, 2H), 4.42 (d, 2H), 4.32 - 4.10 (m, 3H), 4.01 - 3.97 (m, 2H), 3.50 (d, 2H), 1.16 (s, 2H).

¹⁹F NMR (376 MHz, DMSO-*d*₆) δ -55.44 (3F), -63.65 (3F).

LC-MS m/z (ESI) = 524.20 [M+1].

### Example 32

### (S)-4-(trifluoromethyl)-5-(3-(((5-(5-(trifluoromethyl)pyrimidin-2-yl)-4,5,6,7-tetrahydropyrazolo[1,5-a]pyrazin-2-yl)methoxy)methyl)morpholino)pyridazin-3(2H)-one (Compound 32-1)

### (R)-4-(trifluoromethyl)-5-(3-(((5-(5-(trifluoromethyl)pyrimidin-2-yl)-4,5,6,7-tetrahydropyrazolo[1,5-a]pyrazin-2-yl)methoxy)methyl)morpholino)pyridazin-3(2H)-one (Compound 32-II)

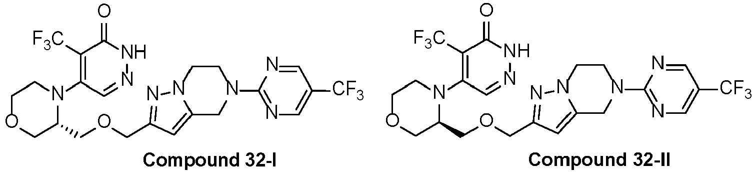

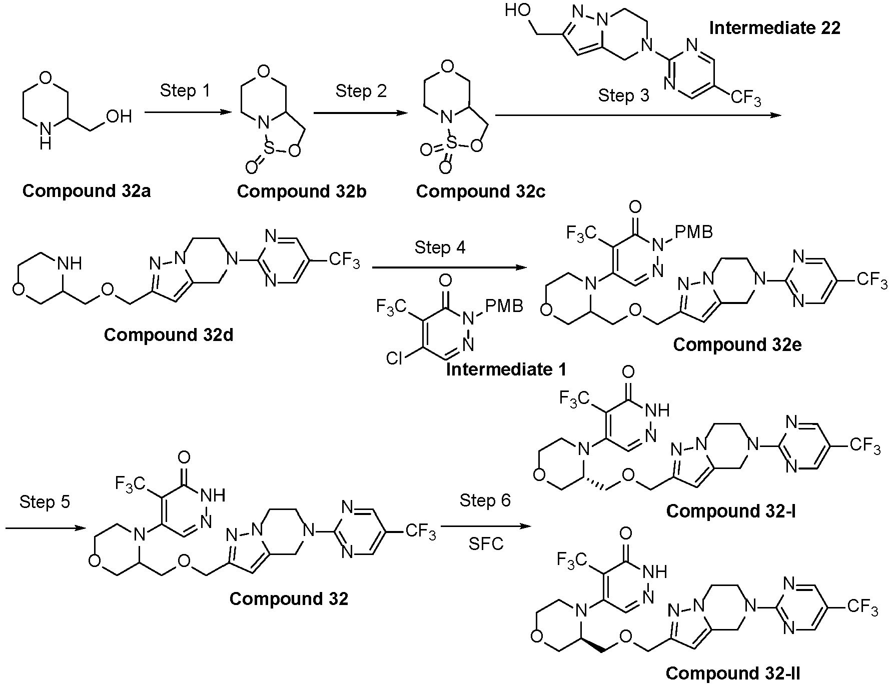

### Step 1:

### Tetrahydro-3H-[1,2,3]oxathiazolo[4,3-c][1,4]oxazine 1-oxide (Compound 32b)

**Compound 32a** (2.0 g, 17.1 mmol, 1.0 equiv) was dissolved in anhydrous dichloromethane (25 mL) in a 50 mL reaction flask, and the reaction system was added with imidazole (3.4 g, 50 mmol, 3.0 equiv) and triethylamine (5.8 mL, 42 mmol, 2.5 equiv) successively at 0 °C. Then the reaction system was added dropwise slowly with thionyl chloride (1.5 mL, 20 mmol, 1.2 equiv); after the addition was completed, the reaction mixture was reacted at room temperature for 2 h. After the reaction was completed, the reaction was quenched with saturated ammonium chloride, the reaction mixture was extracted with ethyl acetate (3 × 60), and the organic phase was concentrated to obtain crude **compound 32b** in the form of a yellow oil (2.0 g, 72% yield).

LC-MS m/z (ESI) = 164.20 [M+1].

### Step 2:

### Tetrahydro-3H-[1,2,3]oxathiazolo[4,3-c][1,4]oxazine 1,1-dioxide (Compound 32c)

Sodium periodate (215 mg, 1 mmol, 1.0 equiv) and ruthenium trichloride (11 mg, 0.05 mmol, 0.05 equiv) were added to a 50 mL reaction flask and dissolved in water (1.5 mL) and acetonitrile (1.5 mL), and the reaction mixture was slowly added with **compound 32b** (163 mg, 1 mmol, 1.0 equiv) and reacted at room temperature for 1 h. The reaction was quenched with water, the reaction mixture was extracted with ethyl acetate (3 × 60 mL), and the organic phase was concentrated to obtain crude **compound 32c** in the form of a yellow oil (130 mg, 72% yield).

LC-MS m/z (ESI) = 180.10 [M+1].

### Step 3:

### 3-(((5-(5-(Trifluoromethyl)pyrimidin-2-yl)-4,5,6,7-tetrahydropyrazolo[1,5-a]pyrazin-2-yl)methoxy)methyl)morpholine (Compound 32d)

The similar synthesis method of **intermediate 11h** was referred to obtain **compound 32d** in the form of a yellow oil (170 mg, 80% yield).

¹H NMR (400 MHz, DMSO-*d*₆) δ 8.81 (s, 2H), 6.19 (s, 1H), 5.03 (s, 2H), 4.47 - 4.31 (m, 4H), 4.17 (t, 2H), 3.76 - 3.58 (m, 3H), 3.35 - 3.24 (m, 3H), 3.16 - 3.06 (m, 1H), 2.93 - 2.82 (m, 1H), 2.76 - 2.63 (m, 2H).

LC-MS m/z (ESI) = 399.20[M+1].

### Step 4:

### 2-(4-Methoxybenzyl)-4-(trifluoromethyl)-5-(3-(((5-(5-(trifluoromethyl)pyrimidin-2-yl)-4,5,6,7-tetrahydropyrazolo[1,5-a]pyrazin-2-yl)methoxy)methyl)morpholino)pyridazin-3(2H)-one (Compound 32e)

The method of **intermediate 3a** was referred to synthesize **compound 32e** in the form of a yellow oil (110 mg, 70% yield).

LC-MS m/z (ESI) = 681.61 [M+1].

### Step 5:

### 4-(Trifluoromethyl)-5-(3-(((5-(5-(trifluoromethyl)pyrimidin-2-yl)-4,5,6,7-tetrahydropyrazolo[1,5-a]pyrazin-2-yl)methoxy)methyl)morpholino)pyridazin-3(2H)-one (Compound 32)

The similar synthesis method of **compound 6** was referred to obtain **compound 32** in the form of a white solid (53 mg, 57% yield).

LC-MS m/z (ESI) = 561.17 [M+1].

### Step 6:

### (S)-4-(trifluoromethyl)-5-(3-(((5-(5-(trifluoromethyl)pyrimidin-2-yl)-4,5,6,7-tetrahydropyrazolo[1,5-a]pyrazin-2-yl)methoxy)methyl)morpholino)pyridazin-3(2H)-one (Compound 32-1)

### (R)-4-(trifluoromethyl)-5-(3-(((5-(5-(trifluoromethyl)pyrimidin-2-yl)-4,5,6,7-tetrahydropyrazolo[1,5-a]pyrazin-2-yl)methoxy)methyl)morpholino)pyridazin-3(2H)-one (Compound 32-II)

**Compound 32** was resolved by SFC to obtain **compound 32-1** (45 mg, 38% yield, RT = 19.471min, 100% ee) and **compound 32-II** (47 mg, 39% yield, RT = 15.946min, 99.8% ee). Chiral HPLC (AS) mobile phase: n-hexane/ethanol = 95/5; column temperature: 35 °C; column pressure: 80 bar; flow rate: 1 mL/min; detector signal channel: 215 nm@4.8 nm; start/stop wavelength of diode array detector: 200-400 nm.

**Compound 32-I:** ¹H NMR (400 MHz, DMSO-*d*₆) δ 12.66 (s, 1H), 8.82 (s, 2H), 7.95 (s, 1H), 6.01 (s, 1H), 5.00 (s, 2H), 4.40 - 4.28 (m, 4H), 4.16 (t, 2H), 3.91 - 3.76 (m, 3H), 3.68 - 3.55 (m, 3H), 3.49 - 3.45 (m, 2H), 3.12 (d, 1H).

**Compound 32-II:** ¹H NMR (400 MHz, DMSO-*d*₆) δ 12.66 (s, 1H), 8.82 (s, 2H), 7.95 (s, 1H), 6.01 (s, 1H), 5.00 (s, 2H), 4.32 (d, 4H), 4.16 (t, 2H), 3.94 - 3.74 (m, 3H), 3.71 - 3.55 (m, 3H), 3.49 - 3.45 (m, 2H), 3.12 (d, 1H).

### Example 33

### (S)-5-((1-((5-(5-(difluoromethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 33)

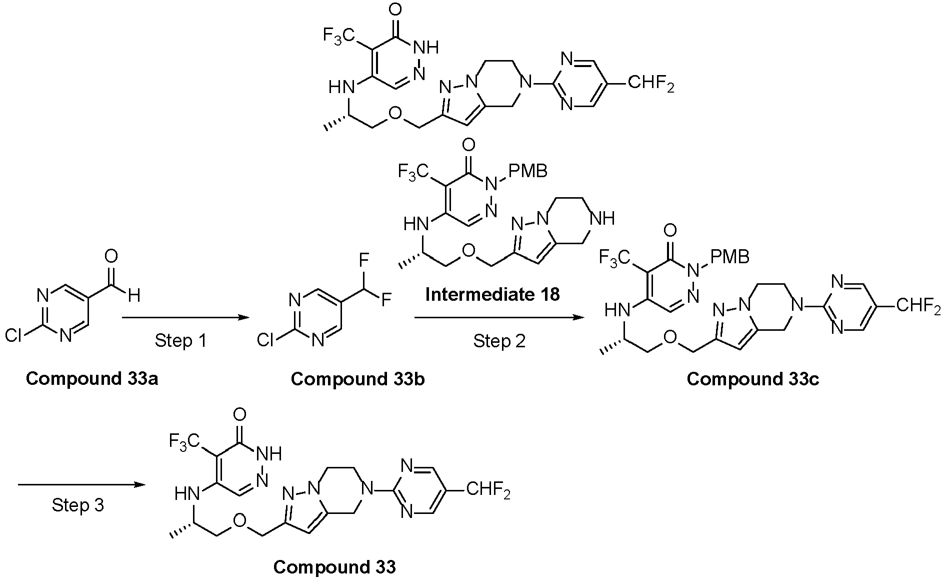

### Step 1:

### 2-Chloro-5-(difluoromethyl)pyrimidine (Compound 33b)

**Compound 33a** (1.0 g, 7.0 mmol, 1.0 equiv) was dissolved in dichloromethane (20 mL), diethylthiotrifluoride (2.33 mL, 17.6 mmol, 2.5 equiv) was added slowly at 0 °C, the ice bath was removed after 10 min, and the reaction mixture was naturally warmed to room temperature and reacted for 2 h. After the reaction was completed, the reaction was quenched by dropwise addition of saturated sodium bicarbonate solution at 0 °C (pH = 8), the reaction mixture was extracted with dichloromethane (3 × 30 mL), and the organic phases were combined, dried over anhydrous sodium sulfate, concentrated and subjected to column chromatography (petroleum ether:ethyl acetate = 2:1) to obtain **compound 33b** in the form of a yellow solid (550 mg, 52% yield).

¹H NMR (400 MHz, DMSO-*d*₆) δ 9.04 (s, 2H), 7.23 (t, 1H).

¹⁹F NMR (377 MHz, DMSO-*d*₆) δ -113.83 (2F).

LCMS m/z (ESI) = 165.2 [M+l].

### Step 2:

### (S)-5-((1-((5-(5-(difluoromethyl)pyrimidin-2-yl)-4,5,6,7-tetrahydropyrazolo[1,5-a]pyrazin-2-yl)methoxy)propan-2-yl)amino)-2-(4-methoxybenzyl)-4-(trifluoromethyl) pyridazin-3(2H)-one (Compound 33c)

The similar synthesis method of **compound 1** was referred to obtain **compound 33c** in the form of a pale yellow solid (85 mg, 67.5% yield).

¹H NMR (400 MHz, DMSO-*d*₆) δ 8.65 (s, 2H), 7.96 (s, 1H), 7.21 (d, 2H), 7.03 (t, 1H) 6.90 - 6.84 (m, 2H), 6.42 - 6.30 (m, 1H), 6.08 (s, 1H), 5.02 (s, 2H), 4.95 (d, 2H), 4.40 (d, 2H), 4.28 (dd, 2H), 4.13 (t, 2H), 3.70 (s, 3H), 3.48 (d, 2H), 2.95 (m, 1H)1.15 - 1.12 (d, 3H).

¹⁹F NMR (377 MHz, DMSO-*d*₆) δ -55.65 (3F), -109.35 (3F).

LCMS m/z (ESI) = 621.2 [M+l].

### Step 3:

### (S)-5-((1-((5-(5-(difluoromethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 33)

The similar synthesis method of **compound 6 was** referred to obtain **compound 33** in the form of a white solid (25 mg, 36% yield).

¹H NMR (400 MHz, DMSO-*d*₆) δ 12.46 (s, 1H), 8.65 (s, 2H), 7.90 (s,1H), 7.01 (t, 1H), 6.28 (dd, 1H), 6.10 (s, 1H), 4.98 (s, 2H), 4.41 (d, 2H), 4.30 (t, 2H), 4.14 (t, 3H), 3.49 (d, 2H), 1.15 (d, 3H).

¹⁹F NMR (377 MHz, DMSO-*d*₆) δ -55.43 (3F), -109.35 (2F).

LCMS m/z (ESI) = 501.2 [M+l].

### Example 34

### (S)-5-((1-((5-(5-(1,1-difluoroethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 34)

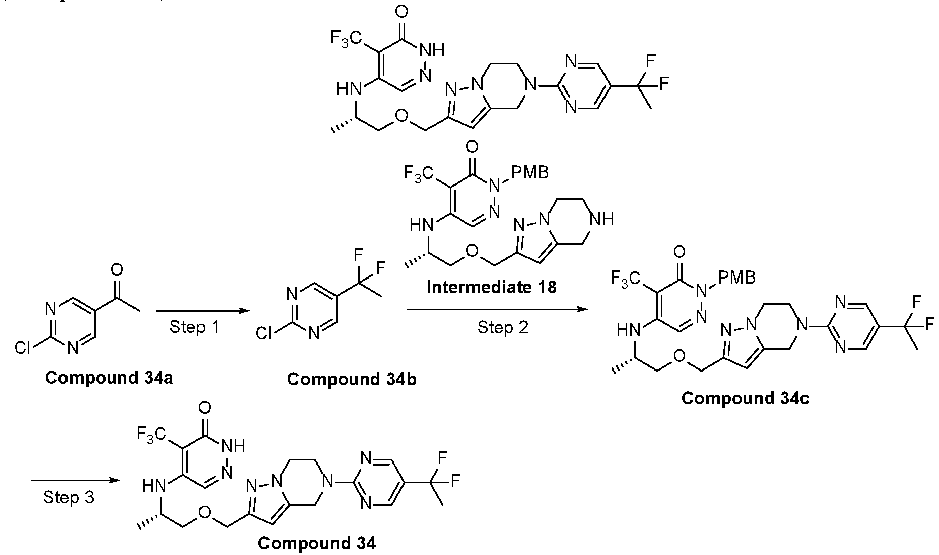

### Step 1:

### 2-Chloro-5-(1,1-difluoroethyl)pyrimidinee (Compound 34b)

The similar synthesis method of **compound 33b** was referred to obtain **compound 34b** in the form of an off-white solid (450 mg, 42.0% yield).

¹H NMR (400 MHz, DMSO-*d*₆) δ 9.05 (s, 2H), 2.07 (t, 3H).

¹⁹F NMR (377 MHz, DMSO-*d*₆) δ -85.52 (2F).

LCMS m/z (ESI) = 179.2 [M+l].

### Step 2:

### (S)-5-((1-((5-(5-(1,1-difluoroethyl)pyrimidin-2-yl)-4,5,6,7-tetrahydropyrazolo[1,5-a]pyrazin-2-yl)methoxy)propan-2-yl)amino)-2-(4-methoxybenzyl)-4-(trifluoromethyl) pyridazin-3(2H)-one (Compound 34c)

The similar synthesis method of **compound 1 was** referred to obtain **compound 34c** in the form of a dark yellow solid (230 mg, 70.0% yield).

LCMS m/z (ESI) = 635.2 [M+l].

### Step 3:

### (S)-5-((1-((5-(5-(1,1-difluoroethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 34)

The similar synthesis method of **compound 6** was referred to obtain **compound 34** in the form of a white solid (90 mg, 80.4% yield).

¹H NMR (400 MHz, DMSO-*d*₆) δ 12.46 (s, 1H), 8.80 (d, 2H), 7.90 (s, 1H), 6.28 (dd, 1H), 6.10 (s, 1H), 4.97 (s, 1H), 4.41 (d, 2H), 4.29 (d, 1H), 4.18 - 4.11 (m, 3H), 3.54 (s, 2H), 3.49 (d, 2H), 2.01 (dd, 16.1 Hz, 3H), 1.15 (d, 3H).

¹⁹F NMR (377 MHz, DMSO-*d*₆) δ -55.43 (3F), -82.61 (2F).

LCMS m/z (ESI) = 515.2 [M+l].

### Example 35

### (S)-5-((1-((5-(5-(difluoromethoxy)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 35)

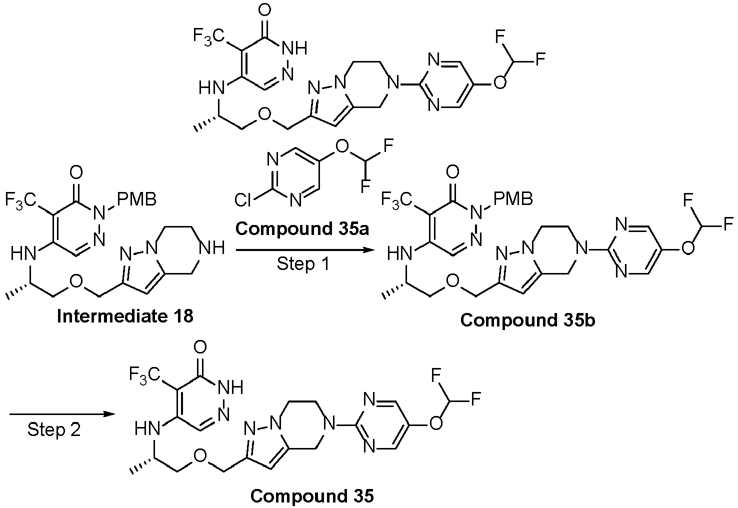

### Step 1:

### (S)-5-((1-((5-(5-(difluoromethoxy)pyrimidin-2-yl)-4,5,6,7-tetrahydropyrazolo[1,5-a]pyrazin-2-yl)methoxy)propan-2-yl)amino)-2-(4-methoxybenzyl)-4-(trifluoromethyl)pyridazin-3(2H)-one (Compound 35b)

The similar synthesis method of **compound 1** was referred to obtain **compound 35b** in the form of a yellow solid (160 mg, 62.0% yield).

LCMS m/z (ESI) = 637.4 [M+l].

### Step 2:

### (S)-5-((1-((5-(5-(difluoromethoxy)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 35)

The similar synthesis method of **compound 6** was referred to obtain **compound 35** in the form of a white solid (70 mg, 55.2% yield).

¹H NMR (400 MHz, DMSO-*d*₆) δ 12.46 (s, 1H), 8.46 (d, 2H), 7.90 (s, 1H), 7.11 (t, 1H), 6.28 (dd, 1H), 6.08 (s, 1H), 4.90 (s, 2H), 4.47 - 4.34 (m, 2H), 4.27 - 4.20 (m, 2H), 4.16 - 4.14 (m, 3H), 3.49 (d, 2H), 1.15 (d, 3H).

¹⁹F NMR (377 MHz, DMSO-*d*₆) δ -55.44 (3F), -82.06 (2F).

LCMS m/z (ESI) = 517.4 [M+l].

### Example 36

### (S)-4-(trifluoromethyl)-5-((5-(5-(5-(trifluoromethyl)pyrimidin-2-yl)-4,5,6,7-tetrahydro-2H-pyrazolo[4,3-c]pyridin-2-yl)pentan-2-yl)amino)pyridazin-3(2H)-one(Compound36)

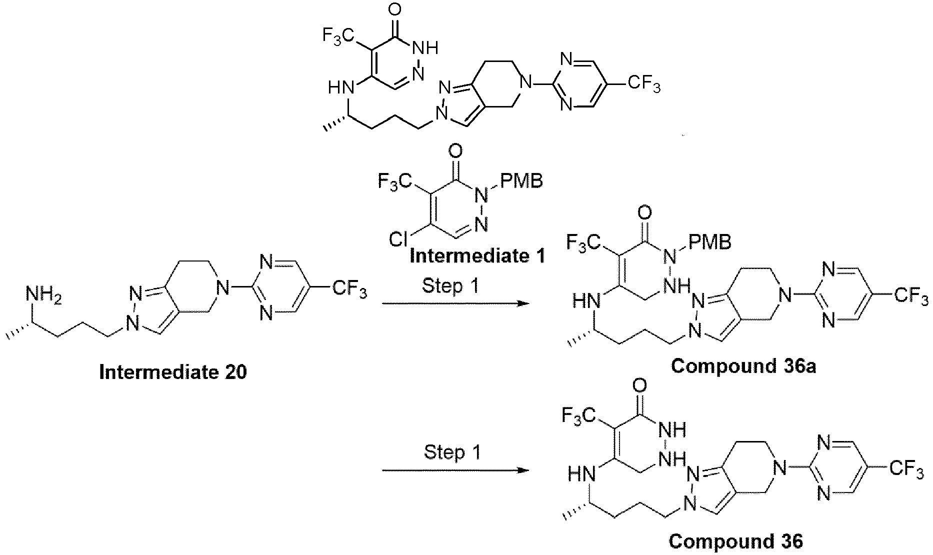

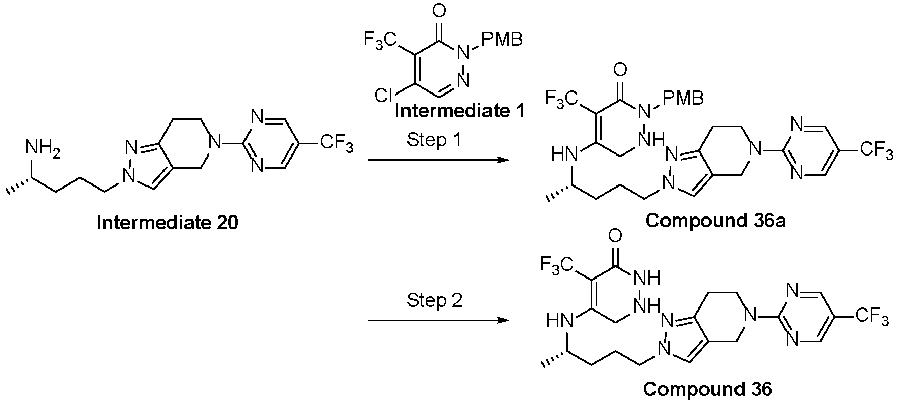

### Step 1:

### (S)-2-(4-methoxybenzyl)-4-(trifluoromethyl)-5-((5-(5-(trifluoromethyl)pyrimidin-2-yl)-4,5,6,7-tetrahydro-2H-pyrazolo[4,3-c]pyridin-2-yl)pentan-2-yl)amino)pyridazin-3(2H)-one (Compound 36a)

The similar synthesis method of **compound 19a** was referred to obtain **compound 36a** in the form of a pale yellow solid (41 mg, 59% yield).

LCMS m/z = 637.24 [M+l].

### Step 2:

### (S)-4-(trifluoromethyl)-5-((5-(5-(5-(trifluoromethyl)pyrimidin-2-yl)-4,5,6,7-tetrahydro-2H-pyrazolo[4,3-c]pyridin-2-yl)pentan-2-yl)amino)pyridazin-3(2H)-one (Compound 36)

The similar synthesis method of **compound 19** was referred to obtain **compound 36** in the form of a white solid (17 mg, 39% yield).

¹H NMR (400 MHz, DMSO-*d*₆) δ12.45 (s, 1H), 8.76 (s, 2H), 7.76(s, 1H),6.70 (d, 1H), 4.74 (d, 1H), 4.61 (d, 4H), 4.12-4.06 (m, 2H), 3.45-3.38 (m, 3H), 1.79 (d, 2H), 1.13 (dd, 3H).

LCMS m/z = 517.18 [M+l].

### Example 37

### (S)-4-(trifluoromethyl)-5-((1-((7-(5-(trifluoromethyl)pyrimidin-2-yl)-5,6,7,8-tetrahydro-[1,2,4]triazolo[1,5-a]pyrazin-2-yl)methoxy)butan-2-yl)amino)pyridazin-3(2H)-one (Compound 37)

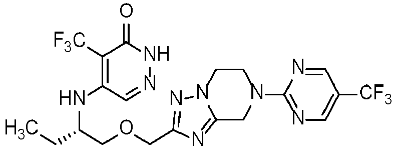

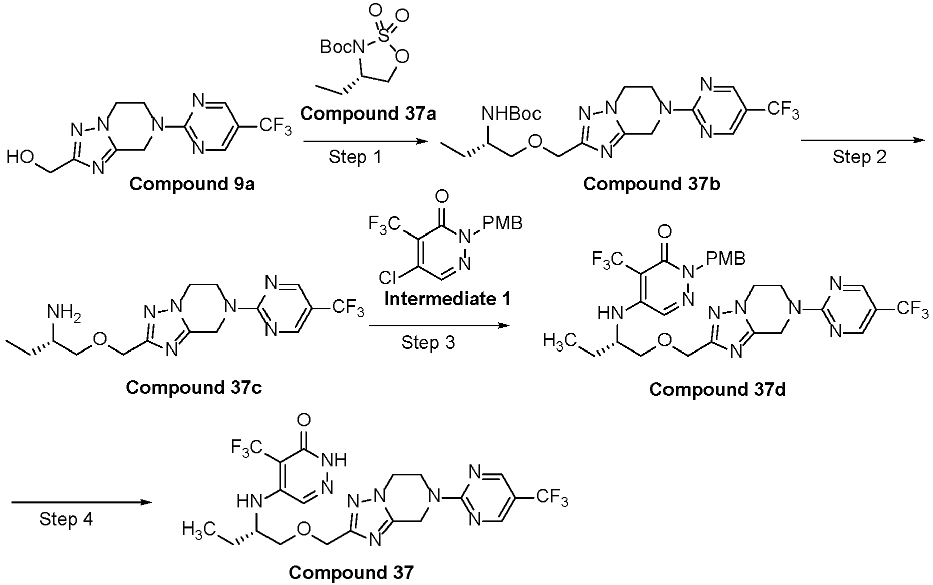

### Step 1:

### Tert-butyl(S)-(1-((7-(5-(trifluoromethyl)pyrimidin-2-yl)-5,6,7,8-tetrahydro-[1,2,4]triazolo[1,5-a]pyrazin-2-yl)methoxy)butan-2-yl)carbamate (Compound 37b)

The similar synthesis method of **intermediate 11h** was referred to obtain **compound 37b** in the form of a yellow solid (230 mg, 48% yield).

¹H NMR (400 MHz, DMSO-*d*₆) δ 12.57 (s, 1H), 8.81 (s, 3H), 6.10 (s, 1H), 5.02 (s, 2H), 4.33 (t, 2H), 4.15 (t, 2H), 3.65 (t, 4H), 3.43 (d, 2H), 3.37 (d, 2H), 2.08 (t, 2H), 1.83 - 1.69 (m, 2H).

¹⁹F NMR (377 MHz, DMSO-*d*₆) δ -59.43 (3F).

LCMS m/z (ESI) = 472.2 [M+l].

### Step 2:

### (S)-1-((7-(5-(trifluoromethyl)pyrimidin-2-yl)-5,6,7,8-tetrahydro-[1,2,4]triazolo[1,5-a]pyrazin-2-yl)methoxy)butan-2-amine (Compound 37c)

The similar synthesis method of **intermediate 11d** was referred to obtain **intermediate 37c** in the form of a white solid (190 mg, 98% yield).

LCMS m/z (ESI) = 372.2 [M+l].

### Step 3:

### (S)-2-(4-methoxybenzyl)-4-(trifluoromethyl)-5-((1-((7-(5-(trifluoromethyl)pyrimidin-2-yl)-5,6,7,8-tetrahydro-[1,2,4]triazolo[1,5-a]pyrazin-2-yl)methoxy)butan-2-yl)amino) pyridazin-3(2H)-one (Compound 37d)

The similar synthesis method of **compound 1** was referred to obtain **compound 37d** in the form of a yellow solid (350 mg, 85% yield).

LCMS m/z (ESI) = 654.4 [M+l].

### Step 4:

### (S)-4-(trifluoromethyl)-5-((1-((7-(5-(trifluoromethyl)pyrimidin-2-yl)-5,6,7,8-tetrahydro-[1,2,4]triazolo[1,5-a]pyrazin-2-yl)methoxy)butan-2-yl)amino)pyridazin-3(2H)-one (Compound 37)

The similar synthesis method of **compound 6** was referred to obtain **compound 37** in the form of a white solid (120 mg, 78% yield).

¹H NMR (400 MHz, DMSO-*d*₆) δ 12.45 (s, 1H), 8.85 (s, 2H), 7.93 (s, 1H), 6.27 (dd, 1H), 5.07 (s, 2H), 4.48 (q, 2H), 4.39 (t, 2H), 4.23 (t, 2H), 3.99 (d, 1H), 3.60 (t, 2H), 1.67 - 1.41 (m, 2H), 0.85 (t, 3H).

¹⁹F NMR (377 MHz, DMSO-*d*₆) δ -55.53 (3F), -59.43 (3F).

LCMS m/z (ESI) = 534.4 [M+l].

### Example 38

### (S)-5-((1-((5-(5-(methylsulfonyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 38)

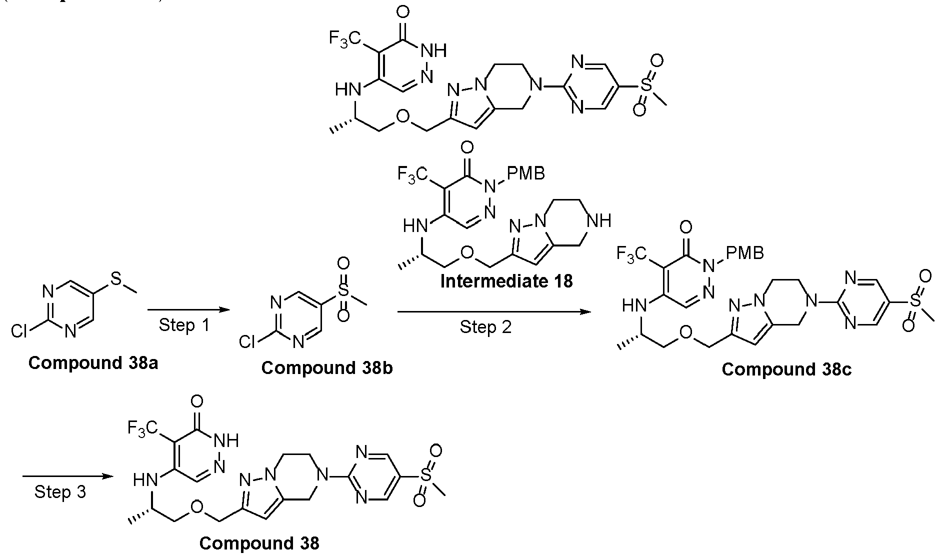

### Step 1:

### 2-Chloro-5-(methylsulfonyl)pyrimidine (Compound 38b)

**Compound 38a** (400 mg, 2.5 mmol, 1.0 equiv) was weighed into a 100 mL reaction flask and dissolved with dichloromethane (15 mL); metachloroperoxybenzoic acid (2.58 g, 14.9 mmol, 6.0 equiv) was added under an ice bath, the ice bath was removed and the reaction mixture was reacted at room temperature for 4 h. The reaction was quenched with saturated sodium thiosulfate (10 mL), the pH of the reaction mixture was adjusted to 8 with saturated sodium bicarbonate, solvent was extracted with dichloromethane (3 × 140 mL), and the organic phases were combined, dried over anhydrous sodium sulfate, concentrated and subjected to column chromatography (petroleum ether:ethyl acetate = 1:2) to obtain **compound 38b** in the form of a white solid (410 mg, 86% yield).

LCMS m/z (ESI) = 193.3 [M+l].

### Step 2:

### (S)-2-(4-methoxybenzyl)-5-((1-((5-(5-(methylsulfonyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 38c)

The similar synthesis method of **compound 1** was referred to obtain **compound 38c** in the form of a yellow solid (340 mg, 85% yield).

LCMS m/z (ESI) = 649.4 [M+l].

### Step 3:

### (S)-5-((1-((5-(5-(methylsulfonyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 38)

The similar synthesis method of **compound 6** was referred to obtain **compound 38** in the form of a white solid (80 mg, 40% yield).

¹H NMR (400 MHz, DMSO-*d*₆) δ 12.46 (s, 1H), 8.82 (s, 2H), 7.90 (s, 1H), 6.28 (dd, 1H), 6.12 (s, 1H), 5.05 (s, 2H), 4.41 (d, 2H), 4.36 (t, 2H), 4.17 (t, 3H), 3.49 (d, 2H), 3.25 (s, 3H), 1.15 (d, 3H).

¹⁹F NMR (377 MHz, DMSO-*d*₆) δ -55.44 (3F).

LCMS m/z (ESI) = 529.4 [M+l].

### Example 39

### (S)-5-((1-((5-(5-(methylthio)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 39)

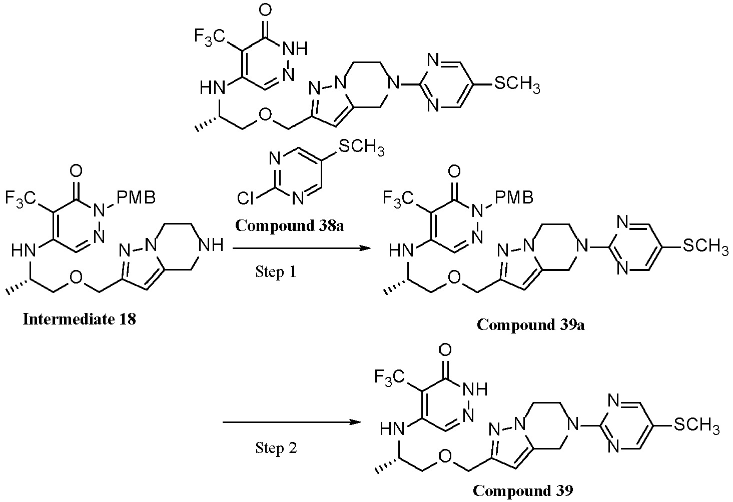

### Step 1:

### (S)-2-(4-methoxybenzyl)-5-((1-((5-(5-(methylthio)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 39a)

The similar synthesis method of **compound 1** was referred to obtain **compound 39a** in the form of a yellow solid (340 mg, 45% yield).

LCMS m/z (ESI) = 617.2 [M+l].

### Step 2:

### (S)-5-((1-((5-(5-(methylthio)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 39)

The similar synthesis method of **compound 6** was referred to obtain **compound 39** in the form of a white solid (75 mg, 83% yield).

¹H NMR (400 MHz, DMSO-*d*₆) δ 12.45 (s, 1H), 8.51 (s, 2H), 7.90 (s, 1H), 6.29 (dd, 1H), 6.08 (s, 1H), 4.91 (s, 2H), 4.44 - 4.35 (m, 2H), 4.23 (dd, 2H), 4.17 (d, 1H), 4.12 (t, 2H), 3.49 (d, 2H), 2.40 (s, 3H), 1.15 (d, 3H).

¹⁹F NMR (377 MHz, DMSO-*d*₆) δ -55.43 (3F).

LCMS m/z (ESI) = 497.2 [M+l].

### Example 40

### (S)-5-((1-((5-(pyrazolo[1,5-a]pyrimidin-5-yl)-4,5,6,7-tetrahydropyrazolo[1,5-a]pyrazin-2-yl)methoxy)propan-2-yl)amino)-4-(trifluoromethyl)pyridazin-3(2H)-one (Compound 40)

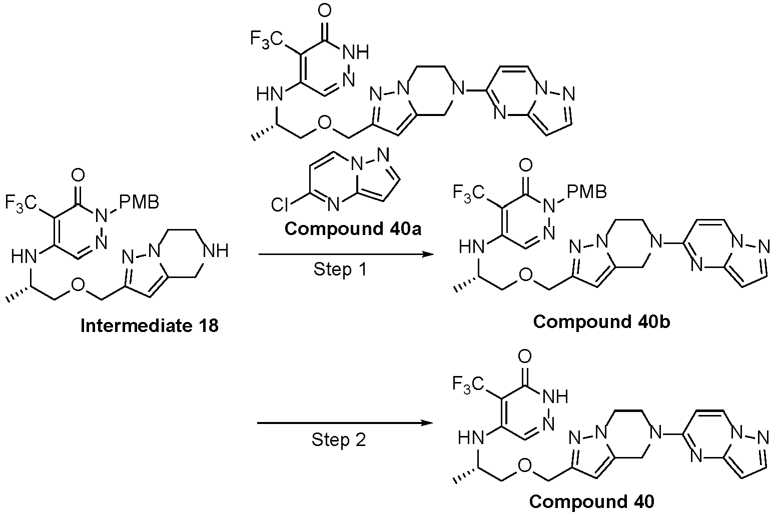

### Step 1:

### (S)-2-(4-methoxybenzyl)-5-((1-((5-(pyrazolo[1,5-a]pyrimidin-5-yl)-4,5,6,7-tetrahydropyrazolo[1,5-a]pyrazin-2-yl)methoxy)propan-2-yl)amino)-4-(trifluoromethyl)pyridazin-3(2H)-one (Compound 40b)

The similar synthesis method of **compound 1** was referred to obtain **compound 40b** in the form of a yellow solid (290 mg, 35% yield).

LCMS m/z (ESI) = 610.3 [M+l].

### Step 2:

### (S)-5-((1-((5-(pyrazolo[1,5-a]pyrimidin-5-yl)-4,5,6,7-tetrahydropyrazolo[1,5-a]pyrazin-2-yl)methoxy)propan-2-yl)amino)-4-(trifluoromethyl)pyridazin-3(2H)-one (Compound 40)

The similar synthesis method of **compound 6** was referred to obtain **compound 40** in the form of a white solid (24 mg, 34% yield).

¹H NMR (400 MHz, DMSO-*d*₆) δ 12.47 (s, 1H), 8.76 (d, 1H), 7.90 (d, 2H), 6.87 (d, 1H), 6.29 (dd, 1H), 6.18 - 6.00 (m, 2H), 4.88 (s, 2H), 4.41 (d, 2H), 4.16 (s, 5H), 3.49 (d, 2H), 1.15 (d, 3H).

¹⁹F NMR (377 MHz, DMSO-*d*₆) δ -55.42 (3F).

LCMS m/z (ESI) = 490.3 [M+l].

### Example 41

### (S)-2-(2-((2-((6-oxo-5-(trifluoromethyl)-1,6-dihydropyridazin-4-yl)amino)propoxy)methyl)-6,7-dihydropyrazolo[1,5-a]pyrazin-5(4H)-yl)-5-(trifluoromethyl)nicotinonitrile (Compound 41)

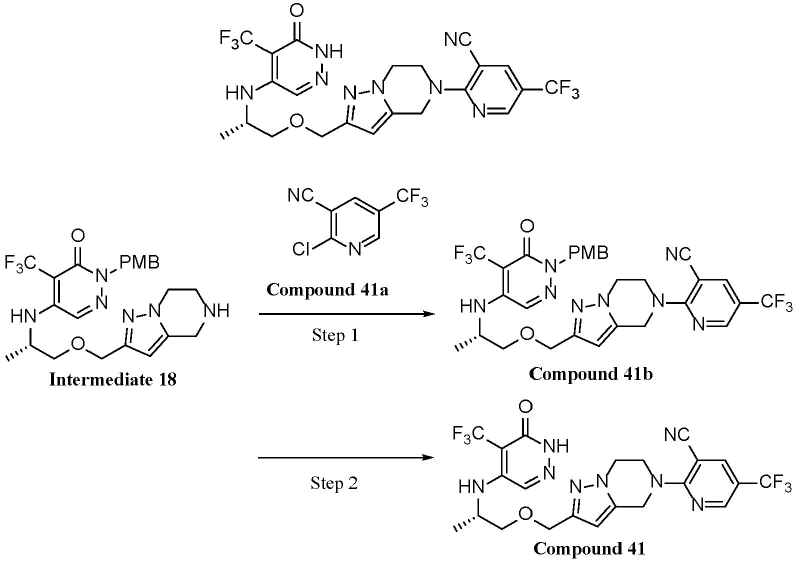

### Step 1:

### (S)-2-(2-((2-((1-(4-methoxybenzyl)-6-oxo-5-(trifluoromethyl)-1,6-dihydropyridazin-4-yl)amino)propoxy)methyl)-6,7-dihydropyrazolo[1,5-a]pyrazin-5(4H)-yl)-5-(trifluoro methyl)nicotinonitrile (Compound 41b)

The similar synthesis method of **compound 1** was referred to obtain **compound 41b** in the form of a yellow solid (380 mg, 74% yield).

LCMS m/z (ESI) = 663.3 [M+l].

### Step 2:

### (S)-2-(2-((2-((6-oxo-5-(trifluoromethyl)-1,6-dihydropyridazin-4-yl)amino)propoxy)methyl)-6,7-dihydropyrazolo[1,5-a]pyrazin-5(4H)-yl)-5-(trifluoromethyl)nicotinonitrile (Compound 41)

The similar synthesis method of **compound 6** was referred to obtain **compound 41** in the form of a white solid (83 mg, 42% yield).

¹H NMR (400 MHz, DMSO-*d*₆) δ 12.46 (s, 1H), 8.77 (s, 1H), 8.64 (d, 1H), 7.91 (s, 1H), 6.41 - 6.23 (m, 1H), 6.12 (s, 1H), 4.99 (s, 2H), 4.46 - 4.37 (m, 2H), 4.27 (s, 4H), 4.16 (s, 1H), 3.50 (d, 2H), 1.15 (d, 3H).

¹⁹F NMR (377 MHz, DMSO-*d*₆) δ -55.44 (3F), -59.80 (3F).

LCMS m/z (ESI) = 543.2 [M+l].

### Example 42

### 5-(((S)-1-(((S)-7-methyl-5-(5-(trifluoromethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 42-1)

### 5-((S)-1-((R)-7-methyl-5-(5-(trifluoromethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 42-II)

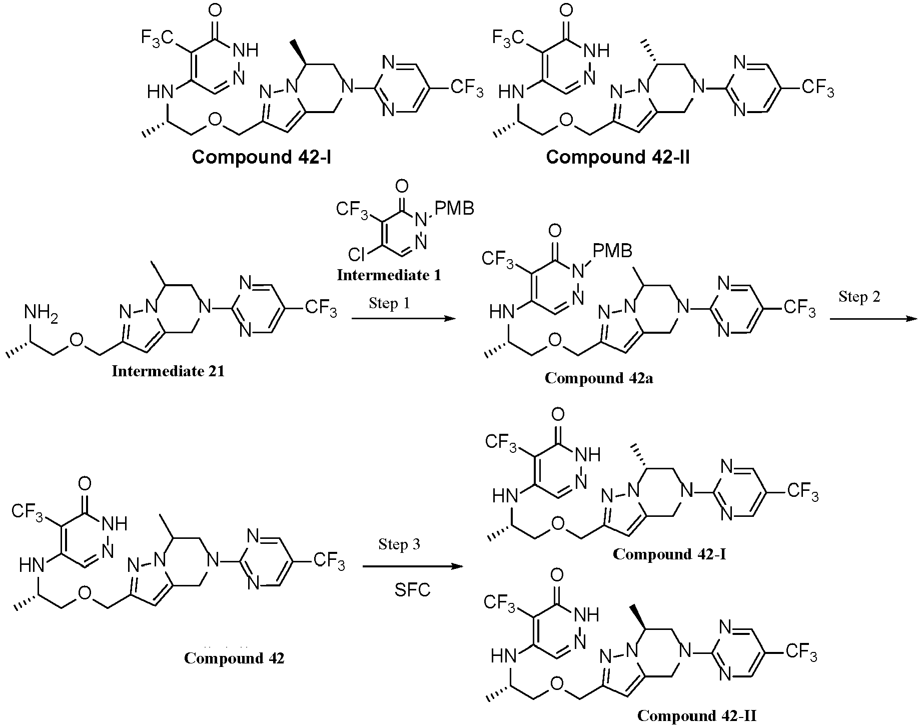

### Step 1:

### 2-(4-Methoxybenzyl)-5-(((2S)-1-((7-methyl-5-(5-(trifluoromethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 42a)

The similar synthesis method of **compound 19a** was referred to obtain **compound 42a** in the form of a pale yellow solid (264 mg, 52% yield).

LCMS m/z = 653.23 [M+l].

### Step 2:

### 5-(((2S)-1-((7-methyl-5-(5-(trifluoromethyl)pyrimidin-2-yl)-4,5,6,7-tetrahydropyrazolo[1,5-a]pyrazin-2-yl)methoxy)propan-2-yl)amino)-4-(trifluoromethyl)pyridazin-3(2H)-one (Compound 42)

The similar synthesis method of **compound 19** was referred to obtain **compound 42** in the form of a white solid (114 mg, 65% yield).

LCMS m/z = 533.18 [M+l].

### Step 3:

**Compound 42** was resolved by SFC to obtain **compound 42-I** (45 mg, 38% yield, RT = 19.471min, 100% ee) and **compound 42-II** (47 mg, 39% yield, RT = 15.946min, 99.8% ee). Chiral HPLC (AS) mobile phase: n-hexane/ethanol = 95/5; column temperature: 35 °C; column pressure: 80 bar; flow rate: 1 mL/min; detector signal channel: 215 nm@4.8 nm; start/stop wavelength of diode array detector: 200-400 nm.

**Compound 42-I:** ¹H NMR (400 MHz, DMSO-*d*₆) δ 12.46 (s, 1H), 8.80 (s, 2H), 7.89 (s, 1H), 6.28 (dd, 1H), 6.08 (s, 1H), 5.11 - 4.90 (m, 2H), 4.45 - 4.33 (m, 4H), 4.20 - 4.10 (m, 1H), 4.05 (dd, 1H), 3.52 - 3.47 (m, 2H), 1.40 (d, 3H), 1.15 (d, 3H).

**Compound 42-II:** ¹H NMR (400 MHz, DMSO-*d*₆) δ 12.47 (s, 1H), 8.82 (s, 2H), 7.91 (s, 1H), 6.29 (dd, 1H), 6.09 (s, 1H), 5.01-4.89 (m, 2H), 4.48 - 4.33 (m, 4H), 4.21 - 4.13 (m, 1H), 4.12 - 4.02 (m, 1H), 3.50 (d, 2H), 1.40 (d, 3H), 1.15 (d, 3H).

### Example 43

### (S)-5-((1-((5-(imidazo[1,2-a]pyrazin-8-yl)-4,5,6,7-tetrahydropyrazolo[1,5-a]pyrazin-2-yl)methoxy)propan-2-yl)amino)-4-(trifluoromethyl)pyridazin-3(2H)-one (Compound 43)

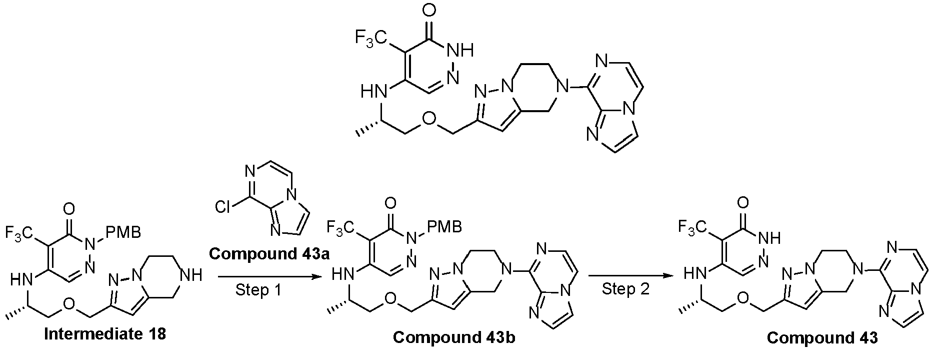

### Step 1:

### (S)-5-((1-((5-(imidazo[1,2-a]pyrazin-8-yl)-4,5,6,7-tetrahydropyrazolo[1,5-a]pyrazin-2-yl)methoxy)propan-2-yl)amino)-2-(4-methoxybenzyl)-4-(trifluoromethyl)pyridazin-3(2H)-one (Compound 43b)

The similar synthesis method of **compound 1** was referred to obtain crude **compound 43b** in the form of a yellow solid (70 mg, 29% yield).

LCMS m/z (ESI) = 610.6 [M+l].

### Step 2:

### (S)-5-((1-((5-(imidazo[1,2-a]pyrazin-8-yl)-4,5,6,7-tetrahydropyrazolo[1,5-a]pyrazin-2-yl)methoxy)propan-2-yl)amino)-4-(trifluoromethyl)pyridazin-3(2H)-one (Compound 43) (S)

The similar synthesis method of **compound 6** was referred to obtain **compound 43** in the form of a white solid (25 mg, 44% yield).

¹H NMR (400 MHz, DMSO-*d*₆) δ 12.47 (s, 1H), 8.02 (d, 1H), 8.00 (d, 1H), 7.91 (s, 1H), 7.63 (d, 1H), 7.40 (d, 1H), 6.30 (dd, 1H), 6.09 (s, 1H), 5.39 - 5.28 (m, 2H), 4.81 - 4.64 (m, 2H), 4.44 - 4.33 (m, 2H), 4.20 (t, 2H), 4.15 (m, 1H), 3.49 (d, 2H), 1.15 (d, 3H).

¹⁹F NMR (377 MHz, DMSO-*d*₆) δ -55.43 (3F).

LCMS m/z (ESI) = 490.4 [M+l].

### Example 44

### (S)-4-bromo-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 44)

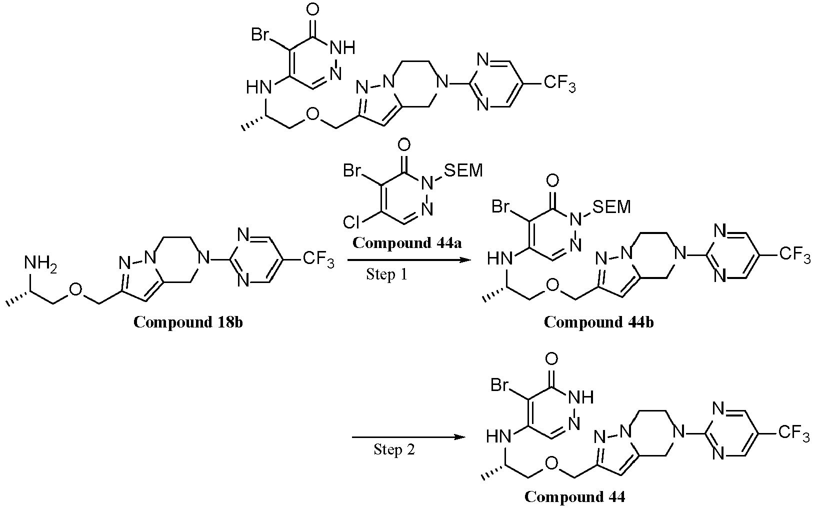

### Step 1:

### (S)-4-bromo-5-((1-((5-(5 -(trifluoromethyl)pyrimidin-2-yl)-4,5,6,7-tetrahydropyrazolo[1,5-a]pyrazin-2-yl)methoxy)propan-2-yl)amino)-2-((2-(trimethylsilyl)ethoxy)methyl)pyridazin-3(2H)-one (Compound 44b)

The similar synthesis of compound 3a was referred to obtain compound 44b in the form of a yellow oil (450 mg, 27% yield).

LCMS m/z (ESI) = 659.5 [M+l].

### Step 2:

### (S)-4-bromo-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 44)

**Compound 44b** (55 mg, 0.083 mmol) was added to 3 mL hydrochloric acid/dioxane (4 mol/L), and the reaction mixture was reacted at room temperature for 3 h. After the reaction was completed, the reaction mixture was concentrated and subjected to column chromatography (DCM/MeOH = 20/1) to obtain **compound 44** in the form of a white solid (20 mg, 45% yield).

¹H NMR (400 MHz, DMSO-*d*₆) δ 12.52 (s, 1H), 8.81 (s, 2H), 7.77 (s, 1H), 6.11 (s, 1H), 5.73 (d, 1H), 5.01 (s, 2H), 4.40 (s, 2H), 4.32 (t, 2H), 4.16 (t, 2H), 4.08( m, 1H), 3.48 (d, 2H), 3.48 (d, 2H), 1.16 (d, 3H).

¹⁹F NMR (377 MHz, DMSO-*d*₆) δ -59.37 (3F).

LCMS m/z (ESI) = 530.3 [M+l].

### Example 45

### (S)-4-cyclopropyl-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 45)

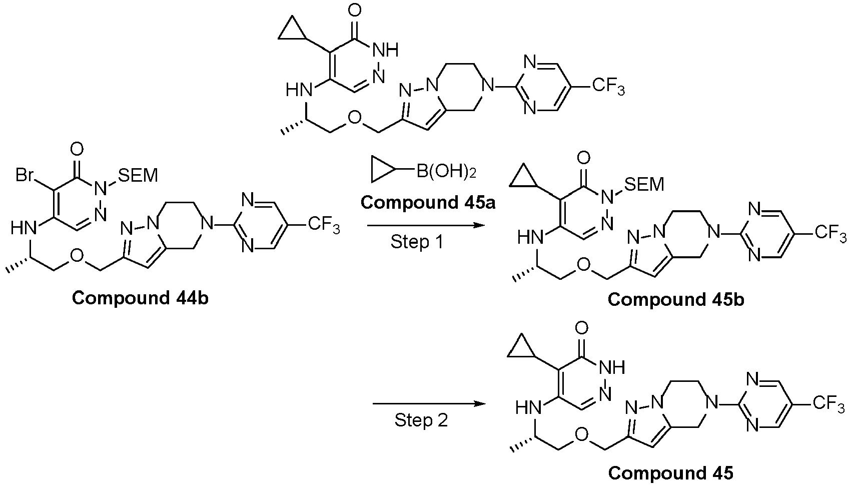

### Step 1:

### (S)-4-cyclopropyl-5-((1-((5-(5-(trifluoromethyl)pyrimidin-2-yl)-4,5,6,7-tetrahydropyrazolo[1,5-a]pyrazin-2-yl)methoxy)propan-2-yl)amino)-2-((2-(trimethylsilyl)ethoxy)methyl)pyridazin-3(2H)-one (Compound 45b)

**Compound 44b** (150 mg, 0.23 mmol), **compound 45a** (39 mg, 0.46 mmol), Pd(OAc)₂ (4.8 mg, 0.023 mmol), tricyclohexylphosphorus (13 mg, 0.046 mmol) and potassium phosphate (138 mg, 0.69 mmol) were weighed into a 20 mL reaction flask. The reaction mixture was dissolved in dioxane (3 mL) and water (0.3 mL), and stirred at 100 °C for 3 h. After the reaction was completed, the reaction mixture was extracted with ethyl acetate (2 × 30 mL), the organic phases were combined, dried over anhydrous sodium sulfate and concentrated, and the crude product was purified by silica gel column (petroleum ether:ethyl acetate = 1:5) to obtain **compound 45b** in the form of a yellow solid (90 mg, 64% yield).

LCMS m/z (ESI) = 621.7 [M+l].

### Step 2:

### (S)-4-cyclopropyl-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 45)

The synthesis method of **compound 44** was referred to obtain **compound 45** in the form of a white solid (22 mg, 28% yield).

¹H NMR (400 MHz, DMSO-*d*₆) δ 11.97 (s, 1H), 8.81 (s, 2H), 7.68 (s, 1H), 6.14 (s, 1H), 5.42 (d, 1H), 5.01 (s, 2H), 4.41 (d, 2H), 4.33 (t, 2H), 4.16 (t, 2H), 3.99 - 3.90 (m, 1H), 3.46 (d, 2H), 1.23 (d, 1H), 1.15 (d, 3H), 0.79 (d, 2H), 0.70 - 0.60 (m, 2H).

¹⁹F NMR (377 MHz, DMSO-*d*₆) δ -59.38 (3F).

LCMS m/z (ESI) = 491.5 [M+l].

### Example 46

### (S)-4-methyl-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 46)

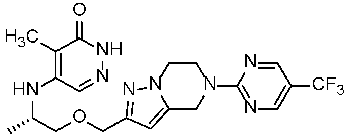

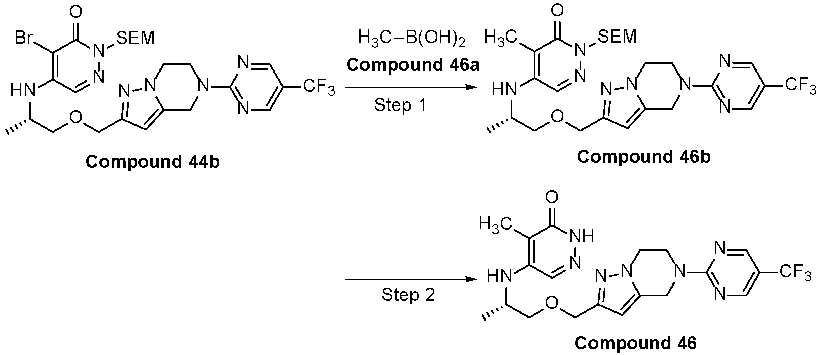

### Step 1:

### (S)-4-methyl-5-((1-((5-(5-(trifluoromethyl)pyrimidin-2-yl)-4,5,6,7-tetrahydropyrazolo[1,5-a]pyrazin-2-yl)methoxy)propan-2-yl)amino)-2-((2-(trimethylsilyl)ethoxy)methyl)pyridazin-3(2H)-one (Compound 46b)

The preparation method of compound **45b** was referred to obtain **compound 46b** in the form of a yellow solid (50 mg, 37% yield).

LCMS m/z (ESI) = 595.6 [M+l].

### Step 2:

### (S)-4-methyl-5-((1-((5-(5-(trifluoromethyl)pyrimidin-2-yl)-4,5,6,7-tetrahydropyrazolo[1,5-a]pyrazin-2-yl)methoxy)propan-2-yl)amino)pyridazin-3(2H)-one (Compound 45)

The preparation method of **compound 44** was referred to obtain **compound 46** in the form of a white solid (15 mg, 38% yield).

¹H NMR (400 MHz, DMSO-*d*₆) δ 12.09 (s, 1H), 8.81 (d, 2H), 7.69 (s, 1H), 6.12 (s, 1H), 5.36 (d, 1H), 5.01 (s, 2H), 4.39 (d, 2H), 4.33 (t, 2H), 4.16 (t, 2H), 3.92 (m, 1H), 3.53 - 3.37 (m, 2H), 1.78 (s, 3H), 1.14 (d, 3H).

¹⁹F NMR (377 MHz, DMSO-*d*₆) δ -59.37 (3F).

LCMS m/z (ESI) = 465.4 [M+l].

### Example 47

### (S)-5-((1-((7-(5-(methylsulfonyl)pyridin-2-yl)-5,6,7,8-tetrahydro-[1,2,4]triazolo[1,5-a]pyrazin-2-yl)methoxy)propan-2-yl)amino)-4-(trifluoromethyl)pyridazin-3(2H)-one (Compound 47)

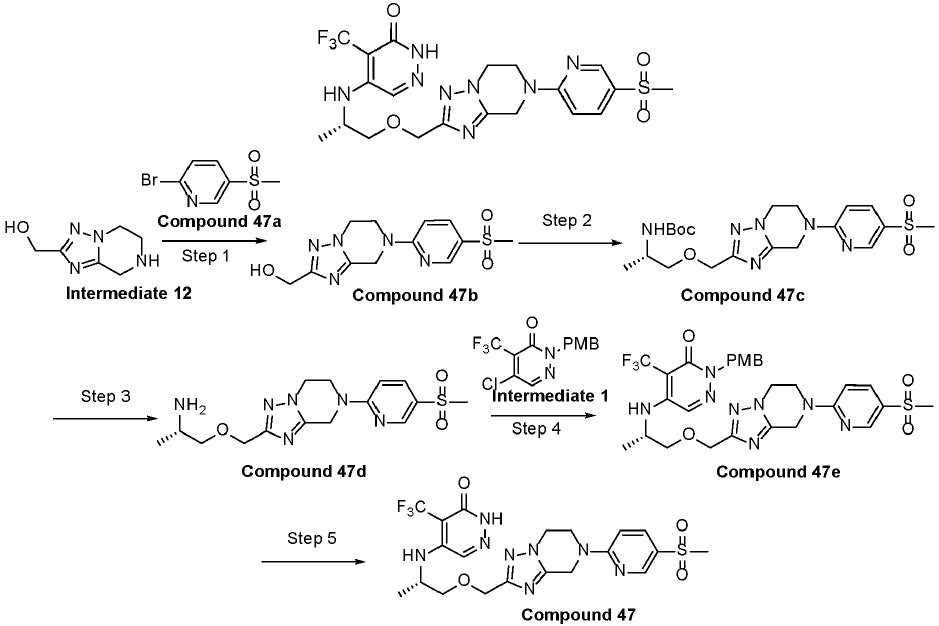

### Step 1:

### (7-(5-(Methylsulfonyl)pyridin-2-yl)-5,6,7,8-tetrahydro-[1,2,4]triazolo[1,5-a]pyrazin-2-yl)methanol (Compound 47b)

The synthesis method of **compound 9a** was referred to obtain **compound 47b** in the form of a white solid (80 mg, 20% yield).

LC-MS m/z (ESI) = 310.09 [M+1].

### Step 2:

### Tert-butyl-(1-((7-(5-(methylsulfonyl)pyridin-2-yl)-5,6,7,8-tetrahydro-[1,2,4]triazolo[1,5-a]pyrazin-2-yl)methoxy)propan-2-yl)carbamate (Compound 47c)

The synthesis method of **intermediate 11h** was referred to obtain **compound 47c** in the form of a yellow oil (60 mg, 35% yield).

LC-MS m/z (ESI) = 467.20[M+1].

### Step 3:

### (S)-1-((7-(5-(methylsulfonyl)pyridin-2-yl)-5,6,7,8-tetrahydro-[1,2,4]triazolo[1,5-a]pyrazin-2-yl)methoxy)propan-2-amine (Compound 47d)

The synthesis method of **intermediate 11i** was referred to obtain **compound 47d** in the form of a white solid (40 mg, 90% yield), and the crude product was used directly in the next step.

LC-MS m/z (ESI) = 367.20 [M+1].

### Step 4:

### (S)-2-(4-methoxybenzyl)-5-((1-((7-(5-(methylsulfonyl)pyridin-2-yl)-5,6,7,8-tetrahydro-[1,2,4]triazolo[1,5-a]pyrazin-2-yl)methoxy)propan-2-yl)amino)-4-(trifluoromethyl)pyridazin-3(2H)-one (Compound 47e)

The synthesis method of **intermediate 3a** was referred to obtain **compound 47e** in the form of a yellow oil (22 mg, 35% yield).

LC-MS m/z (ESI) = 649.21 [M+1].

### Step 5:

### (S)-5-((1-((7-(5-(methylsulfonyl)pyridin-2-yl)-5,6,7,8-tetrahydro-[1,2,4]triazolo[1,5-a]pyrazin-2-yl)methoxy)propan-2-yl)amino)-4-(trifluoromethyl)pyridazin-3(2H)-one (Compound 47)

The synthesis method of **compound 6** was referred to obtain **compound 47** in the form of a white solid (10 mg, 55% yield).

¹H NMR (400 MHz, DMSO-*d*₆) δ 12.47 (s, 1H), 8.59 (d, 1H), 8.02 (dd, 1H), 7.91 (s, 1H), 7.21 (d, 1H), 6.37 - 6.29 (m, 1H), 4.96 (s, 2H), 4.54 - 4.44 (m, 2H), 4.32 - 4.12 (m, 5H), 3.57 (d, 2H), 1.15 (d, 3H).

¹⁹F NMR (376 MHz, DMSO-*d*₆) δ -55.48 (3F).

LC-MS m/z (ESI) = 529.21 [M+1].

### Example 48

### (S)-4-(trifluoromethyl)-5-((1-((5-(trifluoromethyl)pyrimidin-2-yl)-4,5,6,7-tetrahydropyrazolo[1,5-a]pyrazin-2-yl)methoxy)propan-2-yl)oxy)pyridazin-3(2H)-one (Compound 48)

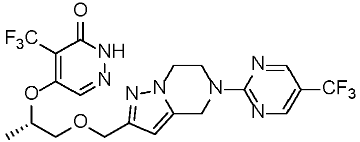

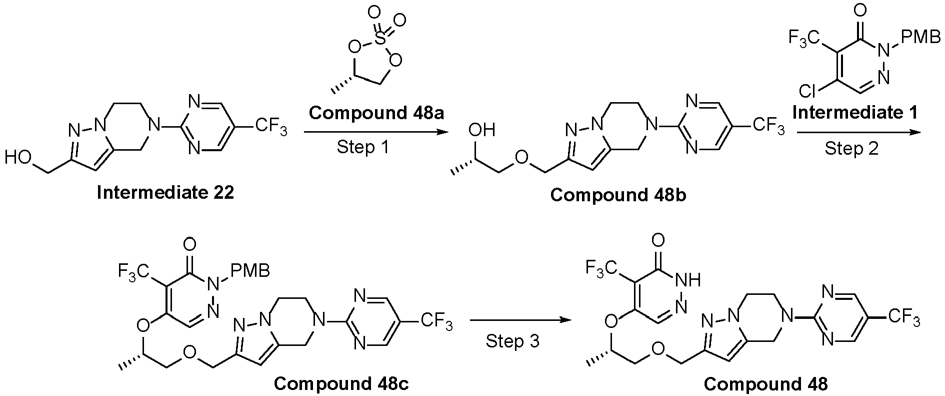

### Step 1:

### (S)-1-((5-(5-(trifluoromethyl)pyrimidin-2-yl)-4,5,6,7-tetrahydropyrazolo[1,5-a]pyrazin-2-yl)methoxy)propan-2-ol (Compound 48b)

**Intermediate 22** (1.0 g, 3.33 mmol) was weighed into a 100 mL reaction flask and dissolved in tetrahydrofuran (20 mL). Under nitrogen atmosphere, the reaction mixture was added with sodium hydride (60%, 334 mg, 8.3 mmol) at 0 °C, stirred for 30 min and then added with compound **48a** (552 mg, 4.0 mmol); after the addition was completed, the reaction mixture was reacted at room temperature overnight. A hydrochloric acid solution (10 mL, 2 N) was added to the reaction mixture under an ice bath; after the addition was completed, the reaction mixture was reacted at 70 °C and stirred for 2 h. The reaction mixture was cooled to room temperature, added with saturated sodium bicarbonate solution to adjust the pH of the reaction mixture to neutral and extracted with ethyl acetate (3 × 40 mL). The organic phases were combined, dried over anhydrous Na₂SO₄ and concentrated to prepare a sample, and the sample was subjected to column chromatography (petroleum ether:ethyl acetate = 1:1) to obtain **compound 48b** in the form of a pale yellow solid (750 mg, 63% yield).

LCMS m/z (ESI) = 358.3 [M+l].

### Step 2:

### (S)-2-(4-methoxybenzyl)-4-(trifluoromethyl)-5-((1-((5-(5-(trifluoromethyl)pyrimidin-2-yl)-4,5,6,7-tetrahydropyrazolo[1,5-a]pyrazin-2-yl)methoxy)propan-2-yl)oxy)pyridazin-3(2H)-one (Compound 48c)

**Compound 48b** (150 mg, 0.42 mmol) and **intermediate 1** (160 mg, 0.51 mmol) were weighed into a 25 mL reaction flask and dissolved in dichloromethane (5 mL). To the reaction flask was added sodium *tert*-butoxide (60 mg, 0.63 mmol) at 0 °C; after the addition was completed, the reaction mixture was stirred at this temperature for 1 h. After the reaction was completed, the reaction mixture was added with water to quench the reaction, extract with ethyl acetate (40 mL × 3), dry over anhydrous sodium sulfate and concentrated. The crude product was subjected to column chromatography (petroleum ether:ethyl acetate = 1:1) to obtain **compound 48c** in the form of a yellow solid (160 mg, 60% yield).

¹H NMR (400 MHz, DMSO-*d*₆) δ 8.81 (s, 2H), 8.33 (s, 1H), 7.33 - 7.18 (m, 2H), 6.96 - 6.82 (m, 2H), 6.04 (s, 1H), 5.13 (d, 2H), 4.97 (s, 2H), 4.38 (d, 2H), 4.45 - 4.35(t, 3H), 4.13 (t, 2H), 3.71 (s, 3H), 3.66 - 3.47 (m, 2H), 1.25 (d, 3H).

LCMS m/z (ESI) = 641.10 [M+l].

### Step 3:

### (S)-4-(trifluoromethyl)-5-((1-((5-(trifluoromethyl)pyrimidin-2-yl)-4,5,6,7-tetrahydropyrazolo[1,5-a]pyrazin-2-yl)methoxy)propan-2-yl)oxy)pyridazin-3(2H)-one (Compound 48)

The synthesis method of **compound 6** was referred to obtain **compound 48** in the form of a white solid (83 mg, 57% yield).

¹H NMR (400 MHz, DMSO-*d*₆) δ 13.23 (s, 1H), 8.81 (d, 2H), 8.27 (s, 1H), 6.06 (s, 1H), 5.16 (s, 1H), 5.00 (s, 2H), 4.39 (d, 2H), 4.32 (t, 2H), 4.15 (t, 2H), 3.62 (dd, 1H), 3.52 (dd, 1H), 1.26 (d, 3H).

¹⁹F NMR (376 MHz, DMSO-*d*₆) δ -59.38 (3F).

LCMS m/z (ESI) = 521.10 [M+l].

### Example 49

### (S)-4-(trifluoromethyl)-5-((1-((5-(trifluoromethyl)pyrimidin-2-yl)-4,5,6,7-tetrahydro pyrazolo[1,5-a]pyrazin-2-yl)methoxy)propan-2-yl-1,1-d₂)amino)pyridazin-3(2H)-one (Compound 49)

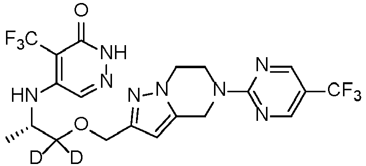

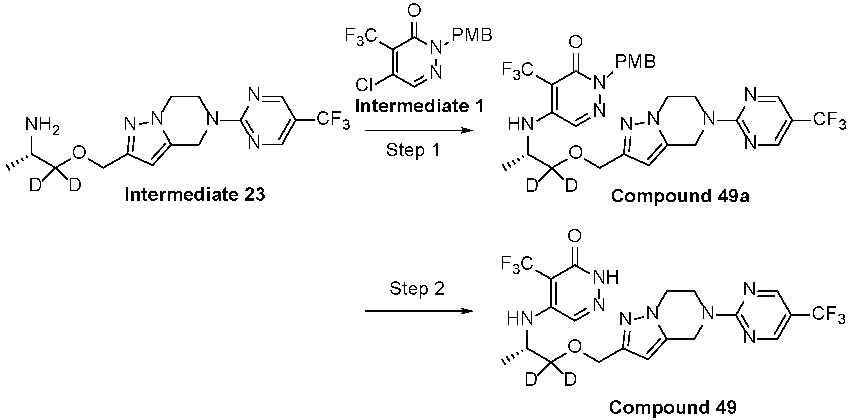

### Step 1:

### (S)-4-(trifluoromethyl)-5-((1-((5-(trifluoromethyl)pyrimidin-2-yl)-4,5,6,7-tetrahydropyrazolo[1,5-a]pyrazin-2-yl)methoxy)propan-2-yl-1,1-d₂)amino)pyridazin-3(2H)-one (Compound 49a)

The synthesis method of **compound 19a** was referred to obtain **compound 49a** in the form of a pale yellow solid (300 mg, 92% yield).

¹H NMR (400 MHz, DMSO-*d*₆) δ 8.81 (d, 2H), 7.95 (s, 1H), 7.26 - 7.17 (m, 2H), 6.93 - 6.83 (m, 2H), 6.34 (d, 1H), 6.09 (s, 1H), 5.02 (s, 2H), 4.98 (d, 2H), 4.40 (d, 2H), 4.34 - 4.26 (m, 2H), 4.14 (t, 2H), 4.03 (q, 1H), 3.70 (s, 3H), 1.14 (d, 3H).

LCMS m/z = 641.30 [M+l].

### Step 2:

### (S)-4-(trifluoromethyl)-5-((1-((5-(trifluoromethyl)pyrimidin-2-yl)-4,5,6,7-tetrahydro pyrazolo[1,5-a]pyrazin-2-yl)methoxy)propan-2-yl-1,1-d₂)amino)pyridazin-3(2H)-one (Compound 49)

The synthesis method of **compound 19** was referred to obtain **compound 49** in the form of a white solid (124 mg, 55% yield).

¹H NMR (400 MHz, DMSO-*d*₆) δ 12.46 (s, 1H), 8.81 (s, 2H), 7.90 (s, 1H), 6.27 (s, 1H), 6.11 (s, 1H), 5.01 (s, 2H), 4.41 (d, 2H), 4.34 - 4.31 (m, 2H), 4.17 - 4.13 (m, 3H), 1.15 (d, 3H).

¹⁹F NMR (376 MHz, DMSO-*d*₆) δ -55.48 (3F), -59.41 (3F).

LCMS m/z = 521.20 [M+l].

### Example 50

### (S)-2-((2-((6-oxo-5-(trifluoromethyl)-1,6-dihydropyridazin-4-yl)amino)propoxy)methyl)-5-(5-(trifluoromethyl)thiazol-2-yl)-6,7-dihydropyrazolo[1,5-a]pyrazin-4(5H)-one (Compound 50)

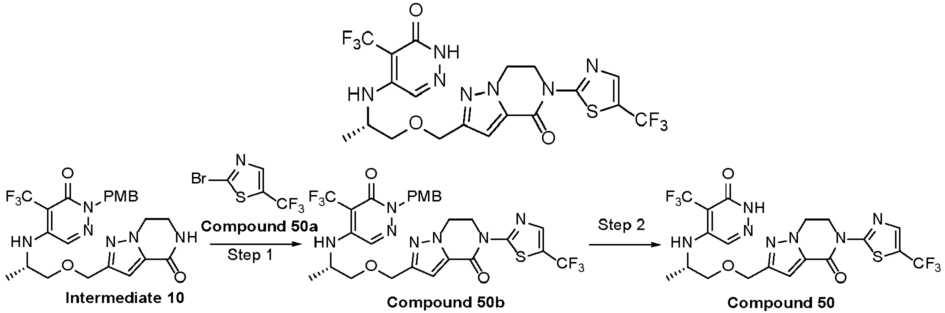

### Step 1:

### (S)-2-((2-((1-(4-methoxybenzyl)-6-oxo-5-(trifluoromethyl)-1,6-dihydropyridazin-4-yl)amino)propoxy)methyl)-5-(5-(trifluoromethyl)thiazol-2-yl)-6,7-dihydropyrazolo[1,5-a]pyrazin-4(5H)-one (Compound 50b)

The synthesis method of **compound 6a** was referred to obtain **compound 50b** in the form of a white solid (220 mg, 53% yield).

LC-MS m/z (ESI) = 658.60 [M+1].

### Step 2:

### (S)-2-((2-((6-oxo-5-(trifluoromethyl)-1,6-dihydropyridazin-4-yl)amino)propoxy)methyl)-5-(5-(trifluoromethyl)thiazol-2-yl)-6,7-dihydropyrazolo[1,5-a]pyrazin-4(5H)-one (Compound 50)

The synthesis method of **compound 6** was referred to obtain **compound 50** in the form of a white solid (43 mg, 49% yield).

¹H NMR (400 MHz, DMSO-*d*₆): δ 12.44 (s, 1H), 8.87 - 8.88 (m, 1H), 6.89 (s, 1H), 6.31 (dd, 1H), 4.65 - 4.43 (m, 5H), 4.30 - 4.09 (m, 1H), 3.55 (d 2H), 1.17 (d, 3H).

LC-MS m/z (ESI) = 537.44 [M+1].

### Example 51

### (S)-4-(trifluoromethyl)-5-(2-(((5-(5-(trifluoromethyl)pyrimidin-2-yl)-4,5,6,7-tetrahydro pyrazolo[1,5-a]pyrazin-2-yl)methoxy)methyl)pyrrolidin-1-yl)pyridazin-3(2H)-one (Compound 51)

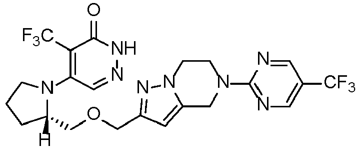

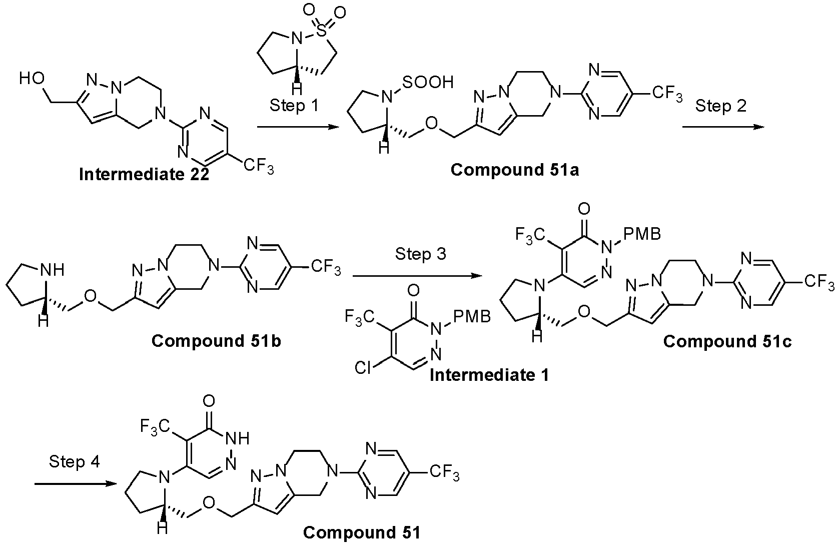

### Step 1:

### (2S)-2-(((5-(5-(trifluoromethyl)pyrimidin-2-yl)-4,5,6,7-tetrahydropyrazolo[1,5-a]pyrazin-2-yl)methoxy)methyl)pyrrolidine-1-sulfinic acid (Compound 51a)

The synthesis method of **compound 27a** was referred to obtain **compound 51a** in the form of a yellow oil (360 mg, 82% yield).

¹H NMR (400 MHz, DMSO-d6) δ 8.81 (s, 2H), 6.17 (s, 1H), 5.03 (s, 2H), 4.33 (d, 4H), 4.24 - 4.15 (m, 2H), 3.58 (dd, 1H), 3.53 - 3.38 (m, 1H), 3.07 (t, 1H), 2.99 - 2.85 (m, 2H), 1.77 - 1.51 (m, 4H).

LC-MS m/z (ESI) = 447.43 [M+1].

### Step 2:

### (S)-2-((pyrrolidin-2-ylmethoxy)methyl)-5-(5-(trifluoromethyl)pyrimidin-2-yl)-4,5,6,7-tetrahydropyrazolo[1,5-a]pyrazine (Compound 51b)

The similar synthesis method of **compound 27b** was referred to obtain **compound 51b** in the form of a yellow oil (268 mg, 91% yield), and the crude product was used directly in the next step.

LC-MS m/z (ESI) = 384.36 [M+1].

### Step 3:

### (S)-2-(4-methoxybenzyl)-4-(trifluoromethyl)-5-(2-(((5-(5-(trifluoromethyl)pyrimidin-2-yl)-4,5,6,7-tetrahydropyrazolo[1,5-a]pyrazin-2-yl)methoxy)methyl)pyrrolidin-1-yl)pyridazin-3(2H)-one (Compound 51c)

The synthesis method of **3a** was referred to obtain **compound 51c** in the form of a yellow oil (480 mg, 72% yield).

LC-MS m/z (ESI) = 666.64 [M+1].

### Step 4:

### (S)-4-(trifluoromethyl)-5-(2-(((5-(5-(trifluoromethyl)pyrimidin-2-yl)-4,5,6,7-tetrahydro pyrazolo[1,5-a]pyrazin-2-yl)methoxy)methyl)pyrrolidin-1-yl)pyridazin-3(2H)-one (Compound 51)

The synthesis method of **compound 6** was referred to obtain **compound 51** in the form of a white solid (80 mg, 56% yield).

¹H NMR (400 MHz, DMSO-*d*₆) δ 12.36 (s, 1H), 8.81 (s, 2H), 8.01 (s, 1H), 6.04 (s, 1H), 4.99 (s, 2H), 4.59 - 4.48 (m, 1H), 4.38 (s, 2H), 4.32 (t, 2H), 4.15 (t, 2H), 3.56 - 3.46 (m, 3H), 3.20 (dd, 1H), 2.12 - 2.02 (m, 1H), 1.91 - 1.86 (m, 1H), 1.70 - 1.55 (m, 2H).

LC-MS m/z (ESI) = 546.49 [M+1].

### Example 52

### (S)-4-(trifluoromethyl)-5-((1-((7-(5-(trifluoromethyl)pyridin-2-yl)-5,6,7,8-tetrahydro-[1,2,4]triazolo[1,5-a]pyrazin-2-yl)methoxy)propan-2-yl-1,1-d₂)amino)pyridazin-3(2H)-one (Compound 52)

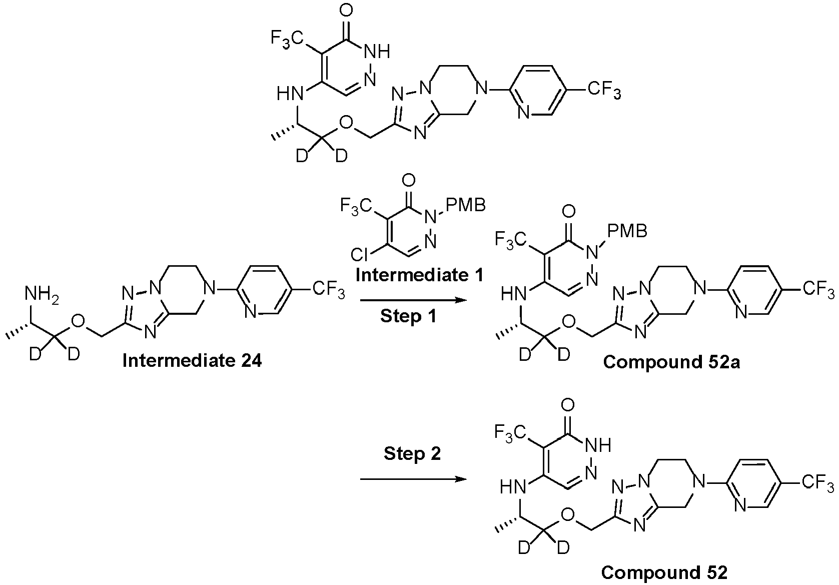

### Step 1:

### (S)-2-(4-methoxybenzyl)-4-(trifluoromethyl)-5-((1-((7-(5-(trifluoromethyl)pyridin-2-yl)-5,6,7,8-tetrahydro-[1,2,4]triazolo[1,5-a]pyrazin-2-yl)methoxy)propan-2-yl-1,1-d₂)amino)pyridazin-3(2H)-one (Compound 52a)

The synthesis method of **compound 19a** was referred to obtain **compound 52a** in the form of a pale yellow solid (340 mg, 87% yield).

LCMS m/z = 642.60 [M+l].

### Step 2:

### (S)-4-(trifluoromethyl)-5-((1-((7-(5-(trifluoromethyl)pyridin-2-yl)-5,6,7,8-tetrahydro-[1,2,4]triazolo[1,5-a]pyrazin-2-yl)methoxy)propan-2-yl-1,1-d₂)amino)pyridazin-3(2H)-one (Compound 52)

The synthesis method of **compound 19** was referred to obtain **compound 52** in the form of a white solid (144 mg, 57% yield).

¹H NMR (400 MHz, DMSO-*d*₆) δ 16.94 (s, 1H), 12.44 (s, 1H), 8.80 (s, 2H), 7.79 (s, 1H), 6.25 (s, 1H), 5.00 (s, 2H), 4.39 (d, 2H), 4.36 - 4.30 (m, 2H), 4.18 - 4.12 (m, 3H), 1.14 (d, 3H).

¹⁹F NMR (376 MHz, DMSO-*d*₆) δ -55.47 (3F), -59.40 (3F).

LCMS m/z = 522.40 [M+l].

### Biological Assays

### 1. Enzymatic biochemical assay of PARP

This assay adopted PARP1, TNKS1, TNKS2, PARP7 and PARP14 chemiluminescence detection kits (BPS, Cat No. 80551/80552/80573/80578/79729/80568) for enzymatic biochemical assay of PARP. The specific scheme was as follows: the 1× histone mixture was added to a 96-well plate at 50 µL per well, and the plate was incubated overnight at 4 °C. The next day, after the plate was washed with PBST, 200 µL of blocking buffer per well was added, and the plate was incubated for 90 min. After the plate was washed with PBST again, 5 µL of inhibitor, 20 µL of 1× PARP buffer and 25 µL of streptavidin-HRP were added to each well, and the plate was incubated at room temperature for 30 min. After the plate was washed with PBST, 100 µL of Elisa ECL substrate A and B mixture was added to each well, chemiluminescence value was immediately read using a plate reader, and IC₅₀ values were calculated. The results are shown in Tables 1 and 2 below.

**Table 1 Enzymatic inhibitory activity of the compounds of the present invention against PARP7**

| **Compound No.** | **Inhibitory activity against PAPR7 (IC₅₀, nM)** |
|---|---|
| **Compound 1** | **B** |
| **Compound 3-I** | A |
| **Compound 3-II** | C |
| **Compound 5** | A |
| **Compound 9** | B |
| **Compound 13** | B |
| **Compound 14** | A |
| **Compound 21** | A |

| | |
|---|---|
| Note: A: IC₅₀ < 10 nM; B: 10 nM < IC₅₀ < 50 nM; C: 50 nM < IC₅₀ < 100 nM. | |

The results show that the compounds of the present invention have remarkable biological inhibition activity on PARP7.

**Table 2 PARP1, TNKS1, TNKS2, PARP7 and PARP14 subtype selectivity**

| **Compound (IC₅₀, nM)** | **Comparative example** | **Compound 1** |
|---|---|---|
| **PARP1** | <20 | >1000 |
| **TNKS1** | >10000 | >10000 |
| **TNKS2** | 3897 | >10000 |
| **PARP14** | >10000 | >10000 |

| | | |
|---|---|---|
| Note: a comparative example was **compound 561** of Patent WO2019212937, which was obtained according to the preparation method of compound 561. | | |

The results show that the compounds of the present invention have higher kinase selectivity compared with the comparative example.

### 2. NCI-H1373 cell proliferation inhibition assay

Human lung adenocarcinoma cells NCI-H1373 (ATCC, CRL-5866^{™}) were cultured in a cell incubator at 37 °C and 5% CO₂ using RPMI-1640 medium containing 10% FBS and 1% bispecific antibody. The cells in the logarithmic growth phase were counted by digestion and inoculated in a 96-well plate at 1500 NCI-H1373 per well, and the plate was placed in an incubator overnight. The next day, test compounds were formulated in 10 mM stock solution by using DMSO and 3-fold diluted in gradient from a maximum concentration of 10 µM by using RPMI-1640 medium, and 10 gradient concentrations were set with 2 parallel wells per well. After 6 days of incubation, 100 µL of Cell Titer Blue working solution was added to each well and the chemiluminescence value was read in a microplate reader. IC₅₀ was calculated using GraphPad Prism7.0 software. The results are shown in Table 3.

**Table 3 NCI-H1373 cell proliferation activity**

| **Compound No.** | **IC₅₀(nM)** |
|---|---|
| **Compound 1** | A |
| **Compound 2** | A |
| **Compound 3-I** | A |
| **Compound 5** | A |
| **Compound 9** | A |
| **Compound 11** | B |
| **Compound 13** | A |
| **Compound 14** | B |
| **Compound 19** | A |
| **Compound 21** | A |
| **Compound 23** | B |
| **Compound 25** | A |
| **Compound 26** | A |
| **Compound 28** | B |
| **Compound 30** | B |
| **Compound 39** | A |

| | |
|---|---|
| Note: A: IC₅₀ < 50 nM; B: 50 nM < IC₅₀ < 100 nM. | |

The results show that the compounds of the present invention have significant inhibition effect on NCI-H1373 cell proliferation.

### 3. Test protocol of metabolic stability in liver microsome

0.5 mg/mL CD1 mouse liver microsome solution, 0.5 µM test compound, 1.0 mM reduced nicotinamide adenine dinucleotide phosphate (NADPH), and 1.0 mM uridine diphosphate-A-glucuronic acid (UDPGA) were added to the incubation plate and mixed, and the plate was incubated at 37 °C for 0, 30, 60, 120 min separately. The positive control group was set as 0.5 µM CYP3A4 probe substrate-testosterone and 0.5 µM UGT probe substrate-7-hydroxycoumarin incubated with microsomes for 0 and 120 min in the presence of NADPH and UDPGA; the negative control group (NC) was set as the test substance incubated with microsomes for 0 and 120 min without any coenzyme. After the reaction was terminated by adding pre-cooled methanol at each termination time, the reaction mixture was centrifuged at 4000 rpm for 10 min, the supernatant was taken and the remaining amount of the parent drug in the sample was measured by LC-MS/MS, the results of which are shown in Table 4.

**Table 4 Stability results in mouse liver microsome**

| **Compound No.** | **T_{1/2}(min)** |
|---|---|
| **Comparative example** | <30 |
| **Compound 1** | >30 |
| **Compound 9** | >30 |
| **Compound 11** | >100 |
| **Compound 26** | >100 |
| **Compound 37** | >100 |

| | |
|---|---|
| Note: a comparative example was **compound 561** of Patent WO2019212937, which was obtained according to the preparation method of compound 561. | |

The results show that the compounds of the present application have significantly better metabolic stability of the liver microsome than the comparative example.

### 4. MDCKII-MDR1 cell permeability assay

50 µL of MDCKII-MDR1 cell suspension with a density of 1.56 × 10⁶ cells/mL was seeded into a Transwell well of a 96-well plate, and the plate was cultured in a cell incubator at 37 °C in 5% CO₂. When the cells were cultured to a certain degree of confluence, the transepithelial electrical resistance value (TEER) was measured, and if the TEER value was greater than 42 ohm•cm², indicating that the cells had grown into a monolayer and met the experimental requirements. The medium in Transwell was discarded, and HBSS equilibration solution was added and incubated at 37 °C for 30 min. Subsequently, an apical to basolateral drug transport rate assay was performed by adding 1 µM test compound solution to the Transwell AB side and a blank equilibration solution to the BL side. When the basolateral to apical drug transport rate assay was performed, the operation steps were reversed. Subsequently, the cells were placed in an incubator for 2 h. After the incubation, samples were taken from the basolateral and apical respectively, and after centrifugation, the supernatant was subjected to LC-MS/MS analysis, and the apparent permeability coefficient (Papp) was calculated, the results of which are shown in Table 5.

**Table 5 MDCKII-MDR1 cell permeability results**

| **Compound No.** | **Papp (A-B) (10-6, cm/s)** | **Papp (B-A) (10-6, cm/s)** | **Efflux Ratio** |
|---|---|---|---|
| **Comparative example** | 5.16 | 49.43 | 9.58 |
| **Compound 13** | 6.67 | 14.76 | 2.28 |
| **Compound 39** | 8.82 | 35.46 | 4.09 |

| | | | |
|---|---|---|---|
| Note: a comparative example was **compound 561** of Patent WO2019212937, which was obtained according to the preparation method of compound 561. | | | |

The results show that the compounds of the present invention have higher cell membrane permeability and lower efflux rate compared with the comparative example.

### 5. In vivo pharmacokinetic studies

The appropriate amount of compound was weighed out and prepared as a clear and transparent solution of 1 mg/mL using 5% DMSO + 30% HP-β-CD. After fasting overnight, ICR male mice were given 30 mpk by intragastric administration, and blood was collected from the jugular plexus at different time points of 0 min, 5 min, 0.25 h, 0.5 h, 1 h, 2 h, 4 h, 8 h and 24 h. After anticoagulation with EDTA-K2, the plasma was separated by centrifugation, and the concentration of the parent drug in the plasma was determined by LC/MS/MS.

The results show that the compounds of the present application showed significantly better pharmacokinetic characteristics in mice than the comparative example.

While specific embodiments of the present invention have been described in detail in the specification, it will be understood by those skilled in the art that the embodiments described above are illustrative and are not to be construed as limiting the present invention, and that various changes and modifications can be made to the present invention without departing from the principles of the present invention, and the technical schemes resulting from these changes and modifications also fall within the protection scope of the appended claims.

## Claims

1. A compound of formula (I) or a pharmaceutically acceptable salt, a stereoisomer or a deuterated compound thereof: 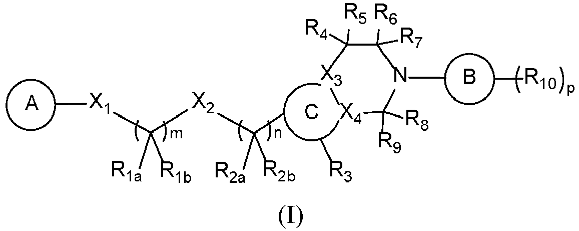 wherein:
X₁ is NH, O, or a 4 to 6-membered heterocycle containing 1 to 3 heteroatoms selected from N and O;
X₂ is O or a bond;
X₃ and X₄ are each independently C or N;
R₁ₐ and R_{1b} are each independently H, D, or a C₁₋₆ alkyl; or R₁ₐ and R_{1b} form a 3 to 5-membered cycloalkyl together with the carbon atom connected thereto;
R₂ₐ and R_{2b} are each independently H, D, or a C₁₋₆ alkyl; or R₂ₐ and R_{2b} form a 3 to 5-membered cycloalkyl together with the carbon atom connected thereto;
R₃ is H, D, a C₁₋₆ alkyl, halogen, or cyano, where the C₁₋₆ alkyl is optionally substituted with 1 to 3 halogens;
R₄ and R₅ are each independently H, D, or a C₁₋₆ alkyl; or R₄ and R₅ form a 3 to 5-membered cycloalkyl together with the carbon atom connected thereto;
R₆ and R₇ are each independently H, D, or a C₁₋₆ alkyl; or R₆ and R₇ form =O on the carbon atom connected thereto;
R₈ and R₉ are each independently H, D, or a C₁₋₆ alkyl; or R₈ and R₉ form =O on the carbon atom connected thereto; or R₈ and R₉ form a 3 to 5-membered cycloalkyl together with the carbon atom connected thereto;
each R₁₀ is independently a C₁₋₆ alkyl, a C₁₋₆ alkoxy, CONR₁₀ₐR_{10b}, halogen, cyano, S(O)₂R_{10c}, SR_{10d}, or a 3 to 5-membered cycloalkyl, where the C₁₋₆ alkyl and C₁₋₆ alkoxy are optionally substituted with 1 to 3 halogens;
R₁₀ₐ, R_{10b}, R_{10c} and R_{10d} are each independently H, D, or a C₁₋₆ alkyl;
A is 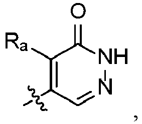 Rₐ is a C₁₋₆ alkyl, a C₃₋₅ cycloalkyl, halogen, or cyano, where the C₁₋₆ alkyl is optionally substituted with 1 to 3 halogens;
B is a 5 to 10-membered carbocycle or heterocycle containing 1 to 3 heteroatoms selected from N, O, and S;
C is a 5 to 6-membered heterocycle containing 1 to 3 heteroatoms of N;
m is 1, 2, or 3;
n is 0, 1, 2, or 3; and
p is 0, 1, 2, or 3.

2. The compound according to claim 1, or a pharmaceutically acceptable salt, a stereoisomer or a deuterated compound thereof, wherein:
X₁ is NH, O, or a 4 to 6-membered heterocycle containing 1 to 3 heteroatoms selected from N and O;
X₂ is O or a bond;
X₃ and X₄ are each independently C or N;
R₁ₐ and R_{1b} are each independently H, D, or a C₁₋₆ alkyl;
R₂ₐ and R_{2b} are each independently H, D, or a C₁₋₆ alkyl; or R₂ₐ and R_{2b} form a 3 to 5-membered cycloalkyl together with the carbon atom connected thereto;
R₃ is H, D, a C₁₋₆ alkyl, halogen, or cyano, where the C₁₋₆ alkyl is optionally substituted with 1 to 3 halogens;
R₄ and R₅ are each independently H, D, or a C₁₋₆ alkyl; or R₄ and R₅ form a 3 to 5 membered cycloalkyl together with the carbon atom connected thereto;
R₆ and R₇ are each independently H, D, or a C₁₋₆ alkyl;
R₈ and R₉ are each independently H, D, or a C₁₋₆ alkyl; or R₈ and R₉ form =O on the carbon atom connected thereto;
each R₁₀ is independently a C₁₋₆ alkyl, a C₁₋₆ alkoxy, CONR₁₀ₐR_{10b}, halogen, cyano, S(O)₂R_{10c}, SR_{10d}, or a 3 to 5-membered cycloalkyl, where the C₁₋₆ alkyl and C₁₋₆ alkoxy are optionally substituted with 1 to 3 halogens;
R₁₀ₐ, R_{10b}, R_{10c} and R_{10d} are each independently H, D, or a C₁₋₆ alkyl;
A is 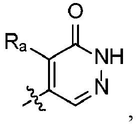 Rₐ is a C₁₋₆ alkyl, a C₃₋₅ cycloalkyl, halogen, or cyano, where the C₁₋₆ alkyl is optionally substituted with 1 to 3 halogens;
B is a 5 to 6-membered carbocycle or heterocycle containing 1 to 3 heteroatoms selected from N, O, and S;
C is a 5 to 6-membered heterocycle containing 1 to 3 heteroatoms of N;
m is 1, 2, or 3;
n is 0, 1, 2, or 3; and
p is 0, 1, 2, or 3.

3. The compound according to claim 1, or a pharmaceutically acceptable salt, a stereoisomer or a deuterated compound thereof, wherein the compound has the structure according to formula (I-1): 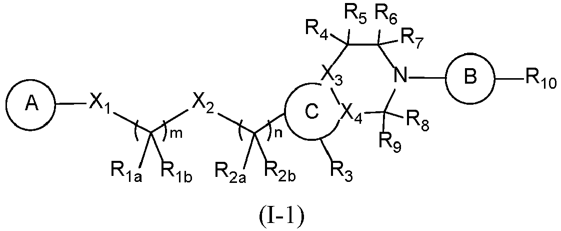 wherein:
X₁ is NH or a 4 to 6-membered heterocycle containing 1 to 3 heteroatoms selected from N and O;
X₂ is O;
X₃ and X₄ are each independently C or N;
R₁ₐ and R_{1b} are each independently H, D, or a C₁₋₆ alkyl;
R₂ₐ and R_{2b} are each independently H, D, or a C₁₋₆ alkyl;
R₃ is H, D, a C₁₋₆ alkyl, or halogen, where the C₁₋₆ alkyl is optionally substituted with 1 to 3 halogens;
R₄ and R₅ are each independently H, D, or a C₁₋₆ alkyl;
R₆ and R₇ are each independently H, D, or a C₁₋₆ alkyl;
R₈ and R₉ are each independently H, D, or a C₁₋₆ alkyl; or R₈ and R₉ form =O on the carbon atom connected thereto;
R₁₀ is a C₁₋₆ alkyl, a C₁₋₆ alkoxy, cyano, or SR_{10d}, where the C₁₋₆ alkyl and C₁₋₆ alkoxy are optionally substituted with 1 to 3 halogens;
R_{10d} is H, D, or a C₁₋₆ alkyl;
A is 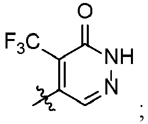
B is a 5 to 6-membered carbocycle or heterocycle containing 1 to 3 heteroatoms selected from N, O, and S;
C is a 5 to 6-membered heterocycle containing 1 to 3 heteroatoms of N;
m is 1, 2, or 3; and
n is 0, 1, 2, or 3.

4. The compound according to claim 1, or a pharmaceutically acceptable salt, a stereoisomer or a deuterated compound thereof, wherein the compound has the structure according to formula (I-2): 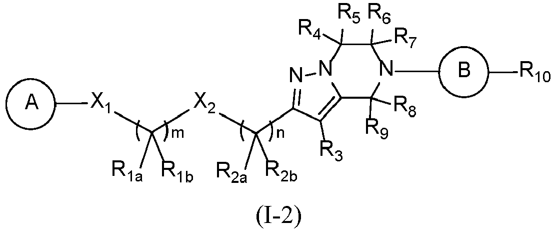 wherein:
X₁ is NH;
X₂ is O;
R₁ₐ and R_{1b} are each independently H, D, or a C₁₋₆ alkyl;
R₂ₐ and R_{2b} are each independently H, D, or a C₁₋₆ alkyl;
R₃ is H, D, a C₁₋₆ alkyl, or halogen, and the C₁₋₆ alkyl is optionally substituted with 1 to 3 halogens;
R₄ and R₅ are each independently H, D, or a C₁₋₆ alkyl;
R₆ and R₇ are each independently H, D, or a C₁₋₆ alkyl;
R₈ and R₉ are each independently H, D, or a C₁₋₆ alkyl; or R₈ and R₉ form =O on the carbon atom connected thereto;
R₁₀ is a C₁₋₆ alkyl, a C₁₋₆ alkoxy, cyano, or SR_{10d}, where the C₁₋₆ alkyl and C₁₋₆ alkoxy are optionally substituted with 1 to 3 halogens;
R_{10d} is H, D, or a C₁₋₆ alkyl;
A is 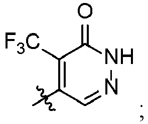
B is a 5 to 6-membered carbocycle or heterocycle containing 1 to 3 heteroatoms selected from N, O, and S;
m is 1, 2, or 3; and
n is 0, 1, 2, or 3.

5. The compound according to claim 4, or a stereoisomer or a deuterated compound thereof, wherein:
X₁ is NH;
X₂ is O;
R₁ₐ and R_{1b} are each independently H, D, or a C₁₋₆ alkyl;
R₂ₐ and R_{2b} are each independently H, D, or a C₁₋₆ alkyl;
R₃ is H, D, a C₁₋₆ alkyl, or halogen, where the C₁₋₆ alkyl is optionally substituted with 1 to 3 halogens;
R₄ and R₅ are each independently H, D, or a C₁₋₆ alkyl;
R₆ and R₇ are each independently H, D, or a C₁₋₆ alkyl;
R₈ and R₉ are each independently H, D, or a C₁₋₆ alkyl, or R₈ and R₉ form =O on the carbon atom connected thereto;
R₁₀ is a C₁₋₆ alkyl, cyano, or SR_{10d}, where the C₁₋₆ alkyl is optionally substituted with 1 to 3 halogens;
R_{10d} is H, D, or a C₁₋₆ alkyl;
A is 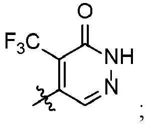
B is a 5 to 6-membered carbocycle or heterocycle containing 1 to 3 heteroatoms selected from N, O, and S;
m is 1, 2, or 3; and
n is 0, 1, 2, or 3.

6. The compound according to claim 5, or a stereoisomer or a deuterated compound thereof, wherein:
X₁ is NH;
X₂ is O;
R₁ₐ and R_{1b} are each independently selected from H, D, or a C₁₋₆ alkyl;
R₂ₐ and R_{2b} are each independently selected from H, D, or a C₁₋₆ alkyl;
R₃ is H, D, a C₁₋₆ alkyl, or halogen, where the C₁₋₆ alkyl is optionally substituted with 1 to 3 halogens;
R₄ and R₅ are each independently H or D;
R₆ and R₇ are each independently H or D;
R₈ and R₉ are each independently H or D;
R₁₀ is CF₃ or SR_{10d};
R_{10d} is H, D, or a C₁₋₆ alkyl;
A is 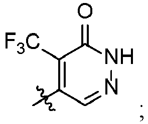
B is 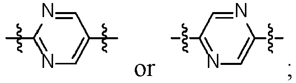 m is 1, 2, or 3; and
n is 0, 1, 2, or 3.

7. The compound according to claim 6, or a stereoisomer or a deuterated compound thereof, wherein:
X₁ is NH;
X₂ is O;
R₁ₐ and R_{1b} are each independently selected from H, D, or a C₁₋₃ alkyl;
R₂ₐ and R_{2b} are each independently selected from H, D, or a C₁₋₃ alkyl;
R₃ is selected from H, D, or CF₃;
R₄ and R₅ are each independently selected from H or D;
R₆ and R₇ are each independently selected from H or D;
R₈ and R₉ are each independently selected from H or D;
R₁₀ is CF₃;
A is 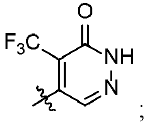
B is 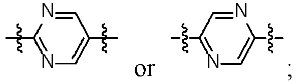 m is 1, 2, or 3; and
n is 0, 1, or 2.

8. The compound according to claim 1, or a pharmaceutically acceptable salt, a stereoisomer or a deuterated compound thereof, wherein the compound has the structure according to formula (I-3): 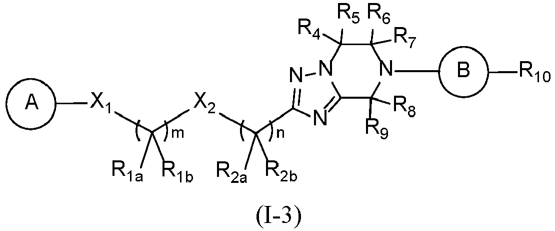 wherein:
X₁ is NH or a 4 to 6-membered heterocycle containing 1 to 3 heteroatoms selected from N and O;
X₂ is O;
R₁ₐ and R_{1b} are each independently H, D, or a C₁₋₆ alkyl;
R₂ₐ and R_{2b} are each independently H, D, or a C₁₋₆ alkyl;
R₄ and R₅ are each independently H, D, or a C₁₋₆ alkyl;
R₆ and R₇ are each independently H, D, or a C₁₋₆ alkyl;
R₈ and R₉ are each independently H, D, or a C₁₋₆ alkyl; or R₈ and R₉ form =O on the carbon atom connected thereto;
R₁₀ is a C₁₋₆ alkyl, a C₁₋₆ alkoxy, cyano, or SR_{10d}, where the C₁₋₆ alkyl and C₁₋₆ alkoxy are optionally substituted with 1 to 3 halogens;
R_{10d} is H, D, or a C₁₋₆ alkyl;
A is 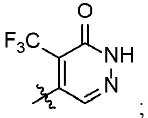
B is a 5 to 6-membered carbocycle or heterocycle containing 1 to 3 heteroatoms selected from N, O, and S;
m is 1, 2, or 3; and
n is 0, 1, 2, or 3.

9. The compound according to claim 8, or a pharmaceutically acceptable salt, a stereoisomer or a deuterated compound thereof, wherein:
X₁ is NH or a 4 to 6-membered heterocycle containing 1 to 3 heteroatoms selected from N and O;
X₂ is O;
R₁ₐ and R_{1b} are each independently H, D, or a C₁₋₃ alkyl;
R₂ₐ and R_{2b} are each independently H, D, or a C₁₋₃ alkyl;
R₄ and R₅ are each independently H, D, or a C₁₋₃ alkyl;
R₆ and R₇ are each independently H, D, or a C₁₋₃ alkyl;
R₈ and R₉ are each independently H, D, or a C₁₋₃ alkyl; or R₈ and R₉ form =O on the carbon atom connected thereto;
R₁₀ is a C₁₋₆ alkyl, cyano, or SR_{10d}, where the C₁₋₆ alkyl is optionally substituted with 1 to 3 halogens;
R_{10d} is H, D, or a C₁₋₆ alkyl;
A is 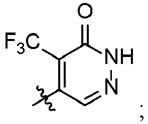
B is 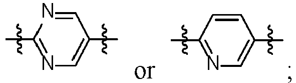 m is 1, 2, or 3; and
n is 0, 1, or 2.

10. The compound according to claim 9, or a pharmaceutically acceptable salt, a stereoisomer or a deuterated compound thereof, wherein:
X₁ is NH;
X₂ is O;
R₁ₐ and R_{1b} are each independently H, D, or a C₁₋₃ alkyl;
R₂ₐ and R_{2b} are each independently H or D;
R₄ and R₅ are each independently H or D;
R₆ and R₇ are each independently H or D;
R₈ and R₉ are each independently H or D;
R₁₀ is CF₃;
A is 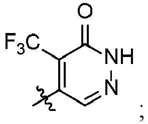
B is 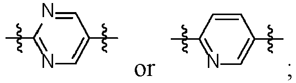 m is 1, 2, or 3; and
n is 0, 1, or 2.

11. A compound of formula (I') or a stereoisomer thereof: 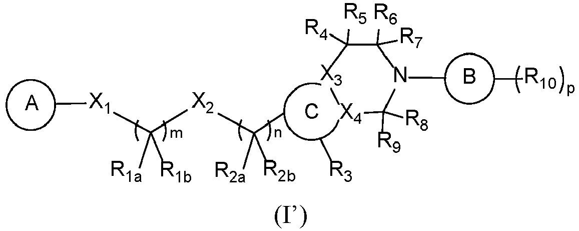 wherein:
X₁ is NH or a 4 to 6-membered heterocycle containing 1 to 3 heteroatoms selected from N and O;
X₂ is O or a bond;
X₃ and X₄ are C or N;
R₁ₐ and R_{1b} are each independently H, D, or a C₁₋₆ alkyl; or R₁ₐ and R_{1b} optionally form a 3 to 5-membered cycloalkyl together with the carbon atom connected thereto;
R₂ₐ and R_{2b} are each independently H, D, or a C₁₋₆ alkyl; or R₂ₐ and R_{2b} optionally form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H or a C₁₋₆ alkyl; or R₄ and R₅ form a 3 to 5-membered cycloalkyl together with the carbon atom connected thereto;
R₆ and R₇ are each independently H or a C₁₋₆ alkyl; or R₆ and R₇ form =O on the carbon atom connected thereto;
R₈ and R₉ are each independently H, D, or a C₁₋₆ alkyl; or R₈ and R₉ form =O on the carbon atom connected thereto; or R₈ and R₉ form a 3 to 5-membered cycloalkyl together with the carbon atom connected thereto;
each R₁₀ is independently a halogen-substituted C₁₋₆ alkyl, a halogen-substituted C₁₋₆ alkoxy, CONR₁₀ₐR_{10b}, cyano, S(O)₂R_{10c}, SR_{10d}, or a 3 to 5-membered cycloalkyl;
R₁₀ₐ, R_{10b}, R_{10c} and R_{10d} are each independently H, D, or a C₁₋₆ alkyl;
A is 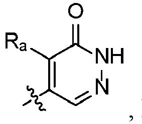 Rₐ is a C₁₋₆ alkyl, a C₃₋₅ cycloalkyl, halogen, or cyano, where the C₁₋₆ alkyl is optionally substituted with 1 to 3 halogens;
B is a 5 to 10-membered carbocycle or heterocycle containing 1 to 3 heteroatoms selected from N, O, and S;
C is a 5 to 6-membered heterocycle containing 1 to 3 heteroatoms of N;
m is 1, 2, or 3;
n is 0, 1, 2, or 3; and
p is 0, 1, 2, or 3.

12. The compound according to claim 11, or a stereoisomer thereof, wherein the compound has the structure according to formula (I-1'): 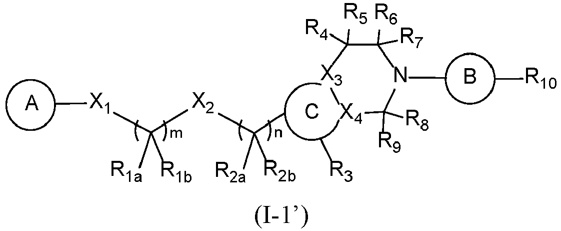 wherein:
X₁ is NH or a 4 to 6-membered heterocycle containing 1 to 3 heteroatoms selected from N and O;
X₂ is O or a bond;
X₃ and X₄ are each independently C or N;
R₁ₐ and R_{1b} are each independently H, D, or a C₁₋₆ alkyl; or R₁ₐ and R_{1b} optionally form a 3 to 5-membered cycloalkyl together with the carbon atom connected thereto;
R₂ₐ and R_{2b} are each independently H, D, or a C₁₋₆ alkyl; or R₂ₐ and R_{2b} optionally form a 3 to 5-membered cycloalkyl together with the carbon atom connected thereto;
R₃ is H, D, a C₁₋₆ alkyl, halogen or cyano, where the C₁₋₆ alkyl is optionally substituted with 1 to 3 halogens;
R₄ and R₅ are each independently H or a C₁₋₆ alkyl; or R₄ and R₈ form a 3 to 5-membered cycloalkyl together with the carbon atom connected thereto;
R₆ and R₇ are each independently H or a C₁₋₆ alkyl; or R₆ and R₇ form =O on the carbon atom connected thereto;
R₈ and R₉ are each independently H, D, or a C₁₋₆ alkyl; or R₈ and R₉ form =O on the carbon atom connected thereto; or R₈ and R₉ form a 3 to 5-membered cycloalkyl together with the carbon atom connected thereto;
R₁₀ is a halogen-substituted C₁₋₆ alkyl, CONR₁₀ₐR_{10b}, cyano, or a 3 to 5-membered cycloalkyl;
R₁₀ₐ and R_{10b} are each independently H, D, or a C₁₋₆ alkyl;
A is 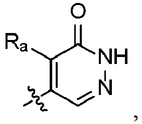 Rₐ is a C₁₋₆ alkyl, a C₃₋₅ cycloalkyl, halogen, or cyano, where the C₁₋₆ alkyl is optionally substituted with 1 to 3 halogens;
B is a 5 to 6-membered carbocycle or heterocycle containing 1 to 3 heteroatoms selected from N, O, and S;
C is a 5 to 6-membered heterocycle containing 1 to 3 heteroatoms of N;
m is 1, 2, or 3; and
n is 0, 1, 2, or 3.

13. A compound of formula (I-2') or a stereoisomer thereof: 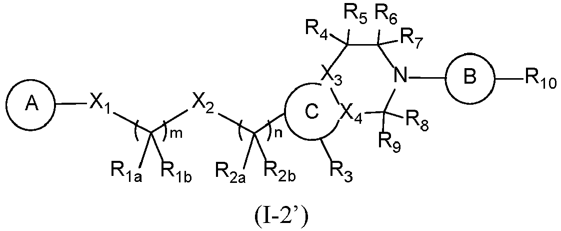 wherein:
X₁ is NH;
X₂ is O or a bond;
X₃ and X₄ are C or N;
R₁ₐ and R_{1b} are each independently H, D, or a C₁₋₆ alkyl; or R₁ₐ and R_{1b} optionally form a 3 to 5-membered cycloalkyl together with the carbon atom connected thereto;
R₂ₐ and R_{2b} are each independently H, D, or a C₁₋₆ alkyl; or R₂ₐ and R_{2b} optionally form a 3 to 5-membered cycloalkyl together with the carbon atom connected thereto;
R₃ is H, a C₁₋₆ alkyl, halogen, or cyano, where the C₁₋₆ alkyl is optionally substituted with 1 to 3 halogens;
R₄ and R₅ are each independently H or a C₁₋₆ alkyl;
R₆ and R₇ are each independently H or a C₁₋₆ alkyl; or R₆ and R₇ form =O on the carbon atom connected thereto;
R₈ and R₉ are each independently H, D, or a C₁₋₆ alkyl; or R₈ and R₉ form =O on the carbon atom connected thereto;
R₁₀ is CF₃, cyano, or a 3 to 5-membered cycloalkyl;
A is 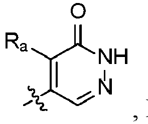 Rₐ is a C₁₋₆ alkyl, a C₃₋₅ cycloalkyl, halogen, or cyano, where the C₁₋₆ alkyl is optionally substituted with 1 to 3 halogens;
B is a 5 to 6-membered carbocycle or heterocycle containing 1 to 3 heteroatoms selected from N, O, and S;
C is a 5 to 6-membered heterocycle containing 1 to 3 heteroatoms of N;
m is 1, 2, or 3; and
n is 0, 1, 2, or 3.

14. The compound according to claim 11, or a stereoisomer thereof, wherein the compound has the structure according to formula (I-3'): 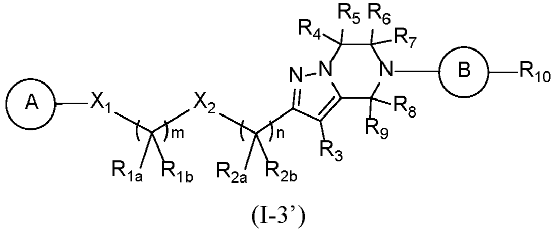 wherein
X₁ is NH;
X₂ is O or a bond;
R₁ₐ and R_{1b} are each independently H, D, or a C₁₋₆ alkyl; or R₁ₐ and R_{1b} optionally form a 3 to 5-membered cycloalkyl together with the carbon atom connected thereto;
R₂ₐ and R_{2b} are each independently H, D, or a C₁₋₆ alkyl; or R₂ₐ and R_{2b} optionally form a 3 to 5-membered cycloalkyl together with the carbon atom connected thereto;
R₃ is H or a C₁₋₆ alkyl;
R₄ and R₅ are each independently H or a C₁₋₆ alkyl;
R₆ and R₇ are each independently H or a C₁₋₆ alkyl;
R₈ and R₉ are each independently H or a C₁₋₆ alkyl;
R₁₀ is CF₃ or a 3 to 5-membered cycloalkyl;
A is 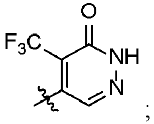
B is 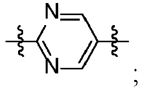
m is 1, 2, or 3; and
n is 0, 1, 2, or 3.

15. The compound according to claim 13, or a stereoisomer thereof, wherein
X₁ is NH;
X₂ is O;
R₁ₐ and R_{1b} are each independently H or a C₁₋₆ alkyl;
R₂ₐ and R_{2b} are each independently H or a C₁₋₆ alkyl;
R₃ is H or a C₁₋₆ alkyl;
R₄ and R₅ are each independently H or a C₁₋₆ alkyl;
R₆ and R₇ are each independently H or a C₁₋₆ alkyl;
R₈ and R₉ are each independently H or a C₁₋₆ alkyl;
R₁₀ is CF₃ or a 3 to 5-membered cycloalkyl;
A is 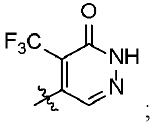
B is 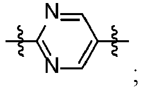
m is 1 or 2; and
n is 0 or 1.

16. The compound according to any one of claims 1 to 15, or a pharmaceutically acceptable salt, a stereoisomer or a deuterated compound thereof, wherein the compound has the following structure: 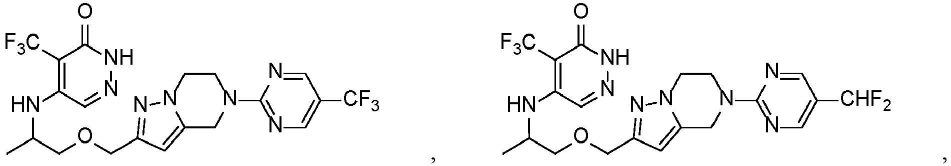 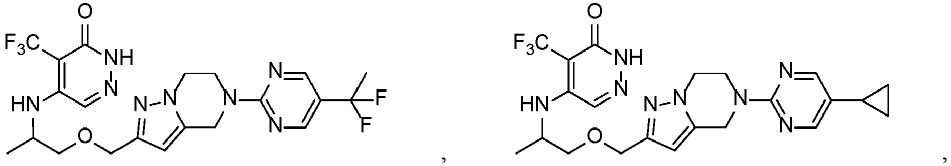 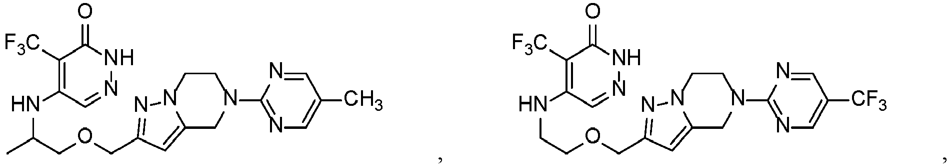 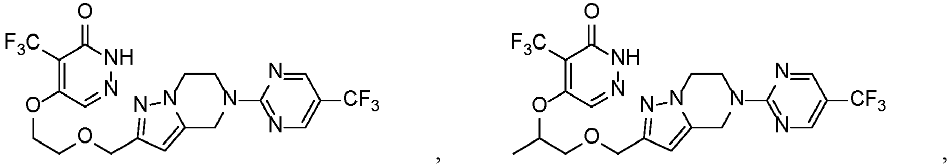 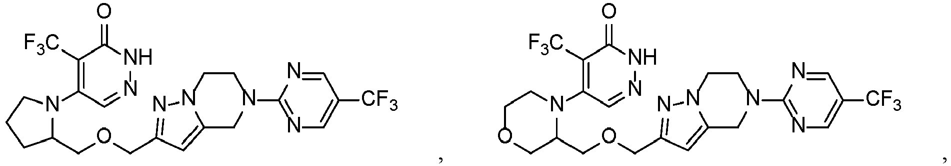 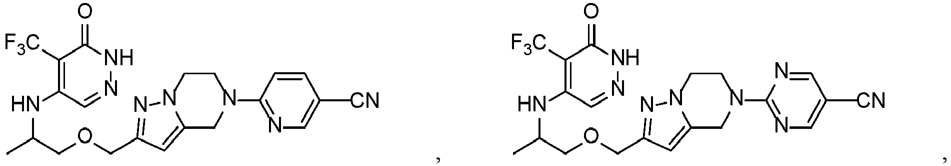 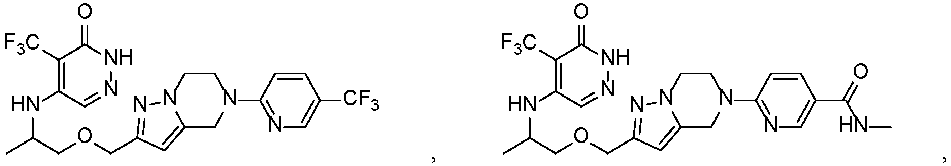 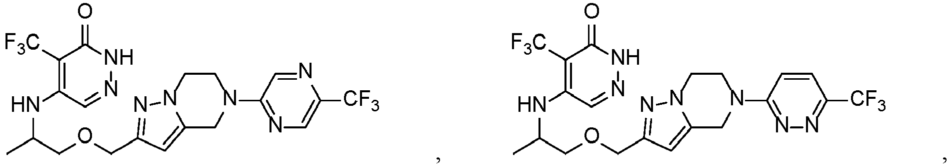 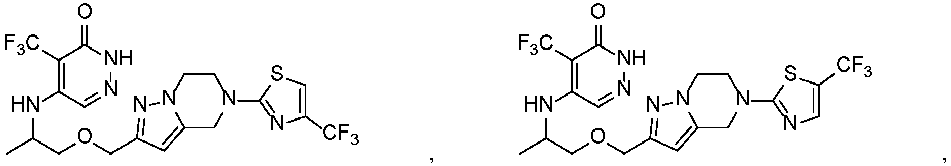 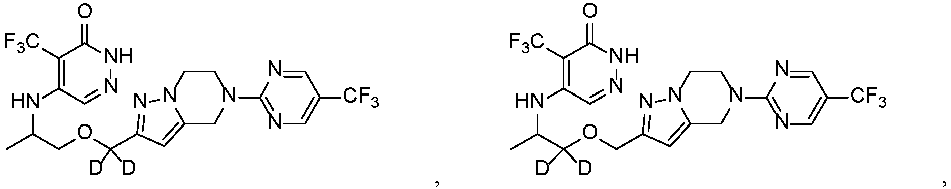 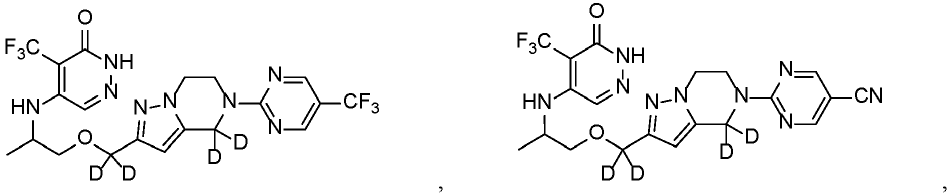 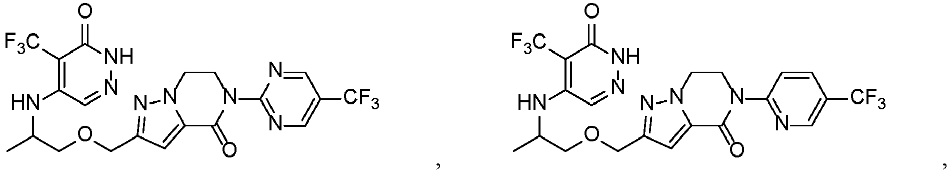 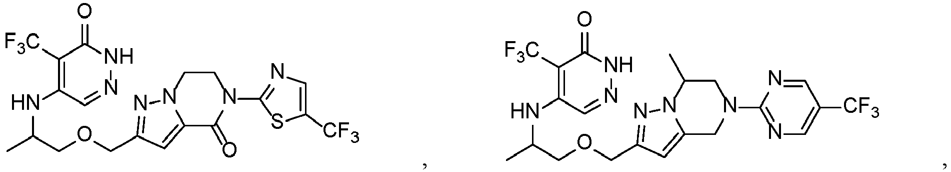 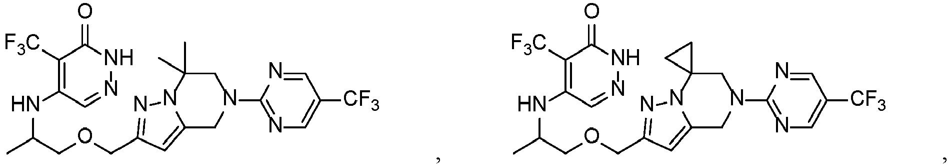 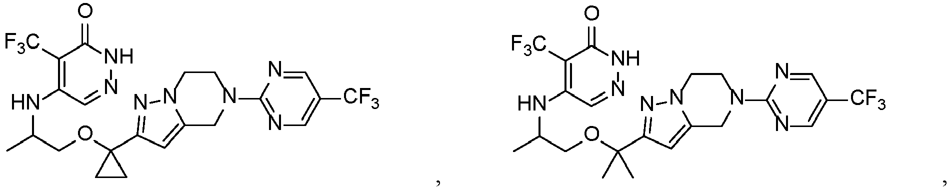 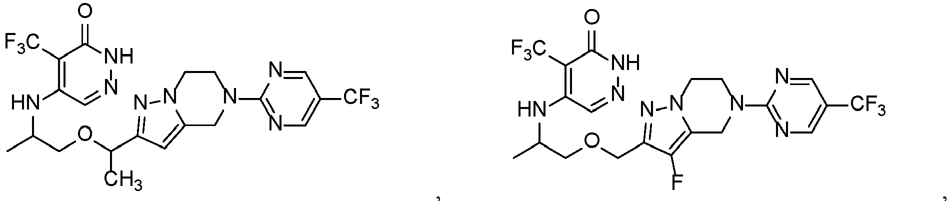 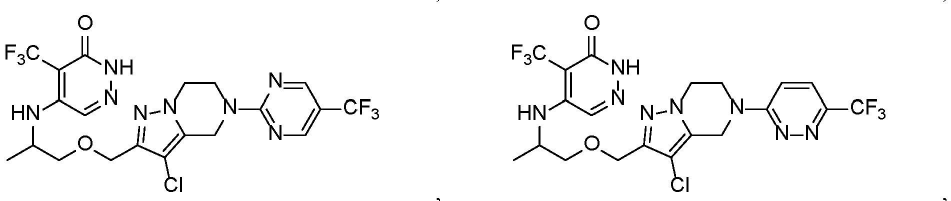 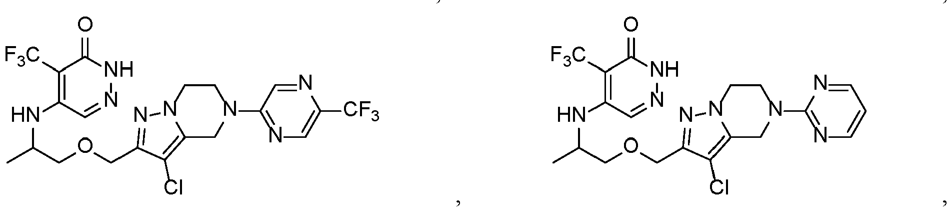 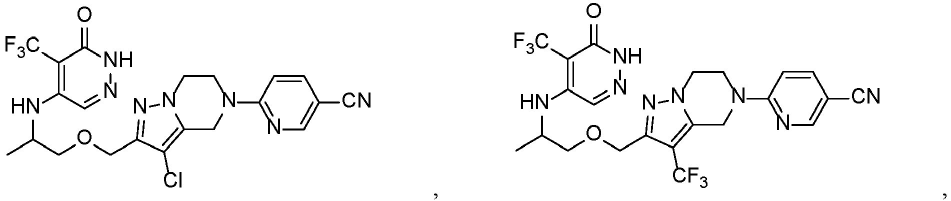 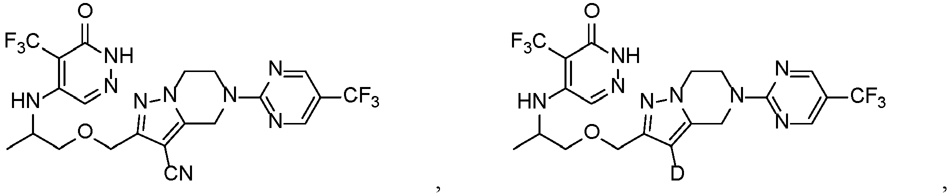 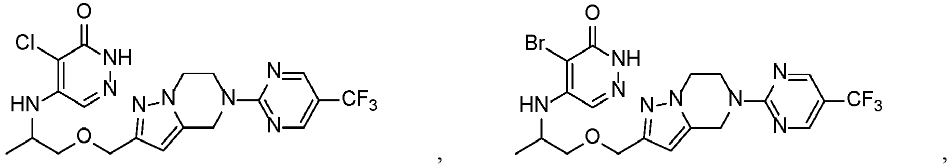 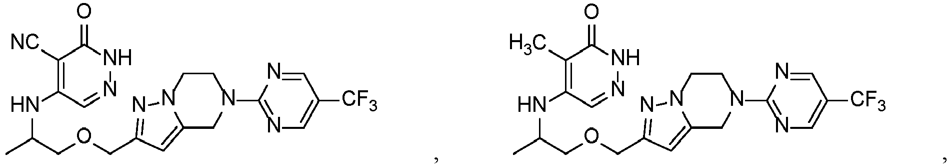 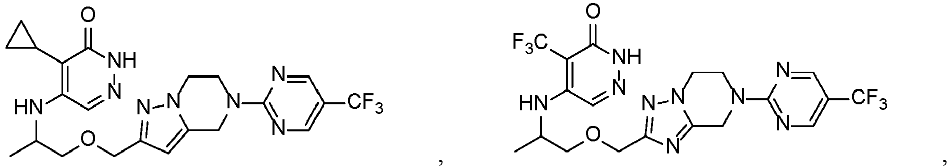 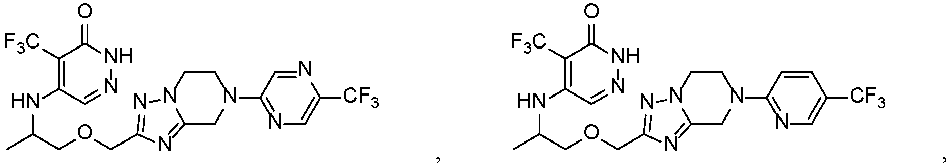 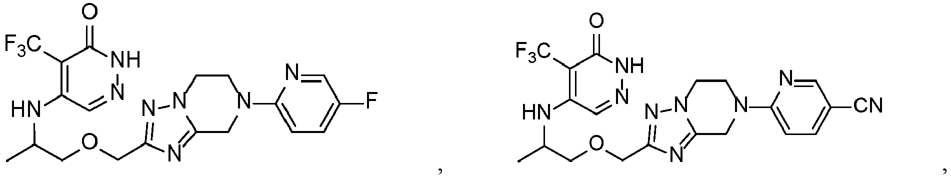 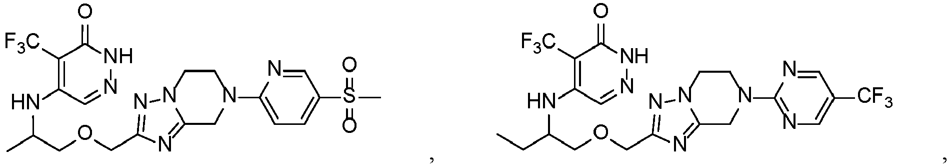 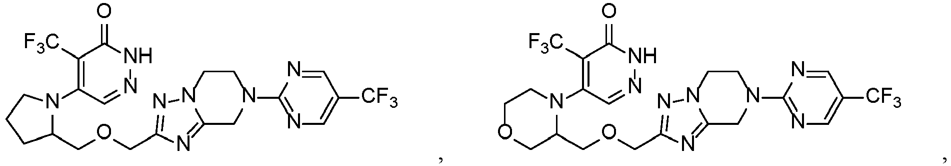 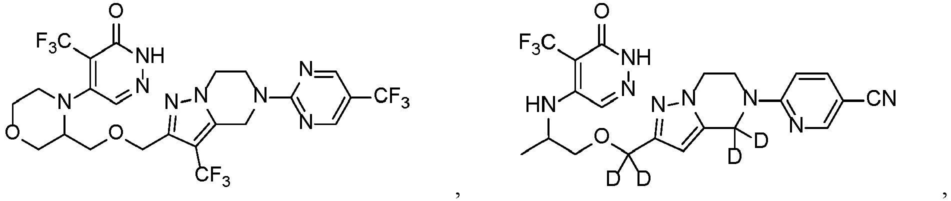 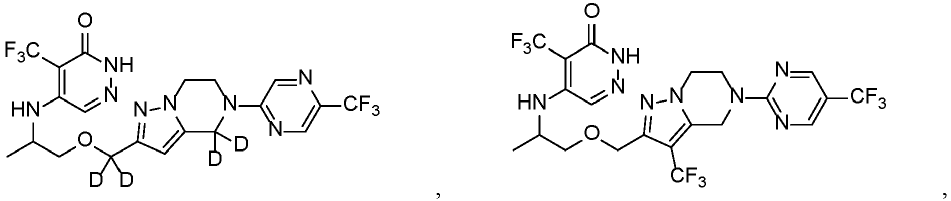 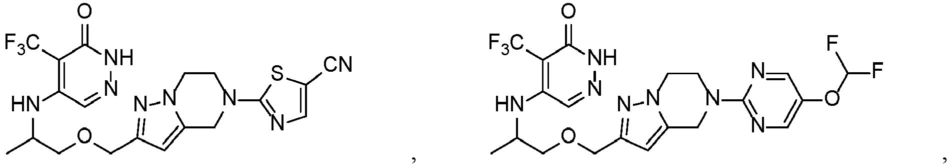 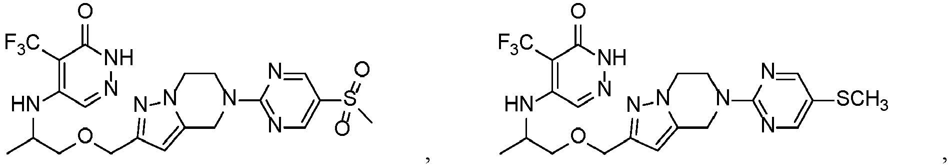 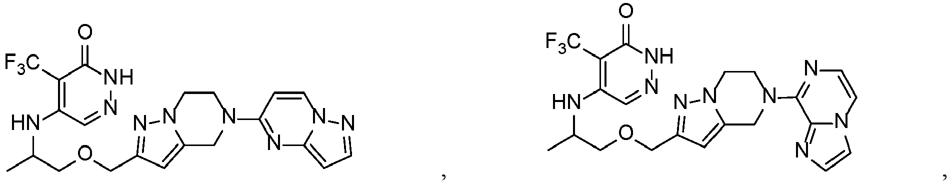 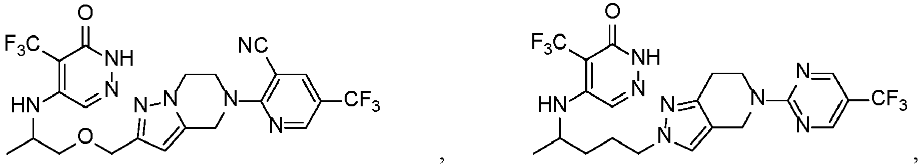 or

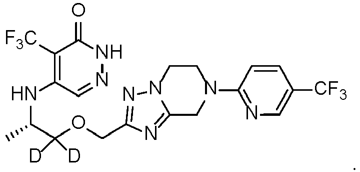

17. A pharmaceutical composition comprising:
(1) the compound according to any one of claims 1 to 16 or a pharmaceutically acceptable salt, a stereoisomer or a deuterated compound thereof;
(2) optionally one or more additional active ingredients; and
(3) a pharmaceutically acceptable carrier and/or excipient.

18. Use of the compound according to any one of claims 1 to 16 or a pharmaceutically acceptable salt, a stereoisomer or a deuterated compound thereof, or the pharmaceutical composition according to claim 17, in the manufacture of an antitumor medicament.

19. An intermediate for preparing the compound according to formula (I), (I-1), (I-2), (I-3), (I'), (I-1'), (I-2') or (I-3') or a pharmaceutically acceptable salt, a stereoisomer or a deuterated compound thereof, wherein the intermediate or a pharmaceutically acceptable salt, a stereoisomer or a deuterated compound thereof has the structure according to formula (I-4): 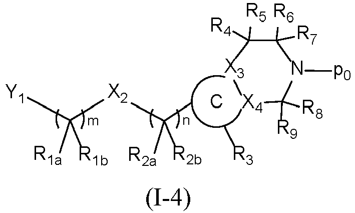 wherein:
Y₁ is -NH₂, -OH, -NHP₁ or a 4 to 6-membered heterocycle containing 1 to 3 heteroatoms selected from N and O;
P₁ is an amino-protecting group, preferably -Boc;
P₀ is H or an amino-protecting group, where the amino-protecting group is preferably - PMB;
X₂ is O or a bond;
X₃ and X₄ are each independently C or N;
R₁ₐ and R_{1b} are each independently H, D, or a C₁₋₆ alkyl;
R₂ₐ and R_{2b} are each independently H, D, or a C₁₋₆ alkyl; or R₂ₐ and R_{2b} form a 3 to 5-membered cycloalkyl together with the carbon atom connected thereto;
R₃ is H, D, a C₁₋₆ alkyl, halogen, or cyano, where the C₁₋₆ alkyl is optionally substituted with 1 to 3 halogens;
R₄ and R₅ are each independently H, D, or a C₁₋₆ alkyl; or R₄ and R₅ form a 3 to 5-membered cycloalkyl together with the carbon atom connected thereto;
R₆ and R₇ are each independently H, D, or a C₁₋₆ alkyl;
R₈ and R₉ are each independently H, D, or a C₁₋₆ alkyl; or R₈ and R₉ form =O on the carbon atom connected thereto;
C is a 5 to 6-membered heterocycle containing 1 to 3 heteroatoms of N;
m is 1, 2, or 3; and
n is 0, 1, 2, or 3.

20. The intermediate according to claim 19, or a pharmaceutically acceptable salt, a stereoisomer or a deuterated compound thereof, wherein the intermediate has the following structure: 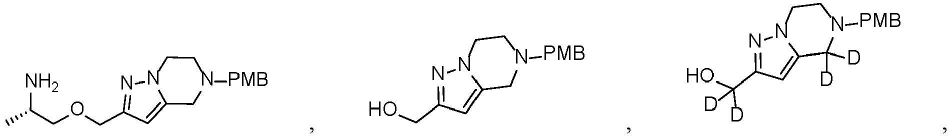 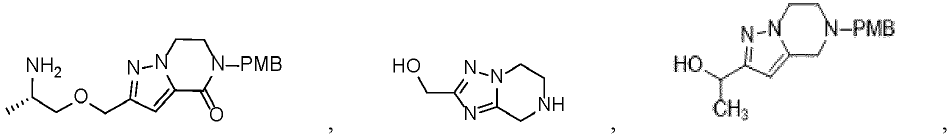 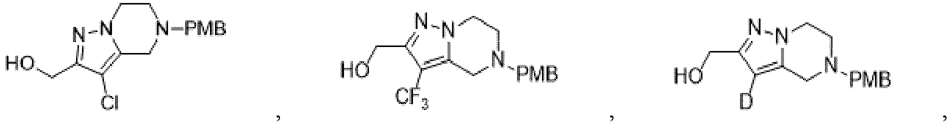 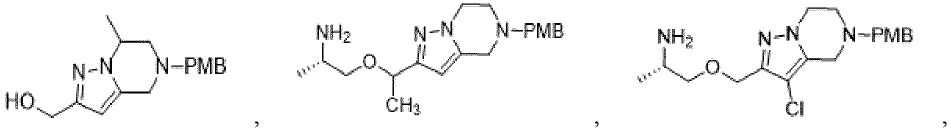 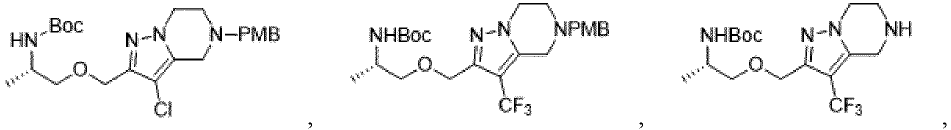 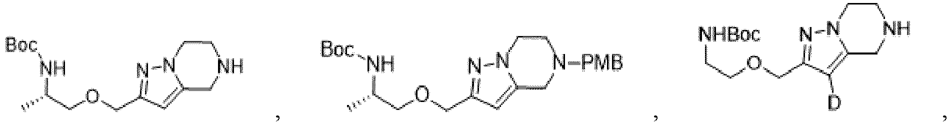 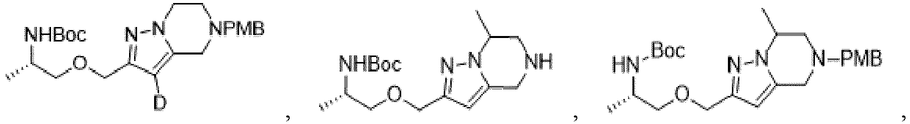 or

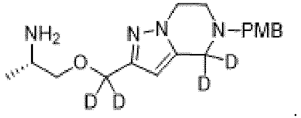

21. An intermediate for preparing the compound according to formula (I), (I-1), (I-2), (I-3), (I'), (I-1'), (I-2') or (I-3') or a pharmaceutically acceptable salt, a stereoisomer or a deuterated compound thereof, wherein the intermediate or a pharmaceutically acceptable salt, a stereoisomer or a deuterated compound thereof has the structure according to formula (I-5): 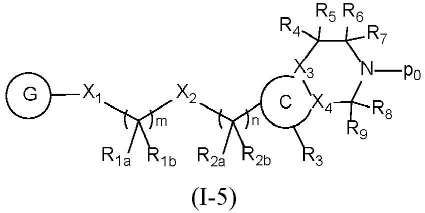 wherein:
X₁ is NH, O, or a 4 to 6-membered heterocycle containing 1 to 3 heteroatoms selected from N and O;
P₀ is H or an amino-protecting group, where the amino-protecting group is preferably - PMB;
X₂ is O or a bond;
X₃ and X₄ are each independently C or N;
R₁ₐ and R_{1b} are each independently H, D, or a C₁₋₆ alkyl;
R₂ₐ and R_{2b} are each independently H, D, or a C₁₋₆ alkyl; or R₂ₐ and R_{2b} form a 3 to 5-membered cycloalkyl together with the carbon atom connected thereto;
R₃ is H, D, a C₁₋₆ alkyl, halogen, or cyano, where the C₁₋₆ alkyl is optionally substituted with 1 to 3 halogens;
R₄ and R₅ are each independently H, D, or a C₁₋₆ alkyl; or R₄ and R₅ form a 3 to 5-membered cycloalkyl together with the carbon atom connected thereto;
R₆ and R₇ are each independently H, D, or a C₁₋₆ alkyl;
R₈ and R₉ are each independently H, D, or a C₁₋₆ alkyl; or R₈ and R₉ form =O on the carbon atom connected thereto;
Gis 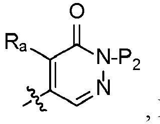 Rₐ is a C₁₋₆ alkyl, a C₃₋₅ cycloalkyl, halogen, or cyano, where the C₁₋₆ alkyl is optionally substituted with 1 to 3 halogens;
P₂ is H or an amino-protecting group, where the amino-protecting group is preferably - SEM or -PMB;
C is a 5 to 6-membered heterocycle containing 1 to 3 heteroatoms of N;
m is 1, 2, or 3; and
n is 0, 1, 2, or 3.

22. The intermediate according to claim 21, or a pharmaceutically acceptable salt, a stereoisomer or a deuterated compound thereof, wherein the intermediate has the following structure:

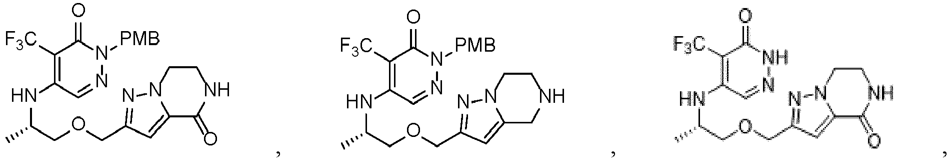

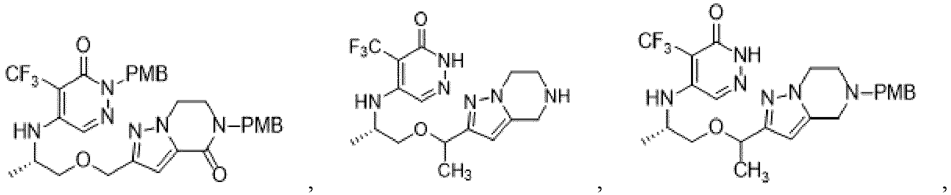

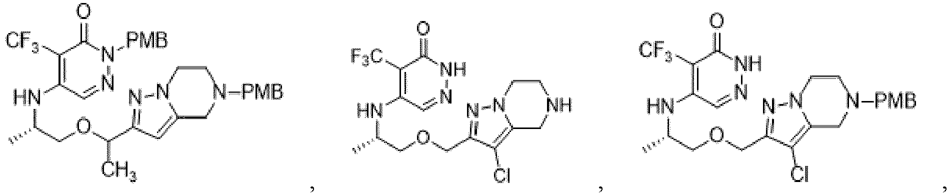

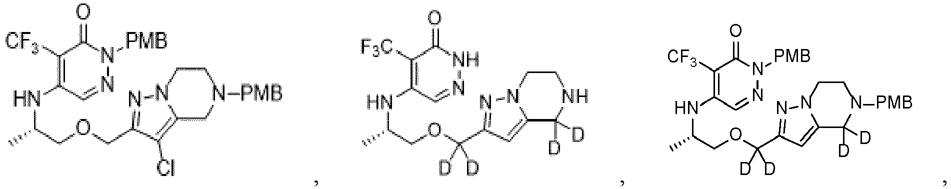

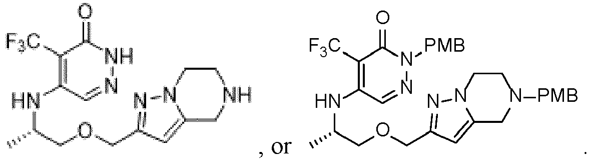

23. An intermediate for preparing the compound according to formula (I), (I-1), (I-2), (I-3), (I'), (I-1'), (I-2') or (I-3') or a pharmaceutically acceptable salt, a stereoisomer or a deuterated compound thereof, wherein the intermediate has the structure according to formula (I-6): 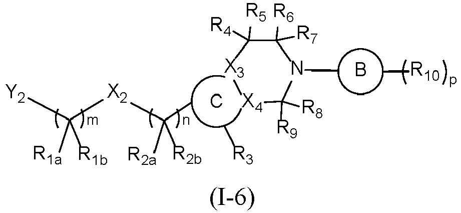 wherein:
Y₂ is NHR_{Y}, OH, or a 4 to 6-membered heterocycle containing 1 to 3 heteroatoms selected from N and O;
R_{Y} is H or an amino-protecting group, where the amino-protecting group is preferably - Boc;
X₂ is O or a bond;
X₃ and X₄ are each independently C or N;
R₁ₐ and R_{1b} are each independently H, D, or a C₁₋₆ alkyl;
R₂ₐ and R_{2b} are each independently H, D, or a C₁₋₆ alkyl; or R₂ₐ and R_{2b} form a 3 to 5-membered cycloalkyl together with the carbon atom connected thereto;
R₃ is H, D, a C₁₋₆ alkyl, halogen, or cyano, where the C₁₋₆ alkyl is optionally substituted with 1 to 3 halogens;
R₄ and R₅ are each independently H, D, or a C₁₋₆ alkyl; or R₄ and R₅ form a 3 to 5-membered cycloalkyl together with the carbon atom connected thereto;
R₆ and R₇ are each independently H, D, or a C₁₋₆ alkyl;
R₈ and R₉ are each independently H, D, or a C₁₋₆ alkyl; or R₈ and R₉ form =O on the carbon atom connected thereto;
each R₁₀ is independently a C₁₋₆ alkyl, a C₁₋₆ alkoxy, CONR₁₀ₐR_{10b}, halogen, cyano, S(O)₂R_{10c}, SR_{10d} or a 3 to 5-membered cycloalkyl, where the C₁₋₆ alkyl and C₁₋₆ alkoxy are optionally substituted with 1 to 3 halogens;
R₁₀ₐ, R_{10b}, R_{10c} and R_{10d} are each independently H, D, or a C₁₋₆ alkyl;
B is a 5 to 10-membered carbocycle or heterocycle containing 1 to 3 heteroatoms selected from N, O, and S;
C is a 5 to 6-membered heterocycle containing 1 to 3 heteroatoms of N;
m is 1, 2, or 3;
n is 0, 1, 2, or 3; and
p is 0, 1, 2, or 3.

24. The intermediate according to claim 23, or a pharmaceutically acceptable salt, a stereoisomer or a deuterated compound thereof, wherein the intermediate has the following structure:

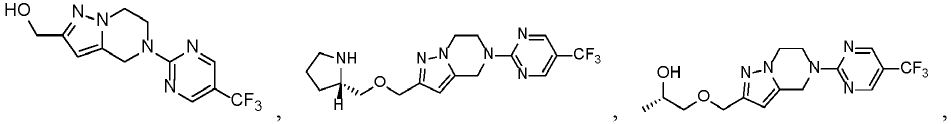

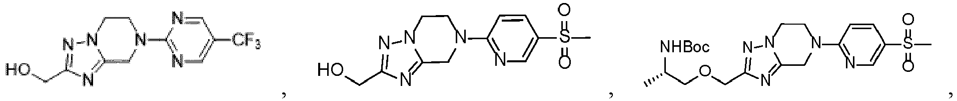

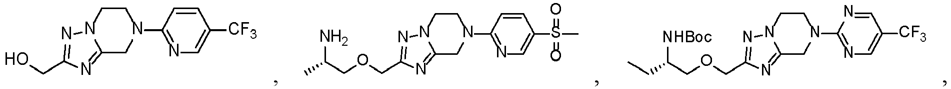

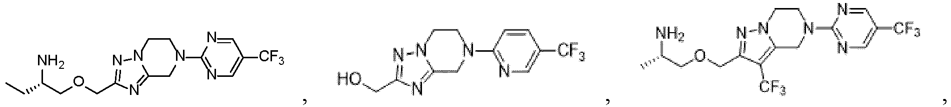

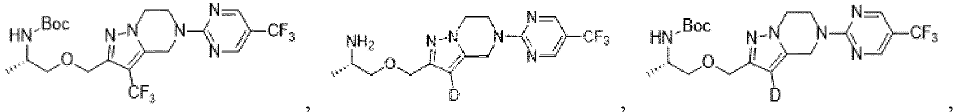

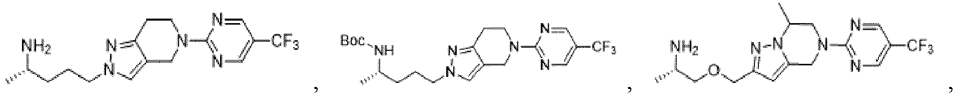

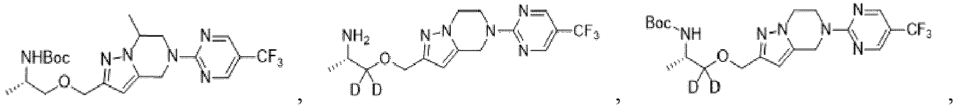

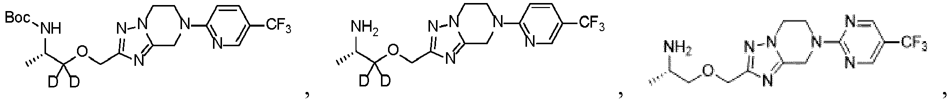

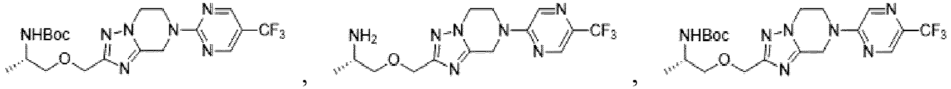

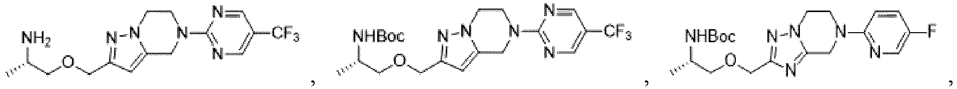

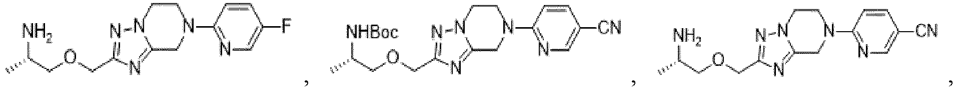

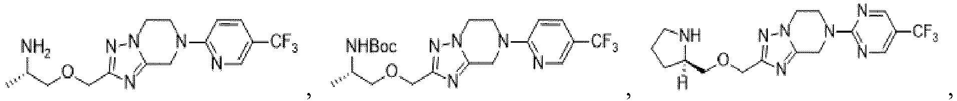

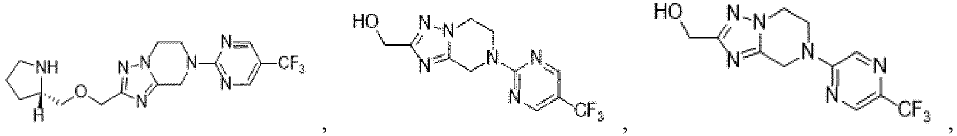

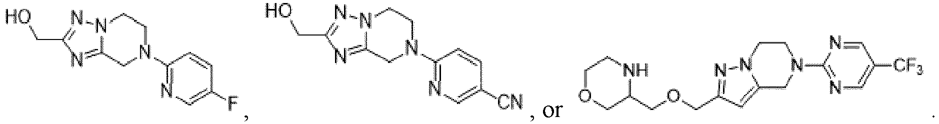

25. An intermediate for preparing the compound according to formula (I), (I-1), (I-2), (I-3), (I'), (I-1'), (I-2') or (I-3') or a pharmaceutically acceptable salt, a stereoisomer or a deuterated compound thereof, wherein the intermediate has the structure according to formula (I-7): 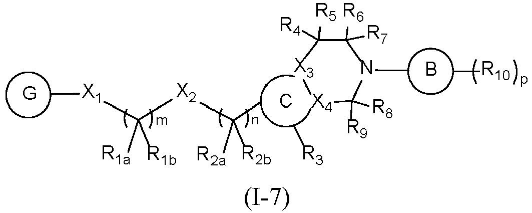 wherein:
X₁ is NH, O, or a 4 to 6-membered heterocycle containing 1 to 3 heteroatoms selected from N and O;
X₂ is O or a bond;
X₃ and X₄ are each independently C or N;
R₁ₐ and R_{1b} are each independently H, D, or a C₁₋₆ alkyl;
R₂ₐ and R_{2b} are each independently H, D, or a C₁₋₆ alkyl; or R₂ₐ and R_{2b} form a 3 to 5-membered cycloalkyl together with the carbon atom connected thereto;
R₃ is H, D, a C₁₋₆ alkyl, halogen, or cyano, where the C₁₋₆ alkyl is optionally substituted with 1 to 3 halogens;
R₄ and R₅ are each independently H, D, or a C₁₋₆ alkyl; or R₄ and R₅ form a 3 to 5-membered cycloalkyl together with the carbon atom connected thereto;
R₆ and R₇ are each independently H, D, or a C₁₋₆ alkyl;
R₈ and R₉ are each independently H, D, or a C₁₋₆ alkyl; or R₈ and R₉ form =O on the carbon atom connected thereto;
each R₁₀ is independently a C₁₋₆ alkyl, a C₁₋₆ alkoxy, CONR₁₀ₐR_{10b}, halogen, cyano, S(O)₂R_{10c}, SR_{10d} or a 3 to 5-membered cycloalkyl, where the C₁₋₆ alkyl and C₁₋₆ alkoxy are optionally substituted with 1 to 3 halogens;
R₁₀ₐ, R_{10b}, R_{10c} and R_{10d} are each independently H, D, or a C₁₋₆ alkyl;
Gis 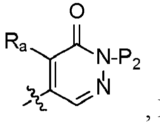 Rₐ is a C₁₋₆ alkyl, a C₃₋₅ cycloalkyl, halogen, or cyano, where the C₁₋₆ alkyl is optionally substituted with 1 to 3 halogens;
P₂ is an amino-protecting group, preferably -SEM or -PMB;
B is a 5 to 10-membered carbocycle or heterocycle containing 1 to 3 heteroatoms selected from N, O, and S;
C is a 5 to 6-membered heterocycle containing 1 to 3 heteroatoms of N;
m is 1, 2, or 3;
n is 0, 1, 2, or 3; and
p is 0, 1, 2, or 3.

26. The intermediate according to claim 25, or a pharmaceutically acceptable salt, a stereoisomer or a deuterated compound thereof, wherein the intermediate has the following structure: 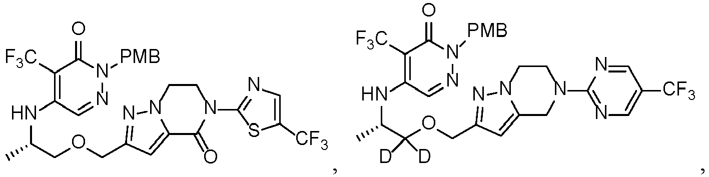 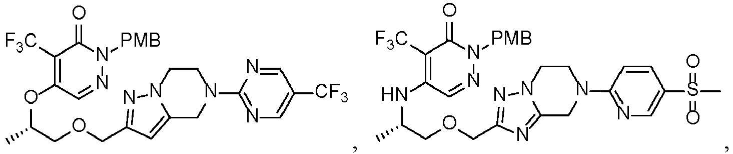 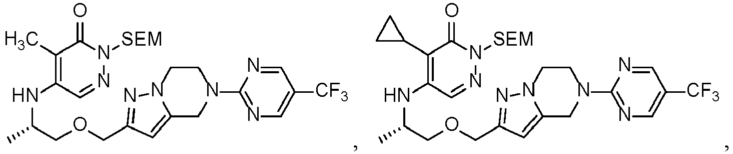 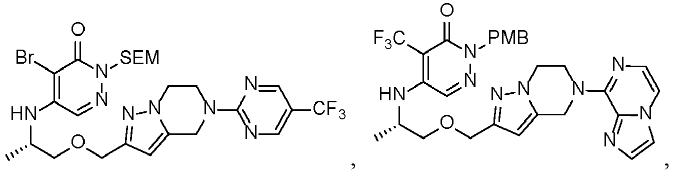 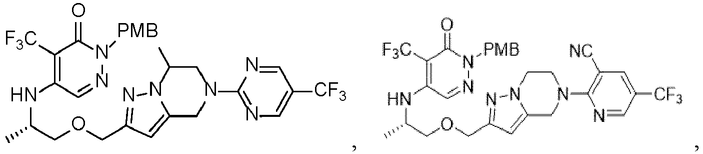 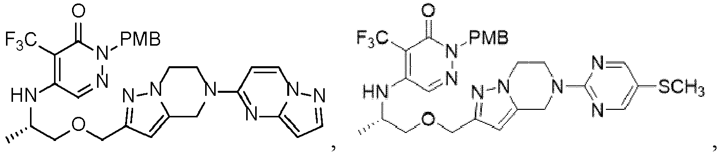 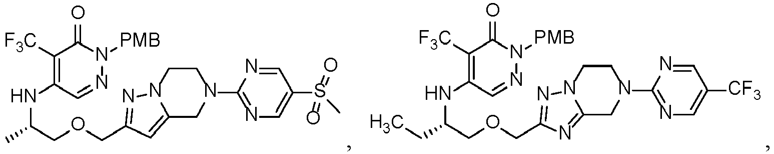 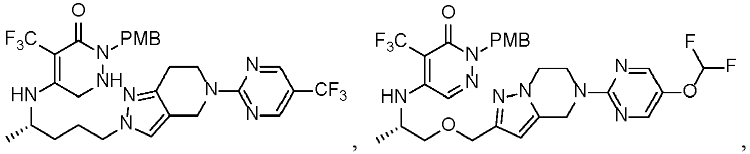 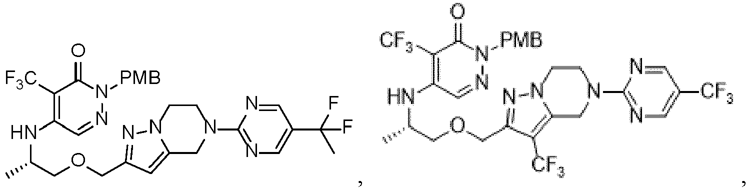 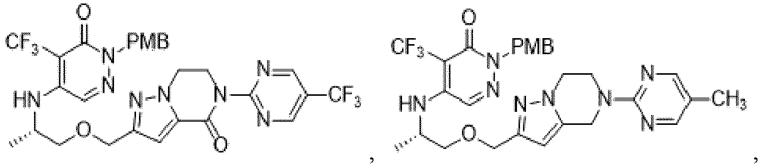 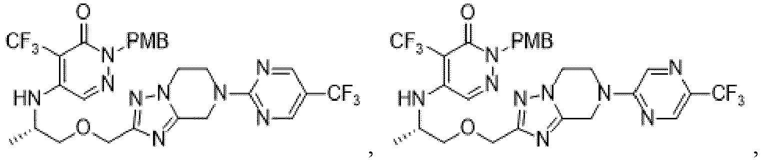 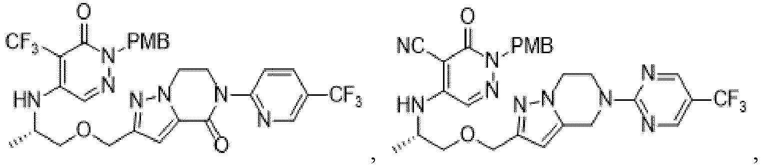 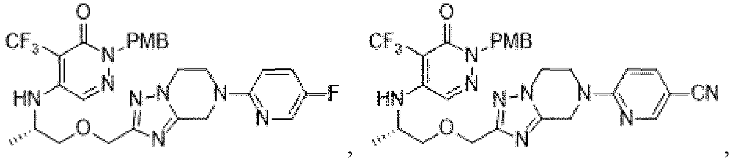 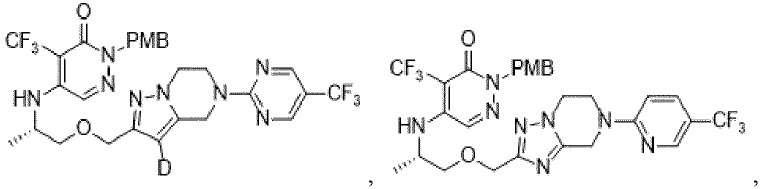 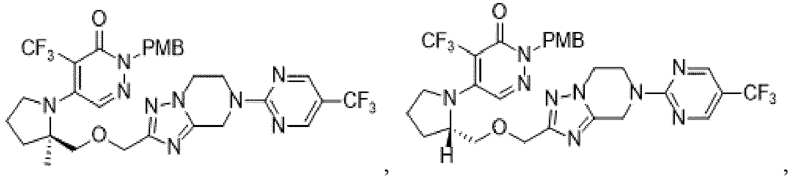 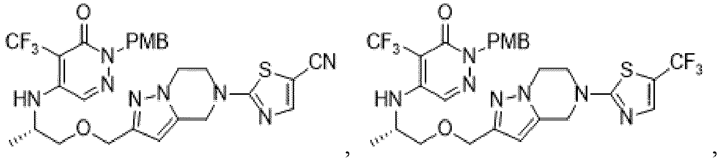 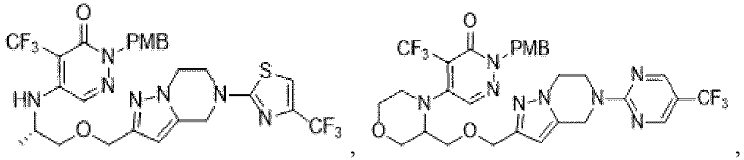 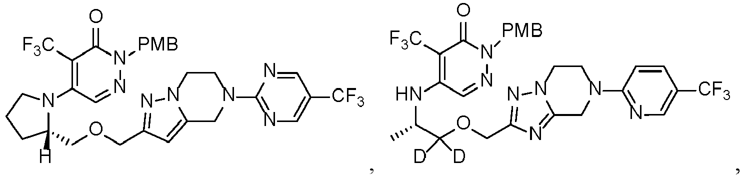 or
